# EUROPEAN PATENT APPLICATION

(11) **EP 2 330 202 A2**
(43) Date of publication of application: **08.06.2011**
(21) Application number: 10015259.4
(22) Date of filing: 05.01.2007
(51) Int. Cl.: C12N 15/82

(54) **Cyst nematode resistant transgenic plants**

(30) Priority: 06.01.2006 US 759182 P
(62) Divisional of application: 07762534.1
(71) Applicant: University Of Georgia Research Foundation, Inc., Athens, GA 30602-7411 (US); NORTH CAROLINA STATE UNIVERSITY, Raleigh, NC 27695-7003 (US); IOWA STATE UNIVERSITY RESEARCH FOUNDATION, INC., Ames, IA 50011-2131 (US)
(72) Inventor: Hussey, Richard S., Athens, GA 30606 (US); Davis, Eric L., Raleigh, NC 27613 (US); Baum, Thomas J., Ames, IA 50010 (US)
(74) Representative: Walker, Ross Thomson

(57) **Abstract**

Compositions and methods for providing cyst nematode resistance are provided. One aspect provides transgenic plants or cells comprising an inhibitory nucleic acid specific for one or more cyst nematode esophageal gland cell polypeptides. Other aspects provide transgenic plants or cells resistant to at least two different cyst nematode species.

## Description

Aspects of the work disclosed herein were supported, in part, by Grant Numbers 95-37302-1918, 98-35302-6881, 2002-35302-12462, 2005-35604-15434, and 2006-35607-16601 awarded by the National Research Initiative of the United States Department of Agriculture. The US government may have certain rights in the claimed subject matter.

### BACKGROUND

### 1. Technical Field

The present disclosure generally relates to compositions for controlling plant parasites, more particularly to nucleic acid compositions for controlling nematode disease.

### 2. Related Art

Nematodes are a very large group of invertebrate animals generally referred to as roundworms, threadworms, eelworms, or nema. Some nematodes are plant parasites and can feed on stems, buds, leaves, and in particular on roots. Cyst nematodes (principally *Heterodera* and *Globodera* spp.) are key pests of major crops. Cyst nematodes are known to infect tobacco, cereals, sugar beets, potato, rice, corn, soybeans and many other crops. *Heterodera schachtii* principally attacks sugar beets, and *Heterodera avenae* has cereals as hosts. *Heterodera zeae* feeds on corn, and *Globodera rostochiensis* and *G. pallida* feed on potatoes. The soybean cyst nematode *(Heterodera glycines)* infests every soybean-producing state in the U.S., with total soybean yield loss estimates approaching $1 billion per year.

The soybean cyst nematode (SCN) is a plant-parasitic nematode that changes shape as it goes through its life cycle. In its juvenile form, SCN penetrates soybean roots. In the root, juveniles become males or females. Males return to the original wormlike shape and leave the roots in search of females. Those that become females lose the ability to move, enlarge into a lemon-shaped "white female," which breaks through the root surface, produces eggs after being fertilized by males, dies, and turns into a brown cyst or egg case. A single nematode cyst may contain several hundred eggs. The majority of eggs remain within the cyst, where they are protected from desiccation and soil predation. There can be more than one generation of SCN during a single growing season, leading to multiple root infestations.

The number of juveniles entering the plant root soon after plant emergence can have a dramatic effect on plant growth and development. Plant damage occurs from juvenile feeding which removes cell materials and disrupts the vascular tissue. In short, SCN infection inhibits the growth and functioning of the soybean root system, interfering with nutrient and water uptake. SCN infection can also suppress nodule formation by nitrogen-fixing bacteria and can increase plant damage when other plant pathogens are present in the soil.

Existing methods for treating or preventing nematode disease include the use of chemicals, pesticides, and fumigants. The use of pre-plant soil fumigants is highly effective in controlling cyst nematodes and other plant-parasitic nematodes. However, the majority of the fumigant-type nematicides is no longer available and is also costly and difficult to apply properly under the prevailing conditions.

Crop rotation has also been used to control nematode disease. Rotating non-host plants can be effective in controlling nematode disease. Unfortunately, these crops are often less valuable. Cover crops grown between the main crops may provide an alternative management strategy. Ryegrain, barley, oats, sudangrass, tall fescue, and annual ryegrass have been shown to be non- or poor nematode hosts. Using cover crops, however, can be costly because the cover crops occupy space that could be used to grow more valuable crops.

Biological control organisms have also been used to try to control nematode disease in crops. Commercially available preparations of biological control organisms are limited in their use to regions that can support the growth of the control organism. Moreover, the outcome of using one organism to control another is unpredictable and subject to a variety of factors such as weather and climate.

While several examples of host resistance genes in diverse crops exist, the availability of host plant resistance is substantially limited with appropriate resistance loci lacking for the majority of our crops (Roberts, P. A. 1992. Journal of Nematology 24:213-227). Another limitation of natural resistance genes is the durability of resistance since resistance-breaking populations of SCN can develop after continuous exposure to resistant cultivars. The SCN is adaptable and can build up on previously resistant cultivars.

SCN juveniles in eggs can survive in the soil under adverse conditions within cysts for long periods of time making SCN difficult to manage. In addition, soil is a difficult environment to manipulate for example to effectively treat with nematicides.

Accordingly, there is need for compositions and methods for controlling, preventing, or reducing cyst nematode disease in plants.

### SUMMARY

Aspects of the present disclosure generally provide transgenic plants or cells, transgenic plant material, and nucleic acid constructs that inhibit the synthesis and activity of esophageal gland cell proteins secreted by cyst nematodes, in particular *Heterodera glycines* also referred to as SCN. In some aspects, the inhibited cyst nematode secretory proteins modulate gene expression of the host plant or host plant cell, formation of a syncytium in the host plant, nematode migration through root tissue of the host plant, cell metabolism of the host plant, signal transduction in the host plant cell, or formation of a feeding tube that enables the nematode to feed from syncytia formed in the host plant. Other aspects provide transgenic cells or plants expressing or containing one or more inhibitory nucleic acids, for example inhibitory double or single stranded RNA, that inhibit or reduce the synthesis of esophageal gland cell proteins secreted by a cyst nematode, for example SCN.

Another aspect provides a transgenic plant or transgenic plant material that comprises inhibitory RNA that down regulates a target cyst nematode parasitism gene transcript in 2, 3, 4, 5, 6, 7, 8, 9, 10, 11, 12, 13, 14, 15, 16, 17, 18, 19, 20, or more *Heterodera spp.* Thus, the present disclosure provides transgenic plants that are resistant to disease caused by multiple cyst nematode species.

Representative cyst riematode esophageal gland cell proteins that are targeted by the disclosed inhibitory nucleic acids include one or more of the proteins encoded by SEQ ID NOs. 1-63 and 113-116. In certain aspects, one or more inhibitory nucleic acids are delivered to a cyst nematode when the nematode enters the transgenic plant or transgenic plant cell, feeds on the transgenic plant or transgenic plant cell, or comes into physical contact with the transgenic plant or transgenic plant cell. Once the inhibitory nucleic acid is internalized by the cyst nematode, the inhibitory nucleic acid interferes with, reduces, or inhibits the synthesis of a target esophageal gland cell protein, for example, by directly or indirectly interfering, reducing, or inhibiting the translation of one or more mRNAs coding for one or more esophageal gland cell proteins.

Yet another aspect provides a plant cell transfected with heterologous nucleic acid encoding an inhibitory nucleic acid specific for one or more cyst nematode esophageal gland cell proteins, wherein the heterologous nucleic acid is expressed in an amount sufficient to reduce or prevent cyst nematode disease. In one aspect, the transgenic plant expresses the inhibitory nucleic acid, and the inhibitory nucleic acid is delivered to a cyst nematode feeding or attempting to feed on the transgenic plant. Generally, the inhibitory nucleic acid is internalized by the nematode. Exemplary methods of internalizing the inhibitory nucleic acid include ingesting the nucleic acid or absorbing the nucleic acid.

Still another aspect provides a transgenic plant comprising an inhibitory nucleic acid specific for one or more cyst nematode parasitism polypeptides, wherein the inhibitory nucleic acid provides resistance to two or more cyst nematode species, for example two or more *Heterodera* or *Globodera* spp..

The details of one or more embodiments are set forth in the accompanying drawings and the description below. Other features, objects, and advantages of the disclosure will be apparent from the description and drawings, and from the claims.

### BRIEF DESCRIPTION OF THE FIGURES

**Figure 1A** shows a nucleotide sequence alignment of the full length cDNA of the 4EO2 parasitism gene between *H. schachtii* (BCN) (SEQ ID NO:113) and *H. glycines* (SCN)(SEQ ID NO:8). Note that the genes are 99% identical between these two species.
**Figure 1B** shows a nucleotide sequence alignment of the full length cDNA of the SYV46 parasitism gene between *H. schachtii* (BCN)(SEQ ID NO:114) and *H. glycines* (SCN)(SEQ ID NO:61). Note that the genes are 93% identical between these two species.
**Figure 1C** shows a nucleotide sequence alignment of the full length cDNA of the 4FO1 parasitism gene between *H. schachtii* (BCN)(SEQ ID NO:115) and *H. glycines* (SCN)(SEQ ID NO:9). Note that the genes are 97% identical between these two species.
**Figure 1D** shows a nucleotide sequence alignment of the full length cDNA of the 5DO8 parasitism gene between *H. schachtii* (BCN))(SEQ ID NO:116) and *H. glycines* (SCN)(SEQ ID NO:14). Note that the genes are 98% identical between these two species.
**Figure 2** shows a bar graph of germination rates of wild-type *Arabidopsis, annAt* mutant *Arabidopsis* and *annAt* mutant *Arabidopsis* expressing 4FO1. Note that expression of the 4FO1 gene partially rescued the tolerance of the *annAt* mutant plants to media conditioned with 75 mM NaCl.
**Figure 3** shows a table demonstrating relative fold decrease in transcript accumulation (column d) compared to actin gene controls as measured by real-time PCR after soaking preparasitic second-stage juvenile (J2) of *H. glycines* in 5.0 mg/ml or 2.5 mg/ml double-stranded RNA. Nucleotides161-445 and 569-835 of HG-pel1 cDNA were used as template to produce dsRNA of 285 (ds285) and 267 (ds267) nucleotides, respectively. Effects of soaking *H. glycines* J2 in dsRNA of green fluorescent protein (GFP) are included as a negative control.
**Figure 4** shows a bar graph demonstrating the average number of *H. schachtii* females on wild-type and four independent segregating transgenic *Arabidopsis* lines expressing partial (170 bp) SYV46 dsRNA. Ntcel7-SYV46-L8 (T₁) and Ntcel7-SYV46-L10 (T₁) show a significant (p<0.007) decrease (*) in females per plant compared to control wild-type plants.
**Figure 5** shows a bar graph demonstrating the number of females per root system on individual T₁ plants in two independent *Arabidopsis thaliana* lines of in planta RNAi to the cyst nematode SYV46 gene as compared to number of females produced in roots of individual wild-type plants.
**Figure 6** shows a bar graph demonstrating the average number of *H. schachtii* females on 3 independent segregating transgenic Arabidopsis lines expressing full-length 4F01 dsRNA Ntcet7-4FO1-4-1 and L4-2 (T₂) and Ntcel7-4FO1-L5-1 and L5-2 (T₂) show a significant (p< 0.03) decrease (*) in females per plant compared to *H. schachtii* infection of roots of wild-type plants.
**Figure 7** shows a bar graph demonstrating the number of H. *schachtii females* per root system on individual T₂ plants in two independent *Arabidopsis thaliana* lines of in planta RNAi to the cyst nematode 4F01 gene compared to number of females produced in roots of wild-type plants.
Figures 8A-B show a bar graph demonstrating the number of *H. schachtii* females (infections) produced on roots of wild-type (WT) and individual primary transformants (To) of Arabidopsis containing the Gmubi-20E03-RNAi (A) and Gmubi-23G12-RNAi (B) constructs.
**Figure 9** shows a bar graph demonstrating the average number of *H. schachtii* females on four transgenic Arabidopsis lines expressing partial dsRNA. Each line was replicated twice in the experiment. All lines, but not all replicates, show a significant (*) decrease in females per plant compared to control wild-type plants.

### DETAILED DESCRIPTION

### 1. Definitions

Before explaining the various embodiments of the disclosure, it is to be understood that the invention is not limited in its application to the details of construction and the arrangement of the components set forth in the following description. Other embodiments can be practiced or carried out in various ways. Also, it is to be understood that the phraseology and terminology employed herein is for the purpose of description and should not be regarded as limiting.

Throughout this disclosure, various publications, patents and published patent specifications are referenced. Where permissible, the disclosures of these publications, patents and published patent specifications are hereby incorporated by reference in their entirety into the present disclosure to more fully describe the state of the art. Unless otherwise indicated, the disclosure encompasses conventional techniques of plant breeding, immunology, molecular biology, microbiology, cell biology and recombinant DNA, which are within the skill of the art. See, e.g., Sambrook and Russell, Molecular Cloning: A Laboratory Manual, 3rd edition (2001); Current Protocols In Molecular Biology [(F. M. Ausubel, et al. eds., (1987)]; Plant Breeding: Principles and Prospects (Plant Breeding, Vol 1) M. D. Hayward, N. O. Bosemark, 1. Romagosa; Chapman & Hall, (1993.); Coligan, Dunn, Ploegh, Speicher and Wingfeld, eds. (1995) CURRENT Protocols in Protein Science (John Wiley & Sons, Inc.); the series Methods in Enzymology (Academic Press, Inc.): PCR 2: A Practical Approach (M. J. MacPherson, B. D. Hames and G. R. Taylor eds. (1995)], Harlow and Lane, eds. (1988) Antibodies, A Laboratory Manual, and Animal Cell Culture [R. I. Freshney, ed. (1987)].

Unless otherwise noted, technical terms are used according to conventional usage. Definitions of common terms in molecular biology may be found in Lewin, Genes VII, published by Oxford University Press, 2000; Kendrew et al. (eds.), The Encyclopedia of Molecular Biology, published by Wiley-Interscience., 1999; and Robert A. Meyers (ed.), Molecular Biology and Biotechnology, a Comprehensive Desk Reference, published by VCH Publishers, Inc., 1995; Ausubel et al. (1987) Current Protocols in Molecular Biology, Green Publishing; Sambrook and Russell. (2001) Molecular Cloning: A Laboratory Manual 3rd. edition.

In order to facilitate understanding of the disclosure, the following definitions are provided:

To "after" the expression of a target gene in a plant or nematode cell means that the level of expression of the target gene in the cell after applying a method of the present invention is different from its expression in the cell before applying the method. To alter gene expression preferably means that the expression of the target gene in the plant or nematode is reduced, preferably strongly reduced, more preferably the expression of the gene is not detectable. The alteration of the expression of an essential gene may result in a knockout mutant phenotype in plant or nematode cells or plants or nematodes derived therefrom. Alternatively, altered expression can included upregulating expression of plant or nematode genes.

"Antisense RNA" is an RNA strand having a sequence complementary to a target gene mRNA, and thought to induce RNAi by binding to the target gene mRNA. "Sense RNA" has a sequence complementary to the antisense RNA, and annealed to its complementary antisense RNA to lead to the production of siRNA. These antisense and sense RNAs have been conventionally synthesized with an RNA synthesizer. In the present invention, these RNAs are intracellularly expressed from DNAs coding for antisense and sense RNAs (antisense and sense code DNAs) respectively leading to the intracellular accumulation of dsRNA and siRNA.

As used herein, "buffer" refers to a buffered solution that resists changes in pH by the action of its acid-base conjugate components.

When referring to expression, "control sequences" means DNA sequences necessary for the expression of an operably linked coding sequence in a particular host organism. Control sequences that are suitable for prokaryotes, for example, include a promoter, optionally an operator sequence, a ribosome binding site, and the like. Eukaryotic cells are known to utilize promoters, polyadenylation signals, and enhancers.

The term "cell" refers to a membrane-bound biological unit capable of replication or division.

The term "construct" refers to a recombinant genetic molecule comprising one or more isolated polynucleotide sequences of the invention.

Genetic constructs used for transgene expression in a host organism comprise in the 5'-3' direction, a promoter sequence; a sequence encoding an inhibitory nucleic acid disclosed herein; and a termination sequence. The open reading frame may be orientated in either a sense or anti-sense direction. The construct may also comprise selectable marker gene(s) and other regulatory elements for expression.

As used herein, the term "control element" or "regulatory element" are used interchangably herein to mean sequences positioned within or adjacent to a promoter sequence so as to influence promoter activity. Control elements may be positive or negative control elements. Positive control elements require binding of a regulatory element for initiation of transcription. Many such positive and negative control elements are known. Where heterologous control elements are added to promoters to alter promoter activity as described herein, they are positioned within or adjacent the promoter sequence so as to aid the promoter's regulated activity in expressing an operationally linked polynucleotide sequence.

The term "cyst nematode" refers to a member of *Heterodera* or *Globodera* spp. and includes, but is not limited to *Heterodera glycines* and *Heteroder schachtii.* Additional *Heterodera* species include but are not limited to *H. avenae, H. bifenestra, H. cajani. H. carotae, H. ciceri, H. cruciferae, H. cynodontis, H. cyperi, H. daverti, H. elachista, H. fici**,** H. galeopsidis, H. goettingiana, H. graminis, H. hordecalis, H. humuli, H. iri, H. latipons, H. lespedeza, H. leucilyma, H. longicaudata, H. mani, , H. maydis, H. medicaginis, H. oryzae, H. oryzicola, H. sacchari, H. salixophila, H. sorghii, H. trifolii, H. urticae, H. vigna, H.* zeae. Representative *Globodera* species include but are not limited to *G. achilleae, G. artemisiae, G. hypolysi, G. leptonepia, G. mali, G. pallida, G. rostochiensis, G. tabacum,* and *G. zelandica.*

The term "heterologous" refers to elements occurring where they are not normally found. For example, a promoter may be linked to a heterologous nucleic acid sequence, e.g., a sequence that is not normally found operably linked to the promoter. When used herein to describe a promoter element, heterologous means a promoter element that differs from that normally found in the native promoter, either in sequence, species, or number. For example, a heterologous control element in a promoter sequence may be a control/regulatory element of a different promoter added to enhance promoter control, or an additional control element of the same promoter.

As used herein, the term "homologues" is generic to "orthologues" and "paralogues".

The term "host plant" refers to a plant subject to nematode disease.

As used herein, the phrase "induce expression" means to increase the amount or rate of transcription and/or translation from specific genes by exposure of the cells containing such genes to an effector or inducer reagent or condition.

An "inducer" is a chemical or physical agent which, when applied to a population of cells, will increase the amount of transcription from specific genes. These are usually small molecules whose effects are specific to particular operons or groups of genes, and can include sugars, phosphate, alcohol, metal ions, hormones, heat, cold, and the like. For example, isopropyl (beta)-D-thiogalactopyranoside (IPTG) and lactose are inducers of the tacll promoter, and L-arabinose is a suitable inducer of the arabinose promoter.

The term "isolated," when used to describe the various compositions disclosed herein, means a substance that has been identified and separated and/or recovered from a component of its natural environment. For example an isolated polypeptide or polynucleotide is free of association with at least one component with which it is naturally associated. Contaminant components of its natural environment are materials that would typically interfere with diagnostic or therapeutic uses for the polypeptide or polynucleotide and may include enzymes, and other proteinaceous or non-proteinaceous solutes. An isolated substance includes the substance *in situ* within recombinant cells. Ordinarily, however, an isolated substance will be prepared by at least one purification step.

An "isolated" nucleic acid molecule or polynucleotide is a nucleic acid molecule that is identified and separated from at least one contaminant nucleic acid molecule with which it is ordinarily associated in the natural source. The isolated nucleic can be, for example, free of association with all components with which it is naturally associated. An isolated nucleic acid molecule is other than in the form or setting in which it is found in nature.

"IPTG" is the compound "isopropyl (beta)-D-thiogalactopyranoside", and is used herein as an inducer of *lac* operon. IPTG binds to a *l*ac repressor effecting a conformational change in the *lac* repressor, which results in dissociation of the *lac* repressor from the *lac* operator. With the *l*ac repressor unbound, an operably linked promoter is activated and downstream genes are transcribed.

The term "lac operator" refers to a nucleic acid sequence that can be bound by a *lac* repressor, *lacl,* as described, for example, in Jacob et al., 1961, J. Mol. Biol., 3: 318-356. A promoter is not activated when the *lac* repressor is bound to the *lac* operator. When the *lac* repressor is induced to dissociate from the operator, the promoter is activated.

The term "leader sequence" refers to a nucleic acid sequence positioned upstream of a coding sequence of interest. Leader sequences described herein contain specific sequences known to bind efficiency to ribosomes, thus delivering a greater efficiency of translation initiation of some potynucleotides.

The term "nematode esophageal glands or nematode esophageal gland cell" refers to three large, transcriptionally active gland cells, one dorsal and two subventral, located in the esophagus of a nematode and that are the principal sources of secretions (parasitism proteins) involved in infection and parasitism of plants by plant-parasitic nematodes in the orders Tylenchida and Aphelenchida.

A nucleic acid sequence or polynucleotide is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading frame. Linking can be accomplished by ligation at convenient restriction sites. If such sites do not exist, synthetic oligonucleotide adaptors or linkers are used in accordance with conventional practice.

The term "orthologues" refers to separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term "paralogues" indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

The term "parasitism proteins, parasitism polypeptides, esophageal polypeptides, or nematode esophageal gland cell secretory polypeptide" refers to the principal molecules involved in nematode parasitism of plants; products of parasitism genes expressed in plant-parasitic nematode esophageal gland cells and injected through their stylet into host tissues to mediate parasitism of plants.

"Percent (%) nucleic acid sequence identity" is defined as the percentage of nucleotides in a candidate sequence that are identical with the nucleotides in a reference nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z,
where W is the number of nucleotides scored as identical matches by the sequence alignment program in that program's alignment of C and D, and where Z is the total number of nucleotides in D. It will he appreciated that where the length of nucleic acid sequence C is not equal to the length of nucleic acid sequence D, the % nucleic acid sequence identity of C to D will not equal the % nucleic acid sequence identity of D to C.

The term "plant" is used in it broadest sense. It includes, but is not limited to, any species of woody, ornamental or decorative, crop or cereal, fruit or vegetable plant, and photosynthetic green algae (e.g., *Chlamydomonas reinhardtii).* It also refers to a plurality of plant cells that are largely differentiated into a structure that is present at any stage of a plant's development. Such structures include, but are not limited to, a fruit, shoot, stem, leaf, flower petal, etc. The term "plant tissue" includes differentiated and undifferentiated tissues of plants including those present in roots, shoots, leaves, pollen, seeds and tumors, as well as cells in culture (e.g., single cells, protoplasts, embryos, callus, etc.). Plant tissue may be in planta, in organ culture, tissue culture, or cell culture. The term "plant part" as used herein refers to a plant structure, a plant organ, or a plant tissue.

A non-naturally occurring plant refers to a plant that does not occur in nature without human intervention. Non-naturally occurring plants include transgenic plants and plants produced by non-transgenic means such as plant breeding.

The term "plant cell" refers to a structural and physiological unit of a plant, comprising a protoplast and a cell wall. The plant cell may be in form of an isolated single cell or a cultured cell, or as a part of higher organized unit such as, for example, a plant tissue, a plant organ, or a whole plant.

The term "plant cell culture" refers to cultures of plant units such as, for example, protoplasts, cell culture cells, cells in plant tissues, pollen, pollen tubes, ovules, embryo sacs, zygotes and embryos at various stages of development.

The term "plant material" refers to leaves, stems, roots, flowers or flower parts, fruits, pollen, egg cells, zygotes, seeds, cuttings, cell or tissue cultures, or any other part or product of a plant.

A "plant organ" refers to a distinct and visibly structured and differentiated part of a plant such as a root, stem, leaf, flower bud, or embryo.

"Plant tissue" refers to a group of plant cells organized into a structural and functional unit. Any tissue of a plant whether in a plant or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

"Plasmids" are designated by a lower case "p" preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

As used herein, "polypeptide" refers generally to peptides and proteins having more than about ten amino acids. The polypeptides can be "exogenous," meaning that they are "heterologous," i.e., foreign to the host cell being utilized, such as human polypeptide produced by a bacterial cell.

The term "promoter" refers to a regulatory nucleic acid sequence, typically located upstream (5') of a gene or protein coding sequence that, in conjunction with various elements, is responsible for regulating the expression of the gene or protein coding sequence. The promoters suitable for use in the constructs of this disclosure are functional in plants and in host organisms used for expressing the inventive polynucleotides. Many plant promoters are publicly known. These include constitutive promoters, inducible promoters, tissue- and cell-specific promoters and developmentally-regulated promoters. Exemplary promoters and fusion promoters are described, e.g., in U.S. Pat. No. 6,717,034, which is herein incorporated by reference in its entirety.

"Purifying" means increasing the degree of purity of a substance in a composition by removing (completely or partially) at least one contaminant from the composition. A "purification step" may be part of an overall purification process resulting in an "essentially pure" composition. An essentially pure composition contains at least about 90% by weight of the substance of interest, based on total weight of the composition, and can contain at least about 95% by weight.

The term "regulatory element" or "control element" refers to DNA sequences controlling initiation of transcription. Examples of control or regulatory elements include, but are not limited to, a TATA box, operators, enhancers, and the like. Regulatory or control elements include negative control elements and positive control elements. A negative control element is one that is removed for activation. Many such negative control elements are known, for example operator/repressor systems. For example, binding of IPTG to the *l*ac repressor dissociates from the *l*ac operator to activate and permit transcription. Other negative elements include the E. *coli* trp and lambda systems. A positive control element is one that is added for activation. Many such positive control elements are known.

Promoters naturally containing both positive and negative regulatory elements are rare. The metE promoter is one example. See, for example, Neidhardt, Ed., 1996, Escherishia coli and Salmonella, Second Ed., pages 1300-1309. Descriptions of known positive and negative control elements can be found, for example, in this reference. Positioning of a positive or negative control element within or adjacent to the promoter to achieve added regulation of the promoter is known, and is described, for example, in *Escherishia coli and Salmonella (Supra)* at pages 1232-1245.

"Small RNA molecules" refer to single stranded or double stranded RNA molecules generally less than 200 nucleotides in length. Such molecules are generally less than 100 nucleotides and usually vary from 10 to 100 nucleotides in length. In a preferred format, small RNA molecules have 11, 12, 13, 14, 15, 16, 17, 18, 19, 20, 21, 22, 23, 24, 25, 26, 27, 28, 29 or 30 nucleotides. Small RNAs include microRNAs (miRNA) and small interfering RNAs (siRNAs). MiRNAs are produced by the cleavage of short stem-loop precursors by Dicer-like enzymes; whereas, siRNAs are produced by the cleavage of long double-stranded RNA molecules. MiRNAs are single-stranded, whereas siRNAs are double-stranded.

The term "siRNA" means a small interfering RNA that is a short-length double-stranded RNA that is not toxic. Generally, there is no particular limitation in the length of siRNA as long as it does not show toxicity. "siRNAs" can be, for example, 15 to 49 bp, preferably 15 to 35 bp, and more preferably 21 to 30 bp long. Alternatively, the double-stranded RNA portion of a final transcription product of siRNA to be expressed can be, for example, 15 to 49 bp, preferably 15 to 35 bp, and more preferably 21 to 30 bp long. The double-stranded RNA portions of siRNAs in which two RNA strands pair up are not limited to the completely paired ones, and may contain nonpairing portions due to mismatch (the corresponding nucleotides are not complementary), bulge (lacking in the corresponding complementary nucleotide on one strand), and the like. Nonpairing portions can be contained to the extent that they do not interfere with siRNA formation. The "bulge" used herein preferably comprise 1 to 2 nonpairing nucleotides, and the double-stranded RNA region of siRNAs in which two RNA strands pair up contains preferably 1 to 7, more preferably 1 to 5 bulges. In addition, the "mismatch" used herein is contained in the double-stranded RNA region of siRNAs in which two RNA strands pair up, preferably 1 to 7, more preferably 1 to 5, in number. In a preferable mismatch, one of the nucleotides is guanine, and the other is uracil. Such a mismatch is due to a mutation from C to T, G to A, or mixtures thereof in DNA coding for sense RNA, but not particularly limited to them. Furthermore, in the present invention, the double-stranded RNA region of siRNAs in which two RNA strands pair up may contain both bulge and mismatched, which sum up to, preferably 1 to 7, more preferably 1 to 5 in number.

The terminal structure of siRNA may be either blunt or cohesive (overhanging) as long as siRNA can silence, reduce, or inhibit the target gene expression due to its RNAi effect. The cohesive (overhanging) end structure is not limited only to the 3' overhang, and the 5' overhanging structure may be included as long as it is capable of inducing the RNAi effect. In addition, the number of overhanging nucleotide is not limited to the already reported 2 or 3, but can be any numbers as long as the overhang is capable of inducing the RNAi effect. For example, the overhang consists of 1 to 8, preferably 2 to 4 nucleotides. Herein, the total length of siRNA having cohesive end structure is expressed as the sum of the length of the paired double-stranded portion and that of a pair comprising overhanging single-strands at both ends. For example, in the case of 19 bp double-stranded RNA portion with 4 nucleotide overhangs at both ends, the total length is expressed as 23 bp. Furthermore, since this overhanging sequence has low specificity to a target gene, it is not necessarily complementary (antisense) or identical (sense) to the target gene sequence. Furthermore, as long as siRNA is able to maintain its gene silencing effect on the target gene, siRNA may contain a low molecular weight RNA (which may be a natural RNA molecule such as tRNA, rRNA or viral RNA, or an artificial RNA molecule), for example, in the overhanging portion at its one end.

In addition, the terminal structure of the "siRNA" is not necessarily the cut off structure at both ends as described above, and may have a stem-loop structure in which ends of one side of double-stranded RNA are connected by a linker RNA. The length of the double-stranded RNA region (stem-loop portion) can be, for example, 15 to 49 bp, preferably 15 to 35 bp, and more preferably 21 to 30 bp long. Alternatively, the length of the double-stranded RNA region that is a final transcription product of siRNAs to be expressed is, for example, 15 to 49 bp, preferably 15 to 35 bp, and more preferably 21 to 30 bp long. Furthermore, there is no particular limitation in the length of the linker as long as it has a length so as not to hinder the pairing of the stem portion. For example, for stable pairing of the stem portion and suppression of the recombination between DNAs coding for the portion, the linker portion may have a clover-leaf tRNA structure. Even though the linker has a length that hinders pairing of the stem portion, it is possible, for example, to construct the linker portion to include introns so that the introns are excised during processing of precursor RNA into mature RNA, thereby allowing pairing of the stem portion. In the case of a stem-loop siRNA, either end (head or tail) of RNA with no loop structure may have a low molecu lar weight RNA. As described above, this low molecular weight RNA may be a natural RNA molecule such as tRNA, rRNA or viral RNA, or an artificial RNA molecule.

"Signal peptide" refers to a short (15-60 amino acids long) amino terminal peptide chain that directs the post translational transport of a protein; usually directs the peptide to the secretory pathway of the cell.

"Soybean cyst nematode or SCN" refers to a nematode belonging to *Heterodera glycines.*

"Sugar beet cyst nematode" refers to a nematode belonging to *Heterodera schachtii.*

"Transformed," "transgenic," "transfected" and "recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed," "non-transgenic," or "non-recombinant" host refers to a wild-type organism, e.g., a bacterium or plant, which does not contain the heterologous nucleic acid molecule.

A "transformed cell" refers to a cell into which has been introduced a nucleic acid molecule, for example by molecular biology techniques. As used herein, the term transformation encompasses all techniques by which a nucleic acid molecule might be introduced into such a cell, plant or animal cell, including transfection with viral vectors, transformation by Agrobacterium, with plasmid vectors, and introduction of naked DNA by electroporation, lipofection, and particle gun acceleration and includes transient as well as stable transformants.

The term "transgenic plant" refers to a plant or tree that contains recombinant genetic material not normally found in plants or trees of this type and which has been introduced into the plant in question (or into progenitors of the plant) by human manipulation. Thus, a plant that is grown from a plant cell into which recombinant DNA is introduced by transformation is a transgenic plant, as are all offspring of that plant that contain the introduced transgene (whether produced sexually or asexually). It is understood that the term transgenic plant encompasses the entire plant or tree and parts of the plant or tree, for instance grains, seeds, flowers, leaves, roots, fruit, pollen, stems etc.

The term "translation initiation enhancer sequence", as used herein, refers to a nucleic acid sequence that can determine a site and efficiency of initiation of translation of a gene (See, for example, McCarthy et al., 1990, Trends in Genetics, 6: 78-85). A translation initiation enhancer sequence can extend to include sequences 5' and 3' to the ribosome binding site. The ribosome binding site is defined to include, minimally, the Shine-Dalgarno region and the start codon, in addition to any bases in between. In addition, the translation initiation enhancer sequence can include an untranslated leader or the end of an upstream cistron, and thus a translational stop codon. See, for example, US Patent No. 5,840,523.

The term "vector" refers to a nucleic acid molecule which is used to introduce a polynucleotide sequence into a host cell, thereby producing a transformed host cell. A "vector" may comprise genetic material in addition to the above-described genetic construct, e.g., one or more nucleic acid sequences that permit it to replicate in one or more host cells, such as origin(s) of replication, selectable marker genes and other genetic elements known in the art (e.g., sequences for integrating the genetic material into the genome of the host cell, and so on).

### 2. Exemplary Embodiments

### 2.1 Nematode Resistant Transgenic Plants or Transgenic Plant Material

It has been discovered that interrupting the feeding cycle of cyst nematodes by down-regulating one or more cyst nematode parasitism genes is an effective method for reducing, preventing, or treating cyst nematode disease in plants. Cyst nematode parasitism genes refers to genes expressed in the esophageal gland cells encoding for secretory proteins exported from the gland cell to be released through the nematode's stylet into host tissue. In particular, it has been discovered that interfering with the expression of proteins secreted by cyst nematodes related to the formation of specialized feeding cells in host plants is an effective method for reducing, treating, or preventing nematode disease in plants. Representative parasitism genes encoding secreted proteins that can be targeted, for example with inhibitory RNA include, but are not limited to those genes listed in Table 1, SEQ ID NOs. 1-63 and 113-116, isoforms, homologues, or a fragment thereof. The inhibitory RNA can also target naturally occurring variations in parasitisim genes, for examples genes or proteins having conservative substitutions in the amino acid sequence or nucleic acid sequence.

SCN is a highly-specialized obligate parasite that transforms plant cells within the vascular and cortical tissues of susceptible soybean roots into an elaborate feeding site called a syncytium which is required for parasite growth and reproduction. Syncytia formation represents one of the most complex responses elicited in plant tissue by any parasite or pathogen. Secretions from the SCN oral spear (stylet) regulate, directly or indirectly, specific host genes affecting plant cell metabolism, protein synthesis, DNA endoreduplication, cell differentiation, and cell wall synthesis and degradation. The coordinated dissolution of adjacent root cell walls produces a multinucleate feeding site where the central vacuoles of the parasitized cells decrease in size, the cytoplasm increases in volume and density, and the cell walls thicken to form elaborate ingrowths, giving the syncytium the phenotype of modified transfer cells. Certain embodiments provide transgenic plants or transgenic plant material comprising inhibitory nucleic acids that inhibit formation of syncitia by nematodes. The inhibitory nucleic acids can be expressed by the plant or can be part of a composition applied to the plant.

Post-embryonic development of SCN is dependent upon plant parasitism for completion and is delineated by molts through four successive juvenile stages to reach reproductive maturity. Preparasitic second-stage juveniles (J2) of SCN hatch from eggs in soil to become the infective stage. These J2 penetrate soybean roots behind the root cap, migrate intracellularly to the root vascular tissue, and must induce a syncytium for feeding to progress to the swollen, sedentary reproductive adult life stage. The J2 inserts its protrusible stylet into a selected vascular parenchyma cell and releases secretions that transform a root cell into an initial syncytial cell that ultimately develops into the full syncytium.

SCN is well-adapted for plant parasitism by possessing, in addition to the stylet, three large and complex esophageal gland cells, one dorsal (DG) and two subventrals (SvG), that are the principal sources of secretions involved in infection and parasitism of soybean. Each esophageal gland is a single large transcriptionally active secretory cell. The two SvG cells are the most active esophageal glands in infective J2 of SCN while the single dorsal gland cell becomes the predominate source of secretions released through the stylet in subsequent parasitic stages.

The collective data provided in at least Examples 2-3 demonstrate that homologous parasitism genes exist between H. *glycines* and *H. schachtii* and that these homologous parasitism genes are expressed in the same pattern between the two cyst nematode species. The data indicate that *H. schachtii* uses similar parasitism genes to infect host plants and suggest that the host plant, *Arabidopsis thaliana,* may be used to assess both *H. glycines* and H. *schachtii* parasitism genes as a model host plant. The strong identity between *H. glycines* and *H. schachtii* parasitism genes indicates that other cyst nematode species contain significantly similar parasitism genes. Accordingly, one of skill in the art would recognize that nematode resistance in plants obtained by inhibiting expression and activity of esophageal gland cell secretory proteins secreted by H. *schachtii* as shown herein, can also be obtained by inhibiting the expression of the similar protein in *H. glycines.*

One embodiment provides a plant or cell comprising one or more inhibitory RNAs specific for one or more mRNAs of one or more cyst nematode parasitism genes. Inhibitory RNAs specific for one or more mRNAs means that the inhibitory RNA down-regulates the expression of a specific RNA. The inhibitory RNA can be single- or double-stranded or a combination thereof. For example, the present disclosure provides transgenic plants that express one or more inhibitory RNAs that down regulate cyst nematode parasitism gene expression when the one or more inhibitory RNAs are absorbed or ingested by a cyst nematode. The transgenic plant can be designed to express inhibitory RNA that down-regulates the target parasitism gene transcript in at least two different cyst nematode species, for example *Heterodera and, Globodera* spp., or a combination thereof. Another embodiment provides a transgenic plant that comprises inhibitory RNA that down regulates the target parasitism gene transcript in 3, 4, 5, 6, 7, 8, 9, 10, 11, 12, 13, 14, 15, 16, 17, 18, 19, 20, or more cyst nematode species. Thus, the present disclosure provides transgenic plants and transgenic plant material that are resistant to disease caused by multiple cyst nematode species.

Another embodiment provides a transgenic plant or transgenic cell conta,ining or expressing one or more inhibitory nucleic acids specific for at least a portion of a nucleic acid encoding one or more esophageal gland cell secretory polypeptides of a cyst nematode, for example a J2 cyst nematode sufficient to down-regulate the expression of the nucleic acid encoding the one or more esophageal gland cell secretory polypeptide. The inhibitory nucleic acid is typically a small inhibitory RNA or microRNA that is specific for mRNA encoding a cyst nematode esophageal gland cell protein or polypeptide. It will be appreciated by one of skill in the art that the inhibitory nucleic acid can be RNA, DNA, or a combination thereof. Additionally, the inhibitory nucleic acid may be single or multi-stranded and may be anti-sense or enzymatic. In one embodiment, the inhibitory nucleic acid interferes with, inhibits, or reduces the translation of a target mRNA. For example, the inhibitory nucleic acid can bind to a target mRNA and induce or promote the degradation of the target mRNA or physically prevent the cellular translational machinery from translating the target mRNA into a functional protein. Inhibition of the secretory polypeptide can be compared to controls, for example plants or cells that do not contain or express the inhibitory nucleic acid. A "control" refers to a sample of material used to produce the transgenic material and differs from the transgenic material in that the control material does not contain or express the inhibitory nucleic acid.

The term "esophageal gland cell protein or polypeptide" refers to a secretory polypeptide encoded by a cyst nematode parasitism gene. In one embodiment, the esophageal gland cell protein or polypeptide to be down-regulated generally is a secreted protein that modulates expression of at least one host plant gene. Exemplary nematode polypeptides that are down-regulated in the disclosed compositions and methods include, but are not limited to polypeptides or fragments thereof encoded by SEQ ID NOs. 1-63 and 113-116 or fragments thereof. The secretory polypeptide can increase or decrease expression of host plant genes either directly or indirectly. For example, direct modulation can occur when the esophageal gland cell protein or polypeptide binds to a host plant nucleic acid, including genomic DNA, RNA, and mRNA. Indirect modulation can occur for example when the polypeptide binds with one or more other proteins or factors to form a complex. The complex can then bind to a host plant nucleic acid to either promote or suppress transcription or translation.

Down-regulation of the secretory protein alleviates or reduces at least one symptom associated with cyst nematode disease. Exemplary symptoms of cyst nematode disease include, but are not limited to the formation of syncytia, lesions, stunting, nutrient and water deficiencies, dieback, and numbers of nematodes infecting a plant. Levels of reduction or inhibition of nematode disease in transgenic plants or cells can be compared to levels of nematode disease in control plants or cells. In one embodiment, the inhibitory nucleic acid reduces, inhibits, alleviates, treats or prevents cyst nematode disease.

In another embodiment, the esophageal gland cell protein or polypeptide to be down-regulated is encoded by a cyst nematode parasitism gene involved in the formation of a syncytium. In still other embodiments, the targeted cyst nematode parasitism gene encodes a polypeptide or nucleic acid involved in cyst nematode migration through root tissue, alters cell metabolism, elicits signal transduction in the recipient cell, or forms a feeding tube that enables the cyst - nematode to feed from the syncytium. Additionally, the esophageal gland cell protein or polypeptide can cause cell wall modifications and potentially interact with signal transduction receptors in the extracellular space, influence cellular metabolism, cell cycle, selective protein degradation, localized defense response, and regulatory activity within the plant cell nucleus.

Exemplary plant genes that are modulated by the esophageal gland cell protein or polypeptide include, but are not limited to genes involved in the formation of specialized nematode feeding cells also known as syncytia. Representative plant genes that can be modulated by cyst nematode esophageal gland cell polypeptides include, but are not limited to *WUN1, POX, CAT, GST, Mia-1, Mia-2, Mia-3, Mia-4, CHS1-CHS3, LOX,* Chitinase, Trypsin inhibitor, Miraculin, *HMGR, TSW12, LEA14, LEMMI9,* C6-19, C27-45, *TAS14, UBC* DB#103, *RPE, ISDGh, IPPP, LPPL, mUCp,* endomembrane protein, 20s proteasome, DAP decarboxylase, *GRP, ENOD40, ATAO1* or combinations thereof (Gheysen, G. and Fenoll, C. 2002. Annual Review of Phytopathology 40:191, which, where permissible, is incorporated by reference in its entirety). Generally, the plant gene is directly or indirectly involved in root cell growth, root cell division or the production of specific nutrients ingested by the parasitic nematode. The gene can be one expressed in a root cell or any other cell of the plant.

In one embodiment, expression of a targeted cyst nematode secretory protein is reduced, inhibited, or blocked, as compared to a control, when the inhibitory nucleic acid is delivered to the cyst nematode. Delivery of the inhibitory nucleic acid can be achieved, for example, when the cyst nematode comes into contact with the inhibitory nucleic acid as the cyst nematode feeds on the transgenic plant or cell. The cyst nematode can ingest the inhibitory nucleic acid during feeding, or the nucleic acid can be transported across a cellular membrane of the nematode by active transport or passive diffusion. It will be appreciated that the inhibitory nucleic acid can be delivered to the cyst nematode in combination or alternation with an agent that induces or promotes the uptake of the inhibitory nucleic acid by the nematode. An exemplary inducing agent includes, but is not limited to octopamine.

In another embodiment, the transgenic plant or transgenic cell expresses the inhibitory nucleic acid in an amount effective to modulate the expression of a cyst nematode esophageal gland cell polypeptide or protein in a cyst nematode when the inhibitory nucleic acid is delivered to the nematode. Expression levels can be decreased by about 10, 20, 30, 40, 50, 60, 70, 80, or 90% compared to a control. Levels of expression of the inhibitory nucleic acid in a transgenic plant or cell can be controlled using methods known in the art, for example using vectors with strong promoters or constitutively active promoters, high copy number vectors, etc. The plant or cell can be stably or transiently transfected. Another embodiment provides a transgenic seed comprising or capable of expressing an inhibitory nucleic acid specific for a cyst nematode esophageal gland secretory polypeptide.

An exemplary cyst nematode includes, but is not limited to members of *Heterodera spp.* Representative species include, but are not limited to *Heterodera glycines,* also referred to as soybean cyst nematode, and *Heterodera schachtii,* also referred to as the sugar beet cyst nematode

Representative host plants that can be transfected with an inhibitory nucleic acid according the present disclosure include, but are not limited to monocots, dicots, tobacco, cereals, sugar beets, potato, rice, corn, and soybeans. Other host plants include members of the phylogenic family *Leguminosae, Chenopodiaceae, Cruciferae,* and *Solanaceae.*

Another embodiment provides a cell containing a nucleic acid encoding an inhibitory nucleic acid specific for an mRNA or fragment thereof, wherein the mRNA encodes a cyst nematode esophageal gland cell protein or polypeptide that directly or indirectly modulates root cell gene expression, syncytia formation, nematode migration through root tissue, cell metabolism, signal transduction, or is involved in the formation of a feeding tube that enables the nematode to feed from the syncytia. The targeted cyst nematode esophageal gland cell protein or polypeptide or esophageal polypeptide can cause cell wall modifications and potentially interact with signal transduction receptors in the extracellular space, influence cellular metabolism, cell cycle, selective protein degradation, localized defense response, and regulatory activity within the plant cell nucleus. The cell can be prokaryotic or eukaryotic, and generally is a plant cell, particularly a root cell.

Yet another embodiment provides transgenic plants or plant cells containing an inhibitory nucleic acid, for example siRNA or microRNA, that down regulates cyst nematode esophageal gland cell proteins when delivered to a nematode feeding on the plant or plant cell. RNA interference is known in the art. See for example, Kreutzer et al., International PCT Publication No. WO 00/44895; Zernicka-Goetz et al, International PCT Publication No. WO 01/36646; Fire, International PCT Publication No. WO 99132619; Plaetinck et al., International PCT Publication No. WO 00/01846; Mello and Fire, International PCT Publication No. WO 01/29058; Deschamps-Depaillette, International PCT Publication No. WO 99/07409; Li et al., International PCT Publication No. WO 00/44914; and Trick et al., US20040098761.

In one embodiment, the nematode is not a member of *Meloidogyne spp.,* for example *Meloidogyne incognita* also known as the root-knot nematode.

In another embodiment, the inhibitory nucleic acid is not directly lethal to embryonic or adult nematodes or is not involved in nematode fertility, but instead inhibits the ability of the nematode to feed on or obtain nutrients from the transgenic plant or plant cell or to produce feeding tubes or modified plant cells for feeding.

In some embodiments, inhibitory double stranded RNA (dsRNA) is derived from an "exogenous template". Such a template may be all or part of a plant or nematode nucleotide sequence; it may be a DNA gene sequence or a cDNA produced from an mRNA isolated from a parasitic nematode, for example by reverse transcriptase. When the template is all or a part of a DNA gene sequence, it is preferred to be from one or more or all exons of the gene. While the dsRNA is derived from an endogenous or exogenous template, there is no limitation on the manner in which it could be synthesized. For example, the siRNA can be chemically synthesized, produced by in vitro transcription; produced by digestion of long dsRNA by an RNase III family enzyme (e.g., Dicer, RNase 111); expressed in cells from an siRNA expression plasmid or viral vector; or expressed in cells from a PCR-derived siRNA expression cassette

SiRNA prepared in vitro is then introduced directly into cells by transfection, electroporation; or by another method. Alternatively, transfection of DNA-based vectors and cassettes that express siRNAs within the cells can be used. RNAI may be synthesized in vitro or in vivo, using manual and/or automated procedures. In vitro synthesis may be chemical or enzymatic, for example using cloned RNA polymerase (e.g., T3, T7, SP6) for transcription of the endogenous DNA (or cDNA) template, or a mixture of both.

In vivo, the dsRNA may be synthesised using recombinant techniques well known in the art (see e.g., Sambrook, et al., Molecular Cloning; A Laboratory Manual, Third Edition (2001). For example, bacterial cells can be transformed with an expression vector which comprises the DNA template from which the dsRNA is to be derived. Alternatively, the cells of a plant for example, in which inhibition of nematode gene expression is required may be transformed with an expression vector or by other means. Bidirectional transcription of one or more copies of the template may be by endogenous RNA polymerase of the transformed cell or by a cloned RNA polymerase (e.g., T3, T7, SP6) coded for by the expression vector or a different expression vector. The use and production of an expression construct are known in the art (see WO98/32016; U.S. Pat. Nos. 5,593,874, 5,698,425, 5712,135, 5,789,214, and 5,804,693). Inhibition of nematode gene expression may be targeted by specific transcription in an organ, tissue, or cell type; an environmental condition (e.g. temperature, chemical); and/or engineering transcription at a developmental stage or age, especially when the dsRNA is synthesized in vivo in the plant cell for example. dsRNA may also be delivered to specific tissues or cell types using known gene delivery systems. Components of these systems include the seed-specific lectin promoter and the flower specific promoter from APETALA3. These vectors are listed solely by way of illustration of the many commercially available and well known vectors that are available to those of skill in the art.

If synthesized outside the cell, the RNA may be purified prior to introduction into the cell. Purification may be by extraction with a solvent (such as phenol/chloroform) or resin, precipitation (for example in ethanol), electrophoresis, chromatography, or a combination thereof. However, purification may result in loss of dsRNA and may therefore be minimal or not carried out at all. The RNA may be dried for storage or dissolved in an aqueous solution, which may contain buffers or salts to promote annealing, and/or stabilization of the RNA strands.

Suitable dsRNA can also contain one or more modified bases, or have a modified backbone to increase stability or for other reasons. For example, the phosphodiester linkages of natural RNA may be modified to include at least one of a nitrogen or sulfur heteroatom. Moreover, dsRNA comprising unusual bases, such as inosine, or modified bases, such as tritylated bases, to name just two examples, can be used. It will be appreciated that a great variety of modifications have been made to RNA that serve many useful purposes known to those of skill in the art. The term dsRNA as it is employed herein embraces such chemically, enzymatically or metabolically modified forms of dsRNA, provided that it is derived from an endogenous template.

The double-stranded structure may be formed by a single self-complementary RNA strand or two separate complementary RNA strands. RNA duplex formation may be initiated either inside or outside the plant cell.

The sequence of at least one strand of the dsRNA contains a region complementary to at least a part of the target mRNA sufficient for the dsRNA to specifically hybridize to the target mRNA. In one embodiment, the siRNA is substantially identical to at least a portion of the target mRNA. "Identity", as known in the art, is the relationship between two or more polynucleotide (or polypeptide) sequences, as determined by comparing the sequences. In the art, identity also means the degree of sequence relatedness between polynucleotide sequences, as determined by the match between strings of such sequences. Identity can be readily calculated (Computational Molecular Biology, Lesk, A. M., ed., Oxford University Press, New York, 1988; Biocomputing: lnformatics and Genome Projects, Smith, D. W., ed., Academic Press, New York, 1993; Computer Analysis of Sequence Data, Part I, Griffin, A. M., and Griffin, H. G., eds., Humana Press, N.J., 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide sequences, the term is well known to skilled artisans (Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., SIAM J. Applied Math., 48: 1073 (1988). Methods commonly employed to determine identity between sequences include, but are not limited to those disclosed in Carillo, H., and Lipman, D., SIAM J. Applied Math., 48:1073 (1988). Preferred methods to determine identity are designed to give the largest match between the sequences tested. Methods to determine identity are codified in computer programs. Computer program methods to determine identity between two sequences include, but are not limited to, GCG program package (Devereux, J., et al., Nucleic Acids Research 12(1): 387 (1984)), BLASTP, BLASTN, and FASTA (Atschul, S. F. et al., J. Molec. Biol. 215: 403 (1990)). Another software package well known in the art for carrying out this procedure is the CLUSTAL program. It compares the sequences of two polynucleotides and finds the optimal alignment by inserting spaces in either sequence as appropriate. The identity for an optimal alignment can also be calculated using a software package such as BLASTx. This program aligns the largest stretch of similar sequence and assigns a value to the fit. For any one pattern comparison several regions of similarity may be found, each having a different score. One skilled in the art will appreciate that two polynucleotides of different lengths may be compared over the entire length of the longer fragment. Alternatively small regions may be compared. Normally sequences of the same length are compared for a useful comparison to be made.

In one embodiment, the inhibitory nucleic acid has 100% sequence identity with at least a part of the target mRNA. However, inhibitory nucleic acids having 70%, 80% or greater than 90% or 95% sequence identity may be used. Thus sequence variations that might be expected due to genetic mutation, strain polymorphism, or evolutionary divergence can be tolerated.

The duplex region of the RNA may have a nucleotide sequence that is capable of hybridizing with a portion of the target gene transcript (e.g., 400 mM NaCl, 40 mM PIPES pH 6.4, 1 mM EDTA, 50° C or 70° C hybridization for 12-16 hours; followed by washing).

While the optimum length of the dsRNA may vary according to the target gene and experimental conditions, the duplex region of the RNA may be at least 19, 20, 21-23, 25, 50, 100, 200, 300, 400 or more bases long.

Target genes are cyst nematode genes encoding secreted esophageal gland cell proteins, in particular secreted proteins that modulate: gene expression of the plant or cell, formation of a syncytium, nematode migration through root tissue of the plant, cell metabolism of the plant, elicits signal transduction in the plant cell, or forms a feeding tube that enables the nematode to feed from syncytia formed in the plant. Typically, the dsRNA or inhibitory nucleic acid is substantially identical to the whole of the target gene, i.e. the coding portion of the gene. However, the dsRNA or inhibitory nucleic acid can be substantially identical to a part of the target gene. The size of this part depends on the particular target gene and can be determined by those skilled in the art by varying the size of the dsRNA and observing whether expression of the gene has been inhibited.

In still another embodiment, the inhibitory nucleic acid can be an antisense nucleic acid specific for mRNA encoding a protein encoded by one or more of SEQ ID Nos 1-63 and 113-116.

Another embodiment provides an isolated nucleic acid selected from the group consisting of SEQ ID NOs:113, 114, 115, and 116 or a vector comprising SEQ ID NOs:113, 114, 115, and 116.

Yet another embodiment provides an isolated host cell comprising the vector a vector comprising SEQ ID NOs:113, 114, 115, and 116. The host cell can be eukaryotic or prokaryotic, preferably a plant cell. The inhibitory nucleic acid can specifically inhibit expression or activity or a nucleic acid selected from the group consisting of SEQ ID NOs:113, 114, 115, and 116.

The inhibitory nucleic acids disclosed here can be in seeds and seed products derived from the transgenic plants described above.

### 2.2 Transgenic Plant Material Compositions

The disclosed transgenic plants and transgenic plant material can be used as a component in feedstock. The feedstock can be in the form of pellets, granules, flakes and the like and can be used to feed domesticated animals such as cattle, sheep, goats, pigs, and pets such as cats and dogs. It will be appreciated that the disclosed transgenic plants and transgenic plant material can also be used to produce foodstuffs for human comsumption.

Another embodiment provides a composition having an inhibitory nucleic acid specific for an mRNA or fragment thereof encoding a polypeptide encoded by one or more of SEQ ID NOs. 1-63 or a fragment or homologues thereof, in an amount sufficient to inhibit expression of the polypeptide encoded by one or more of SEQ ID NOs. 1-63 or homologues thereof when delivered to a cyst nematode, for example when the nematode is feeding on a plant or cell expressing or containing or coated with the inhibitory nucleic acid. The composition can contain one or more nematicides, pesticides, fungicides, or combinations thereof. Representative nematicides include, but are not limited to chloropicrin, methyl bromide, 1,3-dichloropropene, sodium methyl dithiocarbamate, sodium tetrathiocarbonate; and carbamates such as 2-methyl-2-(methylthio)propionaldehyde O-methylcarbamoyloxime (aldicarb), 2,3-Dihydro-2,2-dimethyl-7-benzofuranol methylcarbamate (carbofuran), methyl 2-(dimethylamino)-N-[[(methylamino)carbonyl]oxy]-2-oxoethanimidothioate (oxamyl), 2-methyl-2-(methylsulfonyl)propanal O-[(methylamino)carbonyl]oxime (aldoxycarb), *O,O*-diethyl *O*-[4-(methylsulfinyl)phenyl] phosphorothioate (fensulfothion), O-Ethyl S,S-dipropylphosphorodithioate (ethoprop), and Ethyl-3-methyl-4-(methylthio)phenyl(1-methylethyl)phosphoramidate (phenamiphos). The composition can be formulated to be coated to be coated on a plant, plant part, or seed. In certain aspects the inhibitory nucleic acid is combined with one or more excipients, buffering agents, carriers, etc. Excipients, buffering agents, and carriers are well known in the art.

Standard excipients include gelatin, casein, lecithin, gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidol silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethycellulose phthalate, noncrystalline cellulose, magnesium aluminum silicate, triethanolamine, polyvinyl alcohol, polyvinylpyrrolidone, sugars and starches.

The coating can be formulated as a spray or dip so that the inhibitory nucleic acids remain on the plant material and remain able to inhibit nematode esophageal gland cell secretory protein expression in cyst nematodes as the plant matures and develops. For example, the seed of a plant can be coated with a composition comprising an amount of one or more of the disclosed inhibitory nucleic acids effective to inhibit or reduce nematode disease in the plant in combination with an excipient.

Another embodiment provides a composition comprising an inhibitor of the polypeptide encoded by SEQ ID NOs:1-63 and 113-116 or a combination thereof. The inhibitor can be a small molecule such such as carbon-based compounds including cyclic, heterocylic, and aliphatic compounds. Such compounds can be identified using standard screening assays. The inhibitor can also be an aptamer specific for SEQ ID NOs: 1-63 and 113-116 or a secondary metabolite including but not limited to polyketides, alkaloids, hormones, and oligosaccharides that are products of specialized (multigene) enzyme pathways in cells. Secondary metabolites do not usually regulate "expression" of polypeptides, per se, but they are known to bind to and regulate (inhibit) the "activity" of target (specific) polypeptides in cells.

### 2.3 Methods of Using the Disclosed Inhibitory Nucleic Acids

One embodiment provides a method for providing cyst nematode resistance to a plant by contacting the plant or expressing in the plant one or more inhibitory nucleic acids specific for one or more cyst nematode esophageal gland cell secretory proteins in an amount sufficient to reduce cyst nematode disease. The targeted one or more cyst nematode esophageal gland cell proteins can modulate: gene expression of the plant or cell, formation of a syncytium, nematode migration through root tissue of the plant, cell metabolism of the plant, elicits signal transduction in the plant cell, or forms a feeding tube that enables the nematode to feed from syncytia formed in the plant. Inhibition of one or more of the targeted gland cell proteins, for example those encoded by SEQ ID NOs: 1-63 can provide the plant cell with cyst nematode resistance for example by down regulating one or more cyst nematode gland cell secretory proteins involved in forming syncytia in the plant. One aspect provides inhibitory nucleic acids specific for cyst nematode esophageal gland cell proteins secreted by nematodes, in particular SCN. The inhibitory nucleic acid can be sprayed onto the plant or otherwise delivered to the plant so that the inhibitory nucleic acid comes into contact with a cyst nematode.

Another embodiment provides a method for inhibiting cyst nematode syncytia formation in a plant by expressing in the plant an inhibitory nucleic acid in an amount effective to inhibit the expression by the cyst nematode of a polypeptide encoded by one or more of SEQ ID NOs: 1-63 or a homologue thereof.

Still another embodiment provides a method for inhibiting biological activity of a nematode parasitism gene product by expressing an inhibitory peptide, inhibitory polypeptide, inhibitory nucleic acid, or a combination thereof in a plant, wherein the inhibitory peptide, polypeptide, or inhibitory nucleic acid specifically inhibits the biological activity or expression of the nematode parasitism gene product. Representative parasitic nematodes include, but are not limited to *Heterodera* spp. and *Globorela* spp. The inhibitory peptides or inhibitory polypeptides can specifically associate with the polypeptides encoded by SEQ ID NOs:9-63 and 113-116 in a sequence specific manner or can be conformationally complementary such that the two polypeptides physically interact. An exemplary inhibitory peptide or inhibitory polypeptide includes, but is not limited to an antibody or antibody fragment that specifically binds to the parasitic nematode gene product. The generation of antibodies is known in the art. Based on the nucleic acid sequences provided herein, one of skill in the art could readily produce antibodies to the polypeptides encoded by SEQ ID NOs:1-63 and 113-116. The antibodies could then be cloned and one or more of the antibodies or antigen binding antibody fragments can be expressed in a plant or plant cell so that the antibody binds the polypeptide encoded by one or more of SEQ ID NOs"1-63 and 113-116. Binding of the parasitic nematode gene product by the antibody or antigen binding antibody fragment can inhibit the activity of the parasitic nematode gene product and thereby provide the plant expressing the antibody or antigen binding antibody fragment with resistance to the parasitic nematode.

The inhibitory nucleic acid expressed in the plant or plant cell can be double-stranded RNA, antisense DNA, microRNA, siRNA, an aptamer or a combination thereof. The inhibitory nucleic acid can be specific for mRNA encoded by SEQ ID NOs:1-63 and 113-116. Additionally, the inhibitor can be secondary metabolite including but not limited to polyketides, alkaloids, hormones, and oligosaccharides that are products of specialized (multigene) enzyme pathways in cells.

### 2.4 Methods for Identifying Inhibitors of Nematode Parasitism Gene Products

Methods for identifying inhibitors of the products encoded by SEQ ID NOs:1-63, 113-116 or combinations thereof are also provided. As used herein the term "test compound" or "inhibitor" refers to any molecule that may potentially inhibit the biological function of a cyst nematode parasitism gene product, in particular the products encoded by SEQ ID NOs:1-63, 113-116. The test compound or modulator can be a protein or fragment thereof, a small molecule, or even a nucleic acid molecule. Some test compounds can be compounds that are structurally related to the products encoded by SEQ ID NOs:1-63 and 113-116.

One embodiment provides a method for identifying inhibitors of cyst nematode parasitism gene products by assaying activity of a cyst nematode parasitism gene product, a homo log, or fragment thereof in the presence of a test compound, and selecting the test compound that reduces or inhibits cyst nematode disease in plants compared to a control compound.

In another embodiment, small molecule libraries that are believed to meet the basic criteria for useful inhibitors of cyst nematode parasitism gene products can be screened to identify useful compounds. Screening of such libraries, including combinatorially generated libraries (e.g., expression libraries), is a rapid and efficient way to screen large number of related (and unrelated) compounds for activity. Combinatorial approaches also lend themselves to rapid evolution of potential drugs by the creation of second, third and fourth generation compounds modeled of active, but otherwise undesirable compounds.

Test compounds may include fragments or parts of naturally-occurring compounds, or may be found as active combinations of known compounds, which are otherwise inactive. Compounds isolated from natural sources, such as animals, bacteria, fungi, plant sources, including leaves and bark, and marine samples can be assayed as candidates for the presence of potentially useful pharmaceutical agents. It will be understood that the agents to be screened could also be derived or synthesized from chemical compositions or man-made compounds. Thus, it is understood that the test compound identified by embodiments of the present disclosure may be peptide, polypeptide, polynucleotide, small molecule inhibitors, small molecule inducers, organic or inorganic, or any other compounds that may be designed based on known inhibitors or stimulators.

Other suitable inhibitors include antisense molecules, catalytic nucleic acids such as ribozymes, and antibodies (including single chain antibodies), each of which would be specific for a cyst nematode parasitism gene or gene product, in particular specific for SEQ ID Nos. 1-63, 113-116 or the product encoded by or combinations thereof. For example, an antisense molecule that binds to a translational or transcriptional start site, or splice junctions, are within the scope of a test compound.

In addition to the inhibitor compounds initially identified, other sterically similar compounds may be formulated to mimic the key portions of the structure of the modulators. Such compounds, which may include peptidomimetics of peptide modulators, may be used in the same manner as the initial modulators.

An inhibitor according to the present disclosure may be one which exerts its inhibitory effect upstream, downstream, directly, or indirectly on a cyst nematode parasitism gene or gene product.

In some embodiments, the assays can include random screening of large libraries of test compounds. Alternatively, the assays may be used to focus on particular classes of compounds suspected of modulating the function or expression of occludin in epithelial or endothelial cells, tissues, organs, or systems.

Assays can include determinations of cyst nematode parasitism gene expression, protein expression, protein activity, or binding activity. Other assays can include determinations of nucleic acid transcription or translation, for example mRNA levels, mRNA stability, mRNA degradation, transcription rates, and translation rates, particular of polypeptides involved in cyst nematode disease in plants.

Specific assay endpoints or interactions that may be measured in the disclosed embodiments include, but are not limited to, assaying for nematode disease in plants after contacting the cyst nematode with a suspected inhibitor of one or more of the products of SEQ ID NOs: 1-63, 113-116 or a combination thereof. These assay endpoints may be assayed using standard methods such those disclosed in the Examples or using conventional assays for example, FACS, FACE, ELISA, Northern blotting and/or Western blotting.

Other screening methods include using labeled product encoded by SEQ ID NOs:1-63, 113-116 or a combination or biologically active fragment thereof to identify a test compound. Occludin can be labeled using standard labeling procedures that are well known and used in the art. Such labels include, but are not limited to, radioactive, fluorescent, biological and enzymatic tags.

Another embodiment provides for *in vitro* assays for the identification of inhibitors of products encoded by SEQ ID NOs 1-63, 113-116 or a combination thereof. Such assays generally use isolated molecules, can be run quickly and in large numbers, thereby increasing the amount of information obtainable in a short period of time. A variety of vessels may be used to run the assays, including test tubes, plates, dishes and other surfaces such as dipsticks or beads.

One example of a cell free assay is a binding assay. While not directly addressing function, the ability of an test comopound to bind to a target molecule, for example SEQ ID NOs 1-63, 113-116 or a polypeptide encoded by SEQ ID NOs. 1-63, 113-166, in a specific fashion is strong evidence of a related biological effect. Such a molecule can bind to a cyst nematode parasitic gene product, for example and inhibit or reduce the cyst nematode from forming a syncytium in a plant or plant cell. The binding of a molecule to a target may, in and of itself, be inhibitory, due to steric, allosteric or charge-charge interactions or may downregulate or inactivate the cyst nematode parasitism gene or gene product. The target may be either free in solution, fixed to a support, expressed in or on the surface of a cell. Either the target or the compound may be labeled, thereby permitting determining of binding. Usually, the target will be the labeled species, decreasing the chance that the labeling will interfere with or enhance binding. Competitive binding formats can be performed in which one of the agents is labeled, and one may measure the amount of free label versus bound label to determine the effect on binding.

A technique for high throughput screening of compounds is described in WO 84/03564. Large numbers of small peptide test compounds are synthesized on a solid substrate, such as plastic pins or some other surface. Bound polypeptide is detected by various methods.

### 3. Plant Transformation Technology

DNA molecules and RNA molecules of the present disclosure are incorporated in plant or bacterial cells using conventional recombinant DNA technology. Generally, a DNA or an RNA molecule of the present disclosure is comprised in a transformation vector. A large number of such vector systems known in the art may be used, such as plasmids. The components of the expression system are also modified, e.g., to increase expression of the introduced RNA fragments. For example, truncated sequences, nucleotide substitutions or other modifications may be employed. Expression systems known in the art may be used to transform virtually any plant cell under suitable conditions. A transgene comprising a DNA molecule of the present invention is preferably stably transformed and integrated into the genome of the host cells. Transformed cells are preferably regenerated into whole plants. Detailed description of transformation techniques are within the knowledge of those skilled in the art.

Reporter genes or selectable marker genes may be included in the expression cassette. Examples of suitable reporter genes known in the art can be found in, for example, Jefferson et al. (1991) in Plant Molecular Biology Manual, ed. Gelvin et al. (Kluwer Academic Publishers), pp. 1-33; DeWet et al. (1987) Mol. Cell. Biol. 7:725-737; Goff et al. (1990) EMBO J. 9:2517-2522; Kain et al. (1995) Bio Techniques 19:650-655; and Chiu et al. (1996) Current Biology 6:325-330.

Selectable marker genes for selection of transformed cells or tissues can include genes that confer antibiotic resistance or resistance to herbicides. Examples of suitable selectable marker genes include, but are not limited to, genes encoding resistance to chloramphenicol (Herrera Estrella et al. (1983) EMBO J. 2:987-992); methotrexate (Herrera Estrella et al. (1983) Nature 303:209-213; Meijer et al. (1991) Plant Mol. Biol. 16:807-820); hygromycin (Waldron et al. (1985) Plant Mol. Biol. 5:103-108; Zhijian et al. (1995) Plant Science 108:219-227); streptomycin (Jones et al. (1987) Mol. Gen. Genet 210:86-91); spectinomycin (Bretagne-Sagnard et al. (1996) Transgenic Res. 5:131-137); bleomycin (Hille et al. (1990) Plant Mol. Biol 7:171-176); sulfonamide (Guerineau et al. 1990) Plant Mol. Biol. 15:127-136); bromoxynil (Stalker et al. (1988) Science 242:41 9423); glyphosate (Shaw et al. (1986) Science 233:478481); phosphinothricin (DeBlock et al. (1987) EMBO J. 6:2513-2518).

Other genes that could serve utility in the recovery of transgenic events but might not be required in the final product would include, but are not limited to, examples such as GUS (b-glucoronidase; Jefferson (1987) Plant Mol. Biol. Rep. 5:387), GFP (green florescence protein; Chalfie et al. (1994) Science 263:802), luciferase (Riggs et al. (1987) Nucleic Acids Res. 15(19):8115 and Luehrsen et al. (1992) Methods Enzymol. 216:397-414) and the maize genes encoding for anthocyanin production (Ludwig et al. (1990) Science 247:449).

The expression cassette comprising a promoter sequence operably linked to a heterologous nucleotide sequence of interest can be used to transform any plant. In this manner, genetically modified plants, plant cells, plant tissue, seed, and the like can be obtained.

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway et al. (1986) Biotechniques 4:320-334), electroporation (Riggs et al. (1986) Proc. Natl. Acad. Sci. USA 83:5602-5606, Agrobacterium-mediated transformation (Townsend et al., U.S. Pat. No. 5,563,055; Zhao et al. WO US98/01268), direct gene transfer (Paszkowski et al. (1984) EMBO J. 3:2717-2722), and ballistic particle acceleration (see, for example, Sanford et al., U.S. Pat. No. 4,945,050; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); and McCabe et al. (1988) Biotechnology 6:923-926). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Dafta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Pat. No. 5,240,855; Buising et al., U.S. Pat. Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Bertin) (maize); Klein et al. (1988) Plant Physiol. 91:440-444 (maize); From et al. (1990) Biotechnology 8:833-839 (maize); Hooykaas-Van Slogteren et al. (1984) Nature (London) 311:763-764; Bowen et al., U.S. Pat. No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, N.Y.), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via Agrobacterium tumefaciens); all of which are herein incorporated by reference in their entirety.

The cells that have been transformed may be grown into plants in accordance with conventional techniques. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that constitutive expression of the desired phenotypic characteristic is stably maintained and inherited and then seeds harvested to ensure constitutive expression of the desired phenotypic characteristic has been achieved.

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize 1 n2-2 promoter, which is activated by benzenesulfonamide herbicide safeners, the maize GST promoter, which is activated by hydrophobic electrophilic compounds that are used as pre-emergent herbicides, and the tobacco PR-1 a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Pat. Nos. 5,814,618 and 5,789,156), herein incorporated by reference in their entirety.

Constitutive promoters include, for example, the core promoter of the Rsyn7 promoter and other constitutive promoters disclosed in WO 99/43838 and U.S. Pat. No. 6,072,050; the core CAMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Pat. No. 5,659,026), and the like. Other constitutive promoters include, for example, U.S. Pat. Nos. 5,608,149; 5,608,144; 5,604,121; 5,569,597; 5,466,785; 5,399,680; 5,268,463; 5,608,142.

Where low level expression is desired, weak promoters may be used. Generally, by "weak promoter" is intended a promoter that drives expression of a coding sequence at a low level. By low level is intended at levels of about 1/1000 transcripts to about 1/100,000 transcripts to about 1/500,000 transcripts. Alternatively, it is recognized that weak promoters also encompasses promoters that are expressed in only a few cells and not in others to give a total low level of expression. Where a promoter is expressed at unacceptably high levels, portions of the promoter sequence can be deleted or modified to decrease expression levels.

Such weak constitutive promoters include, for example, the core promoter of the Rsyn7 promoter (WO 99/43838 and U.S. Pat. No. 6,072,050), the core 35S CaMV promoter, and the like. Other constitutive promoters include, for example, U.S. Pat. Nos. 5,608,149; 5,608,144; 5,604,121; 5,569,597; 5,466,785; 5,399,680; 5,268,463; and 5,608,142.

"Tissue-preferred" promoters can be used to target a gene expression within a particular tissue. Tissue-preferred promoters include Yamamoto et al. (1997) Plant J. 12(2)255-265; Kawamata et al. (1997) Plant Cell Physiol. 38(7):792-803; Hansen et al (1997) Mol. Gen. Genet. 254(3):337-343; Russell et al. (1997) Transgenic Res. 6(2):157-168; Rinehart et al. (1996) Plant Physiol. 112(3):1331-1341; Van Camp et al (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol. Biol. 23(6):1129-1138; Matsuoka et al. (1993) Proc Natl. Acad. Sci: USA 90(20):9586-9590; and Guevara-Garcia et al. (1993) Plant J. 4(3):495-505. Such promoters can be modified, if necessary, for weak expression.

"Seed-preferred" promoters include both "seed-specific" promoters (those promoters active during seed development such as promoters of seed storage proteins) as well as "seed-germinating" promoters (those promoters active during seed germination). See Thompson et al. (1989) BioEssays 10:108, herein incorporated by reference. Such seed-preferred promoters include, but are not limited to, Cim1 (cytokinin-induced message); cZ19B1 (maize 19 kDa zein); milps (myo-inositol-1-phosphate synthase); and ce1A (cellulose synthase). Gama-zein is a preferred endosperm-specific promoter. Glob-1 is a preferred embryo-specific promoter. For dicots, seed-specific promoters include, but are not limited to, bean.beta.-phaseolin, napin,.beta.-conglycinin, soybean lectin, cruciferin, and the like. For monocots, seed-specific promoters include, but are not limited to, maize 15 kDa zein, 22 kDa zein, 27 kDa zein, g-zein, waxy, shrunken 1, shrunken 2, globulin 1, etc.

Leaf-specific promoters are known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

Root-preferred promoters are known and may be selected from the many available from the literature or isolated de novo from various compatible species. See, for example, Hire et al. (1992) Plant Mol. Biol. 20(2): 207-218 (soybean root-specific glutamine synthetase gene); Keller and Baumgartner (1991) Plant Cell 3(10):1051-1061 (root-specific control element in the GRP 1.8 gene of French bean); Sanger et al. (1990) Plant Mol. Biol. 14(3):433-443 (root-specific promoter of the mannopine synthase (MAS) gene of Agrobacterium tumefaciens); and Miao et al. (1991) Plant Cell 3(1):) 1-22 (full-length cDNA clone encoding cytosolic glutamine synthetase (GS), which is expressed in roots and root nodules of soybean). See also U.S. Pat. Nos. 5,837,876; 5,750,386; 5,633,363; 5,459,252; 5,401,836; 5,110,732; and 5,023,179.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation may be accomplished by transactivation of a silent plastid-bome transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Pat. No. 5,380,831, herein incorporated by reference.

Plants transformed in accordance with the present disclosure may be monocots or dicots and include, but are not limited to, any nematode host plant.

### 3.1 Construction of Plant Expression Vectors

Nucleic acid sequences intended for expression in transgenic plants are first assembled in expression cassettes behind a suitable promoter expressible in plants. The expression cassettes may also comprise any further sequences required or selected for the expression of the transgene. Such sequences include, but are not restricted to, transcription terminators, extraneous sequences to enhance expression such as introns, vital sequences, and sequences intended for the targeting of the gene product to specific organelles and cell compartments. These expression cassettes can then be easily transferred to the plant transformation vectors described infra. The following is a description of various components of typical expression cassettes.

### 3. 1.1 Promoters

The selection of the promoter used in expression cassettes determine the spatial and temporal expression pattern of the transgene in the transgenic plant. Selected promoters express transgenes in specific cell types (such as leaf epidermal cells, mesophyll cells, root cortex cells) or in specific tissues or organs (roots, leaves or flowers, for example) and the selection reflects the desired location of accumulation of the gene product. Alternatively, the selected promoter drives expression of the gene under various inducing conditions.

Promoters vary in their strength, i.e., ability to promote transcription. Depending upon the host cell system utilized, any one of a number of suitable promoters known in the art may be used. For example, for constitutive expression, the CaMV 35S promoter, the rice actin promoter, or the ubiquitin promoter may be used. For example, for regulatable expression, the chemically inducible PR-1 promoter from tobacco or Arabidopsis may be used (see, e.g., U.S. Pat. No. 5,689,044).

A suitable category of promoters is that which is wound inducible. Numerous promoters have been described which are expressed at wound sites. Preferred promoters of this kind include those described by Stanford et al. Mol. Gen. Genet. 215: 200-208 (1989), Xu et al. Plant Molec. Biol. 22: 573-588 (1993), Logemann et al. Plant Cell 1: 151-158 (1989), Rohrmeier & Lehle, Plant Molec. Biol. 22: 783-792 (1993), Firek et al. Plant Molec. Biol. 22: 129-142 (1993), and Warner et al. Plant J. 3: 191-201 (1993).

Suitable tissue specific expression patterns include green tissue specific, root specific, stem specific, and flower specific. Promoters suitable for expression in green tissue include many which regulate genes involved in photosynthesis, and many of these have been cloned from both monocotyledons and dicotyledons. A suitable promoter is the maize PEPC promoter from the phosphoenol carboxylase gene (Hudspeth & Grula, Plant Molec.Biol. 12: 579-589 (1989)). A suitable promoter for root specific expression is that described by de Framond (FEBS 290: 103-106 (1991); EP 0 452 269 and a root-specific promoter is that from the T-1 gene. A suitable stem specific promoter is that described in U.S. Pat. No. 5,625,136 and which drives expression of the maize trpA gene.

### 3.1.2 Transcriptional Terminators

A variety of transcriptional terminators are available for use in expression cassettes. These are responsible for the termination of transcription beyond the transgene and its correct polyadenylation. Appropriate transcriptional terminators are those that are known to function in plants and include the CaMV 35S terminator, the tm1 terminator, the nopaline synthase terminator and the pea rbcS E9 terminator. These are used in both monocotyledonous and dicotyledonous plants.

### 3.1.3 Sequences for the Enhancement or Regulation of Expression

Numerous sequences have been found to enhance gene expression from within the transcriptional unit and these sequences can be used in conjunction with the genes to increase their expression in transgenic plants. For example, various intron sequences such as introns of the maize Adh1 gene have been shown to enhance expression, particularly in monocotyledonous cells. In addition, a number of non-translated leader sequences derived from viruses are also known to enhance expression, and these are particularly effective in dicotyledonous cells.

### 3.1.4 Coding Sequence Optimization

The coding sequence of the selected gene may be genetically engineered by altering the coding sequence for optimal expression in the crop species of interest. Methods for modifying coding sequences to achieve optimal expression in a particular crop species are well known (see, e.g. Perlak et al., Proc. Natl. Acad. Sci. USA 88: 3324 (1991); and Koziel et al, Bio/technol. 11: 194 (1993)).

Another embodiment provides an RNA molecule directly transformed into the plastid genome. Plastid transformation technology is extensively described in U.S. Pat. Nos. 5,451,513, 5,545,817, and 5,545,818, in PCT application no. WO 95/16783, and in McBride et al. (1994) Proc. Natl. Acad. Sci. USA 91, 7301-7305. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab, Z., Hajdukiewicz, P., and Maliga, P. (1990) Proc. Natl. Acad. Sci. USA 87,8526-8530; Staub, J. M., and Maliga, P. (1992) Plant Cell 4,39-45). The presence of cloning sites between these markers allowed creation of a plastid targeting vector for introduction of foreign DNA molecules (Staub, J. M., and Maliga, P. (1993) EMBO J. 12,601-606). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3'-adenyltransferase (Svab, Z., and Maliga, P. (1993) Proc. Natl. Acad. Sci. USA 90,913-917). Previously, this marker had been used successfully for high-frequency transformation of the plastid genome of the green alga Chlamydomonas reinhardtii (Goldschmidt-Clermont, M. (1991) Nucl. Acids Res. 19: 4083-4089). Other selectable markers useful for plastid transformation are known in the art and are encompassed within the scope of the invention.

### 3.2 Construction of Plant Transformation Vectors

Numerous transformation vectors available for plant transformation are known to those of ordinary skill in the plant transformation arts, and the genes pertinent to this disclosure can be used in conjunction with any such vectors. The selection of vector depends upon the selected transformation technique and the target species for transformation. For certain target species, different antibiotic or herbicide selection markers are preferred. Selection markers used routinely in transformation include the npt11 gene, which confers resistance to kanamycin and related antibiotics (Messing & Vierra. Gene 19: 259-268 (1982); Bevan et al., Nature 304: 184-187 (1983)), the bar gene, which confers resistance to the herbicide phosphinothricin (White et al., Nucl. Acids Res 18: 1062 (1990), Spencer et al. Theor. Appl. Genet 79: 625-631 (1990)), the hph gene, which confers resistance to the antibiotic hygromycin (Blochinger & Diggelmann, Mol Cell Biol 4: 2929-2931), the manA gene, which allows for positive selection in the presence of mannose (Miles and Guest (1984) Gene, 32: 41-48; U.S. Patent No. 5,767,378), and the dhfr gene, which confers resistance to methotrexate (Bourouis et al., EMBO J. 2 (7): 1099-1104 (1983)), and the EPSPS gene, which confers resistance to glyphosate (U.S. Pat. Nos. 4,940,935 and 5,188,642).

Many vectors are available for transformation using *Agrobacterium tumefaciens.* These typically carry at least one T-DNA border sequence and include vectors such as pBIN19 (Bevan, Nucl. Acids Res. (1984). Typical vectors suitable *for Agrobacterium* transformation include the binary vectors pClB200 and pClB2001, as well as the binary vector pCIB 10 and hygromycin selection derivatives thereof. (See, for example, U.S. Pat. No. 5,639,949).

Transformation without the use *of Agrobacterium tumefaciens* circumvents the requirement for T-DNA sequences in the chosen transformation vector and consequently vectors lacking these sequences are utilized in addition to vectors such as the ones described above which contain T-DNA sequences. Transformation techniques that do not rely on Agrobacterium include transformation via particle bombardment, protoplast uptake (e.g. PEG and electroporation) and microinjection. The choice of vector depends largely on the preferred selection for the species being transformed. Typical vectors suitable for non-Agrobacterium transformation include pClB3064, pSOG 19, and pSOG35. (See, for example, U.S. Pat. No. 5,639,949).

### 3.3. Transformation Techniques

Once the DNA sequence of interest is cloned into an expression system, it is transformed into a plant cell. Methods for transformation and regeneration of plants are well known in the art. For example, Ti plasmid vectors have been utilized for the delivery of foreign DNA, as well as direct DNA uptake, liposomes, electroporation, micro-injection, and microprojectiles. In addition, bacteria from the genus Agrobacterium can be utilized to transform plant cells.

Transformation techniques for dicotyledons are well known in the art and include Agrobacterium-based techniques and techniques that do not require *Agrobacterium.* Non Agrobacterium techniques involve the uptake of exogenous genetic material directly by protoplasts or cells. This is accomplished by PEG or electroporation mediated uptake, particle bombardment-mediated delivery, or microinjection. In each case the transformed cells may be regenerated to whole plants using standard techniques known in the art.

Transformation of most monocotyledon species has now become somewhat routine. Preferred techniques include direct gene transfer into protoplasts using PEG or electroporation techniques, particle bombardment into callus tissue or organized structures, as well as Agrobacterium-mediated transformation.

Plants from transformation events are grown, propagated and bred to yield progeny with the desired trait, and seeds are obtained with the desired trait, using processes well known in the art. The methods can result in plant cells comprising the RNA fragments of the present invention, wherein the expression of said target gene in said plant cell is altered by said RNA fragments, a plant and the progeny thereof derived from the plant cell, and seeds derived from the plant.

The disclosed inhibitory nucleic acids or SCN esophageal gland cell secretory polypeptides may be used alone or as a component of a kit having at least one of the reagents necessary to carry out the in vitro or in vivo introduction of RNA to subjects. Suitable components are the dsRNA and a vehicle that promotes introduction of the dsRNA. Such a kit may also include instructions to allow a user of the kit to practice the invention.

Another embodiment provides a method for providing resistance to nematode disease by introducing into a nematode host plant cell an RNA comprising a double stranded structure having a nucleotide sequence which is complementary to at least a part of the target mRNA; and optionally verifying inhibition of expression of the target mRNA.

One embodiment provides a method for treating or preventing nematode disease in a plant by contacting a parasitic nematode in or on the plant with a with dsRNA having a sequence which is complementary to at least a part of a mRNA encoding a nematode secretory protein, for example an esophageal gland cell protein; wherein the secretory protein modulates gene expression of plant.

Still another embodiment provides a plant cell, for example, containing an expression construct, the construct coding for an RNA which forms a double stranded structure having a nucleotide sequence which is complementary to at least a part of a target mRNA that encodes a nematode secretory protein, for example an esophageal gland cell protein, as well as a transgenic plant containing such a cell.

In another embodiment, the RNA fragments are comprised in two different RNA molecules. In this case, the RNA fragments are mixed before being introduced into said cell, e.g. under conditions allowing them to form a double-stranded RNA molecule. In another embodiment, the RNA fragments are introduced into said cell sequentially. Preferably, the time interval between the introduction of each of the RNA molecule is short, preferably less than one hour.

In still another embodiment, the RNA fragments are comprised in one RNA molecule. By using one single RNA molecule, the two complementary RNA fragments are in close proximity such that pairing and double strand formation is favored. In such case, the RNA molecule is preferably capable of folding such that said RNA fragments comprised therein form a double-stranded region. In this case, the complementary parts of the RNA fragments recognize one another, pair with each other and form the double-stranded RNA molecule. In another embodiment, the RNA fragments are incubated under conditions allowing them to form a double-stranded RNA molecule prior to introduction into the cell. In yet another embodiment, the RNA molecule comprises a linker between the sense RNA fragment and the antisense RNA fragment. The linker preferably comprises a RNA sequence encoded by an expression cassette comprising a functional gene, e.g. a selectable marker gene. In another embodiment, the linker comprises a RNA sequence encoded by regulatory sequences, which e.g. comprise intron processing signals.

Another embodiment provides a dsRNA construct having a promoter operably linked to said dsRNA and might further comprise said dsRNA molecule. The promoter can be a heterologous promoter, for example a tissue specific promoter, a developmentally regulated promoter, a constitutive promoter, divergent or an inducible promoter. Termination signal are also optionally included in the DNA molecules.

The single RNA molecule or the two distinct RNA molecules are preferably capable of forming a double-stranded region, in which the complementary parts of the RNA fragments recognize one another, pair with each other and form the double-stranded RNA molecule.

### 4. Chimeric or Fusion Proteins

A further embodiment provides provides chimeric or fusion proteins containing the disclosed nematode esophageal gland cell proteins or fragments thereof. As used herein, a "chimeric protein" or "fusion protein" includes a nematode esophageal gland cell protein or fragment thereof linked to a foreign or heterologous polypeptide. A "foreign polypeptide" is polypeptide that is not substantially homologous to a nematode esophageal gland cell protein or fragment thereof. The foreign polypeptide can be fused to the N-terminus or C-terminus of the nematode esophageal gland cell protein or fragment thereof. Fusion proteins can be useful for tracking or assaying siRNA activity, for example standardizing inhibition activity.

The fusion protein can include a moiety which has a high affinity for a ligand. For example, the fusion protein can be a GST fusion protein in which a nematode esophageal gland cell protein or fragment thereof is fused to the C-terminus of GST. Such fusion proteins can facilitate the purification of the polypeptide. Alternatively, the fusion protein can contain a heterologous signal sequence at its N-terminus. In certain host cells, expression, secretion or transport of a protein can be increased through use of a heterologous signal sequence. For example, in a plant cell, a polypeptide of the invention may be fused with a chloroplast transit peptide. The chloroplast transit peptide allows the polypeptide to be transported from the cytoplasm of the plant cell into the chloroplast. Expression vectors are commercially available that already encode a fusion moiety (e.g., a GST polypeptide). A nucleic acid encoding a nematode esophageal gland cell protein or fragment thereof can be cloned into such an expression vector so that the fusion moiety is linked in-frame to the polypeptide.

The following are only exemplary examples. It should be understood that the invention is not limited to these examples. Other important applications of disclosure would be readily recognized by those of ordinary skill in the art. Other uses which are potentially recognizable by those of prdinary skill in the art are also part of the disclosure.

The references mentioned herein are incorporated in their entirety to the fullest extent permitted by applicable law.

### EXAMPLES

### Example 1: Representative Cyst Nematode Parasitism Genes

The following nucleic acid sequences are exemplary cyst nematode parasitisim genes that can be targeted with an inhibitory nucleic acid. Production of inhibitory nucleic acids based on defined gene sequences is known in the art. The transgenic plants described herein can comprise one or more inhibitory nucleic acids specific for one or more of the following nucleic acids.

### 2A05 - VAP-2

aaaccataaattacaacttaaatcaagcaaaatcaaATGCACTTGATTAAC
TTAATCGCCCTCTTTTTCATGCTTTTCGGCCCATCCGTCCAGCAA
TACACAAAAACGCCAACGAATGAGGACAAAGAAGCGGCTGTCAA
TTGTCACAACAAATTCCGATCGCAATTGGCCCTGGGCAATGCCG
ACAATAAATTGGGCGGCAACAAAATGCCAAAGGCGGGCAACATG
CGTAAGTTTGAATGGGACGAAAACTTGGCCAAACTTGCGGATGA
ATGGGCCAACAAATGCACATTATCGCACTCGTGGAACGGCTGGG
CAGGCGAAAATTTGGCAATGAATGGCGGAACATTTTCGAACAAG
GATGGCTTCGAGTACGCTTGCGGTCGCTGGTGGACGAACTGA
ACCGTTACGGGTTCAACCCGGATCTGATTATGACCGGGGAAAAC
TTCAGTGGCATCGGCCATTGGACTCAGATGGCGTGGGCCGACA
CCGACCGAATTGGCTGTGCCATGGCACAAAACTGCCCAAATACC
AATTGGAAAACATATGTGGTCTGCTGGTATTACACGGGTGGTAAT
TACTTTGGTGCGCCTGTCTATTTGGCCCGGGAGCCGTGCAGCAA
ATGCAAAGCAAATGACAAATGTGACAAAGCCACTGGACTTTGCT
CTCAATGAaagatgaaatattatgaaaattgaattgggataattactaattattgtatctgtatg

tttagtatggtttagtgtttcggtataattttcacttattggtcttaattaacaatgaataaataaatagttt
ctaaaaaaaaaaaaaaaaaaaaaaaaaaa (SEQ ID NO:1)

### 2D01 - Pioneer

GAACCAACCAATACCATTAATTTCATAAATCCGAAGAAATC
CCTCCAAAAATGTCTTCTTCCCCGTCCGTCTCTGTGCTCGCCATC
GTCGCAATTGTCTGTTTGATGTGCCAATGTTGCTTTTCGGCACCG
CATCCGTGCTGTCCCGGCAGTCAAAAAGTGGTTTCACTTATGTC
CAATTACGTTGGCACTTTTGCCAATTCCATTTCCAAGTCATCGCT
TTGTTCGGATGCCCAAAATGTTGCGGAAGCGTTGAAAGGCCAAC
TGATCGGCTGCTCGAATGGCGGCGATCGAACTCTTTTGGCCGAC
ATCGAAGCATCCCTTGCCACTCATTCTGCTGATGAGTGTGCCCT
CAGCCTCGGCTTCGTCCGTGCCATGTTCGCCATTGCCGCCTCCG
CTTCTTCCCATGCCAGCAACAACAGCGAATGGCAGACATTGAGT
GGGCAGTTTGGTCAGAAAGTCACTGAGATTGACTCGAAATGTGC
AGAGTTTGGTATTAGCATTGGCAAAGTGCCCATAAACGGTCCCA
AGGATGTCCATGTTCAAAATGTGCCCAACTCGGAAAGTGTGATTT
NTATGCCTGGATTGGCCGGCTCACACACCCAATGAAAATGCATC
ACTGAAAAGATTTGGTACCTTTTGATTATTGTGCATTAATTAGCTG
TGGATTTTGTAAATTTAGGAAATTATATGTAAATAAATATTAATTC
GTTGAAAAAAAAAAAAAAAAAAA (SEQ ID NO:2)

### 3B05 - CBP

GACAAATAAATTTAATTTTTAATTTTTCAAATCTATTACAA
AATTCAAAAATTAAAACCATGAATTGGATGCATTATTGTTTAATCG
CTTGCTTTGCTTTTTATTATTTCAATACTGTTGAGTCATCCACTAT
TAATTCGGTAACCGTACAAGTGAACAAGATTGAAAACAATGAAAA
TGGAAGACAATTCAATTTGGAATTTACAAACCAAGTTTATGAGCG
AGTGTGCCACGTTGACTTTCGAATTGATCTGCCAGACACAGTAAA
ATTGAACAAATATTCAAAAATGGTGCCAATTCCTGACACCTGCGG

CCAGTACGCATTGCCCAAGAGTTTGGACTTGCTTCCCGGCGAGT
CATTTGATGCACAATTAACACTGCTTGGCCATGATGGGAAGCCG
AATGTGACTGTGCTGAACACGAACAATATTCCAACCAGCAAACAA
TGCAAAAAATGAAAAAGCCTACCCAATTAAGCTAATTGCCCTATC
AGTTCAGCAAGTTAACCAATTAGCATATCATTGCGATCAATTTAAT
TATTGTATTGTCATTAATTTAAAAAAAACATATATAAGTTTGAAGG
TTAAAAAAAAAAAAAAAAAAAAAAAAAAAA (SEQ ID NO:3)

### 3D11 - chitinase

GATCAAACAAAAAATTAAAATGAACAGGTTTTTTACATTATT
ATTTTTTGTATTATTTTTCAATGCCGCAATTAATTTTGTCAGTTCAC
ATCGCATTGTCGGTTATTATCAGGGCATACGTCCATTGACAAATG
ATCAAGCCAAGAAGTTGACCCATCTTATCCTGGCATTTTCAACCC
CTGACTCTCAAGGCAATTTGAGTCCATTGAGCTCTGTGCTTAAAC
AGGCGCTAAAAGCGGGTAAATCCGCTAATGGTGCGCTCAAAGTG
ATGATTGCCATTGGAGGAGGTGGCTTTGATCCGGCCATATTTAC
TTCGTTAGCATCAAACAGTGGCACACGTAAAAGCTTTATTAATAA
CATTGTTTCTTATCTGAAAACCAATGAGCTGGACGGTTGCGACAT
CGACTGGGAGTTCCCAACTTCTAGTGACAAGGCAATCTTTGTGA
CATTTCTGCGCGACTTAAAAAAGGCGATGGCACCCAGCGGCGCT
GTGCTTAGCATGGCATCGGCAGCAAGTGCCTTCTATTTGGACCC
TGGTTACGATTTGCCAGGCATTGAGAGTGCCGTCGATTTCATTAA
CGTGATGTGCTATGACTATTATGGAAGCTGGACCAAAACATCGA
CTGGGCCAAACTCGCCACTGTTTAAGGGTGGCAGTGCCGACCC
ATCGGACACATTGAACAGCAATTGGACAATGAATTATCACTTAAT
GAAAGTGTATAATCGAGCAAAGTTGAACATGGGTGTGCCATTCTA
CGGAAAATCTTGGACCAATGTTGGAGCACCACTAAACGGTGACG
GACTTTGGCGTCAGTTGGGCACTTATGGCACCGAATTAGCCTGG
CGTAACATGGGCAAAAGTTTTGACATGACCAAGACAACGTATCAT
AAAACGGCCAAAACTGCATACATTTATGATACAGCTACCAAAAAT
TTTTTAACCTTTGACAACCCCCAATCACTGAAGGACAAGGCAAAA

TATGTTGCGGAAAAGGGCATTGGTGGGATAATGATATGGTCAAT
TGATCAAGATGACGACAAATTGTCTTTATTGAATTCTGTTTCATAT
TGATTTTGATGTATATATTGTGTTTGAGCCATTAAAATGTGTTAGA
AAAAAAAAAAAAAAAAAAAAAAAAAAA (SEQ ID NO:4)

### 3H07 - UBQ

GACTCATTCCATCGCCGCTCACTCATTTTCTTCCCTTTCCT
TTGTCTTCCATTCATTCCGCTTTTCCCTCCCCATTTCTCATTTGTT
CTTCTCCGCTTCTTCTCTCCCCGATGAATCACTTTGGGCTGACCT
TTCTGTTCGTCGCCGTTTCTCTGCTGACATTGACGGCAAAACG
ATCACTTTGGAGGTGGAGAGCTCGGACACTGTGGACAATGTGAA
GACGAAGATCCAAGAGAAGGAGGGCGTTCCGCCGGATCAGCAA
CGGCTGATCTTCGCCGGCAAACAGCTCGAGGACGGACGAACGT
TGGCCGACTACAACATACAGAAGGAGTCCACGCTCCACTTGGTC
CTCCGTCTCCGGGGCGGAAATGGGAAGAGAAACACGAGTAAGA
ACAAGAAAAGCAACAAAAAGCTTGATCAGAATTGATCAGCAGCG
AACGCACCATCACATGATTGATTGGCACAGTGATTTTCCACCACC
AAATTCACAACACTTTCCCCTTTTTCCGGGTTATCTTTGATTTCCA
TTTTCGGTTATTGTTTTCTGTTTTTCTCCTCTATCTTTTGTAAAATT
CTGTTACTTTGATCTTTAGTGTTTATCATTTATCATCTGCATATATA
GAAGTTATCATAGTCCATATTTCTGTCCCTTTTTATTTATTTTTTTT
GACTATAAAACTTTTGGGCACAAAAAAAAAAAAAAAA (SEQ ID
NO:5)

### 4D06 - Pioneer

ATGTCTTCTCTGCTGCTCTCCATCTTCCCAATTGTGTTTTT
GGTCTGTTGCAATGCAATGCCAAATTTCCCGTGCTGCCCGGCA
GTCAGCAAGTGGTTGCTGTGATGTCCAAATACATTGACACTTTCT
CTTCTGCTGCTGACGAGTCTACAGTATGCTCAACCGCTAAAAGTA
CTGTGGATGGAATAAAAAATGAACTGTCCTATCGCGTGGGATGC

CCAAGCGGAGGAGAAGCACAAATTGTGAATGAAATTGATCAACA
GCTGAAGAACATTGCGAAAATGGAAATCAATTATGAGGACGAGT
GCCCGTACAATTTGGGCTTTGCCCGTGCCATGTTCGACTTGGCC
GCTGCTGCTGCTGGCCATGCGGGGAACGGCACAGAATGGCAAT
ACATGAAAGTACAATTTGAGCAGGAAAGCCAAGCAATCAAAGCA
ATTGGACAAGAAAAGAACTTTGAAGTTACGGATGTGCATTTTGGA
GTCCCAAGCAAAGGGGTTTCTGCACATCAAAATGTGCCGAGTCC
GAGCCATGTGATTGCCAACCCTGGCCAACACAGTTCGGTTGGCC
AAGGAAAGAAAGAAGAACCGTTGTCATCGGACTTCGATTTTTGA
GGACAAACAAATCAGGAGGAAATAGAATAGAAAACCATTTTGTTG
ACATGTCGAACATTTATTTAAAGTAATAAATATTTGGTAATAAGGA
AAAAAAAAAAAAAAAAAAAAAAAAAAA (SEQ ID NO:6)

### 4D09 - Pioneer

ATCATTTGTAATTCATTTTTAAATAAAAATTTCCTTGGACC
GACAAAAATGGCAAAATTCGTTGCCATTGCTCTTCTCGCTGAC
CATTGTTTCGATGGCACTTGCAAAAACTGGCAAAAGTCAAACGG
CAGACGAAGTTGAGGGATTTCGCAATATGAACATCGGCGACAAC
AATAAGGTTGACGCCGGAAAGGAGCCCGCGGCTGATAAAGCAA
CCAAAAAGGGAAAAGCTCAGAAAGCCGGAACGAAATCGGCGGC
GGCCACTAATGAGCCCGCGGCTGCTAAAGGAACTAAAAATGGAA
AAGCCCCGAAAACCAAAGCGAAGCAAGAGGTGGCCACTAATGA
GCCCGCGGCTGCTAATGAATGGAACGACCAATTGATGGGCATGA
GCGTTGAGAAATTTAACGAGGAGCTTGCTGTGTTGTTGCCAAAA
GCCAACACTTTTATGGAAAATGCTTTGAGCTTCATCAATGAACAA
GTGGAAAAAAATGGTATTGCAACTGGCGCTGCCGGGGACTCGT
GCTCGACCGGGATCCCGGCCAACTAGGAGACCAATGCCACAGG
ACGGCACCGGAGGAGGGAAGAAAGGATGACGAGCTCTTTGGAT
GATGAAGGTGGTGGATAATGGATGGTTAGTTGATTGGGGGGATT
TACATTTTTCATTCAACTTTCTTGGGAGTTGCAAATTTCAAAAAT

GTTTTTTTTTTTGAAATTTTTGGTAAACTGATCGGCATTAAAAAAA
AAAAAAAAAAAAAAAAAAAAAAAA (SEQ ID NO:7)

### 4E02 - Pioneer

GATTCCCTCAAAAGCAAAGCCATCCATCCGATCATTCGCT
TCATCTGCCAATCCGCAAAATGTCCCTTTTCCGTCCTCAATCGCT
GCTTCTTCTGGCCGCTCTTTGCCTGTCCTTTGCGCTGCTCTTTGT
CACTTCGTCGGAAGAGGGAGGGCGAGTGAAGCGCGGCGGATG
GCCTTGGGATTGGGCCGGCAAACAACTGTGCAAAACATCGGCAA
ATTGCAAGTGCAAGGATGGCAAAAATTGGGCCAAATGTGTAAAG
TCGGAAGGCTACGCGGCCAGCAATTGTTGCGACAAAAATTACGT
GTGGGCATGTTGCGGGAAGAAGCCCAAACATTGATGAGAGAAG
AGAAAGGGAGAGAATGAGAAGCGAGTTCACATAATTCATTCTGTT
TTGGATTTTTAGTCTTTTTTTTCTTTTTGGGTTTTTTTGGTAAATAT
TTTCTTTAGCAAAAAAAAAAAAAAAAAAAA (SEQ ID NO:8)

### 4FO1 - Annexin

AATCCCAATTTCGCATTCATCTCACTCACTCATAAAATGCT
CCAAAACGGCCTTACCATTCTGCTTCTGATCAGTGTTGTGATCGG
CCATTCCTTGGCCAACCTTGGCCCAACCATCAAACATAATCCTCA
TTTTAAAGCCGTACAAACTGCGCATCATTTGCATGATGCCATTGC
GAAGAAGCACGAGGCCGAAGTTACGCAAGTCATTTGCTCTATTA
GCAACGAACAGCGTCAAGCATTGGCTTTGGAGTTCAAAAAACAA
TTCGGCACTGATCTGATTGCCATGCTGAAAAAGGAGTTCAAAAG
CGACTTTGAAGAACTGATCATTTCTTTGATGCAAACGCCCGCCGT
TTACGATGCCAACCAAATGCGTGCCGCATTGTCCGGCTCCAATG
AGGCGGTGCTAATCGAAATTTTGGCGACGCGCACAAACCGACAA
ATTACGGCGCCGAAGCAGGCGTATGAGCAGTTGGACAGAAGGC
ATCAGCACAATCAGCTGGAGGAGGACATCAAAGCGAAGACGAA
GAGGACCCTTCCAAAATCTGTTGGTGTCTTTGCTCAGCTGCTCTC

GCGAAGAAAAAGTGCGCCCGCAAGCATTGTATTGGCACACGAC
GAGGCCATGAAACTGTTCAGAGAGGGCGAGGGCCGACGGGGC
GTTAACGCCGTGGTGTTCAACCAGGTGTTGGCCACTCGCAGCTT
CGCCCAGCTTCGGGAAACTTTCGAGTTTTACCGACAAGCCGCGC
ACCACGAGATTGAGGAGGGAATTAAGCAAGAATTCAGCGGTCAC
AACGAAGCGGGTTTCTTGGCACTAATCAAATATGTCCGCAACGC
TTCTGTGTTTTTTGCGGATTTGTTGTTCAATTCGATGAAAGGGCT
CGGCACACGCGACTCGGATTTGATTCGTCTGGTCATTTCTCGGT
CTGAGATTGACCTGGCTGACATCAAACACGCTTTTCACACGTTG
CACAAGAAGAGCCTGGAGGAGGCGATCAAAGGGGACACCAGCG
GAGCTTACCGAGACGCACTTTTGGCATTGGTCAAGGGCAACACG
GAGCAGTGATGGAGCAGCGGCAGAAGGGATTTTGCAAGAGATT
AGGATAGATGTAAAAGAGATAAACATGAATACCCAATAAACATCC
TGTAGAAATTATAAAATAAAATTTTTTATTTACAAAAAAAAAAAAA
AAAAAAAAAAAA (SEQ ID NO:9)

### 4G05 - Pioneer

TCAAAAATTCATTCAACCCTAGAATTTATTTTTTTAATAAA
TAAGTTTTTTGTACAAAATGAGCAACTTTATATTTGTCGCCTCTT
TAACTGCAGCGTTTTTAGCTCAGGCCTCGCTCTACCGGCTCCTT
ATGATGCTGAATCGGTGGTATCTTCTGAATTAAATGTTCCACTAC
TTTCAGCTGACGCAAATGTTGAAGCAGCAATTACCAATGAAAGTG
ATGCTGCTGCTGAGATACAAGCTCCATCAATTCCGGTACCAATTG
AACATCAAACTGCTGCTGACATTACTCATCCAACTGAAACTGGCA
ATGAGTCTTCTATTGCATCATCATCATCCACGCCGAAAGTGAGC
AGACGCCAAAAAAAGTGATGAACATGAAAAGCGCGCTGGAAGAN
CGCAGGCAGGCCAACGTGTACGAGGAGAGGCTTCCGTTAGACA
AGCAGCCCAAGACGATCGCTGAAGCGGCAGCAAAAGCAAAGCA
GACGCCCGCCAAACTTCCGGCTGACTACGACGTGAACCGCGTG
GCGGAACGCGCAGCAGCCCGTGTGTACGGGTGGCTTCCGGAAG
ACAAGCAGCCTAAGGCGATCTATGACGCGGCGGAGAAGGCAAA

GAACACGCCCAAACCGCCGGGCGACTACGACGTGGAGCGCGTG
GCGCAAAAGGCGGCCCGGCTCGTCTACGGTGTGCTGCCCATCG
GCATGCAGCCCAACTTCGCCGGCCCTAGCACTGACAAGAGCAAT
GTCGACGACTCGGAGAAACCTTCTGCTGCTGCGGCTGGTGATG
ATGATGATGAAGTCGAAAAAGAGAAGGAATAAGCAAAAAAC
AATGTGAATTTATTATAGGAAAAAAAGAAAAATGGAAGCTTAGAA
ATTTAATATTTTCATTTTTGAACATTTATAAAATTTCAAAATTGACC
GCAAAAAAAAAAAAAAAAAAAAA (SEQ ID NO:10)

### 4G06 - Hexaubiquitin

GATTCCATCGCCCCTCACTCATTTTCTTCCCTTTCCTTTGT
CTTCCATTCATTCCGCTTTTCCCTCCCCATTTCTCAATTGTTCTTC
TCCGCTTCTTCTCTCCCCGATGAATCACTTTGGGCTGACTTTTCT
GTTCGTCGCCGTTTCTCTGCTGGTAATTGCGCCCGGCTGCGATG
GCATGCAAATTTTCGTGAAGACATTGACGGCAAAACGATCACTT
TGGAGGTGGAGAGCTCGGACACTGTGGACAATGTGAAGGAGAA
GATCCAAGAGAAGGAGGGCATTCCGCCGGATCAGCAACGGCTG
ATCTTCGCCGGCAAACAGCTCGAGGACGGACGAACGTTGGCCG
ACTACAACATACAGAAGGAGTCCACGCTCCACTTGGTCCTCCGT
CTCCGGGGCGGAAATGGGAAGAGAAACACGGGTAAGAACAAGA
AAAGCAACAAAAAGCTTGATCAGAATTGATCAGCAGCGGACGCA
CCATCACATGATTGATTGGCACAGTGATTTTCCACCACCAAATTC
ACAACACTTTTCCCTTTTCCGGGTTATCTTTGATTTCCATTTTCGG
TTATTGTTTTCTGTTTTTCTCCTCTATCTTTTGTAAAATTCTGTTAC
TTTGATCTTTAGTGTTTATCATTTATCATCTGCATATATAGAAGTTA
TCATAGTCCATATTTCTGTCCCTTTTTATTTATTTTTTTGACTATA
AAACTTTTGGGCACAAAAAAAAAAAAAAAAAA (SEQ ID NO:11)

### 4G12 - CLAVATA3

ATCCAAATCAACGCGGCACAGTAATAAAATATTAAAAGATC
CGAAAAAATGCCAAACATTTTCAAAATCCTTCTGATTGTGCTTTTG
GCCGTCGTCTCATTCCGCCTCTCGGCTTCTACTGGTGACAAAAA
AACTGCTAATGATGGGAGTGGAAACAACTCATCAGCTGGGATTG
GTACGAAGATCAAAAGAATTGTCACCGCTGGACTGCTCTTCACTT
CCCTGGCGACGGGTGGGGCGGAAGCGATTGGGCGAAGCAATG
CTCAGGGAGGAAATGCCGCCGGATTGGTGCCATCGCATGTGAC
CAATCGCTCAATGGCTCCACCACCTCCTCCTGTGCAATTTGAAAT
GGGGGCAAATCGATTAGAAAAAATGAGGGCACACCTACGCGAAC
TTGCTGAGAAAATGCCGGTCAATGAATCGAAGCGTCTGTCACCG
AGTGGACCCGACCCTCATCATCATTAGGGCCATGGATGGATCTA
ACGGAGGAAGAAAGAATGGATGCTTAGTTTTCAGATTTTATTCTA
TCCTCTTTTATTTATTAGTTTTTCAAAACAAATTTCTTCCACAACTT
TTTAAACTATTTGTTGTTTTGAATCTTTTTAGATAAATCTAAACCCC
AAAAAAAAAAAAAAAAAA (SEQ ID NO:12)

### 5D06 - Pioneer

GAAAATTTGATTTTATACAATAAAAATATTATAATATTTTTGA
ATAAAAATGAAAAATTCTTTCCTCTTCCTGCTTCAAATTTTTATTCT
AACCAACATTTTAACTGAAATACTTTGTGGAGATAAGTCAAGGCC
GTCGACGGAAATCAATGCCAATTTGGGAACAAGGAAAAAGCCTG
AAACAATTACGGCAACAAAAAATGCCAATTTGGGAACAGGAAAAA
TGCATGAAACCGATGGGACCAGCAAAATGCCAAAACATGGCAAA
CCTGTGTCAAATCGAATGGCAGCCAAAAGCACGACGATTAAAAA
TAATAATGAAGCAGCAGGGCCAAGCCAACAATCAAAACAACCTG
CGGCAAACATTACGCCACAGCAAAAGGGGCCAATGCAAAATTCA
AAAAAGCAGCCACCAAACGAATTGTTTGGCAAGAAGCAAAGGTC
AACCCCTGAAGAGATCAAAGCTGGCAAACAACCAGCAATGGAGC
TAATGCCATGCTATCGAGGAATCGGCAAAAAGACGATGCCAACA

ATTGCACAGCGAAATGCCACCATGCTAAAAAGGGGGACAGTTT
GACACGGAGTGCCGACTTTGAGGACCCAATTTTGGCCAATGTAA
AACAAAGCCAAATGTTGATCCGACAAAAATCCAAGGCACCATG
GACTTGGAAATGGACGAATTCTTAAAACTGCACAAGCAAATTCAA
GGACCTCGGCAAAAATTGGTACGAATGGAAAGGAAAAAATGAT
CAAAGAAGCGACCGAAAAGGCAAAAGTGGACCGAAGAAATCGTA
TGTTGGAACGAGAAAAAATGTGGAGCATGAAGAAGGCTGGTGTT
GCTGCACATCAACCAGCATCACCGGCAATTCGTGGACGAACACA
GCAGCAACAACAGCAGAAGCTGCAACCTGACAAAAAGCCGGAAA
AAATGCTGAAACAGCAAAACAAAACACAAAATTTTTCGGCACCTT
CCACCAGCAAACAAATTGTCAATCGAAGGTTGTTGATTGGCCCA
AACAAACCGAGAACTGGCAACAAAATTGGCACTACCAAGCACGG
CATACATTCGGCGGAAATAATGACATCATCGTCGGAAAGCAATG
TGCCAAAAAGTGAAAATTTGGGCAGCAGTGAAATGGAGACGGCG
GAGGAAGCGGCACAAACCTATTTGAACAATTTACGGGTTGATTTA
AATAAAATTCCTTCGATGAAAATTCCATTCCTTGAACCAATAATTG
AAAAATATGTTGATGAATACAAAAAAAGTCGTCAATTATTCGCTGA
ATGCATGCAAAAAATTTTGCGAAAATCGAAAAATCCTAATCAAAC
GGTGAAGATCATCGAATTTAAAAAGACAATTGAAAAAGAAATGAA
TGATTTGTTGAAACATACGGATTTGCCACCAGATCAATTAATTGA
TCTGTTGAACACTCGTGAATGGCTATTGTCGATGAAAAAAGACAT
TGAGTACATGGAACGCATTCTGACCGGACGCGAGAATAATGACA
ACATTTGA (SEQ ID NO:13)

### 5D08 - Pioneer

GGAATTATTTAATAAACAAAAAAACAATTAATTACCGCCAA
TTGCCCATTAGAAGAAATGTTCAGCTCTTCCAATTTGTCTGCTCT
CTTTTTGGCCTCCTCCGTTTTGGCCGTGCTTATAATTGGCATTAA
AATGGACGGACCGACGGAGGCAAAAGGCGCCGCCCCTCCAAAC
GCCGCGGGGCCAATGGGACTTTTGCTTTTATTGAATGGCAAACA
ATCGGCGGCTAATGAAAAGGGAAAAGCGCCCTCTGGCGAAAGT

AAGCCAAATCCGGGGCAGAAGCCGAGCGGAGAACGGCGAAAGA
GGGACGTTTTGGGGCACGCCGGCGGATACGTCGGAGGATGGG
ACCATCCCATTGACTCGACACTTGATTGGGCAAAGAGTCAGTGG
AATGATGCCAATTGGCTCGCCGATGTTGTCAACAGAAACGGATG
GGAAAACACCGGCACTCCAACCGGCGGACGATGAATCAGTGAA
TTGTGCCGACCAAGGAATGAAAGACGGCATTTTTGTTTGGGAATT
TAATTGACTTTTCGGAATCAAACACACTTTCCTTTTTAGTTGCCTA
TTTATTCTAAATTAGCGTTTTTTCTGTTCATTTTCTACGCAAAACAA
ATTTTTTACATATTTTTTGGTTGGGGATTATCCCAATGGTCCCATT
TTTCGCATAAATATGAATTGATAAAAAAAAAAAAAAAAA (SEQ ID
NO:14)

### 6E07 - Pioneer

ATATCACATTTTCATTTAAATTATCCTCCCAAAATGCGTACC
ATTCTCTTCATGGCCATGGTTTGCTTGGTGATGGCTGTCCTAATG
GAAATGGCAAATTCAAAAGTAGTCAAAAAAGACAATAAAAAAGCA
GCAGTGGCGGCATCTCCAGCAAAAGGAAAAGCATCGCCAAAAG
GAGGCAAAAGCCCAGCAAAAGGAAAAGCGGCAAAAGTTAGTAAA
AAAGATAACAAATTGAAAGCAAAGAAAGAAACCAAAGGCATTAAA
GTTAAAAATGCAAAGCCAACAAAAAAAGGTAAATCGGCAAAAGG
CGCATCAAAAACAGCCAAGAAAGTCCAAGCCGCCAAAAAAGCAC
CAGGAAAGGACAAAAGTCGCTAGTTAAGCCAATCGTCCTTAAA
GCACCGGTACCACCCCATAAAATGCACCCGATGATTGAATCCGA
AGCTGTGCCACCGCCCGCCCATGCGCGTTCGCTTGCGACCGTT
CCTTACAGTACACCGGGGGCAGCCGACCGTAACTCATTGCCATC
GTACACTTCGACTGCCACCAATTTGGACATGGCCGATGATAATG
ATGATTATCAGAATTATTACTACGGAACGGAAGACAGCAGCAGG
GAATTTGATGCATCGGCGGAGGAGGAGGATGAACTATATGAGAG
AGGAGAGTCGGGGCAATGAGTGGGCAGACGGACGACTACGGTA
TTAGGCAGTGGACAGAGATGAGTACGGTCATCAACCGGTGGAC
GTGCCGCCATAGTCGGTGATGCTCGACTGAAGAGCATCAATGAA

ATCAATCAGAGATCACCGGAAAATCATTTTTTCGTAATATAGACC
AAAATTTATAGTTGCTAATTTTTGTATATTTTTATTTGTAAAAATAG
TTTTTTATTAATAAATATAACTCTTACAATTATCTCATTTATAATTTT
GCTGAAGTAAAACAACGAAAAAAAAAAAAAAAAAAAAAAAAAAAA
A (SEQ ID NO:15)

### 6F06 - cellulase

CCAACTAATTTTCTCCCAATCTAAAAAGTCTCTCTAATTAA
TACTAATTAGTACAAACTGCTGTTGATTTGATCACAGAATGTTGG
TTCAACTCGTCCTCCTTGCCATCATTGGCATTTCCTTTGTCGGTG
CTGCCGCGCCGCCGTACGGCCAATTGTCCGTCTCCGGCACCAA
ATTGGTTGGCTCAAACGGCAAACCGGTGCAGCTGATCGGCAATT
CGTTGTTCTGGCACCAGTGGTACCCACAATTTTGGAATACTGAAA
CAGTGAAGGCACTCAAATGCAATTGGAATTCCAATGTCGTGCGC
ACCGCAATGGGCGTGGAACAGGGCGGCTATCTGAGTGACGCGA
ACACCGCCTACCGACTGACGGCAGCTGTGATTGAGGCGGCCAT
TGCACAGGGCATTTACGTGATCGTCGATTGGCACGCGCATGAGG
CGAACGCGGACAAAGCGATTGAATTCTTCACCAAAGTTGCGAAA
GCGTACGGCTCCAACCCTCACTTGCTTTACGAAACGTTTAACGA
GCCGTTGGACGTGTCTTGGAACGATGTGCTTGTCCCGTACCATA
AAAAGGTTATTTCTGCAATTCGTGCCATCGACAAAAGAATGTGA
TCATTCTCGGCACTCCCAAATGGTCTCAAGATGTTGACGTGGCG
GCCCAAAATCCGATCAAAGGATTCGGTAATTTGATGTACACTCTC
CACTTCTATGCGTCCAGTCACTTTGTTGATGGACTTGGAAATAAG
CTTAAGACCGCCGTAAACAAGGGTCTTCCGGTGTTCGTCACTGA
GTACGGTACATGCGAAGCGTCTGGCAATGGTAATCTGAATACCA
ATTCAATGTCAAGCTGGTGGAGCCTGCTGGACCAACTGCAAATT
TCGTACGTCAATTGGTCAATCACTGACAAAAGCGAAGCTTGTGC
AGCGCTCACTGGCGGAACATCGGCTGCCAATGTTGGCACTTCCT
CCCGCTGGACGCAGTCTGGCAATATGGTAGCTTCGCAACACAAG
AAAAAATCCACCGGTGTGAACTGCAGCGGTGGTGGTGGCGCTG

CTGCTAAGCCAGCTGCTAAGCCCGCCGCTAAGCCAGCTGCTAAA
TCGAAGGGAAAGTCTTCCAAAGCCAAGAAGTCCGGATGATCAGC
AAATCACAATAAACATAGAAAGTGAATTGAAGACAATATGGTGAT
TCAAAAAACAAATAAGTGCATAATGATAATTTTAAGTATAATTGTA
ATATTCAAAAATATTCTTAGGAGTAAATCGGGCACTGATAAAGCA
TGAACTATTATTAAAAAAAAAAAAAAAAAAAAAAAAAAAAAAAA
AAAAAAA (SEQ ID NO:16)

### 7E05 - Pioneer

ATGTACAAATTTTTATTTTTCCCTCATTTTTGTATCTTAATTA
AGGCAATTTCGGGGCTCCATTGTTGGAATTCGAACATGATTTTGC
TGAGTGAAATGCCGGAAAAGGGCAGTGTCACTGTACACCAATGT
CCGTCCGGGCATCAATGCGTGACGGCAAATTGTTGGCTTGGAGT
CGGCAATTACATTGTCCAAAAATGCGTGCCGGACCAGCCAGGG
GCCAGAAACTATTGCAAAGACTTCAACAACATTTGCCAAATGGGC
TTTTTCGGCATGCCAATTCATTGCTCATCTTGTAAGGGTGATCTG
TGCAACGAAGCGCGTAATTAA (SEQ ID NO:17)

### 8H07 - SKP1

AGAATTTAAAAATCATAAAAATTCAAACTCATCAAATTCACT
CAACATCATCACCAACCGTGAACATTATGCTGAGGATTGCTCTAC
TCATCTCCATTTTGGCACTGTTTGGTGATTGCATGGACATGGGAA
AAAGAAAATTAGGAGGAATCAGTATTAATGAGCCAAGTGAATATG
GAACCAAAGAAAAAGAAGCCATCGCAACAAAAGAAAATGCACAA
ACATCAAAGGACCCGCCGACATCGGCGGGTGGTCAAAATGAAG
CAATCCCTTCACCAAAAAAGCCAAGCCCCAAGGGGAAGTTGAAA
AGCGATTTTGGCCTAAACTTAGCCAAGGCTTTTCCACGGCCGGT
TCCGAAAGGCAGAAGGGGCAAAGAAAAAGTCGGCGAAAAAATAA
GCGTTATGAACACAGCTGAACGCATTGAAAAAATGGACATTGCC
CAAGACAAAGCTAAAATGGAAATAGACAATATAGACACCAATGCC

AAACCCAAACAAAATGAGGATAAAGCTCCAACCATATTCCCCAAA
TGTGCGGACAACGTGGAAGTGGAAGTAGAGCTCGATCGTAATAT
TTTTCGTTTTTCTACTACGCTTGACACAATGATGGAAGATCTTGG
AATGTACACTGCTGAAGGCACAAACCAGAAATTGCCGGTTTCAA
ATGTCAGTAGTACGGTGATGCGAGAAGTGATTGAATGGTGCGAG
CATCACAAAAACGATGCCTCAATAGAGCCAATTTATGAAGAAATT
GCTTTGGATGTGCCAACTGGTAAAGATGCGGAGGCATCCGCACC
AAATGCTCAAGCTGGAGAAGTTGCGGAGGCAGCCGAATCAAATG
CCAAACCAAATAATGAAAAGCGTCTCGTCTTTCCAAGCTGGGAT
GAGAATTTTTTGGATAAGGAATGGCCTGAGCTTGTTGATATAATT
TTAGCAGCCAACTATTTGAACATCAAACTTTTGCTTACCTTCGCG
ACCACAATGGTTGATAACAAGTGGATCAATGGCAAAACGCCGCA
GGAAATTCGCAAGGGATTCGGCGTCGAAGAGCCGTACCCGCCG
GGACATCCGGAATGGGCACGAGTTGAGAAGGAGAACGAGTGGG
AAGAATCGGACGAGGAACGTGAGGCACGCCATGCAAAGGAACG
AGAGGAGGAAGAGGAGCGTGAGAGAAAGGAAGAACAGAAGCGT
AAGGAAGAGGAAGCGGAACGCCTCCATCAGGAACAACTGCAGC
AACAACAGAATCAGGAACAGCAACCTCAGTAGGGACAGCAGCAT
GGTGAAGAACTGGAACACGATGAAGTTATGCATGATGTGGAGGA
AGAGCAAGATGATGAATGGCGAGGAAGAAGATGATGAGTGATGA
GGATGAGGAGGATGTAAAATGATGTGGTGATTAGTGATTTTGAT
GAACCGATGATTCACTTTTCTTGGATCCTGTTGCATAAAACTTGT
TGCAAAAAAAAAAAAAAAAAAAAAAAAAAA (SEQ ID NO:18)

### 10A06 - Zinc finger

GAGAATTTAAAAATCATAAAAATTCAAACTCATCAAATTCAC
TCAACATCATCACCAACCGTGAACATTATGCTGAGGATTGCTCTA
CTCATCTCCATTTTGGCACTGTTTGGTGATTGCATGGACAAGGGA
AAAAGAAAATTAGGAGGAATCAGTATTAATGAGCCAAGTGAATAT
GGAACCAAAGAAAAAGAAGCCATCGCAACAAAAGAAAATGCTCT
AACATCAAAGGACCCGCCGACATCGGCGGGTGGTCAAAATGAA

GCAATCCCTTCACCAAAAAGCCAAGCCCCAAGGGGAAGTTGAA
AAGCGATTTTGGCCTAAACTTAGCCAAGGCTTTTCCACGGCCGG
TTCCGAAAGGAAAAAAAGTTGAAAATACACTATCATCAAAGGACT
CGGCATCTGAAGCTCACAAAGAATTGGCCAAAGAGAAAAATGAA
GAAAATGCACAGACCTCAAAGGTCCCGACATCGATGGAAAGGCA
AAATGAACCAATCCCTTCACCAAAAAGCCAAGCCCCAAGGGGA
AGTCCAAAAGCGATTTCGCCCTAAACTTGGCCAAAGCTTTTCCAC
GACCAGTTGCCGAAGCAAAAATGGGAGAAGAAGCTCAGTCTTCA
AAAGATCCGACAATGAATGCCAAATTTGTGCAATTTGTTTGGATG
CATCGCTTATCACTGACCTTGAAATTGAGCAAATGCCATCATCGC
TTTCACCGCGAATGCGTTGATGGGTGGTTTAAAAACAATGACACA
TGCCCTTATTGTCGTGCTGTAGTTGCAAGCAGATATTTACCAAGA
CCTACGCGTACAGATCGAATTTTTGACGCCAGAATCGAAAACAAA
AGACGCTTCATGGGAGAAGGAGAAGGAAAATACACAATTATTCG
CCCTAACGGAAGTACGCTTATGGTTCACGATAATCATTTTGGAAA
CAATTTTACGGTCGAAAAAACTGAAGAGGGCTCCATTCAACTCAG
TAAAAACGATCGCAAATAGAAAAATTGTTTCAAGATTAATTAAAAA
ATGTCAGGGAGGGTTGCGGAAGCCGGAAAGAATTATGGATAAAA
AACCTAAAAAAAATCGGAGGCTAAACAAATTTCGAAACTCATCTA
AACCGCAATTCGGATCCATATCCCTGATATGTAATATACATTTAT
GCATCAGGGATGCATTCGGAAATATGTATTGGTACTGAGTCGGA
ATTTTTAACGTTTCGGCATANCAATAAATATTTAATCAAAAAAAAA
AAA (SEQ ID NO:19)

### 10A07 - Pioneer

ATATCTATCATATTTTCATTTAAATTATCCTCCCAAAATGCG
CACCATTCTCTTCATAGCCATGGTTTGCTTGGTGATGGCTGTCCT
AATGGAAATGGCAAATTCAAAGGCTGTCAAAAAAGACAACAAAAA
AGGAGCAGTGGCGGCATCACCAGCAAAAGGAAAAGCATCGCCA
AAAGGAGGCAAAAGCCCAGCAAAAGGAAAAGCGGCAAAAAAATT
GAAACCTAAAAAGGATGCTAAAGGCATTAAAGCTAAAAAAGCAAA

GCCAGCAAAGAAAGGCAAAGCGGCAAAAGCAGTAAAGGGAGCG
CCTAAGACAGTCAAAAAACTCGCAATTGCCAAAACAGCACAAGC
AAAAGACAAAAAGTCACCAGCCAAGCCAATGGTTCTAAAAGCAG
TGCCGCCCCACCAAATGCATTTAATGAATGAGAAAGTTCAAACAG
CTGTGTCTCCACCCGCCCATGCTCGTTCACTTGCGACCGTTCCT
TACAGTACACCGGTGGCAGCCGACCGTAACTCACTGCCATCGTA
CACTTCGGATGGCACCAATTTGGACATGGCCCATGAGAATGATG
ATTATCAGAATTATTACTACGGATCGGGAGACAGCAGCAAAGAAT
TTGATGCATCGGCTGAAGAGGAAGATGGACTGTATGAGGAGGG
GGACGGGGCAATGAATTGGGCAAACGGACGACTACCGGATTGG
GCATTGAACAGGAATACGGGCATCAATCGGGGGGAGTGGCCAC
CAAAGTCGGGGATGCTCCAACTGAAAAACACCAAGAATTTTGGA
AAAAACTTATTTTCCAAAATTTTGAAACATTTGGATTTTAATATTCC
CGTTAAAAAACTAAATTTTTTTTTACCCAGCCTCTAATTTTGTAAA
AAAAAAAAAAAAAA (SEQ ID NO:20)

### 10C02 - Pioneer

GATTCATTCCCTCAAAAGCAAAGCCATCCATCCGATCATTC
GCTTCATCTGTCAATCCGCAAAATGTCCCTTCTCCGTCCTCAATC
GCTGCTTCTTGTGCCCGCTCTTTCCCTGTCCTTTGCGCTGCTCTT
TGTCACTTCGTCGGAAGAGGGAGGGCGAGTGAAGCCCGGCGGA
TGCCCTTGGGATTGGGCCGGCAAACAACTGTGCAAAACATCGGC
AAATTGCAAGTGCAAGGATGGCAAAAGTTGGGCCAAATGTGTAA
AGTCGGAAGGCTACGCGGCCAGCAATTGTTGCGACAAAAATTAC
GTGTGGGCATGTTGCGGGAAGAAGCCCAAACATTGATGAGAGAA
GAGAACGGGAGAGAATGAGAAGCGAGTTCCCATAATTCATTCTG
TTTTGGATTTTTAGTCTTTTTTTCTTTTTGGGTTTTTTTTGGTAAAT
ATTTTCTTTAGTTATTAAAAATTGTTTGGTCTAATTATGCCATTGG
AAAAAATGTATTTGTTTTTTACAAAAAAAAAAAAAAAAAAAAA(SEQ
ID NO:21)

### 11A06 - Pioneer

GGCTCCAAAAAAATCCATTCCACATTCCAGCAACCAATA
CCATTAATTTCATAAATCCGAAGAAATCCCTACAAAAATGTCTTCT
TCCCCGTCCGTCTCTGTGCTCGCCATCGTCGCAATTGTCTGTTT
GATGTGCCAATGTTGCTTTTCGGCACCGCATCCGTGCTGTCCCG
GCAGTCAAAAAGTGGTTTCACTTATGTCCAATTACGTTGGCACTT
TTGCCAATTCCATTTCCAAGTCATCGCTTTGTTCGGATGCCCAAA
ATGTTGCGGACGCGTTGAAAGGCCAACTGATCGGCTGCTCGAAT
GGCGGCGATCGAACTCTTTTGGCCGACATCGAAGCATCTCTTGC
CAGTCATTCTGCTGATGAGTGTGCCCACAGCCTCGGCTTCGTCC
GTGCCATGTTCGCCATTGCCGCCTCTGCTTCTTCTCATGCCAGC
AACAACAGCGAATGGCAGGCATTGAGTACACAGTTTGTTCAGAA
AGTCACTGAAATTGACTCGAAATGTGCAGAGTTTGGCATTAGCAT
TGGAAAAGTGCCCATCGATGGCCCCAAGGGAGACCACTCCCAA
CGAAATGTGCCTAGTACGGACAGTGTGATTTCCATGCCCGGATT
GACCGGCTCACACAAACATTGAACTGAATGATGAGTGACGGAAT
GGATAAACTAATT (SEQ ID NO:22)

### 12H04 - Pioneer

ATGGCCCTCTCTGCCCTTCTGCTTCTTCTCCCCCTGCTTC
TCAATGTTCAAAATATCCCAGATGAGTCAGTCCAATCGGATATGA
AGGCTGTTTATTCGGCTATTTCATCGCCGGAACAATGGAAGAACT
CACAAAATTCATTGGCTTCACTCGAATCACAACTGACAGAGCCCC
AAAGAGCACTGGCAAAAATGCATTGGGAATTGGAGACCATCCAA
AAGGAAAAGCCGGAGGCACCGCCACAATTCGACTTGGGACTTTT
CTCGGAAGCTTTGGAAGTGATGGTCGAAATGGACGAAGAAGCGA
AAGAAGTGAAGCTGAGGAAGGACAAACTGATCCGAATGGGCAG
GAGGAGAGGAAGCAAAAAAAAATTGAAGGAAAAGATGACGGAG
GAGGACACAGTGCCGGAAGTGAGGGTGAACGAGAATGGTAAGG
TTGAAGTGAAGGACGGCGCCGGAGGGGAAGGACGGTTGGAAGT

CAGACGAACAAAGGACGAGAAAGGCAGAGAGCAGGTGGTGATC
ACCCTTATGAAGAATGGCGGAACGGAGGGACCAGCGGAAGGAA
CCGCGGAGAAGCCACAAGAGAAGGCCAAGACGGAGGAGGAGG
TACAGAAAAAGAATGATGACAAAAGTACACGACAAGAACAGGAG
GAAGCGAAGAAGACGGAGCAGGAAAACGCCGGAGGGGTGCCA
AAAACTGACTCGGCCAACAGCCACATTCCGGTAATGCCAATGCA
CACCATTTTGTCATCCCCGTCTCCACCGGTGGAGGAGAAGGGCA
AAGCGAGTGCAGAGGAGGCACAAAAAAAGGAGGAGGACAAAAA
CACACGACGTGGAGGGGAGGAGGCAAACAACACGGAGCAGAAC
AAAGTCGGAGGAGTGCCAAAAATGTCAGTTGACAGTCCTAAGTC
GGTCGTGCCAATGCCAATGCACACCATTTTGTCCTCACCAGCCC
CACCGGTCGAGGAGCAGGACAAGGCAAGGGATGCGCTCACAGA
AGGAGCAAATGGAAGGAAAAAGGCGCAAAACAACGAAGAAATGT
TGCTGGTTGCGACGGAGAACAACGGAAGCATGAGAAATACGAAT
AATGGAGGAATATTTGATTTTGTCCGAAAATTTATCTCCAATGTGT
TTGGACGTAAGAAAAGGGACACGGNAAGCGGTGCACAGAGAAA
ATATGACGGGGGAACGAAGGAAAATTCGCAGCATTCAAAATTTG
ATTATGAGCACTGTAGGGAAGTCCGACTCAGAGCCGAAGAGCGA
AGACAAACAGCAGCAGGAAGAGAAAAAGAAGGCGGAGATCAAG
CAGTACCTGGAGAAGGGAGTGGTCAACACGGGTGGAATCAAAA
AGGCCGAGAAACTGGCATATGCTTGGTACTCGGAGCTTCTGTAC
TGGACAACCAAGTGGATCGAGGCATTAGAAAATCGGGTGGCGG
GAGTCAAACCCGAATTGGCACAGCAATTCTTATTCTCCAAAACGG
GATCAGCTGCTTACCAGGAGCTGAAGGAAGAGGTGGACAAATG
CGAGGCGAAGTTGGCCAAACTAAAGGAATGGATCGGCGACTCTT
TCAAGTGAAAATGGAAGAAACGGCTCTTAGAGGTGTATTTTTCTG
A (SEQ ID NO:23)

### 13A06 - Pioneer

TTCCCTTATGGCCATGGTTTGCTTGGTGATGGCTGTCCTA
ATGGAAATGGCAAATTCAAAAGCAGTCAAAAAAGACAATAAAAAA

GGAGCAGTGGCGGCATCACCAGCAAAAGGAAAAGCATCGCCAA
AAGGAGGCAAAAGCCCAGCAAAAGGAAAAGCGGCAAAAGTTAGT
AAAAAAGATAACAAATTGAAAGCAAAGAAAGAAACCAAAGGCATT
AAAGTTAAAAATGCAAAGCCAACAAAAAAAGGTAAATCGGCAAAA
GGCGCACCAAAAACAGCCAAGAAAGTCCAAGCCGCCAAAAAAG
CACCAGGAAAGGACAAAAAGTCGCTAGTTAAGCCAATCGTCCTT
AAAGCACCGGTACCACCCCATAAAATGCACCCGATGATTGAATC
CGAAGCTGTGCCACCGCCCGCCCATGCGCGTTCGCTTGCGACC
GTTCCTTACAGTACACCGGGGGCAGCCGACCGTAACTCATTGCC
ATCGTACACTTCGGCTGGCACCAATTTGGACATGGCCGATGATA
ATGATGATTATCAGAATTATTACTACGGAACGGAAGACGGCAGC
AGGGAATTTGATGCATCGGCGGAGGAGGAGGATGAACTATATGA
NGAGGGAGGTCGGGGCAATGAGTGGGCAGACGGACGACTACG
GTATTGGCAGTGGACAGGATGAGTACGGTCATCAACCGGTGGA
CGTGCCGCCATAGTCGGTGATGCTCGACTGAAGAGCATCAATGA
AATCAATCAGAGATCACCGGAAAATCATTTTTTCGTAATATAGAC
CAAAATTTATAGTTGCTAATTTTTGTATATTTTTATTTGTAAAAATA
GTTTTTTATTAATAAATATAACTCTTACAATTATCTCATTTATAATTT
TGCTGAAGTAAAACAACGAAAAAAAAAA (SEQ ID NO:24)

### 13C08 - cellulase

ATTTTATTTTATCTACAACTCAATAAAAAATGTCCGCTTGGA
TTTGTCCTGGGTTTTTGCTGTTTTTTGTGCTCATTCTGATCACTGA
TCACTGTCCGATGACCCAAGCTGTCACTCAACCGCCCTATGGTC
AATTGGCTGTCAGTGGAAAAAATCTAGTACAAAAAAGTACGAAAA
AAGCCGTGGCATTGCACGGCCTGTCAATGTACTGGAGCCAATGG
GTGCCGAAGTTTTGGGTGAAACAGACCGTCAACAAATTAAAATGT
TCATGCAATGCCAACGTTGTACGCGCAGCAATGGCATCCAGCTT
TGGCGGTTACATTTCAAACCCAACTGCTGAATATAAAAAAATGAC
TGCAATCATTGATGGGGCAATTGACCAAGGCCTTTACGTGGTTG
TCGATTGGCACACGGGCGACGATTTGGCCACCACCGAAATAAAA

TATGCGACTGAATTCTTTACAAAAATTGCTAAAGCCTATAATAAAT
ACCCGCACATTTTGTACGAAATTTGGAATGAGCCCAACAAATTTG
TGGCATGGGAAGCGGTGGTCAAACCATACGCAAAGACGATGATT
GACGTGATTCGTAAATATGACAAAAACAATGTGATCATTGTTGGC
ACGCCAAACTGGGACCAAGATGTTGACATTGTTGCAAAAAGTCC
GTTGAAAGACGCAAATATTATTTACACTATGCACTTTTACGCCGG
CCAACACAAAGATGACATCCGAAACAAAGTCAAAACGGCTTATAA
ATTGGGCCTTCCAATGTTTGTGACTGAGTACGGTTGCTACTCGG
CTGACGGCAATGAAAAAACAAATTTGGAAGAGTTGAAAAAGTGG
ATGGAACTTTTGGACGGCTATGGCATTTCCTACGCCGCGTGGCA
CGTGGCGGACATTGGCGAACAGTCGTCCATACTGACAAAGACTT
CCGAAGTGAACAACATCTGTGACCCGGCCCATTTGACCAACTAC
GGAAAAGCCATCATTAACAAATTGAAATCGCAAAACAATGGTGTG
AAATGCAGTGGTTAGCCAACCAATTTCAATTAATCGGCGGATGTT
ATCGAAAATGTATTCAAAAGTTAAATATTCAAAAAAGTTTACTAAC
CCAATTATTGTTATTAATTTCGGTGTCAATAATTGTAATTGTAATTT
TGAAAACTCTTAATAATTATGTACTATTATTTTAATTCAATTCACTA
GCAAATTTTTTAAATCTAAAAACTTTGAAATGTGAATTTAAATCATT
TAAAAACGAAAAAAAAAAAAAAAA (SEQ ID NO:25)

### 16B09 - Pioneer

ATCCTCTAAGAATTCAAATCCTCTCAGAAAATGGCTTCATC
TTTCTGCTCTTCACTCATTTCCATCGTCGCAATTGTCTGTTTGCTG
TGCAAATGCTGCTTTTCAGCACCCCATCCATGCTGTCCTGGCAG
CCAACATGTTGTTTCGATGATGAAAGATCACACCGGCACATTCTC
CGCTTCGATGCCAAAGTCTTCGCTTTGTCTGAGTGCCGAAAGAG
TCGCCGCTGCGGTGGAAAACCAACTGAAAACAATTTGGTGCCCT
GGCAATGGTGGCCAAACACTCATCAACGAGATCAACGCAGCTCA
ATCATCATCTGATGAGTGTGCTCGCTCTCTCGGCTTCGTCCGTG
CCATGTTCGAAATTGCCGCTTCCGCCGCTTCCCATGTCGGTGCC
AACGCCGAATTGGCCAATTTGGCTGTCCAGTTCCGAGAACAAGT

TGGCACAATTGACACCAACTGTGCTGCGCTGGGCATTCATGTTG
GGCAAATCAGCTTGGGCACTCCCAAAGGAGACCATCCGCAAGT
GCATGACTCTGAGAGTGTGCTTAGTAACCCTGGCACCAGCGGGT
CTCACAAGCGCATTTAAGTGCATTGCGACGATTCCGATGATGTC
ATCATTTGTTGATTGATATGCTATAGAAATTATTTATTAGATAAAA
AATGAATCGTTGCT (SEQ ID NO:26)

### 17C07 - pectate lyase

GATTAAAAAGAATTTCTTCAACTAAAATCACAAACACATA
CAATAAGAAAATGATTCTTTTCATTCTATTGGTCATAACTTTTGTC
CAAATTGGTCAACTGAGTGCTGGCATATGCAAATTTCCAAATCCG
TCAAAAAGTGTTACGGTCCAATCGATGATGACTGTTTCATCGAAT
ACCGACTATAAGAACACTTTGTTTGTCGGCGGTTCCGGCATTTTG
AACGGCGCGTGTGATGTGAACAATGGCAAACTGAAATATTTGAT
GACATTGAAGAATGGCGTGACCATCAAAAATGCCATTCTCGACA
CGCCCGGTTTGGGCATTTACTGCGAGGGCAATTGTGTGCTGGAA
AATATTTACTACAAGCGATTGTGTTACCATGCTACTGGGTTCGGG
TATAAGAGCACCAGCACTTCATACACTTACCAAGTGATTGGCGGT
GCTGGCCAAGGTTCACCGGACAAATATTTCACCCAATCGGGCAA
AGGCACAACCATCATCAAGAATTTCTGCGCAGAAGGCAAATACG
GCAAATTGTGGTGTTCATGCGGGAATTGTGCTTTCCAAACGGCG
CGTACCGTACAAATTTCAAACACCGTCCTTAAGGGGCCCGGACT
TTCGGTTGTCTCGCTTAATTCCAACTACGGTGACAAAATGTCACT
TTCGGGACTGACTTTGCATGGCCAAAAATCATCGAGCACAAAAA
CTAATTACATTTGCCAAGAGTACAAAGGCCTCACTCACATGGCC
GCCATGAGTCCTCAAGCGAATTATGAGCCAACCAAATCGGGCAG
TGGCACATGCGCGTACAGTGCCTCGGCAGTTAAAATTGCGAGCT
AATTTGGAGAAATAAATGAATAAATAATGGAGGGTTGAGTGATGA
AAGTAAATTTAGAAATTAAATGATGTTAATACAACCTGTTAATTGT
CGGACGAATATAAATAAAGATTAAAAAAAAAAAAAAAAAAAAAAA
(SEQ ID NO:27)

### 18H08 - Pioneer

AATGGCATTTCTCCTGTTGTCACTTGCACCCCTCTTCGTTG
CCCTTAGCGGGATTGATGCAAATAAAGTGCCACTGCAATTGTGC
TGTGCTGGTGGCCAAAGCTTGGCTGGCCTTATCTACGACTATTC
TGACCATTTCAGCGAAGTTTTGCAACAGTCGGGAACTGAGATGT
GCACTAGCCTGAAAAAGGACATGACCAAATTGACAACTGCAATT
GAAAAAGCTGAAGGATGCAAGTGCAATCTTGTTGAAAAAATTGAG
AAGGAATTGGATGATGTTGAGGATGATTGCGCCTACAATTTGTCA
CTTCATTCGCGCACTGTTCCACATTGTGGCCATGGCCTCGGAAC
ATTTGGGCGAAAGTTGGAACTCCGAAGCGGCCGAGTTCAACGA
GCAAATTGAAGCCATTGATAGCATTGGACATGATGAACTTAAGAA
GCTTGAGTTTGAAGCGGAATCGGAATTGAAAACACATCCTCGTG
TTGGCCGAAAAAGAATGAACAGATGCTAATTGGGCTGGGCAAAA
TTTGAAGATGTCAATGTGTATTTAGACGAATTAATTTGTCGATCTT
TCATTAGTTTAATGACACATTATGGCAATAATTAATAGAAGAATAA
ATATATTTTTTTG (SEQ ID NO:28)

### 19B10 - Pioneer

TATTTTATTAAAAATGCATTTAAAAATATTTTTATTAATTTTA
TTTGTGACAATTTGCTCGGTTTGGTCACTTTCACCCAAAATCCAC
CACAACTACCACCAACAGCAGCAACAGCACAACATTAAGAATAAA
TTGCCACCAAAAAATATTGGGACAAAACATTTGAAAACGGAAATT
AATGGGCACAACAAACACAAGCACAACCCGGCGAGCAGACACG
GGCATGCTTTGCTTCAAAAAGTGAAAGACCAATTGAATAATGATG
GATGGGCCAGTGAAGATGCAAACAAAACATCGGTATTGCTCTCT
TCACTGTTGGCCATCGAATGCCATATGAAAAAATTGGACAAGAAA
AGGACCAACGACTCGGCCGAGATCAAACATTTGACGGAGCAAAT
CTGTGCCGCCGAGAACAAAGAACAAATTGGCATTCATAAACTGA
TAAATGAAGTTGCGAAAACATTTGAAAAAGATGGAAAGAAAATGG

TAGAACAAATGAAAAGGGACGAGCATACGGTAAAGGTGCATCTT
GACGGGCACTACGGTACGTTGGCCGAGATTTTGCCAAGCGCCA
AAAAGCACGCGAAGGCAACAAGGCTTCGAACGTATCATGCAGTG
CTCCATTTGTTGCATTTGATCACTCCGAAGATGGAGGAGGGACC
GGCCAAAGAAGCGATG (SEQ ID NO:29)

### 19C07 - Pioneer

ATGGGGTTTTTAAAAACTATCGCTTTCCTTTCGCTTTTGGC
ACTTTTTTGCGCAACAATTGCTGAAGAAAATGGGGCGACAGAAG
GCACAGAAGCGGAGGCGACGACAGTGCCAACACTCACCGCAGC
GGCGGAGGGAGGCAAAGGGGGCGAAGAGGAAGTGACCACGGC
GAAGCCGACGGAGACGAGCGAAACGGCACGGGAGGAGGGGGA
GGAGAAGCACAAGGCGAACGAGGGGAAAGCGACTGAGGGAAGT
GGAACCAGATCGTTGTGGCAAAAAGTCACCTGTATGTTGTTCGG
CTTTTTCCCATTTCGCGCCGGCTGTCCGGGCAAAAACGATATAA
AAGCGGGGTCATCATCACATGTGAGCAGCACAATTATGATAATT
GAGGAAAAAGAGGACAGCGAATCGGACGAACAAATGGACGGCG
CCGGAGGGGACGAGGAGAAACGGGGAGGAGAGAACGGCAGCG
AAGCGCAGGGCGGCAAAGGCGCCGCAAGGGACAACGAAATGG
AAGACGAAGAATTGAAAGTGAAAGCCGTGGTGGAAGAGACGAC
GACAAATGAACAGGGCAAAACTGAGACAAACGTCGAACAGAGCG
AAAAGGAGGGGCCGATGAACTGACCGAAAGGACCGACTGATC
GGCAGGAACGTCATCCCTGACGGGGATTTGTCACGGAGAATAG
AAACAAAAAAAGCCACTTGCATTTGCGTTGTTTTATTTTGTTGCTA
CAGTAATCACATAGTTCACCTTTGTAGATGCTTTACTGTACTCTTT
TCATTTTTCTCTCTTTCTGTCCCTCTCTTTCTTTTCTTTCCGCTTTT
GGCTTATAAATTCCTTTTTCTC (SEQ ID NO:30)

### 20E03 - Pioneer

TGATCCGCTAATTATTATTTTGAATTGAGATTTATTAAAACC
TAATTAATTTTTTTCTACTCGAATTTCTTTTATGGCATTTGCCATT
TCAAGTTTAAGCGCTTTAATTTCGCTCGCTTTATTGGCAGCACCA
CAAAACTCGTACCAACAGAAACCTAACGATGAACCGAAGGTCTTT
AATGATATCCAAAACGGGTGTGATTTGATAAATTGTACCGATGGT
CGAACCTGCGGCATTCGAGTTGGGCTCGCAAAATTCGGCAACAG
AAATTTTGAGAAATTTGCTTTTCCAAAATGTATAACAAATGAAACG
GAACTAAACATGAACACGGACAACGATGGAAATGCGCACATTGT
TCCTAATGGACCAGGGTGCCAAATGATACAATGTTCTGTTGGCTA
CCAATGTCAAGTTCGAATTTCAATTTCCAAGCTTGGCAATTTGCC
ATATGCTCAGTCAATTGGAACCTTCCCGCAATGCGTGGGGCCTA
ATGTAAGCTTCAACTCATCAAGTTCAACTATCGAGAATGGCCCAG
GGTGTGACCAACTTCCGACGAAATGTGACGAAGGCACAAAATGC
GTAACTGCCGTCGGAATTGCGAAATATGGCAATTTGCCATGGTC
GCAATTTTTCTGGCCCTTTTGTACATGAAACGATGAAAAGCGCTG
CAACTGTTTTATATTCAGAGGAGCCAAAAAACGACGGAAAATTTA
TTTTTACCTAATTTAACAACTCTTGTGAGTAAATTTGTGATTTAAAA
TGTATTGTGATTTATGATTTTTTGGTAAACTAAAATTCATTTC (SEQ
ID NO:31)

### 20G04 - Pioneer

AAAATGCGCACCATTCTCTTATAGCCATGGTTTGCTTGGT
GATGGCTGTCCTAATGGAAATGGCAAATTCAAAGGCTGTCAAAA
AATACAACAAAAAAGGAGCAGTGGCGGCATCACCAGCAAAAGGA
AAAGCATCGCCAAAAGGAGGCAAAAGCCCAGCAAAAGGAAAAG
CGGCAAAAAAAATTGAAACCTAAAAAGGATGCTAAAGGCATTAAAG
CTAAAAAAGCAAAGCCAGCAAAGAAAAGCAAAGCGGCAAAAGCA
GTAAAGGGAGCGCCTAAGACAGTCAAAAAACTCGCATTTGCCAA
AACAGCAGCGCAAGTGAAAGAAATAAAGTCACCAGCCAAGCCAA

TGGTTCTAAAAGCAGTGCCGCCCCACCAAATGCATTTAATGAATG
AGAAAGTTCAAACTGCTGTGTCTCCACCCGCCCATGCTCGTTCG
CTTGCGACCGTTCCTTACAGTACACCGGTGGCAGCCGACCGTAA
CTCACTGCCATCGTACACTTCGGATGGCACCAATTTGGACATGG
CCCATGAGAATGATGATTATCAGAATTATTACTACGGATCGGGAG
ACAGCAGCAAAGAATTTGATGCATCGGCTGAAGAGGAAGATGGA
CTGTATGAGGAGGGGGACGGGGCAATGAATTGGGCAAACGGAC
GACTACCGGATTGGGCATTGAACAGGAATACGGGCATCAATCGG
GGGGAGTGGCCACCAAAGTCGGGGATGCTCCAACTGAAAAACA
CCAAGAATTTTGGAAAAAACTTATTTCCAAAATTTTGAAACATTT
GGATTTTAATATTCCCGTTAAAAAACTAAATTTTTTTTACCCAGC
CTCTAATTTTGTAAAAAAAAAAAAAAAAAAA (SEQ ID NO:32)

### 21E12 - Pioneer

TCAGTGCCCAACGCAACGCAATGGCCACTTACCTATTCCT
CGCTGCAGCTGCCTTCGGAGCGGGAATTTACTGTTGGAAGACGT
ACCTTGGCCCGATGCTTGACCAACCGAAGAGCGACGACGAGGA
GAAGCCCAAGCCGGAAGGAGGCGAAGAAGCGACGACCGCCGG
CGAGGAGGAGAAGAAGGCTGGCAGCAAGGGCGACAAGCACAA
GAAGAAGGACAAGAAGGGCGAAGGCGCAGCAGCAGCACCAGC
GGCGGACAAAAAGGAGGAGAAGCGCAAGGACAAACACAAATCC
AAGGAGTCGGGGAAGAGCGACGACAGCAAAGCGGGCAAGAAG
GACAAGCACGGGAAGAAGGACAAGAAGGGCAAGAAGAAGGACA
CCGCCGATGCATCCACCGTGGACGAAGCGCTGGGAGAACAGAG
CACAGCGGAGACGAGCCAAGCAGAGGAATAG (SEQ ID NO:33)

### 22C12 - Pioneer

ATGGCTTCATCTTTCCGCTCCTCAATCATTTCCATCGTCGC
AATTGTCTGTTTGCTGTGCAAATGCTGCTTTTCGGCACCCCATCC
ATGCTGTCCTGGCAGCCAACATGTCGTTTCGATGATGAAAGATC

ACACCGGCACATTCTCCGCTTCGATGCCAAAGTCTTCGCTTTGTA
TGAGTGCCGAAAGAGTCGCCGCTGCGGTGGAAAACCAACTGAA
AACAATTTGGTGCCCTGGCAATGGTAGTCAAACACTCATCAACG
AGATCAACGCAACTCAATCATCATCAGATGAGTGTGCTCGCAGT
CTCGGCTTCGTCCGTGCCATGTTCGAAATTGCCGCTTCCGCCGC
TTCCCATGCCGGTGCCAACGCCGATTTGGCTGTCCAGTTCCGAG
AACAAGTTGGCACAATTGACAGCAATTGTGCCGCGTTGGGCATT
CATGTTGGGCAAATCAGCTTGGATGCCCCCAAGGGAGACCATCC
GCAAGTGCATGACTCTGAGAGTGTGCTTAGTAACCCTGGCACCA
GCGGGTCTCACAAGCGCTTTTAA (SEQ ID NO:34)

### 23G12 - Pioneer

TCTCTCTACTAACTGTTGCACATCCCAATTTTACTTTCTTCC
TTAGTAAGTAGGGCTTGGTGCACAATGCGCACTTTTCTGTTCATA
GCCGTAGTCGGGTTGATGTTGGCCGTCATCTTGGAGAATGTAAA
TGCGACAGGGAAAAATTCGCCGACAAAGGAACTTATACATGAGC
AAGGTAACCTACAGAACCATGCTACCGGACAATCGCCGCCAGAC
AGTCCAAACCATAAAAATGACCATGAAAACGAGATTAGTAACCTA
CAGAACCGTGCTACCGGACAATCGCCGCCAGACAGTACAAAAC
GGGAAACAGAACATAAAATTGAGAAAGGAAAACCGTCGCCAGAC
AGTCCAAAGTATGATGAGCAAGCCTTAAAATGGTGAAGAAACTGT
GTGGAACGATGATGCCAACCATGAACAAACCCAAAGCTGTTGTA
GTAGATGTAGAAAAAAACTGTGGTATCATCACGCTATTATTGTTG
TGTAAAAAACGATCCGTTTTTACCTCTTTTCGGGAAAAAAAACTG
GAAAAAATTCACAAAAAAAAAAATCTCACATTGTGCAGATTTTATT
GAAATTAGCCAATTAAAAAAAACAGTGACGAAAAAAAAAAAAAAA
AAAAAAAAAAAAAAAAAAAAAAAAAAAAAAAAAAAAA (SEQ ID
NO:35)

### 24A12 - Pioneer

ATGTCTTCTTCCCCGTCCGTCTCTGTGCTCGCCGTCGTCG
CAATTGTCTGTTTGATGTGCCAATGTTGCTTTTCGGCACCGCATC
CGTGCTGTCCCGGCAGTCAAAAAGTGGTTTCACTTATGTCCAATT
ACGTTGGCACTTTTGCCAATTCCATTTCCAAGTCATCGCTTTGTT
CGGATGCCCAAAATGTTGCGGACGCGTTGAAAGGCCAACTGATC
GGCTGCTCCGCTTCTTCCCATGCCAGCAACAACAGCGAATGGCA
GACATTGAGTGGGCAGTTTGGTCAGAAAGTCACTGAGATTGACT
CGAAATGTGCAGAGTTTGGCATTAGCATTGGCAAAGTGCCCATA
AACGGTCCCAAGGGTGTCCATGTTCAAAATGTGCCCAACTCGGA
AAGTGTGATTTCTATGCCTGGATTGGCCGGCTCACACACCCAAT
GA (SEQ ID NO:36)

### 25A01 - Pioneer

AATTATTTTTTTAATAATAATTTTTTACAAAATGAGCAACTT
TATATTTGTCGCCTTTTTAACTGCAGCGTTTTTTAGCTCAAGCCTC
GCTCTACCGGCTCCTTATGATGCTGAATCGGTGGTATCTTCTGAA
TTAAATGTTCCACTACTTTCAGCTGACGCAAATGTTCAAGCAGCT
GAAAATGTTGAGATACAAATTCCATCAATTCCAGCACCAATTGAA
CAACAAACTGCTGCTCACATTACTCATCCAACTGAAACTGGCAAT
GAGCCTTCTATTGCATCATCATCATCCGCGCCGAAAAGTGAGCA
GACGCCAAAAAAAGTGATGAACATGAAAAGCGCGATGGAAGGC
GCGGCGGCCAACGTGTACGGGGGGCTTCCGTTAGACCAGCAGC
CCAAGACGATCGCTGAAGCGGCAGCAAAAGCAAAGCAGACGCC
CGCCAAACTTCCGGCTGACTACGACGCGAACCGCGTGGCGGAA
CGCGCGGCGGCCCGTGTGTACGGGTGGCTTCCGGAAGACAAG
CAGCCTAAGGCGATCTATGACGCGGCGGAGAAGGCAAAGAACA
CGCCCAAACCGCCGGGCGACTACGACGTGGAGCGCGTGGCGC
AAAAGGCGGCACGGCTCGTCTACGGTGTGCTGCCCATCGGCAT
GCAGCCCAGCTTCGCCGGCCCTAGCACTGACAAGAGCAATGTC

GACGACTCGGAGAAACCTTCTGCTGCTGCGGCTGGTGATGATGA
TGAAGTCGAAAAAAAAAAAAAAAAAAAAAAA (SEQ ID NO:37)

### 25G01 - eng-1

AATTCGCTTTCATCGTTCAAATGTGCCGACTCCAAGCAACT
CATCTGCTCGCTCGACTCTTTCTGCTTCTTGCGCTTTGCACTGCT
CTCGTTAGCTCTCTCACTGCTGTTGCCCCGCCATTCGGCCAATT
GTCCGTTTCCGGCACCAATTTGGTCGGCGCCAACGGACAACCC
GTACAGCTGATCGGCAACTCACTGTTCTGGCACCAATGGTACCC
GCAGTTTTGGAACGCTGACACAGTGAAGGCACTCAAATGCAATT
GGAATGCCAATGTCATCCGGGGGCCATGGGCGTGGACGAGG
GCGGCTATCTGAGTGACGCGAACACGGCTTACAATCTGATGGTG
GCAGTGATCGAAGCGGCCATTTCCAATGGCATTTACGTGATCGT
CGATTGGCATGCCCACAATTCACATCCGGACGAAGCGGTCAAAT
TCTTCACCCGAATTGCTCAAGCGTACGGCTCCTACCCTCACATTT
TGTACGAGGATTTCAACGAGCCGCTGAGCGTTTCGTGGACCGAT
GTGCTGGTGCCATACCACAAAAAGGTCATTGCTGCCATCCGAGC
TATTGACAAGAAAAATGTGATCATTCTCGGCACTCCAACATGGTC
CCAAGACGTGGATGTGGCATCACAGAACCCAATCAAAGACTACC
AAAATCTGATGTACACTCTCCACTTTTACGCGTCCAGTCACTTCA
CGAATGATCTTGGTGCCAAGCTCAAAACAGCCGTGAACAACGGT
TTGCCTGTGTTCGTCACTGAGTACGGCACATGCGAAGCGTCGGG
CAACGGCAACTTGAACACTGACTCGATGTCCAGCTGGTGGACTC
TGCTGGACAGCTTGAAGATTTCATACGCCAACTGGGCAATCTCC
GACAAAAGTGAGGCCTGCTCAGCACTGAGCCCCGGTACAACTG
CTGCCAATGTCGGTGTTTCGTCCCGTTGGACATCCTCCGGAAAT
ATGGTTGCTTCGTACTACAAGAAAAAATCCACCGGCGTAAGCTG
TAGCGGCTCAAGTTCCGGCAGCTCTTCGGGCTCGAGTTCTGGCT
CTTCCGGTTCAAGTTCCGGCAGCTCTTCCGGCTCAAGTTCCGGC
AGCTCTTCCGGCTCAAGTTCGGGCTCCTCTGGTTCGAGCTCAGG
GTCCAGCTCGGGTTCGGGATCCGCCAGCATCTCTGTAGTCCCAT

CCAACACGTGGAATGGCGGTGGTCAGGTCAACTTCGAAATCAAA
AACATCGGGTCCGTGCCATTGTGTGGCGTTGTGTTCAGCGTCTC
TCTTCCCTCAGGGACCACGCTTGGTGGATCGTGGAACATGGAAT
CCGCAGGCTCCGGCCAATACAGCTTGCCAAGTTGGGTCAGAATT
GAGGCCGGAAAATCGAGCAAAGACGCGGGGCTGACATTCAACG
GAAAAGATAAGCCAACGGCGAAAATTGTGACGACGAAGAAATGT
TAG (SEQ ID NO:38)

### 26D05 - cellulase

GAATTTCATTATTTCACTGATTTGCCCATTACTTTTCTTAAT
GAACTCCTCATTTGTCATCTGCTGCTGCTTCCACAACTTTGTGTT
GTTCCTTCTTTTGAGTTGTGTTGTGCCACATGCTTTTGCTTGTTGT
CCTCATGGAAATCTCAGAGTGAAAGGTACGCATTTGGCCGATGA
AAAGGGCGAAACTGTGCAGCTGCGAGGGATGTCTTTGTACTGGA
GCCAATGGGAGTACGGCTCAAAGTTCTTCAACGAGAAGACGGTC
AACTGCCTGAAGTGCAGTTGGCATGCCGACATTGTCCGTGCTCC
GTTAGCTGTGGACCAGGGCGGCTATTTGTCAAATCCAGAACAGG
AGTATGCGAAGGTGAAGAGCGTAGTCCAAGCAGCAATCGACAAA
TGCATATATGTGCTGATTGATTGGCATTACACGAGCAGTGAGAAA
TATACGGACAAAGCGAAGGAGTTCTTCGGCAGAATTAGCACTCT
GTGCGCTGGCAAATGCAACTGTTTGTACGAAACATGGAACGAAC
CCATACAAAACGATTGGTCCTCGCAGTTGAAGCCGTACCACGAG
GAGCTGGTTAAAGTGATTCGGCAGAATGACAAGAACGGAGTGAT
AATCGCTGGAACGCCAAATTATGACCAGGATGTGAAGGCGGTAG
TCAATGACCCAATCAAAGAGCACAATATAATGTACACATTGCATT
TGTACGCGGCATCGCATAAGCAAGAACTGCGCAACACGGCACAA
CAGGCAATCGGCGCGGGAGTGCCGTTGTTTGTCACGGAGTATG
GCACAAGTTCGTACACAGGCGATGGCGGGCCAGACCTTGTCGA
AACACAGAATTGGTACGACTTTTTCAACAAAAACTCCCTCTCGTA
CATCAACTGGGCCATCGACGACAAGCAGGAAGCGTCGGTAGCG
CTGCAGAACACACAGCCCCCAGTCGGGCCGGCCGACGTGTGCA

GCACCGATCGGCTGACCAAATCGGGCAAATTTGTGCACGACCAC
CTCATCGGTGTAAACCAGAAGCCGGATGGCTGTTAAACATCAGA
AAATGGCAAATGATCGGAATCGCGCTCTTTTTTTTACTTTTCT
GGGTCATTTCATCTTTAGCATTAAACCTAATAAAAAAAAAAAAAA
AA (SEQ ID NO:39)

### 27D09 - Pioneer

CGCCCATTCTCTTATAGCCATGGTTTGCTTGGTGATGGCT
GTCCTAATGGAAATGGCAAATTCAAAGGCTGTCAAAAAGACAAC
AAAAAGGAGCAGTGGCGGCATCACCAGCAAAAGGAAAATCATC
GCCAAAAGGAGGCAAAAGCCCAGCAAAAGGAAAAGCGGCAAAA
AAATTGAAACCTAAAAAGGATGCTAAAGGCATTAAAGCTAAAAAA
GCAAAGCCAGCAAAGAAAAGCAAAGCGGCAAAAGCAGTAAAGG
GAGCGCCTAAGACAGTCAAAAAACTCGCAATTGCCAAAACAGCA
GCGCAAGTGAAAGAAATAAAGTCACCAGCCAAGCCAATGGTTCT
AAAAGCAGTGCCGCCCCACCAAATGCATTTAATGAATGAGAAAG
TTCAAACTGCTGTGTCTCCACCCGCCCATGCTCGTTCACTTGCG
ACCGTTCCTTACAGTACACCGATGGCAGCCGACCGTAACTCACT
GCCATCGTACACTTCGGATGGCACCAATTTGGACATGGCCCATG
AGAATGATGATTATCAGAATTATTACTACGGAACGGAAGACAGCA
GCAGAGAATTTGATGCATCGGCGGAGGAGGATGAACTGTAT
GAGGAGGGGGACGGGGCAATGAGTTGGCAGACGGACGACTAC
GGTATTGGCATTGGACAGGAGTACGGTCATCAATCGGTGGAAGT
GCCACCAAAGTCGGTGATGCTCGACTGAAGAACACCAGCAGGAT
TTTTGTAAAAACATATTTCTCAGATTTTTGAACATTTGCATTTTATA
TTCGCTTAGAAGCTAATTTTTTTTATCTAGCTCTAATTATGTAAAA
ATTAATAATTCTTCGAAAAAAAAAAAAAAAAAAAAAAAAAAAA
(SEQ ID NO:40)

### 28B03 - Pioneer

CATGAACATTCTGCTGAAGTCTGTTCTATTCATCTCTCTTC
TGTCACTGTTTGGTGATTGCATGAAAAACTCAGATCAAATTGGAG
TCAAAAGAAAATCAGCAGGAATAAGAATTGAGGAACCAAATGATG
TAAAAGGCAAAGGAAAAGTCGTCGAAACAAGCAGAAAAGGCAAA
GAAAAAGTCGGCGAAAAAATAAGCGTTATGAACACAGCTGAACG
CATTGAAAAAATGGACCTTGCCCAAGACAAAGCTAAAATGGATAT
AGACACCAATGGCAACCCAAACAAAATGAGGAAAATGCTCCTC
CAAAATATGTTTTCATTTGGAGCGGTAACAATGAGAAGAACC
TTGAGGTTGACCTCAGAGTGATCCGTCACTCCAAAAAACTCAGC
GAATTATTCCATGCCAAAAGTGATCAATCCGTCAATGCAAAACAC
GGAAAGCAGGAAGCATTACCGGTTCCGAAACAGGAAGCTGAAAA
AATGCTGAAGGAAGTGGAAGAATGGTACGCACATCACAAAAATG
ATGAACATAAAAAAGCTCCAGTTTTTGTGTTCAAGTGTAAAGACA
ATGTCAGTCTGGTGATAAATGTTCATCTTGATGCAGTCCGGCTTT
CCAAGGAGTTGTCAAATATGCTTGAAGATACTGCCGACGAAGGA
TATCCGAAGCCAATCCCGAGTCCAGTTCAGAATTTCAAAAGTGAC
ATCATGCTAAAACTGATTAAATTGTGTGAAGAATACAAGTACAAAT
TCGATGGCCTAAAGAAACAACTGATGTGCCAACTGGAGAAGTC
GCGGAGGCATCGGCTTCAAATGTCCAAGAGGGTGACGTTGGGG
AGGCATCGGCTTCAAAGGTCAAGGCTGGAACAATTGCGGAGGA
AATCCAAAAATTCGTTCCTCCGCCACACATTATGCAATGGACGT
GAAAACGGACCTTGAGATGGTTCGGGCCGCCGACTTTTATAACA
TCAATCGTTTGATCAACGATGATTACATTGACAATGTGTACGAAA
TCGTAAAAGTCAAATGGATCAATGGCAAAACGCCGCAGGAAATT
CGCAAGGGATTCGGCGTCGAAGAGCCGTACCCGCCGGACATC
CGGAATGGGCACGAGTTGAGAAGGAGAACGAGTGGAAGAATC
GGACGAGGAACGTGAGGCACGCCATGCAAAGGAACGAGAGGAG
GAAGAGGAGCGTGAGAGAAAGGAAGAACAGAAGCGTAAGGAAG
AGGAAGCGGAACGCCTCCATCAGGAACAACTGCAGCAACAACA
GAATCAGGAACAGCAACCTCAGTAGGGACAGCAGCATGGTGAA
GAACTGGAACACGATGAAGTTATGCATGATGTGGAGGAAGAGCA
AGATGATGAATGGCGAGGAAGAAGATGATGAGTGATGAGGATGA

GGAGGATGTAAAATGATGTGGTGATTAGTGATTTTGATGAACCGA
TGATTCACTTTTCTTGGATCCTGTTGCATAAAACTTGTTGCAAAAA
AAAAAAAAAAAAAAAAAAAAAAAA (SEQ ID NO:41)

### 29D09 - Pioneer

ATGTCTTCTCTGCTGCTCTCCATCTTCCCAATTGTGTTTTT
GGTCTGTTGCAATGCAATGCCAAATTTCCCGTGCTGCCCGGGCA
GTCAGCAAGTGGTTGCTGTGATGTCCATTTACATTGACACTTTCT
CTTCTGCTGCTGACGAGTCTACAGTATGCTTTTTCGCTAAAAGTA
CTGTGGATGGAATAAAAAATGAACTGTCCTCTCGCGGCGGATGC
CAAAACGGAGGAGAAGCACAAATTGTGGATGAAATCGATCGACA
GCTGAAGAATATTGCAAAAATGGAGATCAATTATGAGGACGAGT
GCCCGTACAATTTGGGCTTTGCCCGTGCCATGTTCGACTTGGCC
GCTGCTGCTGCTGGCCATGCGGGCAACGGCACAGAATGGCAAT
ACATGAAAGTACAATTTGAGCAGGAAAGCCAAGCAATCAAAGCA
ATTGGACAAGAAAAGAACTTTGAAGTTACGGATGTGCATTTTGGA
GTCCCAAGCAAAGGGGTTTCTGCACATCAAAATGTGCCGAGTCC
GAGCCATGTGATTGCCAATCCTGGCCAACACAGTTCGGTTGGGC
CAAGGAAAGAAAGAAGAACCGTTGTCATCGGACTTCGATTTTTGA
GGACAAACAAATCAGGAGGAAATAGAATAGAAAACCATTTTGTTG
ACATGTCGAACATTTATTTAAAGTAATAAATATTTGGTAATAAGGA
AAAA (SEQ ID NO:42)

### 30C02 - Pioneer

ATGAGGAAACTTCCATTCTTGCTCTTATTTTCTGCTTGTTG
CTACTTGCAACGGAAGGAAGTATATTTCGCCGAAAAAACAGAAA
GAAAAATTCATCGAGCAGCGAACAAAAAACACAAAGAAGAAACC
GAGGGTATGGCCGAAGTCGCACTTTTTCAAATGGCAATGGCATG
TATGGCCAATCGAATGGATTTTCAAATGGCCGATTTGGCGGCTC
CAGTGTCTATTCAAATAGCGGCCGGTCGAATAGCTTTCCAAATG

GTGGATTTGGCGGCTCCAGTGGCTTTTCATCGATGGGACATAAC
TCGGGCGGACTTCACGGCTTTGGGTCCGGCCCACCTTCTGGAG
GCTACGGATACGGAAGCGGTTTTGGTGGAAGAAAATGAAAAAAA
GACCGAAAGTGATCGGCGAGAGAATGATTTGTGCTGTTGCCTTT
TATGCATGCAAAATTTAAATGGAAAAACGCATTTCACCAGCAGAA
CACTTTTTTAA (SEQ ID NO:43)

### 30D08 - Pioneer

ACAACCGCTTCAGTGCGCAACGCAACGCAATGGCCACTTA
CCTATTCATCGCCGCTGCTGCCTTCGGAGCAGGAATTTACTGTT
GGAAGGCGTACCTCGGCCCGATGCTCGACCAACCGAAGAGCGA
CGACGAGGAGAAGAAGGCTGGCAGCAAGGGCGACAAGCACAAG
AAGAAGGACAAGAAGGGCGAAGGCGCAGCAGCAGCACCAGCG
GCGGACAAAAAGGAGGAGAAGCGCAAGGACAAACACCGATCGA
AGGAGGCGGGGAAGAGCGACGACAGCAAAGCGGGCAAGAAGG
ACAAGCACGGGAAGAAGGACAAGAAGGGCAAGAAGAAGGACAC
CGCCGATGCATCCACCGTGGACGAAGCGCTGGGAGAACAGAGC
ACAGCGGAGACGAGCCAAGCAGAGGAATAGCCAGGCCCTGCTG
CGTGCAAATGACAGTGTTCGACGACGACGTCTCGCGACACCAAC
ACACCCGTCCCTTGCTTTCGACCATTCCCTTCCCATCCGATTGCT
GTGTTTTGTCTGTTCCAATGCTTCCTAAATGTTCCCCTTGTGAAC
AGTGATCCTTCCTCATGTGACCTGAATATGTATATATTGTATACC
CATTAATATATTGTGCTTCGCTTGTTCGATATTTGTGAAATCTCAT
TCATATTCATCGTCATTG (SEQ ID NO:44)

### 30E03 - Pioneer

ATCCTCTAAGAATTCACATCCTCTCAGAAAATGGCTTCATC
TTTCTGCTCCTCAATCATTTCCATCGTCGCAATTGTCTGTTTGCT
GTGCAAATGCTGCTTTTCGGCACCCCATCCATGCTGTCCTGGCA
GCCAACATGTTGTTTCGATGATGAAAGATCACACCGGCACATTCT

CCGCTTCGATGCCAAAGTCTTCGCTTTGTCTGAGTGCCGAAAGA
GTCGCCGCTGCGGTGGAAAACCAACTGAAAACAATTTGGTGCCC
TGGCAATGGTGGTCAAACACTCATCAACGAGATCAACGCAGCTC
AATCATCATCTGATGAGTGTGCTCGCTCTCTCGGCTTCATCCGTG
CCATGTTCGAAATTGCCGCTTCCGCCGCTTCCCATGCCGGTGCC
AACGCCGAATTGGCCAATTTGGCTGTCCAGTTCCGAGAACAAGT
TGGCACAATTGACACCAACTGTGCTGCGCTGGGCATTCATGTTG
GGCAAATCAGCTTGGGCACTCCCAAAGGAGACCATCCGCAAGT
GCATGACTCTGAGAGTGTGCTTAGTAACCCTGGCACCAGCGGGT
CTCACAAGCGCATTTAAGTGCATTGCGACGATTCCGATGATGTC
ATCATTTGTTGATTGATATGCTATAGAAATTATTTTATTAGATAAAA
AATGAATCATTACAAAAAAAAAAAAAAAAAAAAAAAAA (SEQ ID
NO:45)

### 30G12 - Pioneer

TAATAAATAAGTTTTTTTGTTCAAAATGAGCAACTTTATATT
TGTTTCCTTTTTAACTGCAGCGTTTTTTAGCTCAGGCCTCGCTCT
ACCGGCTCCTTATGATGCTGAATCGGTGGTATCTTTTGTTTTAAA
TGTTCCACTACTTTCAGCTGACGCAAATGTTGAAGCATCTCCTCC
CAATGAAAGTGATGCTGTTTTTGAGATACAAGCTCCATCAATTCC
GGTACCAATTGAACATCAAACTGCTGCTGACATTACTCATCCAAC
TGAAACTGGCAATGAGTCTTCTATTGCATCATCATCATCCACGCC
GAAAAGTGAGCAGACGCCAAAAAAAGTGATGAACATGAAAAGCG
CGCTGGAAGGCGCGGCGGCCAACGTGTACGGGGGCTTCCGT
TAGACAAGCAGCCCAAGACGATCGCTGAAGCGGCNGCAAAAGC
AAAGCAGACGCCCGCCAAACTTCCGGCTGACTACGACGTGAAC
CGCGTGGCGGAACGCGCAGCAGCCCGTGTGTACGGGTGGCTTC
CGGAAGACAAGCAGCCTAAGGCGATCTATGACGCGGCGGAGAA
TGCAAAGAACACGCCCAAACCGCCGGGCGACTACGACGTGGAG
CGCGTGGCGCAAAAGGCGGCACGGCTCGTCTACGGTGTGCTGC
CCATCGGCATGCAGCCCAACTTCGCCGGCCCTAGCACTGACAA

GAGCAATGTCGACGACTCGGAGAAACCTTCTGCTGCTGCGGCT
GGTGATGATGATGATGAAGTCGAAAAAGAGAAGAAGGAATAAGC
AAAAAACAATGTGAATTTATTATAGGAAAAAAAGAAAAATGGAAG
CTTAGAAATTTAATATTTTCATTTTTGAACATTTATAAAATTTCAAA
ATTAAAAAAAAAAAAAAAAAA (SEQ ID NO:46)

### 32E03 - Pioneer

TTAAAATGCGCGCCGTTCTCTTCCTGGCCATGGTTTGCTT
GGTGATGGCTGTTATTCTTGAGACAGCCAACTCAAAGGCAGTGA
AAAAAGACAATAAGAAAGGAGCAATTACAACGCCAGCAAAAGGA
AAAGCGGCGCCGAAAGGAGCAGCGAAAGGAGGAATTAAAAAG
ACGCAAAATCAAAGGGAAAAGGAAAAAGGACGCCAAAGGCAAA
AAGGACAAAAAAGCTAAACCAGCCAAAGGGAAAGCATCGCCTAA
AAATGACAAAAAACCCCCAGCTGCCAAAGCAAATGACAAAAAGA
GCCCAACCAAACCAATGGCAGCAGTGAAAGCCGTGCCAAAGGC
GGCGGAGAATGCTCAGAAGGACCGTCGAGTGCGGAAGTGGTG
GCGGAGGAAAGCAACTTGGACACTGAGGCGGTCGACGACTTGG
GGGTTGGACCTGAGGCGGTCGACGACTTGGGGGCGGAAGAGT
CGGACTTTGACCAATTGGCAGAGGACGAACTGCTCGAGGACGA
CGCCATGGGCGGCGAGGCGGACGAATGGGAAGGCATGGGGGA
GGAGACGGAGATGGACAGCCAAATGGCACTTTGATGGCCAACTA
ATTGGACAAATTACAAACGGAACAAAACGGGACAATGACACAA
AATATTATTTATCATTTTTGGCATATCATTGAAAATAAATAGAATTT
ATTTTGCAAAAAAAAAAAAAAAAAAAAAAAAAAAA (SEQ ID
NO:47)

### 33A09 - Pioneer

ATGAATTTGATTTTTGAAATATTCGCCTTTGGAATGGACAA
TTTCTCATCGGAAAAAAATGTTTCTCTTCTTCGTCTTTTCTTATTCT
TTTTCTCGACCGTAGTATTATTTGGAAGCGCACACCCTATGGTAA

ACAAATTATGCGAAGACCTAGATAAACCTGAAGGTTGGCAATTAT
TGAAAAAGTTAACAATCGAAAAGTGCCGGATGAGTATAATGTGGA
ATTCCCCAAATGGAAAGACAGCTACAAAAGCATTAAAAAGTTTAT
AA (SEQ ID NO:48)

### 33E05 - Pioneer

ATGCGCACTTTTCTGTTCATAGCTGTAGTCGGGTTGGTGT
TGGCTGTCATCATGGAGAGTGTAAATGCGACTGGAATAATTCG
CCGACAAAGGGGCAAACTCCGCCTGGCAGTCCGAAAGGTGGGC
ACGGCAGCAACAAATCGCCAACGACGCCGCACAGTGCCGGTTC
GCCACACGGCACGCAGCATGGTAACCAACAGAACCATGCTGCC
GGACAAACGCCGCCTGGCAGTCCGAAAGGTGGGCACGGCAGCA
ACAAATCGCCAACGACGCCGCACAGTGCCGGTTCGCCACACGG
CACGCAGCATGGTAACCAACAGAAAAATGCTACCGGACAAACGC
CGCCTGGCAGTCCGAAAGGTGGGCACGGCAGCAACAAATCGGC
AACGACGCCGCACAGTGCCGGTTCGCCACACGGCACGCAGCAT
GGTAACCAACAGAAAAATGCTACCGGACAAACGCCGCCTGGCA
GTCCGAAAGGTGGGCACGGCAACAACAAATCGCCAACGACGCC
GCACAGTGCCGGTTCGCCACACGGCACGCAGCATGGTAACCAA
CAGAACCATGCTGCCGTTTCAAATGTGGTGCAGAACGTTGACAA
CAAACCTCGGCCATCTGCTCCTGCTTCACACCTTGTGCCACCAA
CACCAGGGAACAGCCCTACTGCGCATGACAGTCGCTGA (SEQ
ID NO:49)

### 34B08 ― Pioneer

CTGCACATTCCCCAATTTTATTTCTTCCTTAGTAAGTAGTAT
TGGTGCACAATGCGCACTTTTCTGTTCATAGCCGTAGTCGGGTT
GATGTTGGCCGTCATCTTGGAGAATGTAAATGCGACTGGAAAA
ATTCGCCGACAAAGGACAATCGCCGCCAGGCAGTCCAAAACAT
GAAAAAGACCGTAAAAATGAGCATGGTAACCAACAGAACCATGC

TACCGGAAAATCGCCGCCAGGCAGTCCGAGAGTACAATCGCCG
CCAGGCAGTCCGAGAGGAAAATCGCCGCCAGGCAGTCCGAGAG
TACAATCGCCGCCAGGCAGTCCGAGAGGACAATCGCCGCCAGG
CAGTCCAAAACATGAAAAAGACCATAAAAATGAGCATGGTAACCA
ACAGAACCATGCTACCGTTCCAAATGTGGTGCAGAAAGTTGAAG
TCCACAACCAACCTACGCCATCTGCTGCTGCTTCACACCCTGTG
CCACCAACACCAGGGAACAGCCCTACTGCGCATGACAGTCACCA
CACAAGCCCAAAAGTTGCCCATGTTGCTGCCGCCGCCACTTTGG
GCCATGGACGCCAAGAATGTGCCAATGTGTCGTGTGGTGATGAT
GTAACCGAGGAAATGGACCGCGACGATGATAAGACAGTGGAAC
GCGCGGTCGGCGAGTCACCAATCGGCGTTTCGGAATCCAGCGC
CACGGATGAGGATGATTCTGCTGCCGTTTCGGCTTACAACAAAA
ACAACAGCTCGGCCGCCGCATCAGCGTCAGTTGCTGATGACTGA
GATGGGGGTGATGGTGAAGGAACTGCGTGGAACGATGATGCCA
ACCATGAACAAACACAAAGCTGTTGTAGTAGATGTAGAAAAAAAC
TGTGGTATCATCACGCTATTATTGTTGTGTAAAAACGATCCGTT
TTTACCTCTTTTCGGGAAAAAAACTGGAAAAAATTCACAAAAAAAA
AATCTCACNTAAAAAAAAAAAAAAAAAAAAAAAAA (SEQ ID NO:50)

### 45D07 ― Chorismate mutase

GGCAATTAATTGTCCAATTAAGCGGATGAATTCGTCCGTC
GTTTCATTTTCGCTGTTACTTTTTGGGTTATTCACAATTCGCACTG
CAAAATCGCAATGCGAGAAACATTGCACTAAAAGCAAACCGATG
GGCCAATGCAATGAGGCGGAAGAAGTGATTCTGCGCAACTCCG
ACTGTGCCTTCATGAAGAAAACGGAAACGGCATTCGAATTTGTG
GTCGGAATGAATGGCCAAACGGAGGACAAAACGGCGCCCGGGG
CCAATGCCAATGGGGCATTTTTGTGCTGTAAGGCAACCCAAGGA
ACCGCAACACTTTTCATCGTCGGCGTGGCCAACAAACGGCTGAT
GTTGGCCAAGGACGTGGTCAATTACAAGTTTCATCAAAACATCTC
AATCGATGATTTTGAACGGGAAAAGCAAGTGTTGGAAAGTGTTTC
GGCGCAGGGACAAAAAGCCGGCATTGGGGACAATTATGGAGAA

AAATTCTTCCAGGACCAAATGGACGCCAACAAAATGATTCAGAAA
GGCTCTGTGAAGCTGTGGACCGCCAACAAATCGTTGCCTCCTCA
AAATGTGCCGGATTTACAGAAAGATACCCGGCCCAAAGTGACGG
CGGCGACAGAAGAAATGATTTTGGCACTGAAGGTGTTTCAACAG
TTTCGAAAGAACAAAAATTGTTGGGCATCTGTGGAAAAGGAACTC
TTAAAGTCGCAAAGCTTTTTGAGTTTGGCCGAACCCAACGGAAA
GGACGCAATGCGAAAAGCGGCGGTGCGATTGTGCGCAAAAATG
GAAGCGAAAATTGAGGCACAAATTGACGAAACGGCAAAGAAATT
GTTGGCCTGAGGAGGACGAATTGACGAGCATTTTAGGAAAAAGA
TCCTTTCGTTCCGATTTTGATTCTTTGATCAGTGAAAGTGCGGTA
TTGTCAAAAAAAAAAAAAAAAAAAAAAAAAA (SEQ ID NO:51)

### SYS91 (AF273734)

GAAGGCGTGTCCAAGTTGATCAACGGTATTCCAGTGGCTG
AGACCGTGGTTTACCCAGCCATCGGCGAGACCAAGACTCATATC
ACGGTGTTCACCGATACCACCTGCCCGTACTGCCACAAGCTGCA
CGCCGAAGTGCCTGCGCTGAACAAAATGGGGATCGAAGTGCGC
TACGTCGCGTTCCCGCGCCAGGGCCTGGGCTCGCCGGGTGAC
GAACAGCTGCAAGCCGTATGGTGCTCGGCCGACAAGAAAGCGG
CCATGGACAAAATGGTCGATGGCAAGGAAATCAAATCGGCCAAA
TGCGCCAACCCGGTTTCCAAGCAGTTCGCCCTGGGCCAGTCGA
TTGGTGTGAACGGTACACCGGCCATCGTTTTGGCTGATGGCCAG
GTCATTCCGGGCTACCAGCCTGCGCCACAAGTTGCCAAACTGGC
ACTGGGTGCGAAGTAAGCAGTCATCGTCGAGCCTTTGACGATCA
TGCCCCGTCGAACAGTCGACGGGGCATTAATTGAGAGCCGCAG
TTGTGCGGCTGTTTACCACGGCCGACCTTGAGTCGGCCGTTTCA
TGGGGAGTTCTTCAGTGAAACCGGTCAAAGTAGGCATCTGTGGG
TTAGGG (SEQ ID NO:52)

### SYS16 (AF273730) .

AGAAGAATTTCTCAACCATCATAGGACCGAATAGATGAATA
GTGTTTTATCGATCTCCATTTTATTCCTGCTCAGCCAGGTTCTTTC
CACTGTCTCCTCTAGCGATGTGTTGGAATACACGGATGCTAGCT
TTGACTCGGGAATGCAGCAGCACGACATCGCATTGGCAGAATTC
TATGCCCCCTGGTGTGGACATTGCAAAAAACTCGCTCCGGAATA
CGAAAAAGCGGCCACTAAACTGAAGAACAACGACCCACCAATTC
CACTCATCAAAGTCGATTGTACTGCGGAAAAAGAGACTTGCGAT
AAATTTGGAGTTAGCGGTTTTCCAACATTGAAAATCTTCAGGAAG
GGGC (SEQ ID NO:53)

### SYS86 (AF373733)

GCAATAAAGAGTAAATTATGGATAGGAGATTCACTGTCTTC
CTTGTGATCGCATTGGTTACTTCTATTTATGAAGTGCTTAGTAATG
GAAATTTGAACGATGGCGACGACTCATTTAAACAATTCGATGAAC
TCGAAGAAAACCCAGCGCATAAATATTCAAAAGAGGCCCAGAAG
GGGTTCGAAATGGAGGAGGAGGAAGTCACAATCCGAGAACCTT
CGGGGACGAAGGAATCGTTCAAATTGCCCATCAATATGCCGT
(SEQ ID NO:54)

### SYS79 (AF273732)

CGTNTCAGTTNGAGTTTCATTGAAAACTCAAAAAATGACAA
CAGTCAAATTGCTATGCCTAATTGCCCTATTTGCGGTAGTGCAAC
TTCGTTTTGCATTTGCTATGATGAATCAAAACAACCAACAAGTGA
GCCAAAACAACAGCTCTGACGAAGGAGAAGATGATGATGATGCT
GGTAACGGCGGGCAATATGACACGGTTGCTCCATTGCGTAGCG
ACAGCACTAGTTTCGTTAGCAGCGGCACCTTACCGCCGCAGGG
CAGCACCGGTTTCAGCGGGGTCGGCATGATGCCGCCGCATGCC
CAAAACATGGGAGCAATGGGCATGGGGATGGGCAATACCAGTTT

CGGCAATTTGCAACAACCCACTATGCAAATGCAATATCAAAACCA
GCAAATGATGCAAACCCCGCAAATGATGCAAAACCAGCAAATGC
CGTTTAACCAGAGTGGCAGTTTCAGTGGGGTTGGCATGATGCCT
ATGCAGCACCAAAACATGGGAGCAATGGGCATGATGAATGTTGG
CAGTACCAGTTTTGGCAATTTGCAACAATCCAATATGCAAATGCA
ACATCAAAACCAGCAAATGATGCAAAACCCGC (SEQ ID NO:55)

### SYV80 (AF273736)

ACAAAAATTAATTGGCCGTGACGAAAACAATATCATCAACC
ACAGAAAAGTGAACTGCTCCAGCGCTGCAATGTTGATGCTGTTG
ATGACCATTTTCACTGTGCTGTCCAATGTCAACGAGGTAGCAGC
CGAGGAGCACGAGCTGGTCAGCCGCATTAAGCGCAACGGCTAC
GGATACAGCAGCGGTTATGGTGGTGGCTGTTCAGCCTGCCAAA
GCAGCTGCACCACCTGTGGTTACACATCCTACCATACCGTGCCT
GCGGCTCCCGCTCCCCCGCCACCACCGCCGCCTCCGCCTCCGC
CTGCGCCCGTCTACAGCT (SEQ ID NO:56)

### SYS56 (AF273731)

CAAATTTTTGCATATAACCAAAAAAAAATGCATTTCCAAACT
GCCTTCCTTCTCCTGGTGCCGTTCATTGTTGCCAACATTGTGTTG
GCTGACATGCCCAAAGATGATGCGCCACGCCCGGCGGTGCAAA
TCCGCACCCGTCGTTCCGTGTATGTGGCAATGATGGACGGCATC
GGCGTCACCAAGGAGGCCATGAGATATGCATGCCGGGGATGGC
AAACGAGCTTCTGCACAAAGTTTGGCACTACGCTGGCCGTTGAG
CGTACACGACGTGACACAAATGCCGAGATGATGGGTACCAAGAT
GACTGCCAATGCCGAGCGGAGGCAAAGAAGGAAATGAATGGC
GAGATGGGTGCCAAGAAAGAAATGAATGGCGAGATGATGAACAC
CAAGAAAGACACGAATGCCGAAGTGATGGGCACCAAGACGAAT
GCTAACCCCGAGCGGACACCAAGATGGACGCCGTGGTGATNA
ACACCAAGACGAATGCCAACCCCGAGCGGACACCAAGACGGA

AGCGAATGCTGTCATGGTGAACACCAAGACGGATTCTGCCAACC
CCGAGCGGGACACCAATACCGACGCCGTCATGGTGAACACCAA
GACAAATGTTAACCCCGAGCGGTACACCAAGATTG (SEQ ID
NO:57)

### SYV84 (AF273737)

AAGAATAACATGAAAATAATAACTCTAATTCCAATCTTATTT
TCATTAATAAATTCGGTAGTTGGACAAACAACATGCGCAACACCG
ACAACACCCCCAGTTTGTTACTGTACATGTGAATCAACACCTGGA
GTGTCATCAACAGCACCAACAACGACAGTTGGACCAACAGGAAC
AACAACAACTGGCACACCATTAACAACTGGACAGGGCACATCTG
GACCATNTACACCAGCACCAACAGGCACTCCCGGACAATCTACA
ACAGCACCAACAGGCACGCCTGGACCATCAACACCAGCACCAA
CTGGCACGCCTGGACCATCTACACCAGCACCAACAGGCACACC
CGGACCATCAACACCAACACCAACAGGCACGCCTGGACCATCTA
CACCAGCACCAACAGGAACACCCGGACCATCAACACCAGCACC
AACAGGCACGCCTGGACCATNAACACCAGCACCAACAGGCACG
CCTGGACCATCTACACCAGCACCAACAGGCACACCCGGACCATC
AACACCAGCACCAACAGGCACGCCTGGACCATCTACACCAGCAC
CAACAGGCACACCCGGACCATNAACACCAGNAC (SEQ ID NO:58)

### SYV42 (AF273735)

TGTCGACAGATTTCAAACTTGATACTTGTTGTTGTTGAACA
TATTTCTATACCTCACAGTTCGTCCACATTTTGACTTTCGGACAAA
AACAAAAGACAAAAAACGGTGGCAATGATCTCCAGGAGGGCAAT
TATTATTTGGGCCNTTGCTTTGTTGGCATTAGCAGCGATTTCGCC
CAACTTTTCCACCGCTGACAAAGGAGTTGACGCTGTCGATGCGG
NGGACGAAATCATTGACGACCCAAAAGTGGAGGTGCCGAAGAA
CGGCGTTGGCAAAGGCACCGATGACCAAACAGTGCAGTGGGAG
GAGGAGGCCATCAAACTGGAGGGACTATCAGTGGCCGAGTTCA

AGCAGCTCAGGGAGAGTGCGGAGAAGCATCAATTCCAAGCCGA
GGTCAACCGGATGGTGAAGCTGATCATCAATTCATTGTACCGGA
ACAAGGAGATTTTCCTCCGTGAGCTCATCTCCAACGCGTCGGAT
GCCCTGNACAAAATTCGGCTCATTTCGCTGACAAATTCGACAGC
ACTTGCGGCCACCGAAGAATTGTCCATCAAAATTAAGGCCGACA
NAGAAAATCACATTTT (SEQ ID NO:59)

### SY20 (AF273729)

GTCGNAAATTGCAATGCCTTCATTTAAATTTGTATGTTTCC
AATTTTTGCTAATTTTTGTGCTCACGGAATTGGCACATTCAGAGA
AAGGGTGCCCAAAAACAGAAGATTTAATTGAAATGACCAAACATT
TGCTGACAACTGGGAAGAATGTAATTTCTGGTGAAGCAGCTTCAT
CATCCACCGAAGGAAAGTGCAGTGATACGGTAATGGAAAGTGTT
GTAACAAAAGTTATTGAATTTGGGTGTTATGACGAAATGGAAAAG
AATGACGGCGAAAGAGCGAAAAGCAAAACGAAAAACGCATTTAT
TGAAAGCATTGAAACACAACGAAAAGGTTTTTGCCAACTTTGGCA
AAACGGCAAAAGCATTGGGGAGAAGGAGCAAAAAGATGGCATTT
TGAAAATAGTGAAAGGATTGGGAGGAATTGAGCTGAAGAAAATT
GAACGAATTCTTGAAAATGTTCAAAACATTGGATTATCGCC (SEQ
ID NO:60)

### SYV46/2B10 (AF273728) CLAVATA3

AATATTAAAAGATCCGAAAAAATGCCAAACATTTTCAAAAT
CCTTCTGATTGTGCTTTTGGCCGTCGTCTCATTCCGTCTCTCGGC
TTCTACTGGTGACAAAAAAACTGCTAATGATGGGAGTGGAAACAA
CTCATCAGCTGGGATTGGTACGAAGATCAAAAGAATTGTCACCG
CTGGACTGCTCTTCACTTCCCTGGCGACGGGTGGGGCGGAAGT
GATTGGGCGAAGCAATGCTCAGGGAGGAAATGCCGCCGGATTG
GTGCCATCGCATGTGACCAATCGCTCAATGGCTCCACCACCTCC

TCCTGTGCAATTTGAAATGGGGGCAAATCGATTAGAAAAAATGAG
GGCACACCTACGCG (SEQ ID NO:61)

### SYS7 (AF159590)

AGTCCTAAAGAAAAACGTTCAAATGTTTCGCTTTTCGCTGC
TTCTGCTGCTTGTCCCTCTCGTCCGCTGTGTCCCCAACCCAAAG
CCTTTGTTCGGAATTGGCATCGGTCGCAAGCAAAGCGCGGGAG
CGGAGGGGAAGCTGACCTGCGCTGGAGAGCCGTTGGCGGATGT
GAAGGTGAAACTGTATGATGACGACCGCGGGGTGGACACGGAT
GATCTGATGGGCGAGACGAGAACGGA
CTCAGAGGGACGCTTCAGATTGGAGGGGTACACGCACGAAATC
ACCACCATCGACCCCAAAATTAACATTTATCACGACTGCAACGAC
GGGCTGAAGCCATGCCAGCGCAAAATCTCAATTATGATCCCGGA
CAAATACATCGCCTCGGGCGAACATCCCAATACGTATTACGACG
CGGGCACAGTCGAATTGGAGGGCAAATTCAGTGGCGAGACAAG
GGACTGTCTGCACTAATTGCATTGGAACTGATCATTTGGCACTTT
CTGATCAGTTGGGACTGACCAATTGGAACTCTCTGCACTGATCA
CTTTCCCATTTTTGTTTCTGTTTTGTCACCGATTTTCCCCAAAGTC
TTACACCACCCAATGTGAATTTGCATAAATTTGTCCGCAAAAAA
AAAAAAAA (SEQ ID NO:62)

### SYV55 (AF159591)

AACAAAACAAAACGAACATTTGCCAGCAGTTGGCTAGGAC
AACGGCCCAAAGAGAAGTGAAGTGAATTGCCTCTGCTCTGAACA
CTGATGATGATGGTTCGTTCTTCCTCCTCTTCCTTTTCCCTTCCG
TCGTACTTCCTTTCTCTCCTCCCCCTCCTTCTCCTCCTTTTCTCCC
AATGTTCCCAAATTTCTTTTGCTTGCCAATGCCAATGGGCCCCGC
CAAAGGACAATTATTGTTCCTCCGATTGGGTTGCCCACGTGCAG
GTGATCAAACGACAGGACGGCGTGCGAATGCCGGCGGGGATCA

CCGACCGACAGACGGACTTAAACTCGCGACACGAAGTCAAATAT
TTAAGGATGTTTAAGATCAGCAAACAAATGCCGGTCAATCAGCAG
AACCAAGTGATTCTCCCTGTCAATGTTTATACGGCCACCGAGGAT
GCCGCCTGTGGCATTTTGCTCGAGTCGGGACACCAATATTTGTT
GGCCGGCGATTACGTCAACGGCACAATGCTCACCGGACTGTGC
CGGCAAATTCTGCTCGAAGACCTTAAGGAGTCACGAAAGCACGA
CATTCTCGAGTGGACAGAAGTGCCGCAAAAGCTGAAAGAACAGC
TGGAAAAGCAGGAATTTGATCAGAAATGCAACTGAACAGAATGG
AAAAAGAAAGAGAGGACAAACAATTGCAAATGAATGATCAGAAAA
ATCAAACGGACAGAGAAATGGCCCAATCGGGCACTTTTTGCTGA
ACGAACATTTCCAATAAACGACGAATAAATTTTTACCGGGTTAGC
CCGTGAAAAACGACTTGCTTGTAAAGGTTATTTGCTGCTTATTTA
AAAAAAAAAAAAAAAAAA (SEQ ID NO:63)

A summary of the nematode parasitism genes (SEQ ID NO: 1-51) targeted for inhibition is provided in Table 1. The GenBank accession numbers of each targeted gene is included in the table as well as the location of expression.

**Table 1. Summary of parasitism genes encoding proteins preceded by a signal peptide for secretion and expressed exclusively within the esophageal gland cells of Heterodera glycines**

| Clone^{■} | Accession no. | FL /ORF (bp)^{b} | Highest protein similarity | | Gland expression^{c} | | |
|---|---|---|---|---|---|---|---|
| | | | | BLASTP score/E value | Pre-J2 | Par-J2 | J3-A |
| 2A05 | AY028639 | 683/439 | MI-MSP-1- *Moloidogyne incognita* | 114H1e⁻²⁴ | SvG | SvG | SvG |
| 2B10 | AF273728 | 607/420 | Gland cell protein - *H. glycines* (SYV46) | 0 | -° | DG | DG |
| 2D01 | AF469057 | 711/558 | Pioneer | | - | DG | DG |
| 3B05 | AF469058 | 585/423 | CBP - *H. glycines* | 35/.19 | - | SvG | SvG |
| 3D11 | AF468679 | 1120110533 | Chitinase *- Caenorhabditis elegans* | 27412.7⁻²¹ | - | SvG | SvG |
| 3HO7 | AF473831 | 571/318 | Ubiquitin extension - *Nicotiana tobacco* | 13615e⁻³² | DG | DG | DG |
| 4D06 | AF469063 | 750/615 | Pioneer | | - | DG | DG |
| 4D09 | AF469061 | 738/501 | Pioneer | | DG | DG | DG |
| 4E02 | AF473826 | 449/279 | Pioneer | | SvG | SvG | SvG |
| 4F01 | AF469059 | 1174/1028 | Annexin - C. *elegans* | 242/4e⁻⁶³ | - | DG | DG |
| 4G05 | AF473830 | 926/765 | Pioneer | | - | DG | DG |
| 4G06 | AF469060 | 613/360 | Hexaubiquitin - *Helianthus annuus* (85% identity to 3H07) | 151/1e⁻³⁵ | - | DG | DG |
| 4G12 | AF473827 | 621/417 | Pioneer (91 % identity to 2B10) | | - | DG | DG |
| 5D06* | AF469062 | 1937/1470 | Pioneer | | - | DG | DG |
| 5D08* | AF473828 | 693/441 | Pioneer | | - | DG | DG |
| 6E07* | AF473829 | 10461645 | Pioneer | | - | DG | DG |
| 6F06 | AY043224 | 133311059 | Cellulase - *H. glycines* | 601/1e⁻¹⁷⁰ | SvG | SvG | - |
| 7E05 | AF500023 | 518/330 | Pioneer | | - | DG | DG |
| 8H07* | AF500024 | 1457/1197 | SKP1-like protein *- Arabidopsis thaliane* | 94/3e⁻¹⁸ | - | DG | DG |
| 10A06* | AF502391 | 1239/927 | RING-H2 zinc finger protein - *A. thaliana* | 5013e⁻⁰⁵ | - | DG | DG |
| 10A07* | AF500021 | 837/729 | Pioneer | | - | DG | DG |
| 10C02* | AF500017 | 449/279 | Pioneer (92% identity to 4E02) | | - | SvG | SvG |
| 11A06 | AF500015 | 673/561 | Pioneer (91% identity to 2D01) | | - | DG | DG |
| 12H04 | AF490244 | 1908/1614 | Pioneer | | DG | DG | DG |
| 13A06* | AF500020 | 899/669 | Pioneer (95% identity to 6E07) | | - | DG | DG |
| 13C08 | AF469055 | 1101/1002 | *Cellulase - H. glycines* | 27011e⁻⁷¹ | SvG | SvG | - |
| 16B09 | AF490246 | 676/555 | Pioneer | | - | DG | DG |
| 17C07 | AF520566 | 957/792 | Pectate lyase - *H. glycines* | 461/e-¹²⁹ | SvG | SvG | - |
| | | | | | | | |
| 18H08 | AF490248 | 632/399 | Pioneer | | - | DG | DG |
| 19B10 | AF490249 | 782/665 | Pioneer | | - | DG | DG |
| 19C07 | AF490250 | 660/333 | Pioneer | | - | DG | DG |
| 20E03 | AF490251 | 654/579 | Pioneer | | - | SvG | SvG |
| 20G04* | AF500022 | 816/648 | Pioneer (95% identity to 10A07) | | - | DG | DG |
| 21E12* | AF500028 | 439/354 | Pioneer | | - | DG | DG |
| 22C12 | AF500029 | 676/549 | Pioneer (92% identity to 16B09) | | - | DG | DG |
| 23G12 | AF500033 | 605/321 | Pioneer | | DG | DG | DG |
| 24A12 | AF500034 | 598/441 | Pioneer | | - | DG | DG |
| 25A01 | AF500019 | 750/528 | Pioneer | | - | DG | DG |
| 25G01 | AF006052 | 1600/1428 | *Hg-eng-1-H. glycines* | 0 | SvG | SvG | - |
| 26D05 | AY101191 | 1125/1008 | *Cellulase-Pratylanchus penetrans* | 263/2e⁻⁶⁹ | SvG | SvG | SvG |
| 27D09* | AY101190 | 851/708 | Pioneer (86% identity to 10A07) | | - | DG | DG |
| 28B03 | AF500025 | 1500/1302 | Pioneer | | DG | DG | DG |
| 29D09 | AF500016 | 757/615 | Pioneer (95% identity to 4D06) | | - | DG | DG |
| 30C02 | AF502393 | 537/492 | Pioneer | | - | DG | DG |
| 30D08* | AF500027 | 443/384 | Pioneer (82% identity to 21E12) | | - | DG | DG |
| 30E03 | AF500035 | 675/558 | Pioneer (98% identity to 16B09) | | - | DG | DG |
| 30G12 | AF500018 | 881/717 | Pioneer (93% identity to 4G05) | | - | DG | DG |
| 32E03* | AF500036 | 701/588 | Pioneer | | - | DG | DG |
| 33A09 | Ay125963 | 461/270 | Pioneer | | DG | DG | - |
| 33E05 | AF502392 | 684* | Pioneer | | DG | DG | - |
| 34B08 | AF500037 | 974/735 | Pioneer | | DG | DG | DG |
| 45D07 | AF520565 | 928/819 | Chorismate mutase - *Globodera pallida* | 276/2e⁻⁷³ | DG | DG | DG |

| | | | | | | | |
|---|---|---|---|---|---|---|---|
| ^{a}Clones with an asterisk encode secretory proteins with predicted nuclear localization signals. ^{b}Size of the full-length clone with predicted open reading frame (ORF) size; * indicates not full length.. ^{c}In situ hybridization of cDNA probes to mRNA specifically within the single dorsal esophageal gland cell (DG) or the two subventral esophageal gland cells (SvG) in pre-parasitic second-stage juveniles (Pre-J2), parasitic J2 (Par-J2), or parasitic J3, J4, or young adult stages (J3-A) of *Heterodera glycines.* Novel transcript with no homology to any genes in the public databases. ^{e}Not detected ^{f}Percent identity in the amino acid residues of predicted protein. | | | | | | | |

### Example 2: Heterodera glycines parasitism genes have homologs in other cyst nematodes

This example provides data that the soybean cyst nematode (SCN), *H. glycines,* parasitism genes presented in this application have homologs in the beet cyst nematode (BCN), *Heterodera schachtii,* as evidence of the existence of homologues of the *H. glycines* parasitism genes in multiple cyst nematode species.

### Materials and Methods:

### Nematode culture

*Heterodera schachtii* were propagated on roots of greenhouse-grown cabbage. Eggs were collected as previously described (Goellner et al. (2000) J. Nematology. 32:154-165). To collect pre-parasitic second stage juveniles (pre-J2s), eggs were hatched over water at 28°C on a Baermann pan. Different parasitic stages of *H. schachtii* were collected by root blending and sieving (Ding et al. (1998) Mol. Plant Mircrobe Interaction. 11:952-959) of infected plants.

### RNA extraction

Frozen mixed parasitic stages of *H. schachtii* pellets were ground with Lysis Matrix D beads (Q-Biogene, Irvine, CA, USA) and liquid nitrogen by placing in a mini beadbeater (Biospec Products Inc. Bartlesville, OK). Total RNA was extracted using the Micro-Midi Total RNA purification system (Invitrogen, Carlsbard, CA, USA) following the manufacture's instructions including DNAse digestion with DNAsel.

### Isolation of parasitism H. schachtii cDNA clones

cDNA clones SYV46 (AF273728), 4EO2 (AF473826), 5DO8 (AF473828) and 4FO1 (AF469059) were originally isolated from expressed sequence tags (ESTs) analyses of a cDNA library constructed from mRNA derived from esophageal gland cells of mixed parasitic stages of *H. glycines* (Gao et al. (2003) Molecular Plant-Microbe Interactions. 16:270-276; Wang et al. (2001) Molecular Plant-Microbe Interactions. 14:536-544). To obtain the full length cDNA homolog of SYV46 clones in *H. schachtii,* 3' and 5' cDNA ends were amplified from total RNA using GeneRacer kit (Invitrogen, Carlsbard, CA, USA). 5' RACE was performed using GeneRacer 5'primer and corresponding SYV46 GSP (Table 2) with RACE-ready first strand cDNA template. 3' RACE was performed using Gene Racer 3' primer (oligo dT) and corresponding SYV46 GSP. The RACE products were cloned into pCR4-TOPO vector (Invitrogen, Carlsbard, CA, USA) for sequencing.

cDNA full length clones 4EO2, 5DO8 and 4FO1 was obtained by 3' RACE system. First strand cDNA synthesis was initiated at the poly (A) tail of mRNA using the adapter primer (AP) (Invitrogen, Carlsbard, CA, USA). Amplification of full length cDNA was performed using corresponding 5' GSP (Table 2) and the abridged universal amplification primer (AUAP) (Invitrogen, Carlsbard, CA, USA) homologous to the adapter sequence used to prime the first strand cDNA synthesis. The Race products were cloned into pCR4-TOPO vector (Invitrogen, Carlsbard, CA, USA) for sequencing.

**Table 2: Primers used in amplification of H. schachtii cDNA parasitism genes**

| **Name** | **Sequences 5' to 3'** |
|---|---|
| HgSYV46-1 | CATTTCCTCCCTGAGCATTGCTTA (SEQ ID NO: 64) |
| HgSYV46-2 | TTGTCACCGCTGGACTGCTCTTCACTT (SEQ ID NO: 65) |
| Hg4EO2 | ATGTCCCTTTTCCGTCC (SEQ ID NO: 66) |
| Hg4FO1 | ATGCTCCAAAACGGCCTTAC (SEQ ID NO: 67) |
| Hg5DO8 | ATGTTCAGCTC TTCCAATTTG (SEQ ID NO: 68) |

### Sequence analyses and alignment

Sequence comparison with homologs in *Heterodera glycines* was carried out using NCBI sequence local alignment program www.ncbi.nlm.nih.gov/blast/bl2seq/wblast2.cgi (Tatusova and Madden (1999) FEMS Microbiol. Lett. 174:247-250). Percentage nucleotide and amino acid identities between the two sequences were compared. Protein sequence comparisons of the *H. schachtii* parasitism gene products with others in the database were conducted using NCBI Blastp server http://www_{.}ncbi.nlm.nih.gov/BLAST/.

### Results:

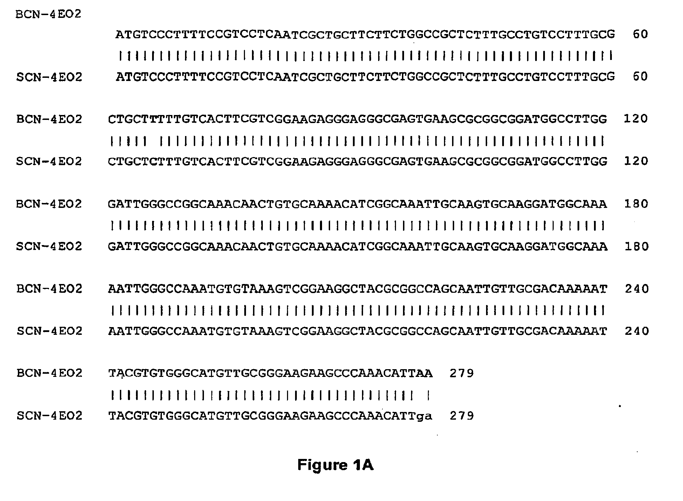

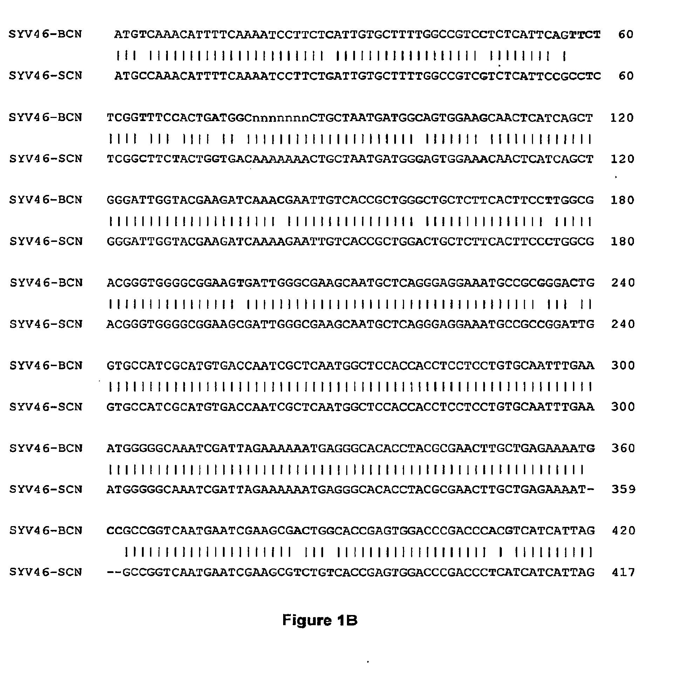

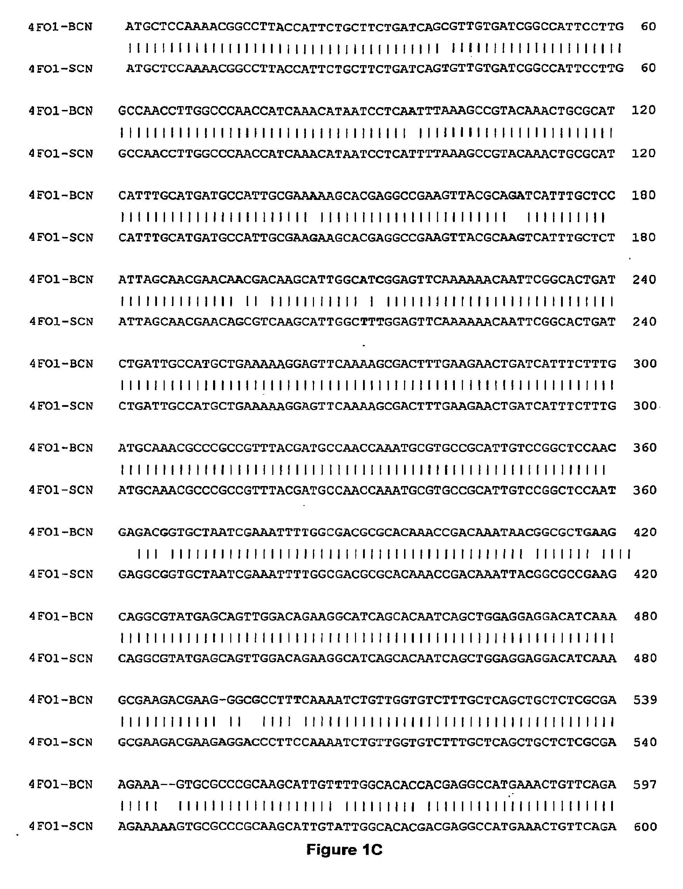

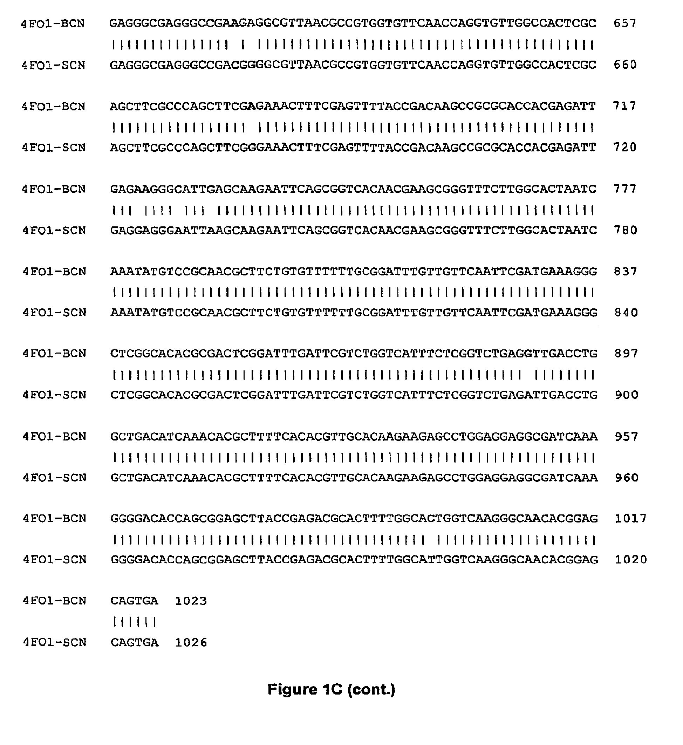

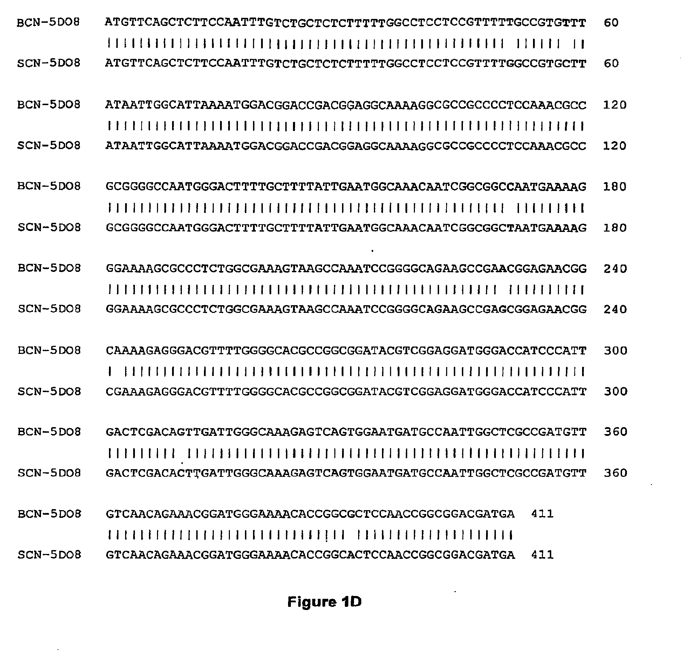

Isolation of *H. schachtii* cDNA homologs of *H. glycines* parasitism genes showed a high (> 93-99%) nucleotide identity (Table 3, Figures 1A-D) with the corresponding soybean cyst nematode (SCN) parasitism gene. The *H. schachtii* cDNA clones also gave a high protein identity with the SCN homologs and similar protein similarities using BLASTP. The data suggests that BCN possess similar parasitism genes as SCN and can therefore be used as a model system to determine putative functions of these genes in *Arabidopsis thaliana* which is a host for *H. schachtii.*

**Table 3: Comparison of BCN (H. schachtii) parasitism gene homologs. High nucleotide and amino acid identities with the SCN (H. glycines) parasitism genes were observed**

| SCN Clones | NCBI Accession Number | Full-length cDNA BCN homologs % nucleotide identity | Full-length BCN homolog % amino acid identity | BLASTP of BCN homolog |
|---|---|---|---|---|
| 4FO1 | AF469059 | 97% | 93% | Annexin, C. *elegans* |
| 5DO8 | AF473828 | 98% | 96% | Pioneer |
| 4EO2 | AF473826 | 99% | 100% | Pioneer, Nuclear localization signal (NLS) |
| SYV46 | AF473827 | 93% | 92% | CLAVATA, *Arabidopsis thaliana* |

### Example 3: Localization of parasitism gene transcripts and products within Heterodera schachtii nematodes

This example provides data regarding the localization of expression of parasitism genes and their products in *H. schachtii,* which provides further evidence of parasitism gene homology among the cyst nematodes.

### Materials and Methods:

### In-situ hybridization

In situ hybridizations were performed on fixed mixed parasitic stages of *H. schachtii* (De Boer et al. (1998) J. Nematology. 30(3):309-312). Specific forward and reverse primers for each cDNA clone were used to synthesize digoxigenin-labeled sense and antisense cDNA probe by asymmetric PCR (Wang et al. (2001) Molecular Plant-Microbe Interactions. 14:536-544). *In situ* hybridization was performed with mixed parasitic stages of *H. schachtii as* described by De Boer et al., (1998). After hybridization, specimens were observed under a light microscope to reveal cDNA probes that hybridize within nematode esophageal gland cells (Wang et al., 2001, De Boer et al., 1998).

**Table 4: Primers used to make DIG-labeled cDNA probes**

| Name | Sequences 5' to 3' |
|---|---|
| HsSYV46-1 | CAATGCTCAGGGAGGAAATG (SEQ ID NO: 69) |
| HsSYV46-2 | CACTCGGTGACAGACGCTTA (SEQ ID NO: 70) |
| Hs4FO1-1 | AAGCAGGCGTATGAGCAGTT (SEQ ID NO: 71) |
| Hs4FO1-2 | GTCGTGTGCCAATACAATGC (SEQ ID NO: 72) |
| Hs5DO8-1 | CGGCTAATGAAAAGGGAAAA (SEQ ID NO: 73) |
| Hs5DO8-2 | TGGCATCATTCCACTGACTC (SEQ ID NO: 74) |
| Hs4EO2-1 | GTCCTCAATCGCTGCTTCTT (SEQ ID NO: 75) |
| Hs4EO2-2 | TCAATGTTTGGGCTTCTTCC (SEQ ID NO: 76) |

### lmmunolocalization

Polyclonal antisera to the selected *H. glycines* parasitism gene products were produced by immunizing rabbits with two synthetic peptides from Eurogentec. Purified polyclonal antibodies were used to localize translated parasitism gene products within pre-fixed mixed parasitic stages of *H. schachtii* (Wang et al. (2005) Molecular Plant Pathology. 6:187-191). Immunodetection of the nematode specimens was observed by indirect immunofluorescence microscopy using fluorescein isothiocyanate (FITC)-conjugated anti-rabbit second antibody.

**Table 5: Synthetic peptide sequences of cyst nematode parasitism gene sequences used to produce Polyclonal antibodies**

| Parasitism gene | Synthetic Peptide sequence |
|---|---|
| SYV46 | STGDKKTANDGSGNN (SEQ ID NO:77) |
| | PVNESKRLSPSGPDPH (SEQ ID NO:78) |
| 4FO1 | EEDIKAKTKRTLPKS (SEQ ID NO:79) |
| | KGLGTRDSDLIRLVI (SEQ ID NO:80) |
| 5DO8 | SKPNPGQKPSGERRK (SEQ ID NO:81) |
| | VNRNGWENTGTPTGGR (SEQ ID NO:82) |

### Results:

The tissue localization and developmental expression patterns of *H. schachtii* parasitism genes were analyzed in various life stages of *H. schachtii* by in-situ hybridization. The digoxigenin-(DIG)-labeled antisense cDNA probes of the selected parasitism genes specifically hybridized with transcripts within the esophageal gland cells of *H. schachtii* parasitic stages. SYV46, 5DO8 and 4FO1 probes hybridized within the dorsal gland cells of parasitic stages (Parasitic-J2, J3, J4) whereas 4EO2 DIG probes specifically bound to the subventral gland cells of both parasitic and pre-parasitic stages of *H. schachtii.* No Hybridization signals were detected with the control sense cDNA probes of each parasitism gene. Polyclonal antisera raised to *H. glycines* SYV46, 5DO8, and 4FO1 parasitism gene products (proteins) were shown to bind to only to the secretory granules of the esophageal glands of parasitic stages of *H. schachtii.*

The collective data demonstrate that homologous parasitism genes exist between *H. glycines* and *H. schachtii* and that they are expressed in the same pattern between the two cyst nematode species. The data indicate that *H. schachtii* uses similar parasitism genes to infect host plants and suggest that the host plant, *Arabidopsis thaliana,* may be used to assess both *H. glycines* and *H. schachtii* parasitism genes as a model host plant. The strong identity between *H. glycines* and *H. schachtii* parasitism genes indicates that other cyst nematode species contain significantly similar parasitism genes.

### Example 4: Expression of the Heterodera glycines cyst nematode parasitism gene 23G12 in soybean hairy roots affects root growth

The effects of cyst nematode parasitism gene products secreted into plant cells can be assessed by expression of each parasitism gene in transformed plant tissues. Both soybean hairy roots (Cho et al. (2000) Planta. 210:195-204); Doyle & Lambert (2003) Mol. Plant-Microbe Interact. 16:123-131) and whole *Arabidopsis thaliana* plants (Wang et al. (2005) Molecular Plant Pathology. 6:187-191) can be transformed with constructs designed to express individual nematode parasitism genes constitutively with promoters like CaMV 35S (Benfey and Chua (1990) Science. 250:949-966) or the Glycine max ubiquitin (Gmubi) promoter (Finer (2006) In Proceedings of the International Symposium of Plant Biotechnology, Institute of Biotechnology, Yeungham University, Korea, pp 17-22). The results presented in Wang et al. (2005) indicate that constitutive expression of HG-SYV46 in wild-type *Arabidopsis thaliana* plants results in consumption of the shoot apical meristem to form a WUSCHEL phenotype that is identical to results obtained by constitutive expression of the plant CLV3 peptide Cyst nematode parasitism genes homologous to known plant genes can also be expressed as described here in available plant mutants for homologous genes to rescue the mutant phenotype as a demonstration of parasitism gene function in plants (Wang et al. (2005)). The results presented in Wang et al. (2005) indicate that constitutive expression of HG-SYV46 in *clv-3* mutant *Arabidopsis* rescues the aberrant floral phenotype of *clv*-3 mutant plants to produce the floral phenotype of wild-type *Arabidopsis* further indicating that HG-SYV46 has a function in plants similar to the plant CLV3 peptide.

Soybean hairy roots have been demonstrated as a successful system to express nematode parasitism genes and obtain an observable phenotype that can indicate the affect of that nematode parasitism gene product on host plant cells (Doyle and Lambert (2003) Mol. Plant-Microbe Interact. 16:123-131). This soybean hairy root system is applicable to nematode parasitism genes with identifiable homologs and to nematode parasitism genes like HG-23G12 (Gao et al., (2003) Mol. Plant-Microbe Interact. 16:270-276) that have no identifiable homolog in public gene databases.

### Materials and Methods:

### 23G12 hairy root gene construct

PCR amplification of full length 23G12 *H. glycines* parasitism gene (AF500033) was performed to generate flanking restriction sites for the enzymes *Bam*HI (5'underlined) and *Sst*l (3'underlined) using the following primers:
a) 5'-AAGGATCCATGCGCACTTTTCTGTTC-3 (SEQ ID NO:83) and
b) 5'-AAGAGCTCTCACCATTTTAAGGCTTGC-3' (SEQ ID NO:84).

Reactions were run for 30 cycles and consisted of the following sequence: 94°C for 2 min, 56°C for 1 min, and 72°C for 1 min. The cycles were preceded by a 94°C denaturation period for 4 min and followed by 72°C final extension period for 7 min. A PCR product of 0.3 kb was digested with *Bam*HI and *Sst*l and subcloned into pBI121 containing the 35S promoter. This vector contains the appropriate border sequence to aid in the transfer of T-DNA into the plant genome and kanamycin-resistance selectable marker to allow for selection of transgenic hairy roots. Restriction analysis and identification and fidelity of the gene construct were confirmed by DNA sequencing.

### Generation and analyses of 23G12 expression in soybean hairy roots

The binary vector pBI121 containing 35S:23G12 gene was introduced into *Agrobacterium rhizogenes* K599 cucumopine strain using electroporation (Savka et al. (1990) Phytopathology. 80:503-508). Plant inoculation was conducted according to Cho and associates (2000), with some modifications. Two weeks after root emergence, 1- to 2-cm-long root tips were transferred and were freed from bacteria by passages on MXB medium (MS (Murashige, T. and Skoog. (1962) Physiol. Plant. 15:473-497) basal nutrient salts, B5 (Gamborg et al. (1968) Exp. Cell Res. 50:151-158) vitamins, and 3% sucrose (pH 5.7)), solidified with 3 g of phytagel per liter. Carbenicillin (500 µg/l) was added to inhibit the growth of *A. rhizogenes* and Kanmycin (50 µg/l) was added to select for transformed roots. To identify putatively transformed hairy roots, total DNA was extracted as described by Edward et al. (1991) Nucleic Acid Res. 19:1349. PCR was performed using the following internal primers: Forward: 5'-AAGAATTCACAGGGAAAAATTCGTGAC-3' (SEQ ID NO:85) and reverse: 5'-AAGGATCCTTCACCATTTTAAGGCTTGC-3' (SEQ ID NO:86) and the PCR program described above. PCR product was electrophoresed on an agarose gel using standard techniques. To evaluate transgene expression, total RNA was extracted with RNeasy Mini kit (QIAgen, Valencia, CA) according to the manufacturer's directions. The extracted RNA was treated with DNAse I, according to the manufacturer's instructions for severely contaminated RNA (DNA-free, Ambion Inc., Austin, TX, U.S.A.). RNA (1 µg) was then reverse-transcribed using the Superscript First Strand Synthesis System for RT-PCR (Invitrogen Life Technologies), according to the manufacturer's instructions for oligo(dT) priming. The resulting cDNA was then used as template for PCR amplification of 23G12 using the internal primers and the PCR program described above.

### Results:

PCR of total DNA from transformed soybean hairy roots using primers specific for the 23G12 gene was used to confirm the presence of this gene in kanamycin selected roots. Expression of the 23G12 gene in transformed hairy roots was conformed by RT-PCR analysis of extracted RNA. Soybean hairy roots that expressed the 23G12 parasitism gene showed a dramatic decrease in formation of lateral roots compared to control soybean hairy roots that did not express the 23G12 gene (data not shown).

### Example 5: Expression of H. schachtii 4FO1 cDNA in selected Arabidopsis mutants

The Arabidopsis annexin gene family is comprised of eight members, *Atann1-Atann8* (Cantero et al. (2006) Plant Physiol. Biochem. 44:13-24) and the nematode 4FO1 cDNA clones shows the highest nucleotide sequence similarities with Atann1. Annexins are a multigene family which are Ca²⁺ dependant binding membrane binding proteins. Several functions have been implicated for plant annexins including Golgi-mediated secretion of plasma membrane and wall material in plant cells (Clark and Roux (1995) Plant Physiol. 126:1072-1084). In addition annexins are thought to be involved in Ca²⁺ channeling and in enzymatic activities.of nucleotide phosphodiesterase and peroxidase (McClung et al. (1994) Biochem. J. 303:709-712). Recently the isolation and characterization of several *AnnAt* knock-out mutants have shown that AnnAt1 is possible involved in the osmotic stress response. The *annAt1* and *atann4* mutants showed hypersensitivity to ABA and osmotic stress induced by NaCI at 75 mM, while *annAt2* did not indicate association with osmotic stress and defection germination (Lee et al., (2004) Plant Cell. 16:1378-1391). In order to determine if the nematode annexin 4FO1 is similar in function to a plant annexin in Arabidopsis, a complementation assays was conducted.

### Materials and Methods:

### T-DNA insertion lines

T-DNA insertion mutants *annAt1* (SALK_015426), *annAt2-1* (SALK_054223), and *annAt4-3* (SALK_073121) were obtained from the Arabidopsis Biological Resource Center (Ohio State University, Columbus, OH). All the mutants and wild-type plants used were in Arabidopsis Col-0 background.

### Complementation of the annAt1 T-DNA insertion mutant

The binary vector, pB121 (Clontech, Palo Alto, CA) containing the β-glucuronidase genes was replaced by the nematode 4FO1 full length coding region. The construct was transformed into *Agrobacterium tumefaciens* strain GV3101 using the floral dip method (Martinez-Trujillo et al. (2004) Plant Molecular Biology Reporter. 22:63-70). Transgenic plants were selected on MS plates contain kanamycin (50µg/ml). PCR with genomic DNA from T1 segregating lines was used to confirm presence of the gene in putative transformants. PCR was conducted using 4FO1 internal primers (forward: 5'AGTTGGACAGAAGGCATCAGCAC3' (SEQ ID NO:87) and reverse: 5'AGAAGCGTTGCGGACATATTTGA 3')(SEQ ID NO:88) to amplify -400bp PCR product.

### Germination Test

Sterilized seeds were plated on MS sucrose (2%) agar medium supplemented with and without NaCl (75mM). Germination rates (%) of plants were scored 8 days post incubation.

### Results:

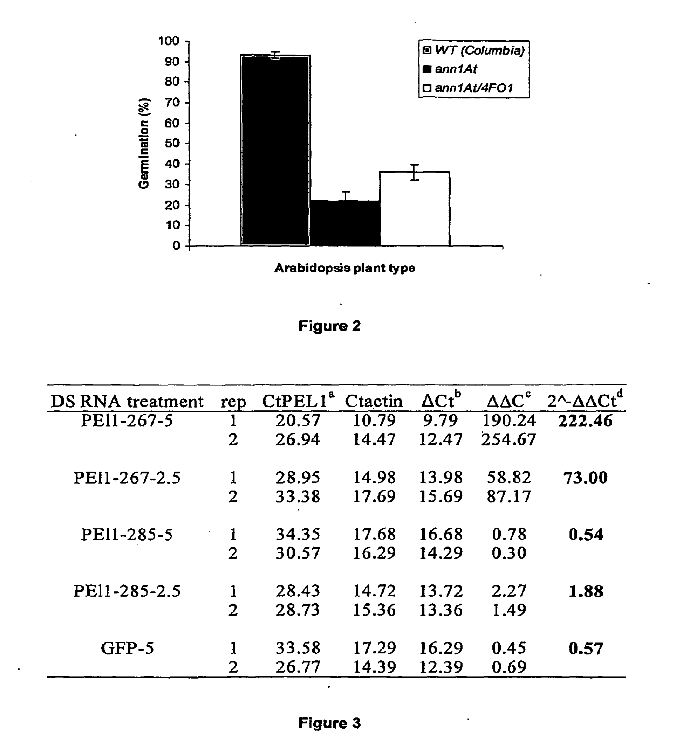

Amplification of an expected 4FO1 PCR product by PCR indicated that the transformation of the 4FO1 gene into the *annAt* mutants of *Arabidopsis* was successful. Transformation with the 4FO1 gene partially complemented the tolerance of the *annAt1* mutant plants to media conditioned with 75 mM NaCI (Figure 2).

The collective results of expression of cyst nematode parasitism genes in plant tissues indicate that the products of cyst nematode genes function in plant cells to alter plant cell maintenance and growth. The results are indicative of the function of each secreted cyst nematode parasitism gene product as it would affect the modification of plant cells during infection of plant roots by cyst nematodes.

### Example 6: RNA interference of a H. glycines parasitism gene by in vitro soaking in dsRNA

Evidence that RNAi to a target nematode gene could be induced by in vitro "soaking" of nematodes in dsRNA of complementary sequence to the target gene was first demonstrated in the bacterial feeding nematode, *C. elegans* (Timmons and Fire (1998) Nature. 395:854). An *in vitro* soaking system to induce ingestion of dsRNA by plant-parasitic nematodes in vitro was subsequently demonstrated to induce RNAi of target genes in the nematode gut and germline (Bakhetia et al. (2005) Mol. Plant-Microbe Interact. 15:099-1106; Urwin et al. (2002) Mol. Plant-Microbe Interact. 15:747-752). This *in vitro* RNAi system was adapted to demonstrate that RNAi of parasitism genes within esophageal gland cells of the root-knot nematode (Huang et al. (2006) Proc. Natl. Acad. Sci. 103:14302-14306; Rosso et al. (2005) Mol. Plant-Microbe Interact. 18:615-620) and potato cyst nematode (Chen et al. (2005) Mol. Plant-Microbe Interact. 18:621-625) could be induced by *in vitro* soaking of preparasitic second-stage juveniles (pre-J2) of each nematode species in dsRNA for ingestion. Evidence is provided herein that ingestion of dsRNA to a target parasitism gene in the esophageal gland cells of *H. glycines* can induce RNAI of the target gene as a result of *in vitro* soaking of pre-J2 of *H. glycines* in dsRNA.

Proof-of-concept that ingestion of dsRNA by *H. glycines* could cause specific RNA interference of a *H. glycines* parasitism gene expressed in the nematode's esophageal gland cells was conducted. The HG-pel1 pectate lyase gene of *H. glycines* (DeBoer et al. (2002) J. Nematology. 30(3)309-312) was chosen as the target parasitism gene for RNAi because the HG-pel1 gene is expressed exclusively within the subventral esophageal glands of *H. glycines* in the pre-parasitic juvenile stage. This allowed *in vitro* soaking of hatched *H. glycines* preparasitic J2 in solution augmented to facilitate the ingestion of HG-pel1 dsRNA and analyses of RNAi affects on treated J2 after ingestion of HG-pel1 dsRNA.

### Materials and Methods:

### Nematode culture and collection

*Heterodera glycines* inbred line OP50 was propagated on roots of greenhouse soybean (*Glycine max*) plants (Goverse et al. (1994) J. Nematology. 26:251-259). Pre-parasitic second-stage juveniles (J2) were hatched from eggs and extracted as described before (Goellner et al. (2000) J. Nematology. 32:154-165). Briefly, to isolate *H. glycines* eggs, the cysts were gently crushed in a glass homogenizer and the eggs were rinsed with water onto a 25 µm sieve. Nematode eggs were stirred in a solution of 0.02% sodium azide for 30 minutes, rinsed with water on a 25µm sieve, and then hatched over water and soybean root exudect at 28 °C on a Baermann pan. After two days, hatched second-stage juveniles (J2) were collected and rinsed with water on a 25 µm sieve and were resuspended in 100 µl water.

### HG-pel1 dsRNA synthesis

Plasmid containing a full-length cDNA clone of an *H. glycines* parasitism gene that encoded pectate lyase (DeBoer et al. (2002) J. Nematology. 34:9-11), HG-pel1 (AY026357), was used as template for RNA synthesis. The plasmid DNA was isolated using the Wizard Plus miniprep DNA purification system (Promega, MD, USA) and used as templates for PCR reactions using the primers shown below. PCR primers (immediately below) were designed to nucleotides 161-445 and 569-835 of HG-pel1 cDNA to produce products for dsRNA synthesis of 285 (ds285) and 267 (ds267) nucleotides, respectively. Green fluorescent protein (GFP) was used as a negative control for dsRNA soaking experiments since it is not present in *H. glycines.* This GFP vector pP114.108 (L3522) is from an enhanced version designed for *C. elegans* from Dr. Andrew Fire, Carnegie Institute of Washington. The PCR amplification was performed as described previously (Wang et al. (2000) J. Biol. Chem. 275:40174-40179) using the following cycle profile: 94 °C for 2 min, followed by 35 cycles of 94 °C 1 min, 69 °C for 40 sec, 72 °C for 1 min, and a final step of 72 °C for 10 min. The PCR products were purified using a Qiagen PCR purification kit and the quality and yield of the reaction was checked by agarose gel electrophoresis. Sense and antisense RNAs were synthesized in a single reaction in vitro using the MEGAscript RNAi kit (Ambion, TX, USA) according to manufacturer's instructions, except that the reactions were incubated for 16 hs to increased RNA yield. The amount and quality of generated dsRNA were estimated by ethidium bromide-staining and agarose gel following standard electrophoresis and quantitated by spectrophotometry. The dsRNA products were ethanol precipitated and resuspended in nuclease free water to a concentration of 10 -15 µg/µl.

### Hg-pel1 dsRNA soaking treatments

The dsRNA soaking protocol of Urwin et al. (2002) Mol. Plant-Microbe Interact. 15:747-752) was used with some modification. Ten-microliter aliquots of the nematode suspension containing 1000 J2 were soaked in dsRNA solution of HG-pel1 ds285, HG-pel1 ds267, or GFP ds269 at final concentrations of 2.5 and 5.0 mg dsRNA/ml, 50 mM final concentration Octopamine (Q-0250, Sigma), 0.2 mg/ml FITC Isomer I (F-7250, Sigma), 0.05% gelatin, 1mM Spermidine (S-2626, Sigma) and sufficient soaking buffer to make a 30 µl final soaking volume. Nematodes were soaked in dsRNA solution for 24 hrs at 28 °C to allow for ingestion of dsRNA and subsequent RNAi effects.

### RNA extraction, reverse transcription (RT-PCR) and PCR amplification

After dsRNA treatment, nematodes were thoroughly washed five times with nuclease free water by centrifugation using standard procedures. Total RNA from 1000 pre-parasitic J2 was isolated using the Rneasy mini Kit from Qiagen (Valencia, CA, USA) according to the manufacture's instructions. Trace amounts of genomic DNA were removed using the RNase-Free DNase set from Qiagen (Valencia, CA, USA) and the Turbo DNA-*free* kit (Ambion, TX, USA) and used in a single oligo-dT primed reverse transcriptase reaction using the High-Capacity cDNA Archive Kit (Applied Biosystems, Foster City, CA, USA) To insure that the RT-PCR amplicon originated from mRNA and did not result from DNA contamination, control reactions were included in which the reverse transcriptase was omitted (NRT). The resulting 60 µl cDNA reaction was stored at -80 C prior to analysis.

### Real time PCR experiments and product analysis

Endogenous mRNA levels were measured by two-step real-time PCR analysis based on SYBR Green detection with the ABI Prism 7000 Sequence Detection system (Applied Biosystems, Foster City, CA, USA) using the SYBR green PCR Kit, according to the manufacturer's instructions. qPCR primers to detect HG-pel1 were design using Primer Express (ABI) software. PCR assay was directed against segments of the target mRNA external to the segment targeted by the dsRNA. The subsequent real-time PCR reaction contained 3 µl of the cDNA in a total volume of 25 µl, consisting of 1x SYBR Green mix and 0.5U of AmpErase uracil N-glycosylate (Applied Biosystems, Foster City, CA, USA), 100 nM forward primer, and 100 nM reverse primer and water to 25 µl. The reactions were performed in the MicroAmp 96-well plate capped with MicroAmp optical caps Perkin-Elmer (Applied Biosystems, Foster City, CA). The PCR reaction was performed using the following program: 50°C for 2 min, 95°C for 10 min followed by 40 cycles of (95°C for 15 sec, 60°C for 1 min). Dissociation curves were carried out at the end of each run for the detection of nonspecific products in the amplification reaction. For each treatment, identical samples were run in triplicate. Quantification of the transcript level was also normalized to the expression of the *H. glycine* actin gene (Accession number AF318603). This housekeeping gene is constitutively expressed in many tissues and has been reported to be a useful internal control in other organisms. The following control reactions were included: PCR negative control without cDNA template (NTC) to confirm that there was no signal from nonspecific PCR products, and a no RT control (NRT) as previous described. Values are obtained from three independent treatment experiments. In order to prevent interassay variation samples with the same primer set were always amplified within one run. Each experimental treatment was run in duplicate. Data analysis, such as the determination of the Threshold cycle (Ct) which represents the starting point of the exponential phase of PCR, and graphic presentation were carried out using the Sequence Detection Software v.1.07 (Applied BioSystems). The amount of target gene relative to the ß actin endogenous control was determined using the ΔΔCt method (Livak and Schmittgen, (2001) Methods. 25:402-408) and expressed as percent of control transcript levels.

### Results:

Ingestion of FITC-labeled dextran by *H. glycines* preparasitic J2 was confirmed in J2 that were incubated in dsRNA soaking solution containing 50 mM octopamine. Greater than 220-fold reduction in Hg-pel1 transcript levels was detected by real-time PCR from RNA extracted from *H. glycines* J2 after 24-hour incubation in 5.0 mg/ml to ingest dsRNA (PEI-267-2) complementary to nucleotides 569-835 of Hg-pel1 cDNA (Figure 3). A 73-fold reduction in HG-pel1 transcript was similarly observed by J2 soaking in 2.5 mg/ml dsRNA267 (PEl1-267-2.5) that was indicative of a quantitative reduction in RNAi effect with reduced levels of dsRNA for ingestion by *H. glycines* J2. Targeting nucleotides 161-445 in Hg-pel1 cDNA for RNAi by soaking *H. glycines* in dsRNA285 at either 5.0 or 2.5 mg/ml (Pel1-285-5 and PEl1-285-2.5, respectively), however, produced no detectable effect on Hg-pel1 transcript levels, nor did soaking J2 in dsGFP (Figure 3).

Ingestion of dsRNA induced by soaking *H. glycines* J2 in vitro in solutions containing 50 mM octopamine induced specific RNAi of the targeted HG-pel1 transcript. The RNAi effect was dependent upon the targeted region of the HG-pel1 transcript complementary to the dsRNA and to the concentration of dsRNA in the soaking solution to be ingested by the nematodes.

### Example 7: Effects of nematode-inducible expression of in planta RNAi to cyst nematode parasitism genes on cyst nematode infection of roots of transformed plants

Expression of RNAi 'in planta' (a.k.a. host-derived gene silencing) to target genes can be developed using vectors designed to produce an intron-spliced hairpin dsRNA in transformed plant tissues (Wesley et al. (2001) Plant J. 27(6):581-90). The potential to disrupt the life cycle of plant-parasitic nematodes by in planta RNAi to a target nematode gene exists, but the choice of target genes is critical to obtaining a significant effect on the nematode. The expression of dsRNA to a target nematode gene can be driven constitutively in planta by promoters such as CaMV 35S (Benfey and Chua (1990) Science. 250:959-966) or Gmubi (Finer (2006) In Proceedings of the International Symposium of Plant Biotechnology, Institute of Biotechnology, Yeungham University, Korea, pp 17-22), or more specifically in nematode feedings sites for ingestion by the nematode by using promoters to genes such as NtCel7 (Goellner et al. (2001) Plant Cell. 13:2241-2255) that are upregulated in nematode feeding sites. Nematode genes that encode biological processes required for metabolism, viability, and reproduction represent a group of potential targets for in planta RNAi, and some success has been achieved (Steeves et al. (2006) Functional Plant Biology. 33:991-999); Yadav et al. (2006) Mol. Biochem. Parasitol. 148:219-222). A novel method to achieve in planta RNAi against nematodes does not target the genes underlying the basic metabolism of the nematode, but rather, disrupts essential secretions from the nematode required to sustain its obligate parasitic interaction with the host by in planta RNAi of the parasitism genes that encode those essential secretions. In planta RNAi to a root-knot nematode parasitism gene has successfully been demonstrated to provide plant resistance to root-knot nematodes (Huang et al., 2006). Given that root-knot nematodes and cyst nematodes share relatively few common parasitism gene sequences (Davis et al. (2004) Trends in Parasitology. 20(3):134-141), and that the majority of cyst nematode parasitism genes have no known homologue (Davis et al., (2004) Trends in Parasitology. 20(3):134-141), it is likely that in planta RNAi to cyst nematode parasitism genes represents a unique means to develop plant resistance specific to cyst nematodes. We present evidence below that in planta RNAi to several different parasitism genes unique to cyst nematodes provides plant resistance to cyst nematodes.

In planta RNAi to four parasitism genes of cyst nematodes (Gao et al., 2003; Wang et al., 2001), SYV46 (AF273728), 4F01 (AF469059), 20E03 (AF490251), and 23G12 (AF500033) was expressed in transformed *Arabidopsis thaliana* plants for analyses of effects on infection of in planta RNAi roots on *H. schachtii.* Constitutive expression of dsRNA to the 20E03 and 23G12 parasitism genes in planta was conducted with the Gmubi promoter (Finer (2006) In Proceedings of the International Symposium of Plant Biotechnology, Institute of Biotechnology, Yeungham University, Korea, pp 17-22), and nematode-inducible expression of the SYV46 and 4FO1 parasitism genes in planta was conducted with the NtCel7 promoter (U.S. Patent 6,906,241 ).

### Materials and Methods:

### Construction of RNAi vector

The CaMV 35S promoter of pHannibal (Wesley et al. (2001) Plant J. 27(6):581-90) was replaced at the Sacl and Xhol restrictions sites with the NtCe17 promoter (U.S. Patent 6,906,241 and where permissible, is incorporated herein by its entirety) that is upregulated in feeding sites of root-knot and cyst nematodes. The sense and antisense cDNA sequences of SYV46 (170bp) and 4FO1 (1023 bp) were amplified from full length cDNA clones. Gene-specific primers with restriction sites Xhol and Kpnl amplified products for the sense orientation and Gene-specific primers with restriction sites BamHI and Hindlll to amplify products in the antisense orientation (pHannibal-Ntcel7-SYV46p; pHannibal-Ntce17-SYV46p). The resultant vector was confirmed by sequences. The pHannibal constructs were sub-cloned at the Notl sites into a binary vector pART27 (pART27-Ntce17-SYV46p; pART27-Ntce17-4F01f1).

### Transformation of RNAI constructs in Arabidopsis

pART27 vectors containing the RNAi intron hairpin loop was electroporated into *Agrobacterium* strain GV3101. Arabidopsis Col-0 was transformed with the silencing vectors using the floral dip method (Martinez-Trujillo et al. (2004) Plant Molecular Biology Reporter. 22:63-70) . Segregating lines were selected on kanamycin (50µg/ml) and transferred to soil for seed production to collect homozygous lines. PCR was conducted with genomic DNA from putative transgenic lines to confirm the presence of the genes. Segregating lines (T₁ for SYV46 and T₂ for 4FO1) were used in the initial screening for in planta RNAi in nematode infection assays.

### RNA extraction

T₁ segregating lines expressing SYV46p dsRNA were ground with Lysis Matrix D beads (Q-Biogene, Irvine, CA, USA) and liquid nitrogen by placing in a mini beadbeater (Biospec Products Inc. Bartlesville, OK). Total RNA was extracted using the RNeasy Plant Mini Kti (Qiagen, Velencia, CA, USA) following the manufacture's instructions including DNAse digestion with DNAsel.

### RT-PCR

Oligo dT primers were used for first strand cDNA synthesis of total RNA using Superscript 11 reverse transcriptase (Invitrogen, Carlsbard, CA, USA) according to the manufacture's instructions. Amplification of SYV46 cDNA was performed with gene specific primers used to amplify the sense orientation in RNAi vector construction pHannibal-Ntce17-SYV46p. Amplification of the loop of the double stranded-hairpin structure was performed using primers plntron-1 5'GACGAAGAAGATAAA AGTTGAGAG 3' (SEQ ID NO:89) and plntron -2 5' TTGATAAATTACAAGCAGATTGGA 3' (SEQ ID NO:90) designed within the intron region of the RNAi construct. Amplification of actin was used as a positive control and PCR performed with RNA as the template served as a negative control.

### Plant material

Seeds of wild-type and (T₁ for SYV46 and T₂ for 4FOI) segregating RNAi silencing lines were surfaced sterilized with 10% sodium hypochlorite and 0.01 % Tween 20 for 3 min and 1 min with 70% ethanol. Seeds were washes 3 times with sterile distilled water to remove residual sterilization solutions. Wild-type Col-O and transgenic RNAi seeds were plated on MS growth media and MS media supplemented with kanamycin (50µg/ml) respectively. Five days post germination the seedlings were aseptically transferred- one plant per well into 12 well culture plates (Costar, Coming, NY) containing 2 ml of modified Knops medium (Sijmons et al. (1991) Plant J. 1:245-254) solidified with 0.8% Daishin agar (Brunschwig Chemie BV, Amsterdam, Netherlands). The plates were sealed with parafilm and allowed to grow in a growth chamber with 15 hours light/9 hours dark days. Seedlings were inoculated 10 days post germination.

### Inoculations and assessment of nematode infection

*Heterodera schachtii* were propagated on roots of greenhouse-grown cabbage and *H. schachtii* eggs were collected as previously described for *H. glycines* (Goverse et al. (1994) J. Nematology. 26:251-259). Eggs were sterilized with 0.02% sodium azide for 30min before placing in a modified Baermann pans for hatching at 28°C. The J2s were surface sterilized for 10min in 0.004% Mercuric chloride, 0.004% sodium azide, and 0.002% Triton X and washed 3 times with sterile distilled water. Nematodes were suspended in 1.5% low-melting point agarose to allow even distribution of nematodes to each plant and to facilitate the penetration of the J2 into the solid growth medium. Approximately 175 J2 were inoculated per plant. At 26 days post inoculation the plants were observed using an inverted light microscope to observe nematode susceptibility. Females per plant root system were counted and used as a measure of nematode susceptibility. RNAi transgenic plant roots and shoots were also compared visually with wild-type to note any phenotypic differences that may indirectly alter the infection of nematodes. Mean values of nematode females/plant were generated from a minimum of seven replicates per Arabidopsis line and analyzed by analysis of variance (ANOVA) and paired *t*-tests.

### Results:

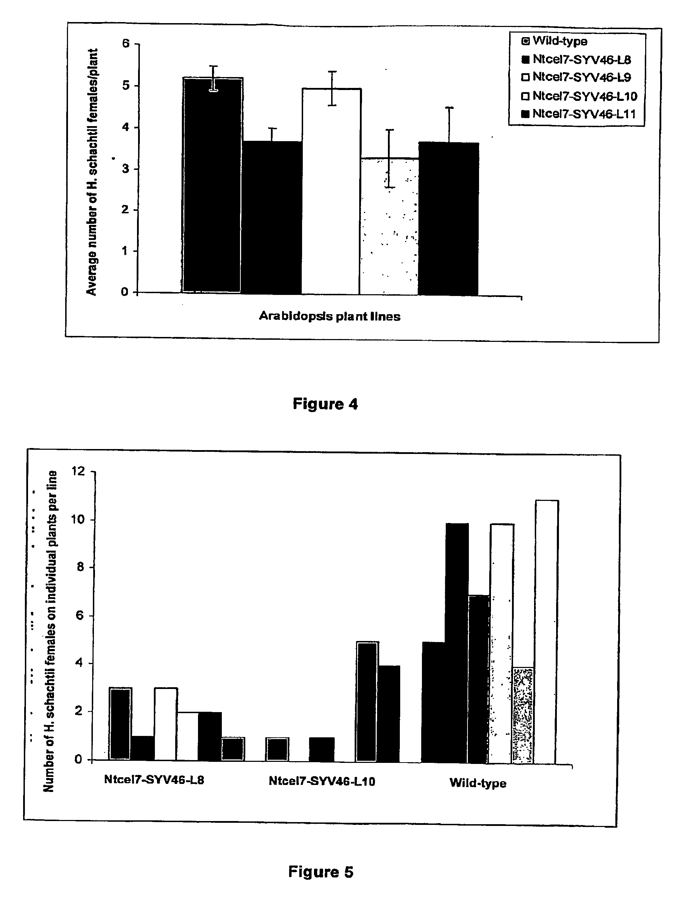

The presence of the NtCe17-4FO1 RNAi and NtCe17-SYV46 RNAi gene construct and expression of the NtCe17-SYV46 RNAi gene construct was confirmed in transformed (T₁ for SYV46 and T₂ for 4FO1) *Arabidopsis thaliana* plants by PCR and RT-PCR, respectively. The Ntce17-SYV46-L8 and Ntce17-SYV46-L10 T₁ lines had a significant (p= 0.0004 and 0.0070, respectively) average reduction in infection by *H. schachtii* compared to infection of roots of wild-type *Arabidopsis* plants (Figure 4). The number of *H. schachtii females* produced on roots of segregating individual Ntce17-SYV46-L8 and Ntce17-SYV46-L10 T₁ lines was as low as a single or zero females per line, respectively (Figure 5).

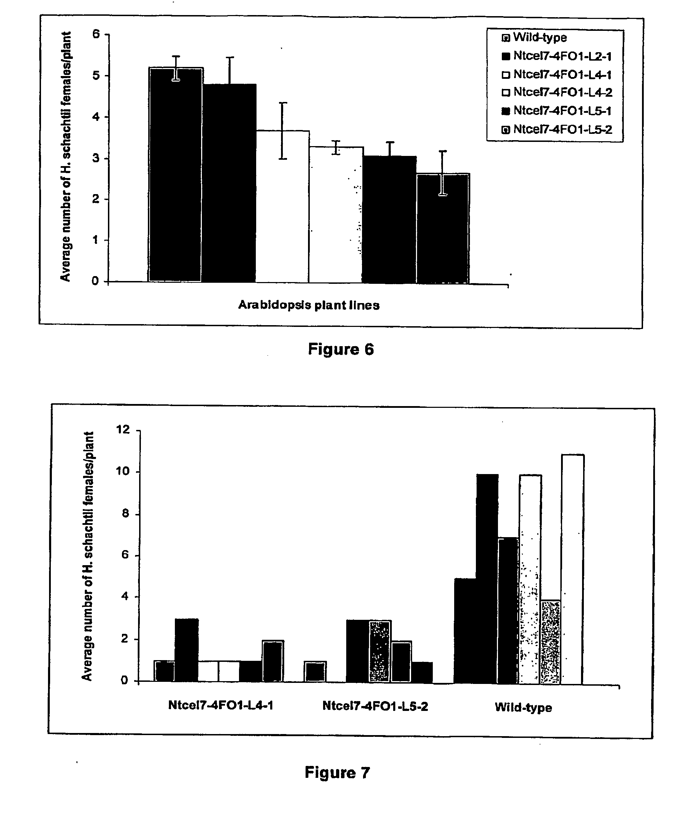

The Ntce17-4F01-L4-1, Ntce17-4F01-L4-2, Ntce17-4F01-L5-1, and Ntce17-4F01-L5-2 T₂ lines had a significant (p= 0.028, 0.003, 0.002, and 0.0004, respectively) average reduction in infection by *H. schachtii* compared to infection of roots of wild-type Arabidopsis plants (Figure 6). The number of *H. schachtii* females produced on roots of segregating individual Ntce17-4F01-L4-1 and Ntce17-4FO1-L5-2 T₂ lines was as low as a single or zero females per line, respectively (Figure 7).

### Example 8: Effects of constitutive expression of in planta RNAi to cyst nematode parasitism genes on cyst nematode infection of roots of transformed plants

### Materials and Methods:

### Generation of gene constructs

The 35S promoter present in pHannibal was removed by digestion with *Sstl-Xhol* and replaced with Gmubi promoter (Finer (2006) In Proceedings of the International Symposium of Plant Biotechnology, Institute of Biotechnology, Yeungham University, Korea, pp 17-22). The Gmubi promoter was provided by Dr. John Finer (lowa State University, IA) in pNSNGmubiX-GFP vector and was isolated by digestion with *Sst*l *Xho*l. pGEM-T easy vectors containing 20E03 and 23G12 were first digested with Eco*R*l. The resulting sense fragments were then gel extracted and subcloned into Eco*R*l digested pHannibal to generate pHannibal-20E03 and phannibal-23G12 plasmids, respectively. To clone the antisense strand of 20E03 gene, PCR amplification of 20E03 antisens gene was performed using the following primers 20E03-F: 5'-TATCTAGAGGCATTTGCCATTTCAAG-3' (SEQ ID NO:91) and 20E03-R: 5'-TAGGATCCTCATGTAGAAAAGGGCC-3 (SEQ ID NO:92). These primers generated Xbal and *BamH*l restriction sites (underlined), respectively. The resulting PCR product was digested with *Xbal* and *Bam*Hl and then subcloned into *Xbal* and *BamH*l digested pHannibal-20E03. To clone the antisense strand of 23G12 gene, PCR was performed using the following primers: 23G12-F: 5'-TATCTAGAGCGCACTTTTCTGTTCATAGC-3' (SEQ ID NO:93) and 23G12-R: 5-ATAAGCTTTCACCATTTTAAGGCTTGCTC-3' (SEQ ID NO:94). These primers generated Xbal and *Hindlll* restriction sites (underlined), respectively. The resulting PCR product was digested with *Xbal* and *Hind*lll and then subcloned into *Xbal* and *Hind*lll digested pHannibal-23G12. The PCR conditions were as described above and identity of the genes was confirmed by sequencing.

### Plant transformation

The constructs made in pHannibal were subcloned as *Not*l fragments in the binary vector pART27 to produce highly effective intro-containing "hairpin" RNA silencing constructs [PART(20E03) and pART27(23G12)]. The pART27-derived constructs were introduced into *Agrobacterium tumefaciens* GV3101 by electroporation and transformed into *A. thaliana* wild type plants by floral dip method (Martinez-Trujillo et al., 2004). To identify putatively transformed *Arabidopsis plants,* total DNA was extracted from plant leaves of . putative transgenic and non-transformed plants as described by Edward et al. (1991). PCR was performed using 23G12 and 20E03 specific primers described above. To evaluate transgene expression, total RNA was extracted with RNeasy Mini kit (QlAgen, Valencia, CA) according to the manufacturer's directions. The extracted RNA was treated with DNase I, according to the manufacturer's instructions for severely contaminated RNA (DNA-free, Ambion Inc., Austin, TX, U.S.A.). RNA (1 µg) was then reverse-transcribed using the Superscript First Strand Synthesis System for RT-PCR (Invitrogen Life Technologies), according to the manufacturer's instructions using the following primer PDK-R: 5'-TTGATAAATTACAAGCAGATTGGA-3' (SEQ ID NO: 95). The resulting cDNA was then used as template for PCR amplification of PDK intron using PDK-R and the following primer PDK-F: 5'-GACGAAGAAGATAAAAGTTGAGAG-3' (SEQ ID NO:96). The PCR program was as described above.

### Plant material

Seeds of wild-type and To RNAi silencing lines (for Gmubi-20E03 and Gmubi-23G12) were surfaced sterilized with 10% sodium hypochlorite and 0.01 % Tween 20 for 3 min and 1 min with 70% ethanol. Seeds were washes 3 times with sterile distilled water to remove residual sterilization solutions. Wild-type Col-O and transgenic RNAi seeds were plated on MS growth media and MS media supplemented with kanamycin (50µg/ml) respectively. Five days post germination the seedlings were aseptically transferred- one plant per well into 12 well culture plates (Costar, Corning, NY) containing 2 ml of modified Knops medium (Sijmons et al., 1991) solidified with 0.8% Daishin agar (Brunschwig Chemie BV, Amsterdam, Netherlands). The plates were sealed with parafilm and allowed to grow in a growth chamber with 15 hours light/9 hours dark days. Seedlings were inoculated 10 days post germination.

### Inoculations and assessment of nematode infection

*Heterodera schachtii* were propagated on roots of greenhouse-grown cabbage and *H. schachtii* eggs were collected as previously described for *H. glycines* (Goverse et al. (1994) J. Nematology. 26:251-259). Eggs were sterilized with 0.02% sodium azide for 30min before placing in a modified Baermann pans for hatching at 28°C. The J2s were surface sterilized for 10min in 0.004% Mercuric chloride, 0.004% sodium azide, and 0.002% Triton X and washed 3 times with sterile distilled water. Nematodes were suspended in 1.5% low-melting point agarose to allow even distribution of nematodes to each plant and to facilitate the penetration of the J2 into the solid growth medium. Approximately 175 J2 were inoculated per plant. At 26 days post inoculation the plants were observed using an inverted light microscope to observe nematode susceptibility. Females per plant root system were counted and used as a measure of nematode susceptibility. RNAi transgenic plant roots and shoots were also compared visually with wild-type to note any phenotypic differences that may indirectly alter the infection of nematodes.

### Results:

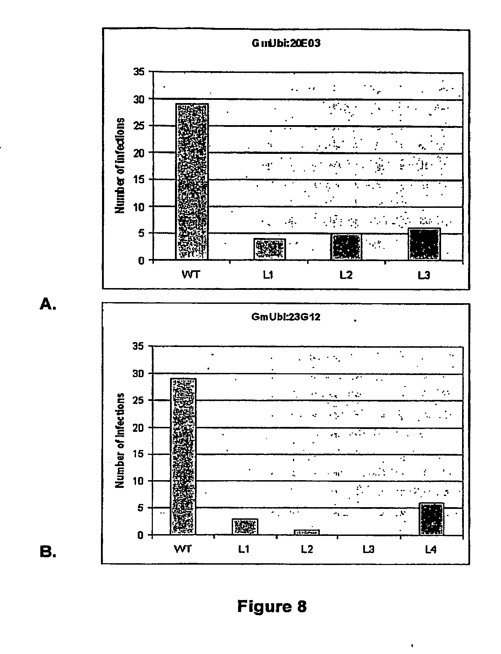

The presence and expression of the Gmubi-20E03 RNAi and Gmubi-23G12 RNAi gene constructs were confirmed in primary transformants (To) of *Arabidopsis thaliana* plants by PCR and RT-PCR, respectively. The number *of H. schachtii* females produced on roots of each individual primary transformant of Gmubi-20E03 RNAi or Gmubi-23G12 RNAi was decreased at least five times or more as compared to the number of *H. schachtii* females produced on roots of wild-type *Arabidopsis* (Figures 8A-B).

Infection of plant roots by cyst nematodes was significantly decreased in plants transformed to express double-stranded RNA of the cyst nematode parasitism genes SYV46 and 4F01 driven by the nematode-inducible NtCel7 promoter as compared to wild-type plants. Individual plants within segregating lines of the SYV46-RNAi (T₁) and 4FO1-RNAi (T₂) had almost no infection by cyst nematodes, indicating that plant lines with strong resistance to cyst nematodes derived from these SYV46-RNAi and 4FO1-RNAi plants can be obtained when brought to homozygosity. Similarly, individual primary plant transformants of the 20E03-RNAi (To) and 23G12-RNAi (Tₒ) driven by the constitutive Gmubi promoter had almost no infection by cyst nematodes, indicating that plant lines with strong resistance to cyst nematodes derived from these 20E03-RNAi and 23G12-RNAi plants can be obtained when brought to homozygosity.

### Example 9: Effects of expression of in planta RNAi to cyst nematode parasitism genes 4G06, 8H07 and 10A06 on cyst nematode infection of roots of transformed plants

### Materials and Methods:

### Vector construction for RNAi knockout experiments

RNAi vector pHannibal (Wesley et al. (2001) Plant J. 27(6):581-90) was used to construct the RNAi constructs. The sense and antisense cDNA sequences of three soybean cyst nematode parasitism genes (Gao et al (2001) Mol. Plant-Microbe Interact. 16:270-276 viz. 10A06 (AF502391), 4G06 (AF469060) and 8H07(AF500024) were amplified from full length cDNA clones and placed under the control of constitutively expressing 35S promoter in pHannibal. The sense fragment was amplified using gene-specific primers having restriction sites Xhol (Forward primer) and EcoRl (Reverse primer) and inserted as Xhol-EcoRl fragment into pHannibal. The antisense fragment was amplified using gene-specific primers having restriction sites Xbal (Forward primer) and Hindlll (Reverse primer) and inserting it as an inverted fragment as Hindill-Xbal into pHannibal. All RNAi constructs were made using the same strategy except for gene 4G06 where Clal restriction site was used in the antisense reverse primer for amplification. The pHannibal vectors were subcloned at Notl sites into a binary vector pART27.

The gene regions were selected on the basis of regions either unique (UR) to that particular gene or conserved regions (CR) across the gene families. The regions and primers used for amplification of these regions are shown in the table below. The restriction sites used in the primers to facilitate the cloning of the PCR product into pHannibal vector are shown bold (Xhol: ctcgag, EcoRl: gaattc, Xbal: tctaga, Hindlll: aagctt, Clal: atcgat)

**Table 6: Gene 10A06_{.} conserved region (CR), base pairs (656 to 758)**

| Primer Name | Sequences | PCR Product (bp) |
|---|---|---|
| 10A06-CR-S-Forward | at**ctcgagttt**ggatgcatcgcttatcactgacctt SEQ ID NO:97 | Sense (103bp) |
| 10AOS-CR-S-Reverse | at**gaattc**catlgtttttaaaccac6catcaacgca SEQ ID NO:98 | |
| 10A06-CR-AS-Forward | at**tctaga**tttggatgcatcgcttatcactgacctt SEQ ID NO:99 | Antisense (103bp) |
| 10A06-CR-AS-Reverse | at**aagctt**cattgtttttaaaccacccatcaacgca SEQ ID NO:100 | |

**Table 7: Gene 4GO6, unique region (UR), base pairs (404 to 556)**

| Primer Name | Sequences | PCR Product (bp) |
|---|---|---|
| 4G06-UR-S-Forward | at**ctcgag**gaaatgggaagagaaaca SEQ ID NO:101 | Sense (153bp) |
| 4G06-UR-S-Reverse | at**gaattc**aagataacccggaaaagg SEQ ID NO:102 | |
| 4G06-UR-AS-Forward | att**ctaga**gaaatgggaagagaaaca SEQ ID NO:103 | Antisense (153bp) |
| 4G06-UR-AS-Reverse | at**atcgat**aagataacccggaaaagg SEQ ID NO:104 | |

**Table 8: Gene 8H07. unique region (UR), base pairs (1079 to 1160)**

| Primer Name | Sequences | PCR Product (bp) |
|---|---|---|
| 8H07-UR-S-Forward | a**tctcgaga**cgagttgagaaggagaa SEQ ID NO:105 | Sense (82bp) |
| 8H07-UR-S-Reverse | at**gaattc**ctcttcctcctctcgttc SEQ ID NO:106 | |
| 8H07-UR-AS-Forward | at**tctaga**acgagttgagaaggagaa SEQ ID NO:107 | Antisense (82bp) |
| 8H07-UR-AS-Reverse | at**aagctt**ctcttcctcctctcgttc SEQ ID NO:108 | |

**Table 9: Gene 8H07, Conserved region (CR), base pairs (515 to 852)**

| Primer Name | Sequences | PCR Product |
|---|---|---|
| 8H07-CR-S-Forward | at**ctcgag**aaccatattccccaaatg SEQ ID N0:109 | Sense (338bp) |
| 8H07-CR-S-Reverse | at**gaattc**tatttggtttggcatttgattcggctg SEQ ID NO:110 | |
| 8H07-CR-AS-Forward | at**tctaga**aaccatattccccaaatg SEQ ID NO:111 | Antisense (338bp) |
| 8H 07-CR-AS-Reverse | a**taagcttt**atttggtttggcatttgattcggctg SEQ ID NO:112 | |

The targeted region of the gene was amplified using MJ research PTC-100 (149 Grove St., Water Town, MA 02172) thermal cycler. The PCR conditions included initial melting temperature of 94° C for 2 minutes followed by 35 cycles of 94° C for 30 seconds, 55 ° C for 45 seconds and 72 ° C for one minute. This was followed by a final extension time of 7 minutes at 72 ° C. The PCR reaction composition included 0.025 units/µl Taq polymerase (New England Biolabs) and Invitrogen (1600 Faraday Av., PO Box 6482, Carlsbad, CA 92008) reagents including 1.5mM MgCl₂ , 200 µM each of dATP, dTTP, dGTP, dCTP nucleotides. The template plasmid DNA concentration was 1 to10 ng/ µl.

DNA was digested using restriction enzymes from New England Biolabs and Invitrogen using the protocol suggested by the manufacturers. Similarly, the ligation of DNA molecules was done essentially as described by the manufacturer Invitrogen using T4 DNA ligase. Plasmid DNA minpreparation was done using Qiagen kits (QIAgen, Valencia, CA) following the instructions described by the manufacturer. All constructs were confirmed by DNA sequencing at lowa State University DNA sequencing facility.

### Transformation of RNAi constructs in Arabidopsis

The binary vector was mobilized into chemical competent *Agrobacterium* strain (C58) using standard heat shock method and plated on LB medium having kanamycin (50µg/ml). Arabidopsis plants were transformed using floral dip method following the method described by Clough and Bent (1998) Plant J. 16:735-43.

### Segregation analysis and obtaining homozygous plants

After the seeds from each plant had been collected, the seeds from 10 individual plants were selected from each construct. Seeds were planted from individual plants onto selectable medium (MS (Murashige and Skoog (1962) Physiol. Plant. 15:473-497)) medium supplemented with 500mg/L MES 2(Nmorpholinoethane. sulphonic acid), 0.1 % sucrose [pH 5.7], solidified with 8gm/L phytoagar (Research Products International Corp. Cat. No. 9002-18-0. 410 N. Business Center Dr., Mt. Prospect, IL 60056). Kanamycin (50mg/L) was added to the medium to select for the kanamycin resistant plant progenies. 300 to 500 seeds from individual plants were plated onto selectable medium-containing 150 mm petridish and grown at 25 ° C at 16/8 hrs light/dark cycle. Kanamycin-resistant plants growing on selectable medium were carefully removed and planted in pots containing soil mixture and transferred to a growth room for further growing till maturity. The seeds were collected from individual plants - these were our T1 seeds. The T1 seeds were further plated on selectable medium to further analyze the segregation of resistant phenotype. T2 kanamycin resistant seeds were harvested from non segregating families and further plated to confirm the resistant phenotypes. The T3 homozygous lines were used in all further assays.

### Plant material

Seeds of wild-type and T3 RNAi silencing lines were surfaced sterilized with 10% sodium hypochlorite and 0.01 % Tween 20 for 3 min and 1 min with 70% ethanol. Seeds were washes 3 times with sterile distilled water to remove residual sterilization solutions. Wild-type C24 and transgenic RNAi seeds were aseptically transferred, one plant per well, into 12 well tissue culture plates (Costar, Coming, NY) containing 1.5 ml of modified Knops medium (Sijmons et al. (1991) Plant J. 1:245-254) solidified with 0.8% Daishin agar (Brunschwig Chemie BV, Amsterdam, Netherlands). The plates were sealed with parafilm and allowed to grow in a growth chamber with 16 hours light/8 hours dark days. Seedlings were inoculated 10 days post germination. The experimental design for these experiments was set up such that each line tested was replicated at least 15 times per experiment with each replicate consisting of a single plant. Experiments were set up following a random block design with all treatments randomized within 12-well plates.

### Inoculations and assessment of nematode infection

*Neterodera schachtii* were propagated on roots of greenhouse-grown canola and H. *schachtii* eggs were collected by breaking open cysts and collecting the eggs on a sieve stack consisting of a #60/#200/#500. Eggs were then cleaned up on a 35% sucrose gradient. Eggs were placed in a modified Baermann pan (hatch chamber) with 3.14 mm ZnSO4 for hatching at 26°C. The J2s where surface sterilized for 1 hour in 0.001% hibitane and 7 minutes in 0.001% Mercuric chloride and washed 3 times with sterile distilled water. Nematodes were suspended in 1.5% low-melting point agarose to allow even distribution of nematodes to each plant and to facilitate the penetration of the J2 into the solid growth medium. Approximately 250 J2 were inoculated per plant. At 14 days post inoculation the plants were observed using a Zeiss Stemi 2000 dissecting microscope. Females per plant root system were counted and used as a measure of nematode susceptibility. RNAi transgenic plant roots and shoots were also compared visually with wild-type to note any phenotypic differences that may indirectly alter the infection of nematodes. Mean values of nematode females/plant were generated from a minimum of fifteen replicates per Arabidopsis line and for three independent experiments. Each experiment was analyzed individually by a modified t-test using the statistical software package SAS. Using SAS, we generated the mean, p-value and standard error for each experiment.

### Results:

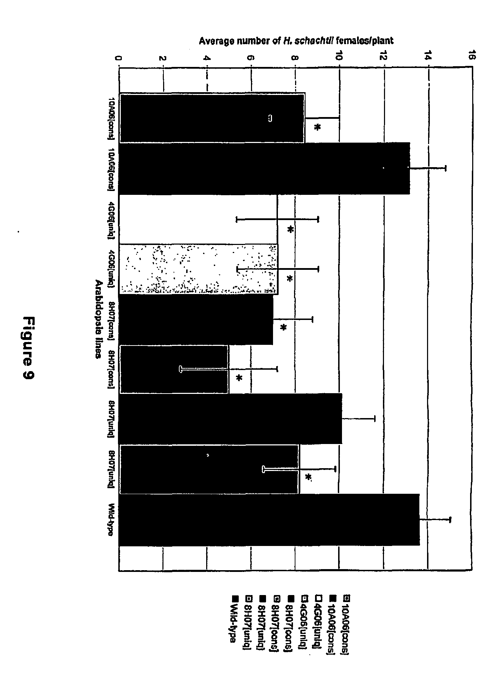

RNAi lines 10A06[conserved], 4G06[unique], 8H07[conserved] and 8H07[unique] were tested against a wild type control line. Within this experiment, all lines were tested twice (Figure 9). For line 10A06[conserved] one of the tests had significant (p=0.0151 ) average reduction in female development by *H. schachtii* compared to wild-type Arabidopsis plants. This was a reduction of 35%, on average, per plant. For line 4G06[unique], both tests had significant (p=0.0061 and 0.0066) average reduction in female development by *H. schachtii* compared to wild-type Arabidopsis plants. This was a reduction of 46%, on average, per plant, for both lines. For line 8H07[conserved], both tests had significant (p=0.005 and 0.0011) average reduction in female development by *H. schachtii* compared to wild-type Arabidopsis plants. This was a reduction of 46% and 62%, respectively. For line 8H07[unique] one of the tests had significant (p=0.013) average reduction in female development by *H. schachtii* compared to wild-type Arabidopsis plants. This was a 38% reduction in females, on average, per plant.

The invention is now described by the following clauses:
1. A transgenic plant or cell comprising:
   an inhibitory nucleic acid specific for at least a portion of a nucleic acid encoding a cyst nematode esophageal gland cell secretory polypeptide.
2. The transgenic plant or cell of clause. 1, wherein the plant or cell is resistant to cyst nematode disease.
3. The transgenic plant or cell of clause 2, wherein the plant or cell is resistant to nematode disease caused by *Heterodera glycines or Heterodea schachtii.*
4. The transgenic plant or cell of clause. 1, wherein the inhibitory nucleic acid is in an amount effective to reduce, inhibit, or prevent expression of the cyst nematode esophageal polypeptide by a cyst nematode feeding on the transgenic plant or cell compared to a control plant or cell.
5. The transgenic plant or cell of clause. 1, wherein the inhibitory nucleic acid is in an amount effective to inhibit cyst nematode disease compared to a control plant or cell.
6. The transgenic plant or cell of clause. 1, wherein the inhibitory nucleic acid is in an amount effective to reduce, inhibit, or prevent cyst nematode disease caused by at least two different cyst nematode species compared to a control plant or cell.
7. The transgenic plant or cell of clause. 1, wherein the cyst nematode esophageal polypeptide or fragment thereof decreases formation of a syncytium, nematode migration through root tissue of the plant, or formation of a feeding tube that enables the nematode to feed from syncytia formed in the plant.
8. The transgenic plant or cell of clause 7, wherein the gene expression of the plant or cell is modulated by the cyst nematode esophageal gland cell polypeptide.
9. The transgenic plant or cell of clause 8, wherein the modulation of gene expression occurs in a root cell.
10. The transgenic plant or cell of clause 1, wherein the nematode is a member of *Heterodera spp or Globodera spp.*
11. The transgenic plant or cell of clause 1, wherein the transgenic plant or cell is any monocot or dicot, or selected from the group consisting of tobacco, cereals, sugar beets, cotton, fruits, fibers, oilseeds, potato, rice, corn, soybeans, vegetables.
12. The transgenic plant or cell of clause 1, wherein the transgenic plant or cell is a member of the phylogenic family *Leguminosae, Chenopodiaceae, Cruciferae,* and *Solanaceae.*
13. The transgenic plant or cell of clause 1, wherein the plant is a soybean.
14. The transgenic plant or cell of clause 1, wherein the inhibitory nucleic acid comprises at least a portion complementary to part or all of mRNA encoding a protein encoded by one or more of SEQ ID NOs 1-63 and 113-116.
15. The transgenic plant or cell of clause 1, wherein the inhibitory nucleic acid comprises a double-stranded or small interfering RNA.
16. The transgenic plant or cell of clause 1, wherein the inhibitory nucleic acid comprises microRNA.
17. The transgenic plant or cell of clause 1, wherein the inhibitory nucleic acid comprises antisense DNA..
18. The transgenic plant or cell of clause 1, wherein the inhibitory nucleic acid inhibits or interferes with the translation of mRNA encoding the cyst nematode esophageal gland cell polypeptide.
19. The transgenic plant or cell of clause 1, wherein the inhibitory nucleic acid induces or promotes the degradation or mRNA encoding the cyst nematode esophageal gland cell polypeptide.
20. The transgenic plant or cell of clause 1, wherein the transgenic plant or cell comprises two or more inhibitory nucleic acids specific for different cyst nematode esophageal gland cell polypeptides.
21. The transgenic plant or cell of clause 1, wherein the inhibitory nucleic acid comprises non-natural or natural nucleotides.
22. The transgenic plant or cell of clause 1, wherein the inhibitory nucleic acid comprises at least one modified internucleotide linkage.
23. A composition comprising:
   an inhibitory nucleic acid specific for an mRNA or fragment thereof encoding a polypeptide encoded by one or-more of SEQ ID NOs:1-63 and 113-116 or a fragment or homologue thereof, in an amount sufficient to inhibit expression of the polypeptide encoded by one or more of SEQ ID NOs:1-63 and 113-116 or homologue thereof when delivered to a nematode.
24. The composition of clause 23, wherein the polypeptide or fragment thereof encoded by one or more of SEQ ID NOs:1-63 and 113-116 and modulates: gene expression of the plant or cell, formation of a syncytium, nematode migration through root tissue of the plant, cell metabolism of the plant, signal transduction in the plant cell, or formation of a feeding tube that enables the nematode to feed from syncytia formed in the plant.
25. The composition of clause 24, wherein the gene expression of the plant or cell is modulated by the polypeptide.
26. The composition of clause 25, wherein the modulation of gene expression occurs in a root cell.
27. The composition of clause 26, wherein the nematode is a member of *Heterodera spp or Globodera spp.*
28. The composition of clause 27, wherein the transgenic plant or cell is a monocot or dicot.
29. The composition of clause 27, wherein the plant or cell is a member of *Leguminosae.*
30. The composition of clause 29 wherein the plant or cell is a soybean.
31. A cell comprising a nucleic acid encoding an inhibitory nucleic acid specific for an mRNA or fragment thereof encoding a cyst nematode esophageal gland cell polypeptide that modulates: gene expression of the plant or cell, formation of a syncytium, nematode migration through root tissue of the plant, cell metabolism of the plant, signal transduction in the plant cell, or formation of a feeding tube that enables the nematode to feed from syncytia formed in the plant.
32. A vector comprising a promoter operably linked to a nucleic acid encoding an inhibitory nucleic acid specific for an mRNA encoding a cyst nematode esophageal gland cell polypeptide that decreases formation of a syncytium, nematode migration through root tissue of the plant, or formation of a feeding tube that enables the nematode to feed from syncytia formed in the plant.
33. The vector of clause 32, wherein the nematode esophageal gland cell protein is encoded by one or more of SEQ ID NOs:1-63 and 113-116 or homologues thereof.
34. A method for providing cyst nematode resistance to a plant comprising:
   expressing in the plant an inhibitory nucleic acid specific for a cyst nematode esophageal gland cell protein.
35. The method of clause 34, wherein the nematode is a member of *Heterodera spp or Globodera spp.*
36. A method for providing cyst nematode resistance to a plant comprising:
   contacting the plant with one or more inhibitory nucleic acids specific for one or more cyst nematode esophageal gland cell proteins in an amount sufficient to reduce cyst nematode disease.
37. The method of clause 36, wherein the one or more cyst nematode esophageal gland cell proteins modulates: gene expression of the plant or cell, formation of a syncytium, nematode migration through root tissue of the plant, cell metabolism of the plant, signal transduction in the plant cell, or formation of a feeding tube that enables the nematodes to feed from syncytia formed in the plant.
38. The method of clause 37, wherein the nematode is a member of *Heterodera spp or Globodera spp.*
39. The method of clause 38, wherein the plant is resistant to nematode disease of at least two different species of cyst nematode.
40. The method of clause 39, wherein the plant is resistant to nematode disease caused by *Heterodera glycines or Heterodera schachtii.*
41. A seed produced by the transgenic plant or cell of clause 1.
42. A fusion protein comprising a polypeptide encoded by SEQ ID NOs:1-63 and 113-116 or fragments thereof and a heterologous polypeptide.
43. A method for inhibiting the biological activity of a nematode parasitism gene product comprising the expression of an inhibitory peptide, polypeptide, or intracellular secondary metabolite in a plant, wherein the inhibitory peptide, polypeptide, or intracellular secondary metabolite specifically inhibits the biological activity or expression of the nematode parasitism gene product.
44. The method of clause 43, wherein the parasitic nematode is a cyst nematode.
45. The method of clause 43, wherein the inhibitory molecule is a peptide.
46. The method of clause 43, wherein the inhibitory molecule is a polypeptide.
47. The method of clause 43, wherein the inhibitory molecule is an intracellular secondary metabolite.
48. An isolated nucleic acid selected from the group consisting of SEQ ID NOs:113, 114, 115, and 116.
49. A vector comprising the nucleic acid of clause 48.
50. An isolated host cell comprising the vector of clause 49.
51. An isolated host cell comprising an inhibitory nucleic acid that specifically inhibits expression or activity of the nucleic acid according to clause 48 in a parasitic nematode.

## Claims

1. A method for providing cyst nematode resistance to a plant comprising:
expressing in the plant dsRNA specific for mRNA for a cyst nematode esophageal gland cell protein, wherein the dsRNA comprises at least 21 nucleotides.

2. The method of claim 1, wherein the dsRNA binds to mRNA encoded by a sequence selected from the group consisting of SEQ ID NOS: 43, 12, 11,9, 18 and 19.

3. The method of any one of the preceding claims wherein the plant is selected from the group consisting of tobacco, cereals, sugar beets, cotton, fruits, fibers, oilseeds, potato, rice, corn, soybeans, and vegetables.

4. The method of any one of claims 1 or 2, wherein the plant is a member of the phylogenic family *Leguminosae, Chenopodiaceae, Cruciferae,* and *Solanaceae,* preferably wherein the plant is a soybean.

5. The method of any one of the preceding claims, wherein the nematode is a member *of Heterodera spp.* or *Globodera spp.*

6. A plant obtainable by the method of any one of the preceding claims.

7. The use of dsRNA which binds to mRNA encoded by a sequence selected from the group consisting of SEQ ID NOS: 43, 12, 11, 9, 18 and 19 in the method of any one of claims 1 to 5.


SEQUENCE LISTING

<110> University of Georgia Research Foundation
North Carolina State University
Iowa State University Research Foundation, Inc.
Hussey, Richard
Davis, Eric
Baum, Thomas

<120> Cyst Nematode Resistant Transgenic Plants

<130> UGA 1208

<150> 60/759,182
<151> 2006-01-06

<160> 117

<170> PatentIn version 3.3

<210> 1
<211> 834
<212> DNA
<213> Heterodera glycines

<400> 1
aaaccataaa ttacaactta aatcaagcaa aatcaaatgc acttgattaa cttaatcgcc 60

ctctttttca tgcttttcgg cccatccgtc cagcaataca caaaaacgcc aacgaatgag 120

gacaaagaag cggctgtcaa ttgtcacaac aaattccgat cgcaattggc cctgggcaat 180

gccgacaata aattgggcgg caacaaaatg ccaaaggcgg gcaacatgcg taagtttgaa 240

tgggacgaaa acttggccaa acttgcggat gaatgggcca acaaatgcac attatcgcac 300

tcgtggaacg gctgggcagg cgaaaatttg gcaatgaatg gcggaacatt ttcgaacaag 360

gatggcttcg agtacgcttg cggtcgctgg tgggacgaac tgaaccgtta cgggttcaac 420

ccggatctga ttatgaccgg ggaaaacttc agtggcatcg gccattggac tcagatggcg 480

tgggccgaca ccgaccgaat tggctgtgcc atggcacaaa actgcccaaa taccaattgg 540

aaaacatatg tggtctgctg gtattacacg ggtggtaatt actttggtgc gcctgtctat 600

ttggcccggg agccgtgcag caaatgcaaa gcaaatgaca aatgtgacaa agccactgga 660

ctttgctctc aatgaaagat gaaatattat gaaaattgaa ttgggataat tactaattat 720

tgtatctgta tgtttagtat ggtttagtgt ttcggtataa ttttcactta ttggtcttaa 780

ttaacaatga ataaataaat agtttctaaa aaaaaaaaaa aaaaaaaaaa aaaa 834

<210> 2
<211> 733
<212> DNA
<213> Heterodera glycines

<220>
<221> misc_feature

<222>  (574)..(574)
<223>  n is a, c, g, or t

<400>  2
gaaccaacca ataccattaa tttcataaat ccgaagaaat ccctccaaaa atgtcttctt      60

ccccgtccgt ctctgtgctc gccatcgtcg caattgtctg tttgatgtgc caatgttgct     120

tttcggcacc gcatccgtgc tgtcccggca gtcaaaaagt ggtttcactt atgtccaatt     180

acgttggcac ttttgccaat tccatttcca agtcatcgct ttgttcggat gcccaaaatg     240

ttgcggaagc gttgaaaggc caactgatcg gctgctcgaa tggcggcgat cgaactcttt     300

tggccgacat cgaagcatcc cttgccactc attctgctga tgagtgtgcc ctcagcctcg     360

gcttcgtccg tgccatgttc gccattgccg cctccgcttc ttcccatgcc agcaacaaca     420

gcgaatggca gacattgagt gggcagtttg gtcagaaagt cactgagatt gactcgaaat     480

gtgcagagtt tggtattagc attggcaaag tgcccataaa cggtcccaag gatgtccatg     540

ttcaaaatgt gcccaactcg gaaagtgtga tttntatgcc tggattggcc ggctcacaca     600

cccaatgaaa atgcatcact gaaaagattt ggtacctttt gattattgtg cattaattag     660

ctgtggattt tgtaaattta ggaaattata tgtaaataaa tattaattcg ttgaaaaaaa     720

aaaaaaaaaa aaa                                                        733

<210>  3
<211>  615
<212>  DNA
<213>  Heterodera glycines

<400>  3
gacaaataaa tttaattttt taattttttc aaatctatta caaaattcaa aaattaaaac      60

catgaattgg atgcattatt gtttaatcgc ttgctttgct ttttattatt tcaatactgt     120

tgagtcatcc actattaatt cggtaaccgt acaagtgaac aagattgaaa acaatgaaaa     180

tggaagacaa ttcaatttgg aatttacaaa ccaagtttat gagcgagtgt gccacgttga     240

ctttcgaatt gatctgccag acacagtaaa attgaacaaa tattcaaaaa tggtgccaat     300

tcctgacacc tgcggccagt acgcattgcc caagagtttg gacttgcttc ccggcgagtc     360

atttgatgca caattaacac tgcttggcca tgatgggaag ccgaatgtga ctgtgctgaa     420

cacgaacaat attccaacca gcaaacaatg caaaaaatga aaaagcctac ccaattaagc     480

taattgccct atcagttcag caagttaacc aattagcata tcattgcgat caatttaatt     540

attgtattgt cattaattta aaaaaaacat atataagttt gaaggttaaa aaaaaaaaaa     600

aaaaaaaaaa aaaaa                                                      615

<210>  4
<211>  1143
<212>  DNA

<213>  Heterodera glycines

<400>  4
gatcaaacaa aaaattaaaa tgaacaggtt ttttacatta ttattttttg tattattttt      60

caatgccgca attaattttg tcagttcaca tcgcattgtc ggttattatc agggcatacg     120

tccattgaca aatgatcaag ccaagaagtt gacccatctt atcctggcat tttcaacccc     180

tgactctcaa ggcaatttga gtccattgag ctctgtgctt aaacaggcgc taaaagcggg     240

taaatccgct aatggtgcgc tcaaagtgat gattgccatt ggaggaggtg gctttgatcc     300

ggccatattt acttcgttag catcaaacag tggcacacgt aaaagcttta ttaataacat     360

tgtttcttat ctgaaaacca atgagctgga cggttgcgac atcgactggg agttcccaac     420

ttctagtgac aaggcaatct ttgtgacatt tctgcgcgac ttaaaaaagg cgatggcacc     480

cagcggcgct gtgcttagca tggcatcggc agcaagtgcc ttctatttgg accctggtta     540

cgatttgcca ggcattgaga gtgccgtcga tttcattaac gtgatgtgct atgactatta     600

tggaagctgg accaaaacat cgactgggcc aaactcgcca ctgtttaagg gtggcagtgc     660

cgacccatcg gacacattga acagcaattg gacaatgaat tatcacttaa tgaaagtgta     720

taatcgagca aagttgaaca tgggtgtgcc attctacgga aaatcttgga ccaatgttgg     780

agcaccacta aacggtgacg gactttggcg tcagttgggc acttatggca ccgaattagc     840

ctggcgtaac atgggcaaaa gttttgacat gaccaagaca acgtatcata aaacggccaa     900

aactgcatac atttatgata cagctaccaa aaatttttta acctttgaca acccccaatc     960

actgaaggac aaggcaaaat atgttgcgga aaagggcatt ggtgggataa tgatatggtc    1020

aattgatcaa gatgacgaca aattgtcttt attgaattct gtttcatatt gattttgatg    1080

tatatattgt gtttgagcca ttaaaatgtg ttagaaaaaa aaaaaaaaaa aaaaaaaaaa    1140

aaa                                                                  1143


<210>  5
<211>  706
<212>  DNA
<213>  Heterodera glycines

<400>  5
   gactcattcc atcgccgctc actcattttc ttccctttcc tttgtcttcc attcattccg      60
   cttttccctc cccatttctc atttgttctt ctccgcttct tctctccccg atgaatcact     120
   ttgggctgac ctttctgttc gtcgccgttt ctctgctgac attgacgggc aaaacgatca     180
   ctttggaggt ggagagctcg gacactgtgg acaatgtgaa gacgaagatc caagagaagg     240
   agggcgttcc gccggatcag caacggctga tcttcgccgg caaacagctc gaggacggac     300
   gaacgttggc cgactacaac atacagaagg agtccacgct ccacttggtc ctccgtctcc     360
   ggggcggaaa tgggaagaga aacacgagta agaacaagaa aagcaacaaa aagcttgatc     420
   agaattgatc agcagcgaac gcaccatcac atgattgatt ggcacagtga ttttccacca 480
   ccaaattcac aacactttcc cctttttccg ggttatcttt gatttccatt ttcggttatt 540
   gttttctgtt tttctcctct atcttttgta aaattctgtt actttgatct ttagtgttta 600
   tcatttatca tctgcatata tagaagttat catagtccat atttctgtcc ctttttattt 660
   attttttttg actataaaac ttttgggcac aaaaaaaaaa aaaaaa 706

<210> 6
<211> 734
<212> DNA
<213> Heterodera glycines

<400> 6
atgtcttctc tgctgctctc catcttccca attgtgtttt tggtctgttg caatgcaatg 60

ccaaatttcc cgtgctgccc gggcagtcag caagtggttg ctgtgatgtc caaatacatt 120

gacactttct cttctgctgc tgacgagtct acagtatgct caaccgctaa aagtactgtg 180

gatggaataa aaaatgaact gtcctatcgc gtgggatgcc caagcggagg agaagcacaa 240

attgtgaatg aaattgatca acagctgaag aacattgcga aaatggaaat caattatgag 300

gacgagtgcc cgtacaattt gggctttgcc cgtgccatgt tcgacttggc cgctgctgct 360

gctggccatg cggggaacgg cacagaatgg caatacatga aagtacaatt tgagcaggaa 420

agccaagcaa tcaaagcaat tggacaagaa aagaactttg aagttacgga tgtgcatttt 480

ggagtcccaa gcaaaggggt ttctgcacat caaaatgtgc cgagtccgag ccatgtgatt 540

gccaaccctg gccaacacag ttcggttggc caaggaaaga aagaagaacc gttgtcatcg 600

gacttcgatt tttgaggaca aacaaatcag gaggaaatag aatagaaaac cattttgttg 660

acatgtcgaa catttattta aagtaataaa tatttggtaa taaggaaaaa aaaaaaaaaa 720

aaaaaaaaaa aaaa 734

<210> 7
<211> 769
<212> DNA
<213> Heterodera glycines

<400> 7
atcatttgta attcattttt taaataaaaa tttccttgga ccgacaaaaa tggcaaaatt 60

cgttgccatt gctcttctct cgctgaccat tgtttcgatg gcacttgcaa aaactggcaa 120

aagtcaaacg gcagacgaag ttgagggatt tcgcaatatg aacatcggcg acaacaataa 180

ggttgacgcc ggaaaggagc ccgcggctga taaagcaacc aaaaagggaa aagctcagaa 240

agccggaacg aaatcggcgg cggccactaa tgagcccgcg gctgctaaag gaactaaaaa 300

tggaaaagcc ccgaaaacca aagcgaagca agaggtggcc actaatgagc ccgcggctgc 360

taatgaatgg aacgaccaat tgatgggcat gagcgttgag aaatttaacg aggagcttgc 420

tgtgttgttg ccaaaagcca acacttttat ggaaaatgct ttgagcttca tcaatgaaca 480

agtggaaaaa aatggtattg caactggcgc tgccggggac tcgtgctcga ccgggatccc 540

ggccaactag gagaccaatg ccacaggacg gcaccggagg agggaagaaa ggatgacgag 600

ctctttggat gatgaaggtg gtggataatg gatggttagt tgattggggg gatttacatt 660

tttcattcaa ctttcttggg agttgcaaat ttcaaaaaat gttttttttt ttgaaatttt 720

tggtaaactg atcggcatta aaaaaaaaaa aaaaaaaaaa aaaaaaaaa 769

<210> 8
<211> 469
<212> DNA
<213> Heterodera glycines

<400> 8
gattccctca aaagcaaagc catccatccg atcattcgct tcatctgcca atccgcaaaa 60

tgtccctttt ccgtcctcaa tcgctgcttc ttctggccgc tctttgcctg tcctttgcgc 120

tgctctttgt cacttcgtcg gaagagggag ggcgagtgaa gcgcggcgga tggccttggg 180

attgggccgg caaacaactg tgcaaaacat cggcaaattg caagtgcaag gatggcaaaa 240

attgggccaa atgtgtaaag tcggaaggct acgcggccag caattgttgc gacaaaaatt 300

acgtgtgggc atgttgcggg aagaagccca aacattgatg agagaagaga aagggagaga 360

atgagaagcg agttcacata attcattctg ttttggattt ttagtctttt ttttcttttt 420

gggttttttt ggtaaatatt ttctttagca aaaaaaaaaa aaaaaaaaa 469

<210> 9
<211> 1200
<212> DNA
<213> Heterodera glycines

<400> 9
aatcccaatt tcgcattcat ctcactcact cataaaatgc tccaaaacgg ccttaccatt 60

ctgcttctga tcagtgttgt gatcggccat tccttggcca accttggccc aaccatcaaa 120

cataatcctc attttaaagc cgtacaaact gcgcatcatt tgcatgatgc cattgcgaag 180

aagcacgagg ccgaagttac gcaagtcatt tgctctatta gcaacgaaca gcgtcaagca 240

ttggctttgg agttcaaaaa acaattcggc actgatctga ttgccatgct gaaaaaggag 300

ttcaaaagcg actttgaaga actgatcatt tctttgatgc aaacgcccgc cgtttacgat 360

gccaaccaaa tgcgtgccgc attgtccggc tccaatgagg cggtgctaat cgaaattttg 420

gcgacgcgca caaaccgaca aattacggcg ccgaagcagg cgtatgagca gttggacaga 480

aggcatcagc acaatcagct ggaggaggac atcaaagcga agacgaagag gacccttcca 540

aaatctgttg gtgtctttgc tcagctgctc tcgcgaagaa aaagtgcgcc cgcaagcatt 600

gtattggcac acgacgaggc catgaaactg ttcagagagg gcgagggccg acggggcgtt 660

aacgccgtgg tgttcaacca ggtgttggcc actcgcagct tcgcccagct tcgggaaact 720

ttcgagtttt accgacaagc cgcgcaccac gagattgagg agggaattaa gcaagaattc 780

agcggtcaca acgaagcggg tttcttggca ctaatcaaat atgtccgcaa cgcttctgtg 840

ttttttgcgg atttgttgtt caattcgatg aaagggctcg gcacacgcga ctcggatttg 900

attcgtctgg tcatttctcg gtctgagatt gacctggctg acatcaaaca cgcttttcac 960

acgttgcaca agaagagcct ggaggaggcg atcaaagggg acaccagcgg agcttaccga 1020

gacgcacttt tggcattggt caagggcaac acggagcagt gatggagcag cggcagaagg 1080

gattttgcaa gagattagga tagatgtaaa agagataaac atgaataccc aataaacatc 1140

ctgtagaaat tataaaataa aatttttatt ttacaaaaaa aaaaaaaaaa aaaaaaaaaa 1200

<210> 10
<211> 951
<212> DNA
<213> Heterodera glycines

<220>
<221> misc_feature
<222> (402)..(402)
<223> n is a, c, g, or t

<400> 10
tcaaaaattc attcaaccct agaatttatt ttttttaata aataagtttt tttgtacaaa 60

atgagcaact ttatatttgt cgcctcttta actgcagcgt tttttagctc aggcctcgct 120

ctaccggctc cttatgatgc tgaatcggtg gtatcttctg aattaaatgt tccactactt 180

tcagctgacg caaatgttga agcagcaatt accaatgaaa gtgatgctgc tgctgagata 240

caagctccat caattccggt accaattgaa catcaaactg ctgctgacat tactcatcca 300

actgaaactg gcaatgagtc ttctattgca tcatcatcat ccacgccgaa aagtgagcag 360

acgccaaaaa aagtgatgaa catgaaaagc gcgctggaag ancgcaggca ggccaacgtg 420

tacgaggaga ggcttccgtt agacaagcag cccaagacga tcgctgaagc ggcagcaaaa 480

gcaaagcaga cgcccgccaa acttccggct gactacgacg tgaaccgcgt ggcggaacgc 540

gcagcagccc gtgtgtacgg gtggcttccg gaagacaagc agcctaaggc gatctatgac 600

gcggcggaga aggcaaagaa cacgcccaaa ccgccgggcg actacgacgt ggagcgcgtg 660

gcgcaaaagg cggcccggct cgtctacggt gtgctgccca tcggcatgca gcccaacttc 720

gccggcccta gcactgacaa gagcaatgtc gacgactcgg agaaaccttc tgctgctgcg 780

gctggtgatg atgatgatga agtcgaaaaa gagaagaagg aataagcaaa aaacaatgtg 840

aatttattat aggaaaaaaa gaaaaatgga agcttagaaa tttaatattt tcatttttga 900

acatttataa aatttcaaaa ttgaccgcaa aaaaaaaaaa aaaaaaaaaa a          951

<210>  11
<211>  746
<212>  DNA
<213>  Heterodera glycines

<400>  11
gattccatcg cccctcactc attttcttcc ctttcctttg tcttccattc attccgcttt      60

tccctcccca tttctcaatt gttcttctcc gcttcttctc tccccgatga atcactttgg     120

gctgactttt ctgttcgtcg ccgtttctct gctggtaatt gcgcccggct gcgatggcat     180

gcaaattttc gtgaagacat tgacgggcaa aacgatcact ttggaggtgg agagctcgga     240

cactgtggac aatgtgaagg agaagatcca agagaaggag ggcattccgc cggatcagca     300

acggctgatc ttcgccggca aacagctcga ggacggacga acgttggccg actacaacat     360

acagaaggag tccacgctcc acttggtcct ccgtctccgg ggcggaaatg ggaagagaaa     420

cacgggtaag aacaagaaaa gcaacaaaaa gcttgatcag aattgatcag cagcggacgc     480

accatcacat gattgattgg cacagtgatt ttccaccacc aaattcacaa cacttttccc     540

ttttccgggt tatctttgat ttccattttc ggttattgtt ttctgttttt ctcctctatc     600

ttttgtaaaa ttctgttact ttgatcttta gtgtttatca tttatcatct gcatatatag     660

aagttatcat agtccatatt tctgtccctt tttatttatt ttttttgact ataaaacttt     720

tgggcacaaa aaaaaaaaaa aaaaaa                                          746

<210>  12
<211>  639
<212>  DNA
<213>  Heterodera glycines

<400>  12
atccaaatca acgcggcaca gtaataaaat attaaaagat ccgaaaaaat gccaaacatt      60

ttcaaaatcc ttctgattgt gcttttggcc gtcgtctcat tccgcctctc ggcttctact     120

ggtgacaaaa aaactgctaa tgatgggagt ggaaacaact catcagctgg gattggtacg     180

aagatcaaaa gaattgtcac cgctggactg ctcttcactt ccctggcgac gggtggggcg     240

gaagcgattg ggcgaagcaa tgctcaggga ggaaatgccg ccggattggt gccatcgcat     300

gtgaccaatc gctcaatggc tccaccacct cctcctgtgc aatttgaaat gggggcaaat     360

cgattagaaa aaatgagggc acacctacgc gaacttgctg agaaaatgcc ggtcaatgaa     420

tcgaagcgtc tgtcaccgag tggacccgac cctcatcatc attagggcca tggatggatc     480

taacggagga agaaagaatg gatgcttagt tttcagattt tattctatcc tcttttattt     540

attagttttt caaaacaaat ttcttccaca actttttaaa ctatttgttg ttttgaatct     600

ttttagataa atctaaaccc caaaaaaaaa aaaaaaaaa 639

<210> 13
<211> 1519
<212> DNA
<213> Heterodera glycines

<400> 13
gaaaatttga ttttatacaa taaaaatatt ataatatttt tgaataaaaa tgaaaaattc 60

tttcctcttc ctgcttcaaa tttttattct aaccaacatt ttaactgaaa tactttgtgg 120

agataagtca aggccgtcga cggaaatcaa tgccaatttg ggaacaagga aaaagcctga 180

aacaattacg gcaacaaaaa atgccaattt gggaacagga aaaatgcatg aaaccgatgg 240

gaccagcaaa atgccaaaac atggcaaacc tgtgtcaaat cgaatggcag ccaaaagcac 300

gacgattaaa aataataatg aagcagcagg gccaagccaa caatcaaaac aacctgcggc 360

aaacattacg ccacagcaaa aggggccaat gcaaaattca aaaaagcagc caccaaacga 420

attgtttggc aagaagcaaa ggtcaacccc tgaagagatc aaagctggca aacaaccagc 480

aatggagcta atgccatgct atcgaggaat cggcaaaaag acgatgccaa caattgcaca 540

gcgaaatgcc accatgctaa aaagggggga cagtttgaca cggagtgccg actttgagga 600

cccaattttg gccaatgtaa aacaaagcca aatgttgatc cgacaaaaat ccaagggcac 660

catggacttg gaaatggacg aattcttaaa actgcacaag caaattcaag gacctcggca 720

aaaattggta cgaatggaaa gggaaaaaat gatcaaagaa gcgaccgaaa aggcaaaagt 780

ggaccgaaga aatcgtatgt tggaacgaga aaaaatgtgg agcatgaaga aggctggtgt 840

tgctgcacat caaccagcat caccggcaat tcgtggacga acacagcagc aacaacagca 900

gaagctgcaa cctgacaaaa agccggaaaa aatgctgaaa cagcaaaaca aaacacaaaa 960

tttttcggca ccttccacca gcaaacaaat tgtcaatcga aggttgttga ttggcccaaa 1020

caaaccgaga actggcaaca aaattggcac taccaagcac ggcatacatt cggcggaaat 1080

aatgacatca tcgtcggaaa gcaatgtgcc aaaaagtgaa aatttgggca gcagtgaaat 1140

ggagacggcg gaggaagcgg cacaaaccta tttgaacaat ttacgggttg atttaaataa 1200

aattccttcg atgaaaattc cattccttga accaataatt gaaaaatatg ttgatgaata 1260

caaaaaaagt cgtcaattat tcgctgaatg catgcaaaaa attttgcgaa aatcgaaaaa 1320

tcctaatcaa acggtgaaga tcatcgaatt taaaaagaca attgaaaaag aaatgaatga 1380

tttgttgaaa catacggatt tgccaccaga tcaattaatt gatctgttga acactcgtga 1440

atggctattg tcgatgaaaa aagacattga gtacatggaa cgcattctga ccggacgcga 1500

gaataatgac aacatttga 1519

<210> 14

<211>  700
<212>  DNA
<213>  Heterodera glycines

<400>  14
ggaattattt aataaacaaa aaaacaatta attaccgcca attgcccatt agaagaaatg      60

ttcagctctt ccaatttgtc tgctctcttt ttggcctcct ccgttttggc cgtgcttata     120

attggcatta aaatggacgg accgacggag gcaaaaggcg ccgcccctcc aaacgccgcg     180

gggccaatgg gacttttgct tttattgaat ggcaaacaat cggcggctaa tgaaaaggga     240

aaagcgccct ctggcgaaag taagccaaat ccggggcaga agccgagcgg agaacggcga     300

aagagggacg ttttggggca cgccggcgga tacgtcggag gatgggacca tcccattgac     360

tcgacacttg attgggcaaa gagtcagtgg aatgatgcca attggctcgc cgatgttgtc     420

aacagaaacg gatgggaaaa caccggcact ccaaccggcg gacgatgaat cagtgaattg     480

tgccgaccaa ggaatgaaag acggcatttt tgtttgggaa tttaattgac ttttcggaat     540

caaacacact ttccttttta gttgcctatt tattctaaat tagcgttttt tctgttcatt     600

ttctacgcaa aacaaatttt ttacatattt tttggttggg gattatccca atggtcccat     660

ttttcgcata aatatgaatt gataaaaaaa aaaaaaaaaa                           700


<210>  15
<211>  975
<212>  DNA
<213>  Heterodera glycines

<400>  15
atatcacatt ttcatttaaa ttatcctccc aaaatgcgta ccattctctt catggccatg      60

gtttgcttgg tgatggctgt cctaatggaa atggcaaatt caaaagtagt caaaaaagac     120

aataaaaaag cagcagtggc ggcatctcca gcaaaaggaa aagcatcgcc aaaaggaggc     180

aaaagcccag caaaaggaaa agcggcaaaa gttagtaaaa aagataacaa attgaaagca     240

aagaaagaaa ccaaaggcat taaagttaaa aatgcaaagc caacaaaaaa aggtaaatcg     300

gcaaaaggcg catcaaaaac agccaagaaa gtccaagccg ccaaaaaagc accaggaaag     360

gacaaaaagt cgctagttaa gccaatcgtc cttaaagcac cggtaccacc ccataaaatg     420

cacccgatga ttgaatccga agctgtgcca ccgcccgccc atgcgcgttc gcttgcgacc     480

gttccttaca gtacaccggg ggcagccgac cgtaactcat tgccatcgta cacttcgact     540

gccaccaatt tggacatggc cgatgataat gatgattatc agaattatta ctacggaacg     600

gaagacagca gcagggaatt tgatgcatcg gcggaggagg aggatgaact atatgagaga     660

ggagagtcgg ggcaatgagt gggcagacgg acgactacgg tattaggcag tggacagaga     720

tgagtacggt catcaaccgg tggacgtgcc gccatagtcg gtgatgctcg actgaagagc     780

atcaatgaaa tcaatcagag atcaccggaa aatcattttt tcgtaatata gaccaaaatt     840

tatagttgct aatttttgta tatttttatt tgtaaaaata gttttttatt aataaatata 900

actcttacaa ttatctcatt tataattttg ctgaagtaaa acaacgaaaa aaaaaaaaaa 960

aaaaaaaaaa aaaaa 975

<210> 16
<211> 1333
<212> DNA
<213> Heterodera glycines

<400> 16
ccaactaatt ttctcccaat ctaaaaaagt ctctctaatt aatactaatt agtacaaact 60

gctgttgatt tgatcacaga atgttggttc aactcgtcct ccttgccatc attggcattt 120

cctttgtcgg tgctgccgcg ccgccgtacg gccaattgtc cgtctccggc accaaattgg 180

ttggctcaaa cggcaaaccg gtgcagctga tcggcaattc gttgttctgg caccagtggt 240

acccacaatt ttggaatact gaaacagtga aggcactcaa atgcaattgg aattccaatg 300

tcgtgcgcac cgcaatgggc gtggaacagg gcggctatct gagtgacgcg aacaccgcct 360

accgactgac ggcagctgtg attgaggcgg ccattgcaca gggcatttac gtgatcgtcg 420

attggcacgc gcatgaggcg aacgcggaca aagcgattga attcttcacc aaagttgcga 480

aagcgtacgg ctccaaccct cacttgcttt acgaaacgtt taacgagccg ttggacgtgt 540

cttggaacga tgtgcttgtc ccgtaccata aaaaggttat ttctgcaatt cgtgccatcg 600

acaaaaagaa tgtgatcatt ctcggcactc ccaaatggtc tcaagatgtt gacgtggcgg 660

cccaaaatcc gatcaaagga ttcggtaatt tgatgtacac tctccacttc tatgcgtcca 720

gtcactttgt tgatggactt ggaaataagc ttaagaccgc cgtaaacaag ggtcttccgg 780

tgttcgtcac tgagtacggt acatgcgaag cgtctggcaa tggtaatctg aataccaatt 840

caatgtcaag ctggtggagc ctgctggacc aactgcaaat ttcgtacgtc aattggtcaa 900

tcactgacaa aagcgaagct tgtgcagcgc tcactggcgg aacatcggct gccaatgttg 960

gcacttcctc ccgctggacg cagtctggca atatggtagc ttcgcaacac aagaaaaaat 1020

ccaccggtgt gaactgcagc ggtggtggtg gcgctgctgc taagccagct gctaagcccg 1080

ccgctaagcc agctgctaaa tcgaagggaa agtcttccaa agccaagaag tccggatgat 1140

cagcaaatca caataaacat agaaagtgaa ttgaagacaa tatggtgatt caaaaaacaa 1200

ataagtgcat aatgataatt ttaagtataa ttgtaatatt caaaaatatt cttaggagta 1260

aatcgggcac tgataaagca tgaactatta ttaaaaaaaa aaaaaaaaaa aaaaaaaaaa 1320

aaaaaaaaaa aaa 1333

<210> 17
<211> 330

<212>  DNA
<213>  Heterodera glycines

<400>  17
atgtacaaat ttttattttt ccctcatttt tgtatcttaa ttaaggcaat ttcggggctc      60

cattgttgga attcgaacat gattttgctg agtgaaatgc cggaaaaggg cagtgtcact     120

gtacaccaat gtccgtccgg gcatcaatgc gtgacggcaa attgttggct tggagtcggc     180

aattacattg tccaaaaatg cgtgccggac cagccagggg ccagaaacta ttgcaaagac     240

ttcaacaaca tttgccaaat gggctttttc ggcatgccaa ttcattgctc atcttgtaag     300

ggtgatctgt gcaacgaagc gcgtaattaa                                      330


<210>  18
<211>  1485
<212>  DNA
<213>  Heterodera glycines

<400>  18
agaatttaaa aatcataaaa attcaaactc atcaaattca ctcaacatca tcaccaaccg      60

tgaacattat gctgaggatt gctctactca tctccatttt ggcactgttt ggtgattgca     120

tggacatggg aaaaagaaaa ttaggaggaa tcagtattaa tgagccaagt gaatatggaa     180

ccaaagaaaa agaagccatc gcaacaaaag aaaatgcaca aacatcaaag gacccgccga     240

catcggcggg tggtcaaaat gaagcaatcc cttcaccaaa aaagccaagc cccaagggga     300

agttgaaaag cgattttggc ctaaacttag ccaaggcttt tccacggccg gttccgaaag     360

gcagaagggg caaagaaaaa gtcggcgaaa aaataagcgt tatgaacaca gctgaacgca     420

ttgaaaaaat ggacattgcc caagacaaag ctaaaatgga aatagacaat atagacacca     480

atgccaaacc caaacaaaat gaggataaag ctccaaccat attccccaaa tgtgcggaca     540

acgtggaagt ggaagtagag ctcgatcgta atatttttcg tttttctact acgcttgaca     600

caatgatgga agatcttgga atgtacactg ctgaaggcac aaaccagaaa ttgccggttt     660

caaatgtcag tagtacggtg atgcgagaag tgattgaatg gtgcgagcat cacaaaaacg     720

atgcctcaat agagccaatt tatgaagaaa ttgctttgga tgtgccaact ggtaaagatg     780

cggaggcatc cgcaccaaat gctcaagctg gagaagttgc ggaggcagcc gaatcaaatg     840

ccaaaccaaa taatgaaaag cgtctcgtct ttccaagctg ggatgagaat tttttggata     900

aggaatggcc tgagcttgtt gatataattt tagcagccaa ctatttgaac atcaaacttt     960

tgcttacctt cgcgaccaca atggttgata acaagtggat caatggcaaa acgccgcagg    1020

aaattcgcaa gggattcggc gtcgaagagc cgtacccgcc gggacatccg gaatgggcac    1080

gagttgagaa ggagaacgag tgggaagaat cggacgagga acgtgaggca cgccatgcaa    1140

aggaacgaga ggaggaagag gagcgtgaga gaaaggaaga acagaagcgt aaggaagagg    1200

aagcggaacg cctccatcag gaacaactgc agcaacaaca gaatcaggaa cagcaacctc 1260

agtagggaca gcagcatggt gaagaactgg aacacgatga agttatgcat gatgtggagg 1320

aagagcaaga tgatgaatgg cgaggaagaa gatgatgagt gatgaggatg aggaggatgt 1380

aaaatgatgt ggtgattagt gattttgatg aaccgatgat tcacttttct tggatcctgt 1440

tgcataaaac ttgttgcaaa aaaaaaaaaa aaaaaaaaaa aaaaa 1485

<210> 19
<211> 1250
<212> DNA
<213> Heterodera glycines

<220>
<221> misc_feature
<222> (1222)..(1222)
<223> n is a, c, g, or t

<400> 19
gagaatttaa aaatcataaa aattcaaact catcaaattc actcaacatc atcaccaacc 60

gtgaacatta tgctgaggat tgctctactc atctccattt tggcactgtt tggtgattgc 120

atggacaagg gaaaaagaaa attaggagga atcagtatta atgagccaag tgaatatgga 180

accaaagaaa aagaagccat cgcaacaaaa gaaaatgctc taacatcaaa ggacccgccg 240

acatcggcgg gtggtcaaaa tgaagcaatc ccttcaccaa aaaagccaag ccccaagggg 300

aagttgaaaa gcgattttgg cctaaactta gccaaggctt ttccacggcc ggttccgaaa 360

ggaaaaaaag ttgaaaatac actatcatca aaggactcgg catctgaagc tcacaaagaa 420

ttggccaaag agaaaaatga agaaaatgca cagacctcaa aggtcccgac atcgatggaa 480

aggcaaaatg aaccaatccc ttcaccaaaa aagccaagcc ccaaggggaa gtccaaaagc 540

gatttcgccc taaacttggc caaagctttt ccacgaccag ttgccgaagc aaaaatggga 600

gaagaagctc agtcttcaaa agatccgaca atgaatgcca aatttgtgca atttgtttgg 660

atgcatcgct tatcactgac cttgaaattg agcaaatgcc atcatcgctt tcaccgcgaa 720

tgcgttgatg ggtggtttaa aaacaatgac acatgccctt attgtcgtgc tgtagttgca 780

agcagatatt taccaagacc tacgcgtaca gatcgaattt ttgacgccag aatcgaaaac 840

aaaagacgct tcatgggaga aggagaagga aaatacacaa ttattcgccc taacggaagt 900

acgcttatgg ttcacgataa tcattttgga aacaatttta cggtcgaaaa aactgaagag 960

ggctccattc aactcagtaa aaacgatcgc aaatagaaaa attgtttcaa gattaattaa 1020

aaaatgtcag ggagggttgc ggaagccgga aagaattatg gataaaaaac ctaaaaaaaa 1080

tcggaggcta aacaaatttc gaaactcatc taaaccgcaa ttcggatcca tatccctgat 1140

atgtaatata catttatgca tcagggatgc attcggaaat atgtattggt actgagtcgg 1200

aatttttaac gtttcggcat ancaataaat atttaatcaa aaaaaaaaaa 1250

<210> 20
<211> 897
<212> DNA
<213> Heterodera glycines

<400> 20
atatctatca tattttcatt taaattatcc tcccaaaatg cgcaccattc tcttcatagc 60

catggtttgc ttggtgatgg ctgtcctaat ggaaatggca aattcaaagg ctgtcaaaaa 120

agacaacaaa aaaggagcag tggcggcatc accagcaaaa ggaaaagcat cgccaaaagg 180

aggcaaaagc ccagcaaaag gaaaagcggc aaaaaaattg aaacctaaaa aggatgctaa 240

aggcattaaa gctaaaaaag caaagccagc aaagaaaggc aaagcggcaa aagcagtaaa 300

gggagcgcct aagacagtca aaaaactcgc aattgccaaa acagcacaag caaaagacaa 360

aaagtcacca gccaagccaa tggttctaaa agcagtgccg ccccaccaaa tgcatttaat 420

gaatgagaaa gttcaaacag ctgtgtctcc acccgcccat gctcgttcac ttgcgaccgt 480

tccttacagt acaccggtgg cagccgaccg taactcactg ccatcgtaca cttcggatgg 540

caccaatttg gacatggccc atgagaatga tgattatcag aattattact acggatcggg 600

agacagcagc aaagaatttg atgcatcggc tgaagaggaa gatggactgt atgaggaggg 660

ggacggggca atgaattggg caaacggacg actaccggat tgggcattga acaggaatac 720

gggcatcaat cggggggagt ggccaccaaa gtcggggatg ctccaactga aaaacaccaa 780

gaattttgga aaaaacttat tttccaaaat tttgaaacat ttggatttta atattcccgt 840

taaaaaacta aatttttttt tacccagcct ctaattttgt aaaaaaaaaa aaaaaaa 897

<210> 21
<211> 530
<212> DNA
<213> Heterodera glycines

<400> 21
gattcattcc ctcaaaagca aagccatcca tccgatcatt cgcttcatct gtcaatccgc 60

aaaatgtccc ttctccgtcc tcaatcgctg cttcttgtgc ccgctctttc cctgtccttt 120

gcgctgctct ttgtcacttc gtcggaagag ggagggcgag tgaagcccgg cggatgccct 180

tgggattggg ccggcaaaca actgtgcaaa acatcggcaa attgcaagtg caaggatggc 240

aaaagttggg ccaaatgtgt aaagtcggaa ggctacgcgg ccagcaattg ttgcgacaaa 300

aattacgtgt gggcatgttg cgggaagaag cccaaacatt gatgagagaa gagaacggga 360

gagaatgaga agcgagttcc cataattcat tctgttttgg atttttagtc tttttttctt 420

tttgggtttt ttttggtaaa tattttcttt agttattaaa aattgtttgg tctaattatg 480

ccattggaaa aaatgtattt gttttttaca aaaaaaaaaa aaaaaaaaaa 530

<210> 22
<211> 673
<212> DNA
<213> Heterodera glycines

<400> 22
ggctccaaaa aaaatccatt ccacattcca gcaaccaata ccattaattt cataaatccg 60

aagaaatccc tacaaaaatg tcttcttccc cgtccgtctc tgtgctcgcc atcgtcgcaa 120

ttgtctgttt gatgtgccaa tgttgctttt cggcaccgca tccgtgctgt cccggcagtc 180

aaaaagtggt ttcacttatg tccaattacg ttggcacttt tgccaattcc atttccaagt 240

catcgctttg ttcggatgcc caaaatgttg cggacgcgtt gaaaggccaa ctgatcggct 300

gctcgaatgg cggcgatcga actcttttgg ccgacatcga agcatctctt gccagtcatt 360

ctgctgatga gtgtgcccac agcctcggct tcgtccgtgc catgttcgcc attgccgcct 420

ctgcttcttc tcatgccagc aacaacagcg aatggcaggc attgagtaca cagtttgttc 480

agaaagtcac tgaaattgac tcgaaatgtg cagagtttgg cattagcatt ggaaaagtgc 540

ccatcgatgg ccccaaggga gaccactccc aacgaaatgt gcctagtacg gacagtgtga 600

tttccatgcc cggattgacc ggctcacaca aacattgaac tgaatgatga gtgacggaat 660

ggataaacta att 673

<210> 23
<211> 1614
<212> DNA
<213> Heterodera glycines

<220>
<221> misc_feature
<222> (1157)..(1157)
<223> n is a, c, g, or t

<400> 23
atggccctct ctgcccttct gcttcttctc cccctgcttc tcaatgttca aaatatccca 60

gatgagtcag tccaatcgga tatgaaggct gtttattcgg ctatttcatc gccggaacaa 120

tggaagaact cacaaaattc attggcttca ctcgaatcac aactgacaga gccccaaaga 180

gcactggcaa aaatgcattg ggaattggag accatccaaa aggaaaagcc ggaggcaccg 240

ccacaattcg acttgggact tttctcggaa gctttggaag tgatggtcga aatggacgaa 300

gaagcgaaag aagtgaagct gaggaaggac aaactgatcc gaatgggcag gaggagagga 360

agcaaaaaaa aattgaagga aaagatgacg gaggaggaca cagtgccgga agtgagggtg 420

aacgagaatg gtaaggttga agtgaaggac ggcgccggag gggaaggacg gttggaagtc 480

agacgaacaa aggacgagaa aggcagagag caggtggtga tcacccttat gaagaatggc 540

ggaacggagg gaccagcgga aggaaccgcg gagaagccac aagagaaggc caagacggag 600

gaggaggtac agaaaaagaa tgatgacaaa agtacacgac aagaacagga ggaagcgaag 660

aagacggagc aggaaaacgc cggaggggtg ccaaaaactg actcggccaa cagccacatt 720

ccggtaatgc caatgcacac cattttgtca tccccgtctc caccggtgga ggagaagggc 780

aaagcgagtg cagaggaggc acaaaaaaag gaggaggaca aaaacacacg acgtggaggg 840

gaggaggcaa acaacacgga gcagaacaaa gtcggaggag tgccaaaaat gtcagttgac 900

agtcctaagt cggtcgtgcc aatgccaatg cacaccattt tgtcctcacc agccccaccg 960

gtcgaggagc aggacaaggc aagggatgcg ctcacagaag gagcaaatgg aaggaaaaag 1020

gcgcaaaaca acgaagaaat gttgctggtt gcgacggaga acaacggaag catgagaaat 1080

acgaataatg gaggaatatt tgattttgtc cgaaaattta tctccaatgt gtttggacgt 1140

aagaaaaggg acacggnaag cggtgcacag agaaaatatg acgggggaac gaaggaaaat 1200

tcgcagcatt caaaatttga ttatgagcac tgtagggaag tccgactcag agccgaagag 1260

cgaagacaaa cagcagcagg aagagaaaaa gaaggcggag atcaagcagt acctggagaa 1320

gggagtggtc aacacgggtg gaatcaaaaa ggccgagaaa ctggcatatg cttggtactc 1380

ggagcttctg tactggacaa ccaagtggat cgaggcatta gaaaatcggg tggcgggagt 1440

caaacccgaa ttggcacagc aattcttatt ctccaaaacg ggatcagctg cttaccagga 1500

gctgaaggaa gaggtggaca aatgcgaggc gaagttggcc aaactaaagg aatggatcgg 1560

cgactctttc aagtgaaaat ggaagaaacg gctcttagag gtgtattttt ctga 1614

<210> 24
<211> 909
<212> DNA
<213> Heterodera glycines

<220>
<221> misc_feature
<222> (613)..(613)
<223> n is a, c, g, or t

<400> 24
ttcccttatg gccatggttt gcttggtgat ggctgtccta atggaaatgg caaattcaaa 60

agcagtcaaa aaagacaata aaaaaggagc agtggcggca tcaccagcaa aaggaaaagc 120

atcgccaaaa ggaggcaaaa gcccagcaaa aggaaaagcg gcaaaagtta gtaaaaaaga 180

taacaaattg aaagcaaaga aagaaaccaa aggcattaaa gttaaaaatg caaagccaac 240

aaaaaaaggt aaatcggcaa aaggcgcacc aaaaacagcc aagaaagtcc aagccgccaa 300

aaaagcacca ggaaaggaca aaaagtcgct agttaagcca atcgtcctta aagcaccggt 360

accaccccat aaaatgcacc cgatgattga atccgaagct gtgccaccgc ccgcccatgc 420

gcgttcgctt gcgaccgttc cttacagtac accgggggca gccgaccgta actcattgcc 480

atcgtacact tcggctggca ccaatttgga catggccgat gataatgatg attatcagaa 540

ttattactac ggaacggaag acggcagcag ggaatttgat gcatcggcgg aggaggagga 600

tgaactatat gangagggag gtcggggcaa tgagtgggca gacggacgac tacggtattg 660

gcagtggaca ggatgagtac ggtcatcaac cggtggacgt gccgccatag tcggtgatgc 720

tcgactgaag agcatcaatg aaatcaatca gagatcaccg gaaaatcatt ttttcgtaat 780

atagaccaaa atttatagtt gctaattttt gtatattttt atttgtaaaa atagtttttt 840

attaataaat ataactctta caattatctc atttataatt ttgctgaagt aaaacaacga 900

aaaaaaaaa 909

<210> 25
<211> 1278
<212> DNA
<213> Heterodera glycines

<400> 25
attttatttt atctacaact caataaaaaa tgtccgcttg gatttgtcct gggtttttgc 60

tgttttttgt gctcattctg atcactgatc actgtccgat gacccaagct gtcactcaac 120

cgccctatgg tcaattggct gtcagtggaa aaaatctagt acaaaaaagt acgaaaaaag 180

ccgtggcatt gcacggcctg tcaatgtact ggagccaatg ggtgccgaag ttttgggtga 240

aacagaccgt caacaaatta aaatgttcat gcaatgccaa cgttgtacgc gcagcaatgg 300

catccagctt tggcggttac atttcaaacc caactgctga atataaaaaa atgactgcaa 360

tcattgatgg ggcaattgac caaggccttt acgtggttgt cgattggcac acgggcgacg 420

atttggccac caccgaaata aaatatgcga ctgaattctt tacaaaaatt gctaaagcct 480

ataataaata cccgcacatt ttgtacgaaa tttggaatga gcccaacaaa tttgtggcat 540

gggaagcggt ggtcaaacca tacgcaaaga cgatgattga cgtgattcgt aaatatgaca 600

aaaacaatgt gatcattgtt ggcacgccaa actgggacca agatgttgac attgttgcaa 660

aaagtccgtt gaaagacgca aatattattt acactatgca cttttacgcc ggccaacaca 720

aagatgacat ccgaaacaaa gtcaaaacgg cttataaatt gggccttcca atgtttgtga 780

ctgagtacgg ttgctactcg gctgacggca atgaaaaaac aaatttggaa gagttgaaaa 840

agtggatgga acttttggac ggctatggca tttcctacgc cgcgtggcac gtggcggaca 900

ttggcgaaca gtcgtccata ctgacaaaga cttccgaagt gaacaacatc tgtgacccgg 960

cccatttgac caactacgga aaagccatca ttaacaaatt gaaatcgcaa aacaatggtg 1020

tgaaatgcag tggttagcca accaatttca attaatcggc ggatgttatc gaaaatgtat 1080

tcaaaagtta aatattcaaa aaagtttact aacccaatta ttgttattaa tttcggtgtc 1140

aataattgta attgtaattt tgaaaactct taataattat gtactattat tttaattcaa 1200

ttcactagca aattttttaa atctaaaaac tttgaaatgt gaatttaaat catttaaaaa 1260

cgaaaaaaaa aaaaaaaa 1278

<210> 26
<211> 676
<212> DNA
<213> Heterodera glycines

<400> 26
atcctctaag aattcaaatc ctctcagaaa atggcttcat ctttctgctc ttcactcatt 60

tccatcgtcg caattgtctg tttgctgtgc aaatgctgct tttcagcacc ccatccatgc 120

tgtcctggca gccaacatgt tgtttcgatg atgaaagatc acaccggcac attctccgct 180

tcgatgccaa agtcttcgct ttgtctgagt gccgaaagag tcgccgctgc ggtggaaaac 240

caactgaaaa caatttggtg ccctggcaat ggtggccaaa cactcatcaa cgagatcaac 300

gcagctcaat catcatctga tgagtgtgct cgctctctcg gcttcgtccg tgccatgttc 360

gaaattgccg cttccgccgc ttcccatgtc ggtgccaacg ccgaattggc caatttggct 420

gtccagttcc gagaacaagt tggcacaatt gacaccaact gtgctgcgct gggcattcat 480

gttgggcaaa tcagcttggg cactcccaaa ggagaccatc cgcaagtgca tgactctgag 540

agtgtgctta gtaaccctgg caccagcggg tctcacaagc gcatttaagt gcattgcgac 600

gattccgatg atgtcatcat ttgttgattg atatgctata gaaattattt tattagataa 660

aaaatgaatc gttgct 676

<210> 27
<211> 978
<212> DNA
<213> Heterodera glycines

<400> 27
gattaaaaaa gaatttcttc aactaaaatc acaaacacat acaataagaa aatgattctt 60

ttcattctat tggtcataac ttttgtccaa attggtcaac tgagtgctgg catatgcaaa 120

tttccaaatc cgtcaaaaag tgttacggtc caatcgatga tgactgtttc atcgaatacc 180

gactataaga acactttgtt tgtcggcggt tccggcattt tgaacggcgc gtgtgatgtg 240

aacaatggca aactgaaata tttgatgaca ttgaagaatg gcgtgaccat caaaaatgcc 300

attctcgaca cgcccggttt gggcatttac tgcgagggca attgtgtgct ggaaaatatt 360

tactacaagc gattgtgtta ccatgctact gggttcgggt ataagagcac cagcacttca 420

tacacttacc aagtgattgg cggtgctggc caaggttcac cggacaaata tttcacccaa 480

tcgggcaaag gcacaaccat catcaagaat ttctgcgcag aaggcaaata cggcaaattg 540

tggtgttcat gcgggaattg tgctttccaa acggcgcgta ccgtacaaat ttcaaacacc 600

gtccttaagg ggcccggact ttcggttgtc tcgcttaatt ccaactacgg tgacaaaatg 660

tcactttcgg gactgacttt gcatggccaa aaatcatcga gcacaaaaac taattacatt 720

tgccaagagt acaaaggcct cactcacatg gccgccatga gtcctcaagc gaattatgag 780

ccaaccaaat cgggcagtgg cacatgcgcg tacagtgcct cggcagttaa aattgcgagc 840

taatttggag aaataaatga ataaataatg gagggttgag tgatgaaagt aaatttagaa 900

attaaatgat gttaatacaa cctgttaatt gtcggacgaa tataaataaa gattaaaaaa 960

aaaaaaaaaa aaaaaaaa 978

<210> 28
<211> 632
<212> DNA
<213> Heterodera glycines

<400> 28
aatggcattt ctcctgttgt cacttgcacc cctcttcgtt gcccttagcg ggattgatgc 60

aaataaagtg ccactgcaat tgtgctgtgc tggtggccaa agcttggctg gccttatcta 120

cgactattct gaccatttca gcgaagtttt gcaacagtcg ggaactgaga tgtgcactag 180

cctgaaaaag gacatgacca aattgacaac tgcaattgaa aaagctgaag gatgcaagtg 240

caatcttgtt gaaaaaattg agaaggaatt ggatgatgtt gaggatgatt gcgcctacaa 300

tttgtcactt cattcgcgca ctgttccaca ttgtggccat ggcctcggaa catttgggcg 360

aaagttggaa ctccgaagcg gccgagttca acgagcaaat tgaagccatt gatagcattg 420

gacatgatga acttaagaag cttgagtttg aagcggaatc ggaattgaaa acacatcctc 480

gtgttggccg aaaaagaatg aacagatgct aattgggctg ggcaaaattt gaagatgtca 540

atgtgtattt agacgaatta atttgtcgat ctttcattag tttaatgaca cattatggca 600

ataattaata gaagaataaa tatatttttt tg 632

<210> 29
<211> 679
<212> DNA
<213> Heterodera glycines

<400> 29
   tattttatta aaaatgcatt taaaaatatt tttattaatt ttatttgtga caatttgctc 60
   ggtttggtca ctttcaccca aaatccacca caactaccac caacagcagc aacagcacaa 120
   cattaagaat aaattgccac caaaaaatat tgggacaaaa catttgaaaa cggaaattaa 180
   tgggcacaac aaacacaagc acaacccggc gagcagacac gggcatgctt tgcttcaaaa 240
   agtgaaagac caattgaata atgatggatg ggccagtgaa gatgcaaaca aaacatcggt 300
   attgctctct tcactgttgg ccatcgaatg ccatatgaaa aaattggaca agaaaaggac 360
   caacgactcg gccgagatca aacatttgac ggagcaaatc tgtgccgccg agaacaaaga 420
   acaaattggc attcataaac tgataaatga agttgcgaaa acatttgaaa aagatggaaa 480
   gaaaatggta gaacaaatga aaagggacga gcatacggta aaggtgcatc ttgacgggca 540
   ctacggtacg ttggccgaga ttttgccaag cgccaaaaag cacgcgaagg caacaaggct 600
   tcgaacgtat catgcagtgc tccatttgtt gcatttgatc actccgaaga tggaggaggg 660
   accggccaaa gaagcgatg 679

<210> 30
<211> 848
<212> DNA
<213> Heterodera glycines

<400> 30
atggggtttt taaaaactat cgctttcctt tcgcttttgg cacttttttg cgcaacaatt 60

gctgaagaaa atggggcgac agaaggcaca gaagcggagg cgacgacagt gccaacactc 120

accgcagcgg cggagggagg caaagggggc gaagaggaag tgaccacggc gaagccgacg 180

gagacgagcg aaacggcacg ggaggagggg gaggagaagc acaaggcgaa cgaggggaaa 240

gcgactgagg gaagtggaac cagatcgttg tggcaaaaag tcacctgtat gttgttcggc 300

tttttcccat ttcgcgccgg ctgtccgggc aaaaacgata taaaagcggg gtcatcatca 360

catgtgagca gcacaattat gataattgag gaaaaagagg acagcgaatc ggacgaacaa 420

atggacggcg ccggagggga cgaggagaaa cggggaggag agaacggcag cgaagcgcag 480

ggcggcaaag gcgccgcaag ggacaacgaa atggaagacg aagaattgaa agtgaaagcc 540

gtggtggaag agacgacgac aaatgaacag ggcaaaactg agacaaacgt cgaacagagc 600

gaaaaggagg ggccgatgaa ctgaccgaaa gggaccgact gatcggcagg aacgtcatcc 660

ctgacgggga tttgtcacgg agaatagaaa caaaaaaagc cacttgcatt tgcgttgttt 720

tattttgttg ctacagtaat cacatagttc acctttgtag atgctttact gtactctttt 780

catttttctc tctttctgtc cctctctttc ttttctttcc gcttttggct tataaattcc 840

tttttctc 848

<210> 31
<211> 805
<212> DNA
<213> Heterodera glycines

<400> 31
tgatccgcta attattattt tgaattgaga tttattaaaa cctaattaat tttttttcta 60

ctcgaatttc ttttatggca tttgccattt caagtttaag cgctttaatt tcgctcgctt 120

tattggcagc accacaaaac tcgtaccaac agaaacctaa cgatgaaccg aaggtcttta 180

atgatatcca aaacgggtgt gatttgataa attgtaccga tggtcgaacc tgcggcattc 240

gagttgggct cgcaaaattc ggcaacagaa attttgagaa atttgctttt ccaaaatgta 300

taacaaatga aacggaacta aacatgaaca cggacaacga tggaaatgcg cacattgttc 360

ctaatggacc agggtgccaa atgatacaat gttctgttgg ctaccaatgt caagttcgaa 420

tttcaatttc caagcttggc aatttgccat atgctcagtc aattggaacc ttcccgcaat 480

gcgtggggcc taatgtaagc ttcaactcat caagttcaac tatcgagaat ggcccagggt 540

gtgaccaact tccgacgaaa tgtgacgaag gcacaaaatg cgtaactgcc gtcggaattg 600

cgaaatatgg caatttgcca tggtcgcaat ttttctggcc cttttgtaca tgaaacgatg 660

aaaagcgctg caactgtttt atattcagag gagccaaaaa acgacggaaa atttattttt 720

acctaattta acaactcttg tgagtaaatt tgtgatttaa aatgtattgt gatttatgat 780

tttttggtaa actaaaattc atttc 805

<210> 32
<211> 868
<212> DNA
<213> Heterodera glycines

<400> 32
   aaaatgcgca ccattctctt atagccatgg tttgcttggt gatggctgtc ctaatggaaa 60
   tggcaaattc aaaggctgtc aaaaaataca acaaaaaagg agcagtggcg gcatcaccag 120
   caaaaggaaa agcatcgcca aaaggaggca aaagcccagc aaaaggaaaa gcggcaaaaa 180
   aattgaaacc taaaaaggat gctaaaggca ttaaagctaa aaaagcaaag ccagcaaaga 240
   aaagcaaagc ggcaaaagca gtaaagggag cgcctaagac agtcaaaaaa ctcgcatttg 300
   ccaaaacagc agcgcaagtg aaagaaataa agtcaccagc caagccaatg gttctaaaag 360
   cagtgccgcc ccaccaaatg catttaatga atgagaaagt tcaaactgct gtgtctccac 420
   ccgcccatgc tcgttcgctt gcgaccgttc cttacagtac accggtggca gccgaccgta 480
   actcactgcc atcgtacact tcggatggca ccaatttgga catggcccat gagaatgatg 540
   attatcagaa ttattactac ggatcgggag acagcagcaa agaatttgat gcatcggctg 600
   aagaggaaga tggactgtat gaggaggggg acggggcaat gaattgggca aacggacgac 660
   taccggattg ggcattgaac aggaatacgg gcatcaatcg gggggagtgg ccaccaaagt 720
   cggggatgct ccaactgaaa aacaccaaga attttggaaa aaacttattt tccaaaattt 780
   tgaaacattt ggattttaat attcccgtta aaaaactaaa ttttttttta cccagcctct 840
   aattttgtaa aaaaaaaaaa aaaaaaaa 868

<210> 33
<211> 455
<212> DNA
<213> Heterodera glycines

<400> 33
tcagtgccca acgcaacgca atggccactt acctattcct cgctgcagct gccttcggag 60

cgggaattta ctgttggaag acgtaccttg gcccgatgct tgaccaaccg aagagcgacg 120

acgaggagaa gcccaagccg gaaggaggcg aagaagcgac gaccgccggc gaggaggaga 180

agaaggctgg cagcaagggc gacaagcaca agaagaagga caagaagggc gaaggcgcag 240

cagcagcacc agcggcggac aaaaaggagg agaagcgcaa ggacaaacac aaatccaagg 300

agtcggggaa gagcgacgac agcaaagcgg gcaagaagga caagcacggg aagaaggaca 360

agaagggcaa gaagaaggac accgccgatg catccaccgt ggacgaagcg ctgggagaac 420

agagcacagc ggagacgagc caagcagagg aatag 455

<210> 34
<211> 549
<212> DNA
<213> Heterodera glycines

<400> 34
atggcttcat ctttccgctc ctcaatcatt tccatcgtcg caattgtctg tttgctgtgc 60

aaatgctgct tttcggcacc ccatccatgc tgtcctggca gccaacatgt cgtttcgatg 120

atgaaagatc acaccggcac attctccgct tcgatgccaa agtcttcgct ttgtatgagt 180

gccgaaagag tcgccgctgc ggtggaaaac caactgaaaa caatttggtg ccctggcaat 240

ggtagtcaaa cactcatcaa cgagatcaac gcaactcaat catcatcaga tgagtgtgct 300

cgcagtctcg gcttcgtccg tgccatgttc gaaattgccg cttccgccgc ttcccatgcc 360

ggtgccaacg ccgatttggc tgtccagttc cgagaacaag ttggcacaat tgacagcaat 420

tgtgccgcgt tgggcattca tgttgggcaa atcagcttgg atgcccccaa gggagaccat 480

ccgcaagtgc atgactctga gagtgtgctt agtaaccctg gcaccagcgg gtctcacaag 540

cgcttttaa 549

<210> 35
<211> 657
<212> DNA
<213> Heterodera glycines

<400> 35
tctctctact aactgttgca catcccaatt ttactttctt ccttagtaag tagggcttgg 60

tgcacaatgc gcacttttct gttcatagcc gtagtcgggt tgatgttggc cgtcatcttg 120

gagaatgtaa atgcgacagg gaaaaattcg ccgacaaagg aacttataca tgagcaaggt 180

aacctacaga accatgctac cggacaatcg ccgccagaca gtccaaacca taaaaatgac 240

catgaaaacg agattagtaa cctacagaac cgtgctaccg gacaatcgcc gccagacagt 300

acaaaacggg aaacagaaca taaaattgag aaaggaaaac cgtcgccaga cagtccaaag 360

tatgatgagc aagccttaaa atggtgaaga aactgtgtgg aacgatgatg ccaaccatga 420

acaaacccaa agctgttgta gtagatgtag aaaaaaactg tggtatcatc acgctattat 480

tgttgtgtaa aaaacgatcc gtttttacct cttttcggga aaaaaaactg gaaaaaattc 540

acaaaaaaaa aaatctcaca ttgtgcagat tttattgaaa ttagccaatt aaaaaaaaca 600

gtgacgaaaa aaaaaaaaaa aaaaaaaaaa aaaaaaaaaa aaaaaaaaaa aaaaaaa 657

<210> 36
<211> 441
<212> DNA
<213> Heterodera glycines

<400> 36
atgtcttctt ccccgtccgt ctctgtgctc gccgtcgtcg caattgtctg tttgatgtgc 60

caatgttgct tttcggcacc gcatccgtgc tgtcccggca gtcaaaaagt ggtttcactt 120

atgtccaatt acgttggcac ttttgccaat tccatttcca agtcatcgct ttgttcggat 180

gcccaaaatg ttgcggacgc gttgaaaggc caactgatcg gctgctccgc ttcttcccat 240

gccagcaaca acagcgaatg gcagacattg agtgggcagt ttggtcagaa agtcactgag 300

attgactcga aatgtgcaga gtttggcatt agcattggca aagtgcccat aaacggtccc 360

aagggtgtcc atgttcaaaa tgtgcccaac tcggaaagtg tgatttctat gcctggattg 420

gccggctcac acacccaatg a 441

<210> 37
<211> 774
<212> DNA
<213> Heterodera glycines

<400> 37
   aattattttt tttaataata attttttaca aaatgagcaa ctttatattt gtcgcctttt 60
   taactgcagc gttttttagc tcaagcctcg ctctaccggc tccttatgat gctgaatcgg 120
   tggtatcttc tgaattaaat gttccactac tttcagctga cgcaaatgtt caagcagctg 180
   aaaatgttga gatacaaatt ccatcaattc cagcaccaat tgaacaacaa actgctgctc 240
   acattactca tccaactgaa actggcaatg agccttctat tgcatcatca tcatccgcgc 300
   cgaaaagtga gcagacgcca aaaaaagtga tgaacatgaa aagcgcgatg gaaggcgcgg 360
   cggccaacgt gtacgggggg cttccgttag accagcagcc caagacgatc gctgaagcgg 420
   cagcaaaagc aaagcagacg cccgccaaac ttccggctga ctacgacgcg aaccgcgtgg 480
   cggaacgcgc ggcggcccgt gtgtacgggt ggcttccgga agacaagcag cctaaggcga 540
   tctatgacgc ggcggagaag gcaaagaaca cgcccaaacc gccgggcgac tacgacgtgg 600
   agcgcgtggc gcaaaaggcg gcacggctcg tctacggtgt gctgcccatc ggcatgcagc 660
   ccagcttcgc cggccctagc actgacaaga gcaatgtcga cgactcggag aaaccttctg 720
   ctgctgcggc tggtgatgat gatgaagtcg aaaaaaaaaa aaaaaaaaaa aaaa 774

<210> 38
<211> 1451
<212> DNA
<213> Heterodera glycines

<400> 38
aattcgcttt catcgttcaa atgtgccgac tccaagcaac tcatctgctc gctcgactct 60

ttctgcttct tgcgctttgc actgctctcg ttagctctct cactgctgtt gccccgccat 120

tcggccaatt gtccgtttcc ggcaccaatt tggtcggcgc caacggacaa cccgtacagc 180

tgatcggcaa ctcactgttc tggcaccaat ggtacccgca gttttggaac gctgacacag 240

tgaaggcact caaatgcaat tggaatgcca atgtcatccg gggggccatg ggcgtggacg 300

agggcggcta tctgagtgac gcgaacacgg cttacaatct gatggtggca gtgatcgaag 360

cggccatttc caatggcatt tacgtgatcg tcgattggca tgcccacaat tcacatccgg 420

acgaagcggt caaattcttc acccgaattg ctcaagcgta cggctcctac cctcacattt 480

tgtacgagga tttcaacgag ccgctgagcg tttcgtggac cgatgtgctg gtgccatacc 540

acaaaaaggt cattgctgcc atccgagcta ttgacaagaa aaatgtgatc attctcggca 600

ctccaacatg gtcccaagac gtggatgtgg catcacagaa cccaatcaaa gactaccaaa 660

atctgatgta cactctccac ttttacgcgt ccagtcactt cacgaatgat cttggtgcca 720

agctcaaaac agccgtgaac aacggtttgc ctgtgttcgt cactgagtac ggcacatgcg 780

aagcgtcggg caacggcaac ttgaacactg actcgatgtc cagctggtgg actctgctgg 840

acagcttgaa gatttcatac gccaactggg caatctccga caaaagtgag gcctgctcag 900

cactgagccc cggtacaact gctgccaatg tcggtgtttc gtcccgttgg acatcctccg 960

gaaatatggt tgcttcgtac tacaagaaaa aatccaccgg cgtaagctgt agcggctcaa 1020

gttccggcag ctcttcgggc tcgagttctg gctcttccgg ttcaagttcc ggcagctctt 1080

ccggctcaag ttccggcagc tcttccggct caagttcggg ctcctctggt tcgagctcag 1140

ggtccagctc gggttcggga tccgccagca tctctgtagt cccatccaac acgtggaatg 1200

gcggtggtca ggtcaacttc gaaatcaaaa acatcgggtc cgtgccattg tgtggcgttg 1260

tgttcagcgt ctctcttccc tcagggacca cgcttggtgg atcgtggaac atggaatccg 1320

caggctccgg ccaatacagc ttgccaagtt gggtcagaat tgaggccgga aaatcgagca 1380

aagacgcggg gctgacattc aacggaaaag ataagccaac ggcgaaaatt gtgacgacga 1440

agaaatgtta g 1451

<210> 39

<211>  1149
<212>  DNA
<213>  Heterodera glycines

<400>  39
gaatttcatt atttcactga tttgcccatt acttttctta atgaactcct catttgtcat      60

ctgctgctgc ttccacaact ttgtgttgtt ccttcttttg agttgtgttg tgccacatgc     120

ttttgcttgt tgtcctcatg gaaatctcag agtgaaaggt acgcatttgg ccgatgaaaa     180

gggcgaaact gtgcagctgc gagggatgtc tttgtactgg agccaatggg agtacggctc     240

aaagttcttc aacgagaaga cggtcaactg cctgaagtgc agttggcatg ccgacattgt     300

ccgtgctccg ttagctgtgg accagggcgg ctatttgtca aatccagaac aggagtatgc     360

gaaggtgaag agcgtagtcc aagcagcaat cgacaaatgc atatatgtgc tgattgattg     420

gcattacacg agcagtgaga aatatacgga caaagcgaag gagttcttcg gcagaattag     480

cactctgtgc gctggcaaat gcaactgttt gtacgaaaca tggaacgaac ccatacaaaa     540

cgattggtcc tcgcagttga agccgtacca cgaggagctg gttaaagtga ttcggcagaa     600

tgacaagaac ggagtgataa tcgctggaac gccaaattat gaccaggatg tgaaggcggt     660

agtcaatgac ccaatcaaag agcacaatat aatgtacaca ttgcatttgt acgcggcatc     720

gcataagcaa gaactgcgca acacggcaca acaggcaatc ggcgcgggag tgccgttgtt     780

tgtcacggag tatggcacaa gttcgtacac aggcgatggc gggccagacc ttgtcgaaac     840

acagaattgg tacgactttt tcaacaaaaa ctccctctcg tacatcaact gggccatcga     900

cgacaagcag gaagcgtcgg tagcgctgca gaacacacag cccccagtcg ggccggccga     960

cgtgtgcagc accgatcggc tgaccaaatc gggcaaattt gtgcacgacc acctcatcgg    1020

tgtaaaccag aagccggatg gctgttaaac atcagaaaat ggcaaatgat cggaatcgcg    1080

cgctcttttt tttacttttc tgggtcattt catctttagc attaaaccta ataaaaaaaa    1140

aaaaaaaaa                                                            1149


<210>  40
<211>  880
<212>  DNA
<213>  Heterodera glycines

<400>  40
cgcccattct cttatagcca tggtttgctt ggtgatggct gtcctaatgg aaatggcaaa      60

ttcaaaggct gtcaaaaaag acaacaaaaa aggagcagtg gcggcatcac cagcaaaagg     120

aaaatcatcg ccaaaaggag gcaaaagccc agcaaaagga aaagcggcaa aaaaattgaa     180

acctaaaaag gatgctaaag gcattaaagc taaaaaagca aagccagcaa agaaaagcaa     240

agcggcaaaa gcagtaaagg gagcgcctaa gacagtcaaa aaactcgcaa ttgccaaaac     300

agcagcgcaa gtgaaagaaa taaagtcacc agccaagcca atggttctaa aagcagtgcc     360

gccccaccaa atgcatttaa tgaatgagaa agttcaaact gctgtgtctc cacccgccca 420

tgctcgttca cttgcgaccg ttccttacag tacaccgatg gcagccgacc gtaactcact 480

gccatcgtac acttcggatg gcaccaattt ggacatggcc catgagaatg atgattatca 540

gaattattac tacggaacgg aagacagcag cagagaattt gatgcatcgg cggaggagga 600

ggatgaactg tatgaggagg gggacggggc aatgagttgg cagacggacg actacggtat 660

tggcattgga caggagtacg gtcatcaatc ggtggaagtg ccaccaaagt cggtgatgct 720

cgactgaaga acaccagcag gatttttgta aaaacatatt tctcagattt ttgaacattt 780

gcattttata ttcgcttaga agctaatttt ttttatctag ctctaattat gtaaaaatta 840

ataattcttc gaaaaaaaaa aaaaaaaaaa aaaaaaaaaa 880

<210> 41
<211> 1523
<212> DNA
<213> Heterodera glycines

<400> 41
catgaacatt ctgctgaagt ctgttctatt catctctctt ctgtcactgt ttggtgattg 60

catgaaaaac tcagatcaaa ttggagtcaa aagaaaatca gcaggaataa gaattgagga 120

accaaatgat gtaaaaggca aaggaaaagt cgtcgaaaca agcagaaaag gcaaagaaaa 180

agtcggcgaa aaaataagcg ttatgaacac agctgaacgc attgaaaaaa tggaccttgc 240

ccaagacaaa gctaaaatgg atatagacac caatggcaaa cccaaacaaa atgaggaaaa 300

tgctcctcca aaatatgttt tcatttggag cggtaacaat gagaagaaga accttgaggt 360

tgacctcaga gtgatccgtc actccaaaaa actcagcgaa ttattccatg ccaaaagtga 420

tcaatccgtc aatgcaaaac acggaaagca ggaagcatta ccggttccga aacaggaagc 480

tgaaaaaatg ctgaaggaag tggaagaatg gtacgcacat cacaaaaatg atgaacataa 540

aaaagctcca gtttttgtgt tcaagtgtaa agacaatgtc agtctggtga taaatgttca 600

tcttgatgca gtccggcttt ccaaggagtt gtcaaatatg cttgaagata ctgccgacga 660

aggatatccg aagccaatcc cgagtccagt tcagaatttc aaaagtgaca tcatgctaaa 720

actgattaaa ttgtgtgaag aatacaagta caaattcgat ggcctaaaga aaacaactga 780

tgtgccaact ggagaagtcg cggaggcatc ggcttcaaat gtccaagagg gtgacgttgg 840

ggaggcatcg gcttcaaagg tcaaggctgg aacaattgcg gaggaaatcc aaaaattcgt 900

tcctccgcca cacattatgc aatgggacgt gaaaacggac cttgagatgg ttcgggccgc 960

cgacttttat aacatcaatc gtttgatcaa cgatgattac attgacaatg tgtacgaaat 1020

cgtaaaagtc aaatggatca atggcaaaac gccgcaggaa attcgcaagg gattcggcgt 1080

cgaagagccg tacccgccgg gacatccgga atgggcacga gttgagaagg agaacgagtg 1140

ggaagaatcg gacgaggaac gtgaggcacg ccatgcaaag gaacgagagg aggaagagga 1200

gcgtgagaga aaggaagaac agaagcgtaa ggaagaggaa gcggaacgcc tccatcagga 1260

acaactgcag caacaacaga atcaggaaca gcaacctcag tagggacagc agcatggtga 1320

agaactggaa cacgatgaag ttatgcatga tgtggaggaa gagcaagatg atgaatggcg 1380

aggaagaaga tgatgagtga tgaggatgag gaggatgtaa aatgatgtgg tgattagtga 1440

ttttgatgaa ccgatgattc acttttcttg gatcctgttg cataaaactt gttgcaaaaa 1500

aaaaaaaaaa aaaaaaaaaa aaa 1523

<210> 42
<211> 711
<212> DNA
<213> Heterodera glycines

<400> 42
atgtcttctc tgctgctctc catcttccca attgtgtttt tggtctgttg caatgcaatg 60

ccaaatttcc cgtgctgccc gggcagtcag caagtggttg ctgtgatgtc catttacatt 120

gacactttct cttctgctgc tgacgagtct acagtatgct ttttcgctaa aagtactgtg 180

gatggaataa aaaatgaact gtcctctcgc ggcggatgcc aaaacggagg agaagcacaa 240

attgtggatg aaatcgatcg acagctgaag aatattgcaa aaatggagat caattatgag 300

gacgagtgcc cgtacaattt gggctttgcc cgtgccatgt tcgacttggc cgctgctgct 360

gctggccatg cgggcaacgg cacagaatgg caatacatga aagtacaatt tgagcaggaa 420

agccaagcaa tcaaagcaat tggacaagaa aagaactttg aagttacgga tgtgcatttt 480

ggagtcccaa gcaaaggggt ttctgcacat caaaatgtgc cgagtccgag ccatgtgatt 540

gccaatcctg gccaacacag ttcggttggg ccaaggaaag aaagaagaac cgttgtcatc 600

ggacttcgat ttttgaggac aaacaaatca ggaggaaata gaatagaaaa ccattttgtt 660

gacatgtcga acatttattt aaagtaataa atatttggta ataaggaaaa a 711

<210> 43
<211> 492
<212> DNA
<213> Heterodera glycines

<400> 43
atgaggaaac ttccattctt gctcttattt tctgcttgtt gctacttgca acggaaggaa 60

gtatatttcg ccgaaaaaac agaaagaaaa attcatcgag cagcgaacaa aaaacacaaa 120

gaagaaaccg agggtatggc cgaagtcgca ctttttcaaa tggcaatggc atgtatggcc 180

aatcgaatgg attttcaaat ggccgatttg gcggctccag tgtctattca aatagcggcc 240

ggtcgaatag ctttccaaat ggtggatttg gcggctccag tggcttttca tcgatgggac 300

ataactcggg cggacttcac ggctttgggt ccggcccacc ttctggaggc tacggatacg 360

gaagcggttt tggtggaaga aaatgaaaaa aagaccgaaa gtgatcggcg agagaatgat 420

ttgtgctgtt gccttttatg catgcaaaat ttaaatggaa aaacgcattt caccagcaga 480

acactttttt aa 492

<210> 44
<211> 669
<212> DNA
<213> Heterodera glycines

<400> 44
acaaccgctt cagtgcgcaa cgcaacgcaa tggccactta cctattcatc gccgctgctg 60

ccttcggagc aggaatttac tgttggaagg cgtacctcgg cccgatgctc gaccaaccga 120

agagcgacga cgaggagaag aaggctggca gcaagggcga caagcacaag aagaaggaca 180

agaagggcga aggcgcagca gcagcaccag cggcggacaa aaaggaggag aagcgcaagg 240

acaaacaccg atcgaaggag gcggggaaga gcgacgacag caaagcgggc aagaaggaca 300

agcacgggaa gaaggacaag aagggcaaga agaaggacac cgccgatgca tccaccgtgg 360

acgaagcgct gggagaacag agcacagcgg agacgagcca agcagaggaa tagccaggcc 420

ctgctgcgtg caaatgacag tgttcgacga cgacgtctcg cgacaccaac acacccgtcc 480

cttgctttcg accattccct tcccatccga ttgctgtgtt ttgtctgttc caatgcttcc 540

taaatgttcc ccttgtgaac agtgatcctt cctcatgtga cctgaatatg tatatattgt 600

atacccatta atatattgtg cttcgcttgt tcgatatttg tgaaatctca ttcatattca 660

tcgtcattg 669

<210> 45
<211> 700
<212> DNA
<213> Heterodera glycines

<400> 45
atcctctaag aattcacatc ctctcagaaa atggcttcat ctttctgctc ctcaatcatt 60

tccatcgtcg caattgtctg tttgctgtgc aaatgctgct tttcggcacc ccatccatgc 120

tgtcctggca gccaacatgt tgtttcgatg atgaaagatc acaccggcac attctccgct 180

tcgatgccaa agtcttcgct ttgtctgagt gccgaaagag tcgccgctgc ggtggaaaac 240

caactgaaaa caatttggtg ccctggcaat ggtggtcaaa cactcatcaa cgagatcaac 300

gcagctcaat catcatctga tgagtgtgct cgctctctcg gcttcatccg tgccatgttc 360

gaaattgccg cttccgccgc ttcccatgcc ggtgccaacg ccgaattggc caatttggct 420

gtccagttcc gagaacaagt tggcacaatt gacaccaact gtgctgcgct gggcattcat 480

gttgggcaaa tcagcttggg cactcccaaa ggagaccatc cgcaagtgca tgactctgag 540

agtgtgctta gtaaccctgg caccagcggg tctcacaagc gcatttaagt gcattgcgac 600

gattccgatg atgtcatcat ttgttgattg atatgctata gaaattattt tattagataa 660

aaaatgaatc attacaaaaa aaaaaaaaaa aaaaaaaaaa 700

<210> 46
<211> 900
<212> DNA
<213> Heterodera glycines

<220>
<221> misc_feature
<222> (433)..(433)
<223> n is a, c, g, or t

<400> 46
taataaataa gtttttttgt tcaaaatgag caactttata tttgtttcct ttttaactgc 60

agcgtttttt agctcaggcc tcgctctacc ggctccttat gatgctgaat cggtggtatc 120

ttttgtttta aatgttccac tactttcagc tgacgcaaat gttgaagcat ctcctcccaa 180

tgaaagtgat gctgtttttg agatacaagc tccatcaatt ccggtaccaa ttgaacatca 240

aactgctgct gacattactc atccaactga aactggcaat gagtcttcta ttgcatcatc 300

atcatccacg ccgaaaagtg agcagacgcc aaaaaaagtg atgaacatga aaagcgcgct 360

ggaaggcgcg gcggccaacg tgtacggggg gcttccgtta gacaagcagc ccaagacgat 420

cgctgaagcg gcngcaaaag caaagcagac gcccgccaaa cttccggctg actacgacgt 480

gaaccgcgtg gcggaacgcg cagcagcccg tgtgtacggg tggcttccgg aagacaagca 540

gcctaaggcg atctatgacg cggcggagaa tgcaaagaac acgcccaaac cgccgggcga 600

ctacgacgtg gagcgcgtgg cgcaaaaggc ggcacggctc gtctacggtg tgctgcccat 660

cggcatgcag cccaacttcg ccggccctag cactgacaag agcaatgtcg acgactcgga 720

gaaaccttct gctgctgcgg ctggtgatga tgatgatgaa gtcgaaaaag agaagaagga 780

ataagcaaaa aacaatgtga atttattata ggaaaaaaag aaaaatggaa gcttagaaat 840

ttaatatttt catttttgaa catttataaa atttcaaaat taaaaaaaaa aaaaaaaaaa 900

<210> 47
<211> 730
<212> DNA
<213> Heterodera glycines

<400> 47
ttaaaatgcg cgccgttctc ttcctggcca tggtttgctt ggtgatggct gttattcttg 60

agacagccaa ctcaaaggca gtgaaaaaag acaataagaa aggagcaatt acaacgccag 120

caaaaggaaa agcggcgccg aaaggagcag cgaaaggagg aattaaaaaa gacgcaaaat 180

caaagggaaa aggaaaaaag gacgccaaag gcaaaaagga caaaaaagct aaaccagcca 240

aagggaaagc atcgcctaaa aatgacaaaa aacccccagc tgccaaagca aatgacaaaa 300

agagcccaac caaaccaatg gcagcagtga aagccgtgcc aaaggcggcg gagaatgctc 360

agaagggacc gtcgagtgcg gaagtggtgg cggaggaaag caacttggac actgaggcgg 420

tcgacgactt gggggttgga cctgaggcgg tcgacgactt gggggcggaa gagtcggact 480

ttgaccaatt ggcagaggac gaactgctcg aggacgacgc catgggcggc gaggcggacg 540

aatgggaagg catgggggag gagacggaga tggacagcca aatggcactt tgatggccaa 600

ctaattggac aaattacaaa cggaacaaaa cgggacaaat gacacaaaat attatttatc 660

atttttggca tatcattgaa aataaataga atttattttg caaaaaaaaa aaaaaaaaaa 720

aaaaaaaaaa 730

<210> 48
<211> 270
<212> DNA
<213> Heterodera glycines

<400> 48
atgaatttga tttttgaaat attcgccttt ggaatggaca atttctcatc ggaaaaaaat 60

gtttctcttc ttcgtctttt cttattcttt ttctcgaccg tagtattatt tggaagcgca 120

caccctatgg taaacaaatt atgcgaagac ctagataaac ctgaaggttg gcaattattg 180

aaaaagttaa caatcgaaaa gtgccggatg agtataatgt ggaattcccc aaatggaaag 240

acagctacaa aagcattaaa aagtttataa 270

<210> 49
<211> 684
<212> DNA
<213> Heterodera glycines

<400> 49
atgcgcactt ttctgttcat agctgtagtc gggttggtgt tggctgtcat catggagagt 60

gtaaatgcga ctgggaataa ttcgccgaca aaggggcaaa ctccgcctgg cagtccgaaa 120

ggtgggcacg gcagcaacaa atcgccaacg acgccgcaca gtgccggttc gccacacggc 180

acgcagcatg gtaaccaaca gaaccatgct gccggacaaa cgccgcctgg cagtccgaaa 240

ggtgggcacg gcagcaacaa atcgccaacg acgccgcaca gtgccggttc gccacacggc 300

acgcagcatg gtaaccaaca gaaaaatgct accggacaaa cgccgcctgg cagtccgaaa 360

ggtgggcacg gcagcaacaa atcggcaacg acgccgcaca gtgccggttc gccacacggc 420

acgcagcatg gtaaccaaca gaaaaatgct accggacaaa cgccgcctgg cagtccgaaa 480

ggtgggcacg gcaacaacaa atcgccaacg acgccgcaca gtgccggttc gccacacggc 540

acgcagcatg gtaaccaaca gaaccatgct gccgtttcaa atgtggtgca gaacgttgac 600

aacaaacctc ggccatctgc tcctgcttca caccttgtgc caccaacacc agggaacagc 660

cctactgcgc atgacagtcg ctga 684

<210> 50
<211> 999
<212> DNA
<213> Heterodera glycines

<220>
<221> misc_feature
<222> (974)..(974)
<223> n is a, c, g, or t

<400> 50
ctgcacattc ccccaattta tttcttcctt agtaagtagt attggtgcac aatgcgcact 60

tttctgttca tagccgtagt cgggttgatg ttggccgtca tcttggagaa tgtaaatgcg 120

actgggaaaa attcgccgac aaagggacaa tcgccgccag gcagtccaaa acatgaaaaa 180

gaccgtaaaa atgagcatgg taaccaacag aaccatgcta ccggaaaatc gccgccaggc 240

agtccgagag tacaatcgcc gccaggcagt ccgagaggaa aatcgccgcc aggcagtccg 300

agagtacaat cgccgccagg cagtccgaga ggacaatcgc cgccaggcag tccaaaacat 360

gaaaaagacc ataaaaatga gcatggtaac caacagaacc atgctaccgt tccaaatgtg 420

gtgcagaaag ttgaagtcca caaccaacct acgccatctg ctgctgcttc acaccctgtg 480

ccaccaacac cagggaacag ccctactgcg catgacagtc accacacaag cccaaaagtt 540

gcccatgttg ctgccgccgc cactttgggc catggacgcc aagaatgtgc caatgtgtcg 600

tgtggtgatg atgtaaccga ggaaatggac cgcgacgatg ataagacagt ggaacgcgcg 660

gtcggcgagt caccaatcgg cgtttcggaa tccagcgcca cggatgagga tgattctgct 720

gccgtttcgg cttacaacaa aaacaacagc tcggccgccg catcagcgtc agttgctgat 780

gactgagatg ggggtgatgg tgaaggaact gcgtggaacg atgatgccaa ccatgaacaa 840

acacaaagct gttgtagtag atgtagaaaa aaactgtggt atcatcacgc tattattgtt 900

gtgtaaaaaa cgatccgttt ttacctcttt tcgggaaaaa aactggaaaa aattcacaaa 960

aaaaaaatct cacntaaaaa aaaaaaaaaa aaaaaaaaa 999

<210> 51
<211> 954
<212> DNA
<213> Heterodera glycines

<400> 51
ggcaattaat tgtccaatta agcggatgaa ttcgtccgtc gtttcatttt cgctgttact 60

ttttgggtta ttcacaattc gcactgcaaa atcgcaatgc gagaaacatt gcactaaaag 120

caaaccgatg ggccaatgca atgaggcgga agaagtgatt ctgcgcaact ccgactgtgc 180

cttcatgaag aaaacggaaa cggcattcga atttgtggtc ggaatgaatg gccaaacgga 240

ggacaaaacg gcgcccgggg ccaatgccaa tggggcattt ttgtgctgta aggcaaccca 300

aggaaccgca acacttttca tcgtcggcgt ggccaacaaa cggctgatgt tggccaagga 360

cgtggtcaat tacaagtttc atcaaaacat ctcaatcgat gattttgaac gggaaaagca 420

agtgttggaa agtgtttcgg cgcagggaca aaaagccggc attggggaca attatggaga 480

aaaattcttc caggaccaaa tggacgccaa caaaatgatt cagaaaggct ctgtgaagct 540

gtggaccgcc aacaaatcgt tgcctcctca aaatgtgccg gatttacaga aagatacccg 600

gcccaaagtg acggcggcga cagaagaaat gattttggca ctgaaggtgt ttcaacagtt 660

tcgaaagaac aaaaattgtt gggcatctgt ggaaaaggaa ctcttaaagt cgcaaagctt 720

tttgagtttg gccgaaccca acggaaagga cgcaatgcga aaagcggcgg tgcgattgtg 780

cgcaaaaatg gaagcgaaaa ttgaggcaca aattgacgaa acggcaaaga aattgttggc 840

ctgaggagga cgaattgacg agcattttag gaaaaagatc ctttcgttcc gattttgatt 900

ctttgatcag tgaaagtgcg gtattgtcaa aaaaaaaaaa aaaaaaaaaa aaaa 954

<210> 52
<211> 612
<212> DNA
<213> Heterodera glycines

<400> 52
gaaggcgtgt ccaagttgat caacggtatt ccagtggctg agaccgtggt ttacccagcc 60

atcggcgaga ccaagactca tatcacggtg ttcaccgata ccacctgccc gtactgccac 120

aagctgcacg ccgaagtgcc tgcgctgaac aaaatgggga tcgaagtgcg ctacgtcgcg 180

ttcccgcgcc agggcctggg ctcgccgggt gacgaacagc tgcaagccgt atggtgctcg 240

gccgacaaga aagcggccat ggacaaaatg gtcgatggca aggaaatcaa atcggccaaa 300

tgcgccaacc cggtttccaa gcagttcgcc ctgggccagt cgattggtgt gaacggtaca 360

ccggccatcg ttttggctga tggccaggtc attccgggct accagcctgc gccacaagtt 420

gccaaactgg cactgggtgc gaagtaagca gtcatcgtcg agcctttgac gatcatgccc 480

cgtcgaacag tcgacggggc attaattgag agccgcagtt gtgcggctgt ttaccacggc 540

cgaccttgag tcggccgttt catggggagt tcttcagtga aaccggtcaa agtaggcatc 600

tgtgggttag gg 612

<210> 53
<211> 356
<212> DNA
<213> Heterodera glycines

<400>  53
agaagaattt ctcaaccatc ataggaccga atagatgaat agtgttttat cgatctccat      60

tttattcctg ctcagccagg ttctttccac tgtctcctct agcgatgtgt tggaatacac     120

ggatgctagc tttgactcgg gaatgcagca gcacgacatc gcattggcag aattctatgc     180

cccctggtgt ggacattgca aaaaactcgc tccggaatac gaaaaagcgg ccactaaact     240

gaagaacaac gacccaccaa ttccactcat caaagtcgat tgtactgcgg aaaaagagac     300

ttgcgataaa tttggagtta gcggttttcc aacattgaaa atcttcagga aggggc         356


<210>  54
<211>  261
<212>  DNA
<213>  Heterodera glycines

<400>  54
gcaataaaga gtaaattatg gataggagat tcactgtctt ccttgtgatc gcattggtta      60

cttctattta tgaagtgctt agtaatggaa atttgaacga tggcgacgac tcatttaaac     120

aattcgatga actcgaagaa aacccagcgc ataaatattc aaaagaggcc cagaaggggt     180

tcgaaatgga ggaggaggaa gtcacaatcc gagaaccttc ggggacgaag gaatcgttca     240

aattgcccat caatatgccg t                                               261


<210>  55
<211>  602
<212>  DNA
<213>  Heterodera glycines


<220>
<221>  misc_feature
<222>  (4)..(4)
<223>  n is a, c, g, or t

<220>
<221>  misc_feature
<222>  (11)..(11)
<223>  n is a, c, g, or t

<400>  55
cgtntcagtt ngagtttcat tgaaaactca aaaaatgaca acagtcaaat tgctatgcct      60

aattgcccta tttgcggtag tgcaacttcg ttttgcattt gctatgatga atcaaaacaa     120

ccaacaagtg agccaaaaca acagctctga cgaaggagaa gatgatgatg atgctggtaa     180

cggcgggcaa tatgacacgg ttgctccatt gcgtagcgac agcactagtt tcgttagcag     240

cggcacctta ccgccgcagg gcagcaccgg tttcagcggg gtcggcatga tgccgccgca     300

tgcccaaaac atgggagcaa tgggcatggg gatgggcaat accagtttcg gcaatttgca     360

acaacccact atgcaaatgc aatatcaaaa ccagcaaatg atgcaaaccc cgcaaatgat     420

gcaaaaccag caaatgccgt ttaaccagag tggcagtttc agtggggttg gcatgatgcc     480

tatgcagcac caaaacatgg gagcaatggg catgatgaat gttggcagta ccagttttgg 540

caatttgcaa caatccaata tgcaaatgca acatcaaaac cagcaaatga tgcaaaaccc 600

gc 602

<210> 56
<211> 320
<212> DNA
<213> Heterodera glycines

<400> 56
acaaaaatta attggccgtg acgaaaacaa tatcatcaac cacagaaaag tgaactgctc 60

cagcgctgca atgttgatgc tgttgatgac cattttcact gtgctgtcca atgtcaacga 120

ggtagcagcc gaggagcacg agctggtcag ccgcattaag cgcaacggct acggatacag 180

cagcggttat ggtggtggct gttcagcctg ccaaagcagc tgcaccacct gtggttacac 240

atcctaccat accgtgcctg cggctcccgc tcccccgcca ccaccgccgc ctccgcctcc 300

gcctgcgccc gtctacagct 320

<210> 57
<211> 643
<212> DNA
<213> Heterodera glycines

<220>
<221> misc_feature
<222> (477)..(477)
<223> n is a, c, g, or t

<400> 57
caaatttttg catataacca aaaaaaaatg catttccaaa ctgccttcct tctcctggtg 60

ccgttcattg ttgccaacat tgtgttggct gacatgccca aagatgatgc gccacgcccg 120

gcggtgcaaa tccgcacccg tcgttccgtg tatgtggcaa tgatggacgg catcggcgtc 180

accaaggagg ccatgagata tgcatgccgg ggatggcaaa cgagcttctg cacaaagttt 240

ggcactacgc tggccgttga gcgtacacga cgtgacacaa atgccgagat gatgggtacc 300

aagatgactg ccaatgccga gcgggaggca aagaaggaaa tgaatggcga gatgggtgcc 360

aagaaagaaa tgaatggcga gatgatgaac accaagaaag acacgaatgc cgaagtgatg 420

ggcaccaaga cgaatgctaa ccccgagcgg gacaccaaga tggacgccgt ggtgatnaac 480

accaagacga atgccaaccc cgagcgggac accaagacgg aagcgaatgc tgtcatggtg 540

aacaccaaga cggattctgc caaccccgag cgggacacca ataccgacgc cgtcatggtg 600

aacaccaaga caaatgttaa ccccgagcgg tacaccaaga ttg 643

<210> 58

<211>  601
<212>  DNA
<213>  Heterodera glycines

<220>
<221>  misc_feature
<222>  (227)..(227)
<223>  n is a, c, g, or t

<220>
<221>  misc_feature
<222>  (458)..(458)
<223>  n is a, c, g, or t

<220>
<221>  misc_feature
<222>  (590)..(590)
<223>  n is a, c, g, or t

<220>
<221>  misc_feature
<222>  (599)..(599)
<223>  n is a, c, g, or t

<400>  58
aagaataaca tgaaaataat aactctaatt ccaatcttat tttcattaat aaattcggta      60

gttggacaaa caacatgcgc aacaccgaca acacccccag tttgttactg tacatgtgaa      120

tcaacacctg gagtgtcatc aacagcacca acaacgacag ttggaccaac aggaacaaca      180

acaactggca caccattaac aactggacag ggcacatctg gaccatntac accagcacca      240

acaggcactc ccggacaatc tacaacagca ccaacaggca cgcctggacc atcaacacca      300

gcaccaactg gcacgcctgg accatctaca ccagcaccaa caggcacacc cggaccatca      360

acaccaacac caacaggcac gcctggacca tctacaccag caccaacagg aacacccgga      420

ccatcaacac cagcaccaac aggcacgcct ggaccatnaa caccagcacc aacaggcacg      480

cctggaccat ctacaccagc accaacaggc acacccggac catcaacacc agcaccaaca      540

ggcacgcctg gaccatctac accagcacca acaggcacac ccggaccatn aacaccagna      600

c                                                                     601

<210>  59
<211>  584
<212>  DNA
<213>  Heterodera glycines

<220>
<221>  misc_feature
<222>  (145)..(145)
<223>  n is a, c, g, or t

<220>
<221>  misc_feature
<222>  (221)..(221)

<223>  n is a, c, g, or t

<220>
<221>  misc_feature
<222>  (487)..(487)
<223>  n is a, c, g, or t

<220>
<221>  misc_feature
<222>  (569)..(569)
<223>  n is a, c, g, or t

<400>  59
tgtcgacaga tttcaaactt gatacttgtt gttgttgaac atatttctat acctcacagt      60

tcgtccacat tttgactttc ggacaaaaac aaaagacaaa aaacggtggc aatgatctcc     120

aggagggcaa ttattatttg ggccnttgct ttgttggcat tagcagcgat ttcgcccaac     180

ttttccaccg ctgacaaagg agttgacgct gtcgatgcgg nggacgaaat cattgacgac     240

ccaaaagtgg aggtgccgaa gaacggcgtt ggcaaaggca ccgatgacca aacagtgcag     300

tgggaggagg aggccatcaa actggaggga ctatcagtgg ccgagttcaa gcagctcagg     360

gagagtgcgg agaagcatca attccaagcc gaggtcaacc ggatggtgaa gctgatcatc     420

aattcattgt accggaacaa ggagattttc ctccgtgagc tcatctccaa cgcgtcggat     480

gccctgnaca aaattcggct catttcgctg acaaattcga cagcacttgc ggccaccgaa     540

gaattgtcca tcaaaattaa ggccgacana gaaaatcaca tttt                      584


<210>  60
<211>  483
<212>  DNA
<213>  Heterodera glycines


<220>
<221>  misc_feature
<222>  (5)..(5)
<223>  n is a, c, g, or t

<400>  60
gtcgnaaatt gcaatgcctt catttaaatt tgtatgtttc caatttttgc taatttttgt      60

gctcacggaa ttggcacatt cagagaaagg gtgcccaaaa acagaagatt taattgaaat     120

gaccaaacat ttgctgacaa ctgggaagaa tgtaatttct ggtgaagcag cttcatcatc     180

caccgaagga aagtgcagtg atacggtaat ggaaagtgtt gtaacaaaag ttattgaatt     240

tgggtgttat gacgaaatgg aaaagaatga cggcgaaaga gcgaaaagca aaacgaaaaa     300

cgcatttatt gaaagcattg aaacacaacg aaaaggtttt tgccaacttt ggcaaaacgg     360

caaaagcatt ggggagaagg agcaaaaaga tggcattttg aaaatagtga aaggattggg     420

aggaattgag ctgaagaaaa ttgaacgaat tcttgaaaat gttcaaaaca ttggattatc     480

gcc                                                                   483

<210> 61
<211> 364
<212> DNA
<213> Heterodera glycines

<400> 61
aatattaaaa gatccgaaaa aatgccaaac attttcaaaa tccttctgat tgtgcttttg 60

gccgtcgtct cattccgtct ctcggcttct actggtgaca aaaaaactgc taatgatggg 120

agtggaaaca actcatcagc tgggattggt acgaagatca aaagaattgt caccgctgga 180

ctgctcttca cttccctggc gacgggtggg gcggaagtga ttgggcgaag caatgctcag 240

ggaggaaatg ccgccggatt ggtgccatcg catgtgacca atcgctcaat ggctccacca 300

cctcctcctg tgcaatttga aatgggggca aatcgattag aaaaaatgag ggcacaccta 360

cgcg 364

<210> 62
<211> 647
<212> DNA
<213> Heterodera glycines

<400> 62
agtcctaaag aaaaacgttc aaatgtttcg cttttcgctg cttctgctgc ttgtccctct 60

cgtccgctgt gtccccaacc caaagccttt gttcggaatt ggcatcggtc gcaagcaaag 120

cgcgggagcg gaggggaagc tgacctgcgc tggagagccg ttggcggatg tgaaggtgaa 180

actgtatgat gacgaccgcg gggtggacac ggatgatctg atgggcgaga cgagaacgga 240

ctcagaggga cgcttcagat tggaggggta cacgcacgaa atcaccacca tcgaccccaa 300

aattaacatt tatcacgact gcaacgacgg gctgaagcca tgccagcgca aaatctcaat 360

tatgatcccg gacaaataca tcgcctcggg cgaacatccc aatacgtatt acgacgcggg 420

cacagtcgaa ttggagggca aattcagtgg cgagacaagg gactgtctgc actaattgca 480

ttggaactga tcatttggca ctttctgatc agttgggact gaccaattgg aactctctgc 540

actgatcact ttcccatttt tgtttctgtt ttgtcaccga ttttccccaa agtcttacac 600

cacccaatgt gaatttgcat aaatttgtcc gcaaaaaaaa aaaaaaa 647

<210> 63
<211> 857
<212> DNA
<213> Heterodera glycines

<400> 63
aacaaaacaa aacgaacatt tgccagcagt tggctaggac aacggcccaa agagaagtga 60

agtgaattgc ctctgctctg aacactgatg atgatggttc gttcttcctc ctcttccttt 120

tcccttccgt cgtacttcct ttctctcctc cccctccttc tcctcctttt ctcccaatgt 180

tcccaaattt cttttgcttg ccaatgccaa tgggccccgc caaaggacaa ttattgttcc 240

tccgattggg ttgcccacgt gcaggtgatc aaacgacagg acggcgtgcg aatgccggcg 300

gggatcaccg accgacagac ggacttaaac tcgcgacacg aagtcaaata tttaaggatg 360

tttaagatca gcaaacaaat gccggtcaat cagcagaacc aagtgattct ccctgtcaat 420

gtttatacgg ccaccgagga tgccgcctgt ggcattttgc tcgagtcggg acaccaatat 480

ttgttggccg gcgattacgt caacggcaca atgctcaccg gactgtgccg gcaaattctg 540

ctcgaagacc ttaaggagtc acgaaagcac gacattctcg agtggacaga agtgccgcaa 600

aagctgaaag aacagctgga aaagcaggaa tttgatcaga aatgcaactg aacagaatgg 660

aaaaagaaag agaggacaaa caattgcaaa tgaatgatca gaaaaatcaa acggacagag 720

aaatggccca atcgggcact ttttgctgaa cgaacatttc caataaacga cgaataaatt 780

tttaccgggt tagcccgtga aaaacgactt gcttgtaaag gttatttgct gcttatttaa 840

aaaaaaaaaa aaaaaaa 857

<210> 64
<211> 24
<212> DNA
<213> Heterodera glycines

<400> 64
catttcctcc ctgagcattg ctta 24

<210> 65
<211> 27
<212> DNA
<213> Heterodera glycines

<400> 65
ttgtcaccgc tggactgctc ttcactt 27

<210> 66
<211> 17
<212> DNA
<213> Heterodera glycines

<400> 66
atgtcccttt tccgtcc 17

<210> 67
<211> 20
<212> DNA
<213> Heterodera glycines

<400> 67
atgctccaaa acggccttac 20

<210> 68

<211>  20
<212>  DNA
<213>  Heterodera glycines

<400>  68
atgctccaaa acggccttac                                                    20


<210>  69
<211>  21
<212>  DNA
<213>  Heterodera glycines

<400>  69
atgttcagct cttccaattt g                                                  21


<210>  70
<211>  20
<212>  DNA
<213>  Heterodera glycines

<400>  70
caatgctcag ggaggaaatg                                                    20


<210>  71
<211>  20
<212>  DNA
<213>  Heterodera glycines

<400>  71
cactcggtga cagacgctta                                                    20


<210>  72
<211>  20
<212>  DNA
<213>  Heterodera glycines

<400>  72
aagcaggcgt atgagcagtt                                                    20


<210>  73
<211>  20
<212>  DNA
<213>  Heterodera glycines

<400>  73
gtcgtgtgcc aatacaatgc                                                    20


<210>  74
<211>  20
<212>  DNA
<213>  Heterodera glycines

<400>  74
cggctaatga aaagggaaaa                                                    20


<210>  75

<211>  20
<212>  DNA
<213>  Heterodera glycines

<400>  75
tggcatcatt ccactgactc                                                    20


<210>  76
<211>  20
<212>  DNA
<213>  Heterodera glycines

<400>  76
gtcctcaatc gctgcttctt                                                    20


<210>  77
<211>  20
<212>  DNA
<213>  Heterodera glycines

<400>  77
tcaatgtttg ggcttcttcc                                                    20


<210>  78
<211>  15
<212>  PRT
<213>  Heterodera glycines

<400>  78

Ser Thr Gly Asp Lys Lys Thr Ala Asn Asp Gly Ser Gly Asn Asn
1               5                   10                  15


<210>  79
<211>  16
<212>  PRT
<213>  Heterodera glycines

<400>  79

Pro Val Asn Glu Ser Lys Arg Leu Ser Pro Ser Gly Pro Asp Pro His
1               5                   10                  15


<210>  80
<211>  15
<212>  PRT
<213>  Heterodera glycines

<400>  80

Glu Glu Asp Ile Lys Ala Lys Thr Lys Arg Thr Leu Pro Lys Ser
1               5                   10                  15


<210>  81
<211>  15
<212>  PRT
<213>  Heterodera glycines

<400>  81

Lys Gly Leu Gly Thr Arg Asp Ser Asp Leu Ile Arg Leu Val Ile
1               5                   10                  15


<210>  82
<211>  15
<212>  PRT
<213>  Heterodera glycines

<400>  82

Ser Lys Pro Asn Pro Gly Gln Lys Pro Ser Gly Glu Arg Arg Lys
1               5                   10                  15


<210>  83
<211>  16
<212>  PRT
<213>  Heterodera glycines

<400>  83

Val Asn Arg Asn Gly Trp Glu Asn Thr Gly Thr Pro Thr Gly Gly Arg
1               5                   10                  15


<210>  84
<211>  26
<212>  DNA
<213>  artificial sequence

<220>
<223>  Synthetic primer

<400>  84
aaggatccat gcgcactttt ctgttc                                          26


<210>  85
<211>  27
<212>  DNA
<213>  artificial sequence

<220>
<223>  Synthetic primer

<400>  85
aagagctctc accattttaa ggcttgc                                         27


<210>  86
<211>  27
<212>  DNA
<213>  artificial sequence

<220>
<223>  Synthetic primer

<400>  86
aagaattcac agggaaaaat tcgtgac                                         27

<210>  87
<211>  28
<212>  DNA
<213>  artificial sequence

<220>
<223>  Synthetic primer

<400>  87
aaggatcctt caccatttta aggcttgc                                          28


<210>  88
<211>  23
<212>  DNA
<213>  artificial sequence

<220>
<223>  Synthetic primer

<400>  88
agttggacag aaggcatcag cac                                               23


<210>  89
<211>  23
<212>  DNA
<213>  artificial sequence

<220>
<223>  Synthetic primer

<400>  89
agaagcgttg cggacatatt tga                                               23


<210>  90
<211>  24
<212>  DNA
<213>  artificial sequence

<220>
<223>  Synthetic primer

<400>  90
gacgaagaag ataaaagttg agag                                              24


<210>  91
<211>  24
<212>  DNA
<213>  artificial sequence

<220>
<223>  Synthetic primer

<400>  91
ttgataaatt acaagcagat tgga                                              24


<210>  92

<211>  26
<212>  DNA
<213>  artificial sequence

<220>
<223>  Synthetic primer

<400>  92
tatctagagg catttgccat ttcaag                                          26


<210>  93
<211>  25
<212>  DNA
<213>  artificial sequence

<220>
<223>  Synthetic primer

<400>  93
taggatcctc atgtagaaaa gggcc                                           25


<210>  94
<211>  29
<212>  DNA
<213>  artificial sequence

<220>
<223>  Synthetic primer

<400>  94
tatctagagc gcacttttct gttcatagc                                       29


<210>  95
<211>  29
<212>  DNA
<213>  artificial sequence

<220>
<223>  Synthetic primer

<400>  95
ataagctttc accattttaa ggcttgctc                                       29


<210>  96
<211>  24
<212>  DNA
<213>  artificial sequence

<220>
<223>  Synthetic primer

<400>  96
ttgataaatt acaagcagat tgga                                            24


<210>  97
<211>  24
<212>  DNA
<213>  artificial sequence

<220>
<223>  Synthetic primer

<400>  97
gacgaagaag ataaaagttg agag                                              24


<210>  98
<211>  36
<212>  DNA
<213>  artificial sequence

<220>
<223>  Synthetic primer

<400>  98
atctcgagtt tggatgcatc gcttatcact gacctt                                 36


<210>  99
<211>  36
<212>  DNA
<213>  artificial sequence

<220>
<223>  Synthetic primer

<400>  99
atgaattcca ttgtttttaa accacccatc aacgca                                 36


<210>  100
<211>  36
<212>  DNA
<213>  artificial sequence

<220>
<223>  Synthetic primer

<400>  100
attctagatt tggatgcatc gcttatcact gacctt                                 36


<210>  101
<211>  36
<212>  DNA
<213>  artificial sequence

<220>
<223>  Synthetic primer

<400>  101
ataagcttca ttgtttttaa accacccatc aacgca                                 36


<210>  102
<211>  26
<212>  DNA
<213>  artificial sequence

<220>
<223>  Synthetic primer

<400>  102
atctcgagga aatgggaaga gaaaca                                          26


<210>  103
<211>  26
<212>  DNA
<213>  artificial sequence

<220>
<223>  Synthetic primer

<400>  103
atgaattcaa gataacccgg aaaagg                                          26


<210>  104
<211>  26
<212>  DNA
<213>  artificial sequence

<220>
<223>  Synthetic primer

<400>  104
attctagaga aatgggaaga gaaaca                                          26


<210>  105
<211>  26
<212>  DNA
<213>  artificial sequence

<220>
<223>  Synthetic primer

<400>  105
atatcgataa gataacccgg aaaagg                                          26


<210>  106
<211>  26
<212>  DNA
<213>  artificial sequence

<220>
<223>  Synthetic primer

<400>  106
atctcgagac gagttgagaa ggagaa                                          26


<210>  107
<211>  26
<212>  DNA
<213>  artificial sequence

<220>
<223>  Synthetic primer

<400>  107
atgaattcct cttcctcctc tcgttc                                          26

<210> 108
<211> 26
<212> DNA
<213> artificial sequence

<220>
<223> Synthetic primer

<400> 108
attctagaac gagttgagaa ggagaa 26


<210> 109
<211> 26
<212> DNA
<213> artificial sequence

<220>
<223> Synthetic primer

<400> 109
ataagcttct cttcctcctc tcgttc 26


<210> 110
<211> 26
<212> DNA
<213> artificial sequence

<220>
<223> Synthetic primer

<400> 110
atctcgagaa ccatattccc caaatg 26


<210> 111
<211> 35
<212> DNA
<213> artificial sequence

<220>
<223> Synthetic primer

<400> 111
atgaattcta tttggtttgg catttgattc ggctg 35


<210> 112
<211> 26
<212> DNA
<213> artificial sequence

<220>
<223> Synthetic primer

<400> 112
attctagaaa ccatattccc caaatg 26


<210> 113

<211>  35
<212>  DNA
<213>  artificial sequence

<220>
<223>  Synthetic primer

<400>  113
ataagcttta tttggtttgg catttgattc ggctg                                35


<210>  114
<211>  279
<212>  DNA
<213>  Heterodera schachtii

<400>  114
atgtcccttt tccgtcctca atcgctgctt cttctggccg ctctttgcct gtcctttgcg      60

ctgctttttg tcacttcgtc ggaagaggga gggcgagtga agcgcggcgg atggccttgg      120

gattgggccg gcaaacaact gtgcaaaaca tcggcaaatt gcaagtgcaa ggatggcaaa     180

aattgggcca aatgtgtaaa gtcggaaggc tacgcggcca gcaattgttg cgacaaaaat     240

tacgtgtggg catgttgcgg gaagaagccc aaacattaa                            279


<210>  115
<211>  420
<212>  DNA
<213>  Heterodera schachtii


<220>
<221>  misc_feature
<222>  (79)..(85)
<223>  n is a, c, g, or t

<400>  115
atgtcaaaca ttttcaaaat ccttctcatt gtgcttttgg ccgtcctctc attcagttct      60

tcggtttcca ctgatggcnn nnnnnctgct aatgatggca gtggaagcaa ctcatcagct     120

gggattggta cgaagatcaa acgaattgtc accgctgggc tgctcttcac ttccttggcg     180

acgggtgggg cggaagtgat tgggcgaagc aatgctcagg gaggaaatgc cgcgggactg     240

gtgccatcgc atgtgaccaa tcgctcaatg gctccaccac ctcctcctgt gcaatttgaa     300

atgggggcaa atcgattaga aaaaatgagg gcacacctac gcgaacttgc tgagaaaatg     360

ccgccggtca atgaatcgaa gcgactggca ccgagtggac ccgacccacg tcatcattag     420


<210>  116
<211>  1023
<212>  DNA
<213>  Heterodera schachtii

<400>  116
atgctccaaa acggccttac cattctgctt ctgatcagcg ttgtgatcgg ccattccttg      60

gccaaccttg gcccaaccat caaacataat cctcaattta aagccgtaca aactgcgcat 120

catttgcatg atgccattgc gaaaaagcac gaggccgaag ttacgcagat catttgctcc 180

attagcaacg aacaacgaca agcattggca tcggagttca aaaaacaatt cggcactgat 240

ctgattgcca tgctgaaaaa ggagttcaaa agcgactttg aagaactgat catttctttg 300

atgcaaacgc ccgccgttta cgatgccaac caaatgcgtg ccgcattgtc cggctccaac 360

gagacggtgc taatcgaaat tttggcgacg cgcacaaacc gacaaataac ggcgctgaag 420

caggcgtatg agcagttgga cagaaggcat cagcacaatc agctggagga ggacatcaaa 480

gcgaagacga agggcgcctt tcaaaatctg ttggtgtctt tgctcagctg ctctcgcgaa 540

gaaagtgcgc ccgcaagcat tgttttggca caccacgagg ccatgaaact gttcagagag 600

ggcgagggcc gaagaggcgt taacgccgtg gtgttcaacc aggtgttggc cactcgcagc 660

ttcgcccagc ttcgagaaac tttcgagttt taccgacaag ccgcgcacca cgagattgag 720

aagggcattg agcaagaatt cagcggtcac aacgaagcgg gtttcttggc actaatcaaa 780

tatgtccgca acgcttctgt gttttttgcg gatttgttgt tcaattcgat gaaagggctc 840

ggcacacgcg actcggattt gattcgtctg gtcatttctc ggtctgaggt tgacctggct 900

gacatcaaac acgcttttca cacgttgcac aagaagagcc tggaggaggc gatcaaaggg 960

gacaccagcg gagcttaccg agacgcactt ttggcactgg tcaagggcaa cacggagcag 1020

tga 1023

<210> 117
<211> 411
<212> DNA
<213> Heterodera schachtii

<400> 117
atgttcagct cttccaattt gtctgctctc tttttggcct cctccgtttt tgccgtgttt 60

ataattggca ttaaaatgga cggaccgacg gaggcaaaag gcgccgcccc tccaaacgcc 120

gcggggccaa tgggactttt gcttttattg aatggcaaac aatcggcggc caatgaaaag 180

ggaaaagcgc cctctggcga aagtaagcca aatccggggc agaagccgaa cggagaacgg 240

caaaagaggg acgttttggg gcacgccggc ggatacgtcg gaggatggga ccatcccatt 300

gactcgacag ttgattgggc aaagagtcag tggaatgatg ccaattggct cgccgatgtt 360

gtcaacagaa acggatggga aaacaccggc gctccaaccg gcggacgatg a 411

SEQUENCE LISTING

<110>  University of Georgia Research Foundation
       North Carolina State University
       Iowa State University Research Foundation, Inc.
       Hussey, Richard
       Davis, Eric
       Baum, Thomas

<120>  Cyst Nematode Resistant Transgenic Plants

<130>  UGA 1208

<150>  60/759,182
<151>  2006-01-06

<160>  117

<170>  PatentIn version 3.3

<210>  1
<211>  834
<212>  DNA
<213>  Heterodera glycines

<400>  1
aaaccataaa ttacaactta aatcaagcaa aatcaaatgc acttgattaa cttaatcgcc      60

ctctttttca tgcttttcgg cccatccgtc cagcaataca caaaaacgcc aacgaatgag     120

gacaaagaag cggctgtcaa ttgtcacaac aaattccgat cgcaattggc cctgggcaat     180

gccgacaata aattgggcgg caacaaaatg ccaaaggcgg gcaacatgcg taagtttgaa     240

tgggacgaaa acttggccaa acttgcggat gaatgggcca acaaatgcac attatcgcac     300

tcgtggaacg gctgggcagg cgaaaatttg gcaatgaatg gcggaacatt ttcgaacaag     360

gatggcttcg agtacgcttg cggtcgctgg tgggacgaac tgaaccgtta cgggttcaac     420

ccggatctga ttatgaccgg ggaaaacttc agtggcatcg gccattggac tcagatggcg     480

tgggccgaca ccgaccgaat tggctgtgcc atggcacaaa actgcccaaa taccaattgg     540

aaaacatatg tggtctgctg gtattacacg ggtggtaatt actttggtgc gcctgtctat     600

ttggcccggg agccgtgcag caaatgcaaa gcaaatgaca aatgtgacaa agccactgga     660

ctttgctctc aatgaaagat gaaatattat gaaaattgaa ttgggataat tactaattat     720

tgtatctgta tgtttagtat ggtttagtgt ttcggtataa ttttcactta ttggtcttaa     780

ttaacaatga ataaataaat agtttctaaa aaaaaaaaaa aaaaaaaaaa aaaa           834


<210>  2
<211>  733
<212>  DNA
<213>  Heterodera glycines

<220>
<221>  misc_feature
<222>  (574)..(574)
<223>  n is a, c, g, or t

<400>  2
gaaccaacca ataccattaa tttcataaat ccgaagaaat ccctccaaaa atgtcttctt      60

ccccgtccgt ctctgtgctc gccatcgtcg caattgtctg tttgatgtgc caatgttgct     120

tttcggcacc gcatccgtgc tgtcccggca gtcaaaaagt ggtttcactt atgtccaatt     180

acgttggcac ttttgccaat tccatttcca agtcatcgct ttgttcggat gcccaaaatg     240

ttgcggaagc gttgaaaggc caactgatcg gctgctcgaa tggcggcgat cgaactcttt     300

tggccgacat cgaagcatcc cttgccactc attctgctga tgagtgtgcc ctcagcctcg     360

gcttcgtccg tgccatgttc gccattgccg cctccgcttc ttcccatgcc agcaacaaca     420

gcgaatggca gacattgagt gggcagtttg gtcagaaagt cactgagatt gactcgaaat     480

gtgcagagtt tggtattagc attggcaaag tgcccataaa cggtcccaag gatgtccatg     540

ttcaaaatgt gcccaactcg gaaagtgtga tttntatgcc tggattggcc ggctcacaca     600

cccaatgaaa atgcatcact gaaaagattt ggtacctttt gattattgtg cattaattag     660

ctgtggattt tgtaaattta ggaaattata tgtaaataaa tattaattcg ttgaaaaaaa     720

aaaaaaaaaa aaa                                                        733

<210>  3
<211>  615
<212>  DNA
<213>  Heterodera glycines

<400>  3
gacaaataaa tttaattttt taattttttc aaatctatta caaaattcaa aaattaaaac      60

catgaattgg atgcattatt gtttaatcgc ttgctttgct ttttattatt tcaatactgt     120

tgagtcatcc actattaatt cggtaaccgt acaagtgaac aagattgaaa acaatgaaaa     180

tggaagacaa ttcaatttgg aatttacaaa ccaagtttat gagcgagtgt gccacgttga     240

ctttcgaatt gatctgccag acacagtaaa attgaacaaa tattcaaaaa tggtgccaat     300

tcctgacacc tgcggccagt acgcattgcc caagagtttg gacttgcttc ccggcgagtc     360

atttgatgca caattaacac tgcttggcca tgatgggaag ccgaatgtga ctgtgctgaa     420

cacgaacaat attccaacca gcaaacaatg caaaaaatga aaaagcctac ccaattaagc     480

taattgccct atcagttcag caagttaacc aattagcata tcattgcgat caatttaatt     540

attgtattgt cattaattta aaaaaaacat atataagttt gaaggttaaa aaaaaaaaaa     600

aaaaaaaaaa aaaaa                                                      615

<210>  4
<211>  1143
<212>  DNA
<213>  Heterodera glycines

<400>  4
   gatcaaacaa aaaattaaaa tgaacaggtt ttttacatta ttattttttg tattattttt      60
   caatgccgca attaattttg tcagttcaca tcgcattgtc ggttattatc agggcatacg     120
   tccattgaca aatgatcaag ccaagaagtt gacccatctt atcctggcat tttcaacccc     180
   tgactctcaa ggcaatttga gtccattgag ctctgtgctt aaacaggcgc taaaagcggg     240
   taaatccgct aatggtgcgc tcaaagtgat gattgccatt ggaggaggtg gctttgatcc     300
   ggccatattt acttcgttag catcaaacag tggcacacgt aaaagcttta ttaataacat     360
   tgtttcttat ctgaaaacca atgagctgga cggttgcgac atcgactggg agttcccaac     420
   ttctagtgac aaggcaatct ttgtgacatt tctgcgcgac ttaaaaaagg cgatggcacc     480
   cagcggcgct gtgcttagca tggcatcggc agcaagtgcc ttctatttgg accctggtta     540
   cgatttgcca ggcattgaga gtgccgtcga tttcattaac gtgatgtgct atgactatta     600
   tggaagctgg accaaaacat cgactgggcc aaactcgcca ctgtttaagg gtggcagtgc     660
   cgacccatcg gacacattga acagcaattg gacaatgaat tatcacttaa tgaaagtgta     720
   taatcgagca aagttgaaca tgggtgtgcc attctacgga aaatcttgga ccaatgttgg     780
   agcaccacta aacggtgacg gactttggcg tcagttgggc acttatggca ccgaattagc     840
   ctggcgtaac atgggcaaaa gttttgacat gaccaagaca acgtatcata aaacggccaa     900
   aactgcatac atttatgata cagctaccaa aaatttttta acctttgaca acccccaatc     960
   actgaaggac aaggcaaaat atgttgcgga aaagggcatt ggtgggataa tgatatggtc    1020
   aattgatcaa gatgacgaca aattgtcttt attgaattct gtttcatatt gattttgatg    1080
   tatatattgt gtttgagcca ttaaaatgtg ttagaaaaaa aaaaaaaaaa aaaaaaaaaa    1140
   aaa                                                                  1143

<210>  5
<211>  706
<212>  DNA
<213>  Heterodera glycines

<400>  5
   gactcattcc atcgccgctc actcattttc ttccctttcc tttgtcttcc attcattccg      60
   cttttccctc cccatttctc atttgttctt ctccgcttct tctctccccg atgaatcact     120
   ttgggctgac ctttctgttc gtcgccgttt ctctgctgac attgacgggc aaaacgatca     180
   ctttggaggt ggagagctcg gacactgtgg acaatgtgaa gacgaagatc caagagaagg     240
   agggcgttcc gccggatcag caacggctga tcttcgccgg caaacagctc gaggacggac 300
   gaacgttggc cgactacaac atacagaagg agtccacgct ccacttggtc ctccgtctcc 360
   ggggcggaaa tgggaagaga aacacgagta agaacaagaa aagcaacaaa aagcttgatc 420
   agaattgatc agcagcgaac gcaccatcac atgattgatt ggcacagtga ttttccacca 480
   ccaaattcac aacactttcc cctttttccg ggttatcttt gatttccatt ttcggttatt 540
   gttttctgtt tttctcctct atcttttgta aaattctgtt actttgatct ttagtgttta 600
   tcatttatca tctgcatata tagaagttat catagtccat atttctgtcc ctttttattt 660
   attttttttg actataaaac ttttgggcac aaaaaaaaaa aaaaaa 706

<210> 6
<211> 734
<212> DNA
<213> Heterodera glycines

<400> 6
atgtcttctc tgctgctctc catcttccca attgtgtttt tggtctgttg caatgcaatg 60

ccaaatttcc cgtgctgccc gggcagtcag caagtggttg ctgtgatgtc caaatacatt 120

gacactttct cttctgctgc tgacgagtct acagtatgct caaccgctaa aagtactgtg 180

gatggaataa aaaatgaact gtcctatcgc gtgggatgcc caagcggagg agaagcacaa 240

attgtgaatg aaattgatca acagctgaag aacattgcga aaatggaaat caattatgag 300

gacgagtgcc cgtacaattt gggctttgcc cgtgccatgt tcgacttggc cgctgctgct 360

gctggccatg cggggaacgg cacagaatgg caatacatga aagtacaatt tgagcaggaa 420

agccaagcaa tcaaagcaat tggacaagaa aagaactttg aagttacgga tgtgcatttt 480

ggagtcccaa gcaaaggggt ttctgcacat caaaatgtgc cgagtccgag ccatgtgatt 540

gccaaccctg gccaacacag ttcggttggc caaggaaaga aagaagaacc gttgtcatcg 600

gacttcgatt tttgaggaca aacaaatcag gaggaaatag aatagaaaac cattttgttg 660

acatgtcgaa catttattta aagtaataaa tatttggtaa taaggaaaaa aaaaaaaaaa 720

aaaaaaaaaa aaaa 734

<210> 7
<211> 769
<212> DNA
<213> Heterodera glycines

<400> 7
atcatttgta attcattttt taaataaaaa tttccttgga ccgacaaaaa tggcaaaatt 60

cgttgccatt gctcttctct cgctgaccat tgtttcgatg gcacttgcaa aaactggcaa 120

aagtcaaacg gcagacgaag ttgagggatt tcgcaatatg aacatcggcg acaacaataa 180

ggttgacgcc ggaaaggagc ccgcggctga taaagcaacc aaaaagggaa aagctcagaa 240

agccggaacg aaatcggcgg cggccactaa tgagcccgcg gctgctaaag gaactaaaaa 300

tggaaaagcc ccgaaaacca aagcgaagca agaggtggcc actaatgagc ccgcggctgc 360

taatgaatgg aacgaccaat tgatgggcat gagcgttgag aaatttaacg aggagcttgc 420

tgtgttgttg ccaaaagcca acacttttat ggaaaatgct ttgagcttca tcaatgaaca 480

agtggaaaaa aatggtattg caactggcgc tgccggggac tcgtgctcga ccgggatccc 540

ggccaactag gagaccaatg ccacaggacg gcaccggagg agggaagaaa ggatgacgag 600

ctctttggat gatgaaggtg gtggataatg gatggttagt tgattggggg gatttacatt 660

tttcattcaa ctttcttggg agttgcaaat ttcaaaaaat gttttttttt ttgaaatttt 720

tggtaaactg atcggcatta aaaaaaaaaa aaaaaaaaaa aaaaaaaaa 769

<210> 8
<211> 469
<212> DNA
<213> Heterodera glycines

<400> 8
gattccctca aaagcaaagc catccatccg atcattcgct tcatctgcca atccgcaaaa 60

tgtccctttt ccgtcctcaa tcgctgcttc ttctggccgc tctttgcctg tcctttgcgc 120

tgctctttgt cacttcgtcg gaagagggag ggcgagtgaa gcgcggcgga tggccttggg 180

attgggccgg caaacaactg tgcaaaacat cggcaaattg caagtgcaag gatggcaaaa 240

attgggccaa atgtgtaaag tcggaaggct acgcggccag caattgttgc gacaaaaatt 300

acgtgtgggc atgttgcggg aagaagccca aacattgatg agagaagaga aagggagaga 360

atgagaagcg agttcacata attcattctg ttttggattt ttagtctttt ttttcttttt 420

gggttttttt ggtaaatatt ttctttagca aaaaaaaaaa aaaaaaaaa 469

<210> 9
<211> 1200
<212> DNA
<213> Heterodera glycines

<400> 9
aatcccaatt tcgcattcat ctcactcact cataaaatgc tccaaaacgg ccttaccatt 60

ctgcttctga tcagtgttgt gatcggccat tccttggcca accttggccc aaccatcaaa 120

cataatcctc attttaaagc cgtacaaact gcgcatcatt tgcatgatgc cattgcgaag 180

aagcacgagg ccgaagttac gcaagtcatt tgctctatta gcaacgaaca gcgtcaagca 240

ttggctttgg agttcaaaaa acaattcggc actgatctga ttgccatgct gaaaaaggag 300

ttcaaaagcg actttgaaga actgatcatt tctttgatgc aaacgcccgc cgtttacgat 360

gccaaccaaa tgcgtgccgc attgtccggc tccaatgagg cggtgctaat cgaaattttg 420

gcgacgcgca caaaccgaca aattacggcg ccgaagcagg cgtatgagca gttggacaga 480

aggcatcagc acaatcagct ggaggaggac atcaaagcga agacgaagag gacccttcca 540

aaatctgttg gtgtctttgc tcagctgctc tcgcgaagaa aaagtgcgcc cgcaagcatt 600

gtattggcac acgacgaggc catgaaactg ttcagagagg gcgagggccg acggggcgtt 660

aacgccgtgg tgttcaacca ggtgttggcc actcgcagct tcgcccagct tcgggaaact 720

ttcgagtttt accgacaagc cgcgcaccac gagattgagg agggaattaa gcaagaattc 780

agcggtcaca acgaagcggg tttcttggca ctaatcaaat atgtccgcaa cgcttctgtg 840

ttttttgcgg atttgttgtt caattcgatg aaagggctcg gcacacgcga ctcggatttg 900

attcgtctgg tcatttctcg gtctgagatt gacctggctg acatcaaaca cgcttttcac 960

acgttgcaca agaagagcct ggaggaggcg atcaaagggg acaccagcgg agcttaccga 1020

gacgcacttt tggcattggt caagggcaac acggagcagt gatggagcag cggcagaagg 1080

gattttgcaa gagattagga tagatgtaaa agagataaac atgaataccc aataaacatc 1140

ctgtagaaat tataaaataa aattttttat ttacaaaaaa aaaaaaaaaa aaaaaaaaaa 1200

<210> 10
<211> 951
<212> DNA
<213> Heterodera glycines

<220>
<221> misc_feature
<222> (402)..(402)
<223> n is a, c, g, or t

<400> 10
tcaaaaattc attcaaccct agaatttatt ttttttaata aataagtttt tttgtacaaa 60

atgagcaact ttatatttgt cgcctcttta actgcagcgt tttttagctc aggcctcgct 120

ctaccggctc cttatgatgc tgaatcggtg gtatcttctg aattaaatgt tccactactt 180

tcagctgacg caaatgttga agcagcaatt accaatgaaa gtgatgctgc tgctgagata 240

caagctccat caattccggt accaattgaa catcaaactg ctgctgacat tactcatcca 300

actgaaactg gcaatgagtc ttctattgca tcatcatcat ccacgccgaa aagtgagcag 360

acgccaaaaa aagtgatgaa catgaaaagc gcgctggaag ancgcaggca ggccaacgtg 420

tacgaggaga ggcttccgtt agacaagcag cccaagacga tcgctgaagc ggcagcaaaa 480

gcaaagcaga cgcccgccaa acttccggct gactacgacg tgaaccgcgt ggcggaacgc 540

gcagcagccc gtgtgtacgg gtggcttccg gaagacaagc agcctaaggc gatctatgac 600

gcggcggaga aggcaaagaa cacgcccaaa ccgccgggcg actacgacgt ggagcgcgtg 660

gcgcaaaagg cggcccggct cgtctacggt gtgctgccca tcggcatgca gcccaacttc 720

gccggcccta gcactgacaa gagcaatgtc gacgactcgg agaaaccttc tgctgctgcg 780

gctggtgatg atgatgatga agtcgaaaaa gagaagaagg aataagcaaa aaacaatgtg 840

aatttattat aggaaaaaaa gaaaaatgga agcttagaaa tttaatattt tcatttttga 900

acatttataa aatttcaaaa ttgaccgcaa aaaaaaaaaa aaaaaaaaaa a 951

<210> 11
<211> 746
<212> DNA
<213> Heterodera glycines

<400> 11
gattccatcg cccctcactc attttcttcc ctttcctttg tcttccattc attccgcttt 60

tccctcccca tttctcaatt gttcttctcc gcttcttctc tccccgatga atcactttgg 120

gctgactttt ctgttcgtcg ccgtttctct gctggtaatt gcgcccggct gcgatggcat 180

gcaaattttc gtgaagacat tgacgggcaa aacgatcact ttggaggtgg agagctcgga 240

cactgtggac aatgtgaagg agaagatcca agagaaggag ggcattccgc cggatcagca 300

acggctgatc ttcgccggca aacagctcga ggacggacga acgttggccg actacaacat 360

acagaaggag tccacgctcc acttggtcct ccgtctccgg ggcggaaatg ggaagagaaa 420

cacgggtaag aacaagaaaa gcaacaaaaa gcttgatcag aattgatcag cagcggacgc 480

accatcacat gattgattgg cacagtgatt ttccaccacc aaattcacaa cacttttccc 540

ttttccgggt tatctttgat ttccattttc ggttattgtt ttctgttttt ctcctctatc 600

ttttgtaaaa ttctgttact ttgatcttta gtgtttatca tttatcatct gcatatatag 660

aagttatcat agtccatatt tctgtccctt tttatttatt ttttttgact ataaaacttt 720

tgggcacaaa aaaaaaaaaa aaaaaa 746

<210> 12
<211> 639
<212> DNA
<213> Heterodera glycines

<400> 12
atccaaatca acgcggcaca gtaataaaat attaaaagat ccgaaaaaat gccaaacatt 60

ttcaaaatcc ttctgattgt gcttttggcc gtcgtctcat tccgcctctc ggcttctact 120

ggtgacaaaa aaactgctaa tgatgggagt ggaaacaact catcagctgg gattggtacg 180

aagatcaaaa gaattgtcac cgctggactg ctcttcactt ccctggcgac gggtggggcg 240

gaagcgattg ggcgaagcaa tgctcaggga ggaaatgccg ccggattggt gccatcgcat 300

gtgaccaatc gctcaatggc tccaccacct cctcctgtgc aatttgaaat gggggcaaat 360

cgattagaaa aaatgagggc acacctacgc gaacttgctg agaaaatgcc ggtcaatgaa 420

tcgaagcgtc tgtcaccgag tggacccgac cctcatcatc attagggcca tggatggatc 480

taacggagga agaaagaatg gatgcttagt tttcagattt tattctatcc tcttttattt 540

attagttttt caaaacaaat ttcttccaca actttttaaa ctatttgttg ttttgaatct 600

ttttagataa atctaaaccc caaaaaaaaa aaaaaaaaa 639

<210> 13
<211> 1519
<212> DNA
<213> Heterodera glycines

<400> 13
gaaaatttga ttttatacaa taaaaatatt ataatatttt tgaataaaaa tgaaaaattc 60

tttcctcttc ctgcttcaaa tttttattct aaccaacatt ttaactgaaa tactttgtgg 120

agataagtca aggccgtcga cggaaatcaa tgccaatttg ggaacaagga aaaagcctga 180

aacaattacg gcaacaaaaa atgccaattt gggaacagga aaaatgcatg aaaccgatgg 240

gaccagcaaa atgccaaaac atggcaaacc tgtgtcaaat cgaatggcag ccaaaagcac 300

gacgattaaa aataataatg aagcagcagg gccaagccaa caatcaaaac aacctgcggc 360

aaacattacg ccacagcaaa aggggccaat gcaaaattca aaaaagcagc caccaaacga 420

attgtttggc aagaagcaaa ggtcaacccc tgaagagatc aaagctggca aacaaccagc 480

aatggagcta atgccatgct atcgaggaat cggcaaaaag acgatgccaa caattgcaca 540

gcgaaatgcc accatgctaa aaagggggga cagtttgaca cggagtgccg actttgagga 600

cccaattttg gccaatgtaa aacaaagcca aatgttgatc cgacaaaaat ccaagggcac 660

catggacttg gaaatggacg aattcttaaa actgcacaag caaattcaag gacctcggca 720

aaaattggta cgaatggaaa gggaaaaaat gatcaaagaa gcgaccgaaa aggcaaaagt 780

ggaccgaaga aatcgtatgt tggaacgaga aaaaatgtgg agcatgaaga aggctggtgt 840

tgctgcacat caaccagcat caccggcaat tcgtggacga acacagcagc aacaacagca 900

gaagctgcaa cctgacaaaa agccggaaaa aatgctgaaa cagcaaaaca aaacacaaaa 960

tttttcggca ccttccacca gcaaacaaat tgtcaatcga aggttgttga ttggcccaaa 1020

caaaccgaga actggcaaca aaattggcac taccaagcac ggcatacatt cggcggaaat 1080

aatgacatca tcgtcggaaa gcaatgtgcc aaaaagtgaa aatttgggca gcagtgaaat 1140

ggagacggcg gaggaagcgg cacaaaccta tttgaacaat ttacgggttg atttaaataa 1200

aattccttcg atgaaaattc cattccttga accaataatt gaaaaatatg ttgatgaata 1260

caaaaaaagt cgtcaattat tcgctgaatg catgcaaaaa attttgcgaa aatcgaaaaa 1320

tcctaatcaa acggtgaaga tcatcgaatt taaaaagaca attgaaaaag aaatgaatga 1380

tttgttgaaa catacggatt tgccaccaga tcaattaatt gatctgttga acactcgtga 1440

atggctattg tcgatgaaaa aagacattga gtacatggaa cgcattctga ccggacgcga 1500

gaataatgac aacatttga 1519

<210> 14
<211> 700
<212> DNA
<213> Heterodera glycines

<400> 14
ggaattattt aataaacaaa aaaacaatta attaccgcca attgcccatt agaagaaatg 60

ttcagctctt ccaatttgtc tgctctcttt ttggcctcct ccgttttggc cgtgcttata 120

attggcatta aaatggacgg accgacggag gcaaaaggcg ccgcccctcc aaacgccgcg 180

gggccaatgg gacttttgct tttattgaat ggcaaacaat cggcggctaa tgaaaaggga 240

aaagcgccct ctggcgaaag taagccaaat ccggggcaga agccgagcgg agaacggcga 300

aagagggacg ttttggggca cgccggcgga tacgtcggag gatgggacca tcccattgac 360

tcgacacttg attgggcaaa gagtcagtgg aatgatgcca attggctcgc cgatgttgtc 420

aacagaaacg gatgggaaaa caccggcact ccaaccggcg gacgatgaat cagtgaattg 480

tgccgaccaa ggaatgaaag acggcatttt tgtttgggaa tttaattgac ttttcggaat 540

caaacacact ttccttttta gttgcctatt tattctaaat tagcgttttt tctgttcatt 600

ttctacgcaa aacaaatttt ttacatattt tttggttggg gattatccca atggtcccat 660

ttttcgcata aatatgaatt gataaaaaaa aaaaaaaaaa 700

<210> 15
<211> 975
<212> DNA
<213> Heterodera glycines

<400> 15
atatcacatt ttcatttaaa ttatcctccc aaaatgcgta ccattctctt catggccatg 60

gtttgcttgg tgatggctgt cctaatggaa atggcaaatt caaaagtagt caaaaaagac 120

aataaaaaag cagcagtggc ggcatctcca gcaaaaggaa aagcatcgcc aaaaggaggc 180

aaaagcccag caaaaggaaa agcggcaaaa gttagtaaaa aagataacaa attgaaagca 240

aagaaagaaa ccaaaggcat taaagttaaa aatgcaaagc caacaaaaaa aggtaaatcg 300

gcaaaaggcg catcaaaaac agccaagaaa gtccaagccg ccaaaaaagc accaggaaag 360

gacaaaaagt cgctagttaa gccaatcgtc cttaaagcac cggtaccacc ccataaaatg 420

cacccgatga ttgaatccga agctgtgcca ccgcccgccc atgcgcgttc gcttgcgacc 480

gttccttaca gtacaccggg ggcagccgac cgtaactcat tgccatcgta cacttcgact 540

gccaccaatt tggacatggc cgatgataat gatgattatc agaattatta ctacggaacg 600

gaagacagca gcagggaatt tgatgcatcg gcggaggagg aggatgaact atatgagaga 660

ggagagtcgg ggcaatgagt gggcagacgg acgactacgg tattaggcag tggacagaga 720

tgagtacggt catcaaccgg tggacgtgcc gccatagtcg gtgatgctcg actgaagagc 780

atcaatgaaa tcaatcagag atcaccggaa aatcattttt tcgtaatata gaccaaaatt 840

tatagttgct aatttttgta tatttttatt tgtaaaaata gttttttatt aataaatata 900

actcttacaa ttatctcatt tataattttg ctgaagtaaa acaacgaaaa aaaaaaaaaa 960

aaaaaaaaaa aaaaa 975

<210> 16
<211> 1333
<212> DNA
<213> Heterodera glycines

<400> 16
ccaactaatt ttctcccaat ctaaaaaagt ctctctaatt aatactaatt agtacaaact 60

gctgttgatt tgatcacaga atgttggttc aactcgtcct ccttgccatc attggcattt 120

cctttgtcgg tgctgccgcg ccgccgtacg gccaattgtc cgtctccggc accaaattgg 180

ttggctcaaa cggcaaaccg gtgcagctga tcggcaattc gttgttctgg caccagtggt 240

acccacaatt ttggaatact gaaacagtga aggcactcaa atgcaattgg aattccaatg 300

tcgtgcgcac cgcaatgggc gtggaacagg gcggctatct gagtgacgcg aacaccgcct 360

accgactgac ggcagctgtg attgaggcgg ccattgcaca gggcatttac gtgatcgtcg 420

attggcacgc gcatgaggcg aacgcggaca aagcgattga attcttcacc aaagttgcga 480

aagcgtacgg ctccaaccct cacttgcttt acgaaacgtt taacgagccg ttggacgtgt 540

cttggaacga tgtgcttgtc ccgtaccata aaaaggttat ttctgcaatt cgtgccatcg 600

acaaaaagaa tgtgatcatt ctcggcactc ccaaatggtc tcaagatgtt gacgtggcgg 660

cccaaaatcc gatcaaagga ttcggtaatt tgatgtacac tctccacttc tatgcgtcca 720

gtcactttgt tgatggactt ggaaataagc ttaagaccgc cgtaaacaag ggtcttccgg 780

tgttcgtcac tgagtacggt acatgcgaag cgtctggcaa tggtaatctg aataccaatt 840

caatgtcaag ctggtggagc ctgctggacc aactgcaaat ttcgtacgtc aattggtcaa 900

tcactgacaa aagcgaagct tgtgcagcgc tcactggcgg aacatcggct gccaatgttg 960

gcacttcctc ccgctggacg cagtctggca atatggtagc ttcgcaacac aagaaaaaat 1020

ccaccggtgt gaactgcagc ggtggtggtg gcgctgctgc taagccagct gctaagcccg 1080

ccgctaagcc agctgctaaa tcgaagggaa agtcttccaa agccaagaag tccggatgat 1140

cagcaaatca caataaacat agaaagtgaa ttgaagacaa tatggtgatt caaaaaacaa 1200

ataagtgcat aatgataatt ttaagtataa ttgtaatatt caaaaatatt cttaggagta 1260

aatcgggcac tgataaagca tgaactatta ttaaaaaaaa aaaaaaaaaa aaaaaaaaaa 1320

aaaaaaaaaa aaa 1333

<210> 17
<211> 330
<212> DNA
<213> Heterodera glycines

<400> 17
atgtacaaat ttttattttt ccctcatttt tgtatcttaa ttaaggcaat ttcggggctc 60

cattgttgga attcgaacat gattttgctg agtgaaatgc cggaaaaggg cagtgtcact 120

gtacaccaat gtccgtccgg gcatcaatgc gtgacggcaa attgttggct tggagtcggc 180

aattacattg tccaaaaatg cgtgccggac cagccagggg ccagaaacta ttgcaaagac 240

ttcaacaaca tttgccaaat gggctttttc ggcatgccaa ttcattgctc atcttgtaag 300

ggtgatctgt gcaacgaagc gcgtaattaa 330

<210> 18
<211> 1485
<212> DNA
<213> Heterodera glycines

<400> 18
agaatttaaa aatcataaaa attcaaactc atcaaattca ctcaacatca tcaccaaccg 60

tgaacattat gctgaggatt gctctactca tctccatttt ggcactgttt ggtgattgca 120

tggacatggg aaaaagaaaa ttaggaggaa tcagtattaa tgagccaagt gaatatggaa 180

ccaaagaaaa agaagccatc gcaacaaaag aaaatgcaca aacatcaaag gacccgccga 240

catcggcggg tggtcaaaat gaagcaatcc cttcaccaaa aaagccaagc cccaagggga 300

agttgaaaag cgattttggc ctaaacttag ccaaggcttt tccacggccg gttccgaaag 360

gcagaagggg caaagaaaaa gtcggcgaaa aaataagcgt tatgaacaca gctgaacgca 420

ttgaaaaaat ggacattgcc caagacaaag ctaaaatgga aatagacaat atagacacca 480

atgccaaacc caaacaaaat gaggataaag ctccaaccat attccccaaa tgtgcggaca 540

acgtggaagt ggaagtagag ctcgatcgta atatttttcg tttttctact acgcttgaca 600

caatgatgga agatcttgga atgtacactg ctgaaggcac aaaccagaaa ttgccggttt 660

caaatgtcag tagtacggtg atgcgagaag tgattgaatg gtgcgagcat cacaaaaacg 720

atgcctcaat agagccaatt tatgaagaaa ttgctttgga tgtgccaact ggtaaagatg 780

cggaggcatc cgcaccaaat gctcaagctg gagaagttgc ggaggcagcc gaatcaaatg 840

ccaaaccaaa taatgaaaag cgtctcgtct ttccaagctg ggatgagaat tttttggata 900

aggaatggcc tgagcttgtt gatataattt tagcagccaa ctatttgaac atcaaacttt 960

tgcttacctt cgcgaccaca atggttgata acaagtggat caatggcaaa acgccgcagg 1020

aaattcgcaa gggattcggc gtcgaagagc cgtacccgcc gggacatccg gaatgggcac 1080

gagttgagaa ggagaacgag tgggaagaat cggacgagga acgtgaggca cgccatgcaa 1140

aggaacgaga ggaggaagag gagcgtgaga gaaaggaaga acagaagcgt aaggaagagg 1200

aagcggaacg cctccatcag gaacaactgc agcaacaaca gaatcaggaa cagcaacctc 1260

agtagggaca gcagcatggt gaagaactgg aacacgatga agttatgcat gatgtggagg 1320

aagagcaaga tgatgaatgg cgaggaagaa gatgatgagt gatgaggatg aggaggatgt 1380

aaaatgatgt ggtgattagt gattttgatg aaccgatgat tcacttttct tggatcctgt 1440

tgcataaaac ttgttgcaaa aaaaaaaaaa aaaaaaaaaa aaaaa 1485

<210> 19
<211> 1250
<212> DNA
<213> Heterodera glycines

<220>
<221> misc_feature
<222> (1222)..(1222)
<223> n is a, c, g, or t

<400> 19
gagaatttaa aaatcataaa aattcaaact catcaaattc actcaacatc atcaccaacc 60

gtgaacatta tgctgaggat tgctctactc atctccattt tggcactgtt tggtgattgc 120

atggacaagg gaaaaagaaa attaggagga atcagtatta atgagccaag tgaatatgga 180

accaaagaaa aagaagccat cgcaacaaaa gaaaatgctc taacatcaaa ggacccgccg 240

acatcggcgg gtggtcaaaa tgaagcaatc ccttcaccaa aaaagccaag ccccaagggg 300

aagttgaaaa gcgattttgg cctaaactta gccaaggctt ttccacggcc ggttccgaaa 360

ggaaaaaaag ttgaaaatac actatcatca aaggactcgg catctgaagc tcacaaagaa 420

ttggccaaag agaaaaatga agaaaatgca cagacctcaa aggtcccgac atcgatggaa 480

aggcaaaatg aaccaatccc ttcaccaaaa aagccaagcc ccaaggggaa gtccaaaagc 540

gatttcgccc taaacttggc caaagctttt ccacgaccag ttgccgaagc aaaaatggga 600

gaagaagctc agtcttcaaa agatccgaca atgaatgcca aatttgtgca atttgtttgg 660

atgcatcgct tatcactgac cttgaaattg agcaaatgcc atcatcgctt tcaccgcgaa 720

tgcgttgatg ggtggtttaa aaacaatgac acatgccctt attgtcgtgc tgtagttgca 780

agcagatatt taccaagacc tacgcgtaca gatcgaattt ttgacgccag aatcgaaaac 840

aaaagacgct tcatgggaga aggagaagga aaatacacaa ttattcgccc taacggaagt 900

acgcttatgg ttcacgataa tcattttgga aacaatttta cggtcgaaaa aactgaagag 960

ggctccattc aactcagtaa aaacgatcgc aaatagaaaa attgtttcaa gattaattaa 1020

aaaatgtcag ggagggttgc ggaagccgga aagaattatg gataaaaaac ctaaaaaaaa 1080

tcggaggcta aacaaatttc gaaactcatc taaaccgcaa ttcggatcca tatccctgat 1140

atgtaatata catttatgca tcagggatgc attcggaaat atgtattggt actgagtcgg 1200

aatttttaac gtttcggcat ancaataaat atttaatcaa aaaaaaaaaa 1250

<210> 20
<211> 897
<212> DNA
<213> Heterodera glycines

<400> 20
atatctatca tattttcatt taaattatcc tcccaaaatg cgcaccattc tcttcatagc 60

catggtttgc ttggtgatgg ctgtcctaat ggaaatggca aattcaaagg ctgtcaaaaa 120

agacaacaaa aaaggagcag tggcggcatc accagcaaaa ggaaaagcat cgccaaaagg 180

aggcaaaagc ccagcaaaag gaaaagcggc aaaaaaattg aaacctaaaa aggatgctaa 240

aggcattaaa gctaaaaaag caaagccagc aaagaaaggc aaagcggcaa aagcagtaaa 300

gggagcgcct aagacagtca aaaaactcgc aattgccaaa acagcacaag caaaagacaa 360

aaagtcacca gccaagccaa tggttctaaa agcagtgccg ccccaccaaa tgcatttaat 420

gaatgagaaa gttcaaacag ctgtgtctcc acccgcccat gctcgttcac ttgcgaccgt 480

tccttacagt acaccggtgg cagccgaccg taactcactg ccatcgtaca cttcggatgg 540

caccaatttg gacatggccc atgagaatga tgattatcag aattattact acggatcggg 600

agacagcagc aaagaatttg atgcatcggc tgaagaggaa gatggactgt atgaggaggg 660

ggacggggca atgaattggg caaacggacg actaccggat tgggcattga acaggaatac 720

gggcatcaat cggggggagt ggccaccaaa gtcggggatg ctccaactga aaaacaccaa 780

gaattttgga aaaaacttat tttccaaaat tttgaaacat ttggatttta atattcccgt 840

taaaaaacta aatttttttt tacccagcct ctaattttgt aaaaaaaaaa aaaaaaa 897

<210> 21
<211> 530
<212> DNA
<213> Heterodera glycines

<400> 21
gattcattcc ctcaaaagca aagccatcca tccgatcatt cgcttcatct gtcaatccgc 60

aaaatgtccc ttctccgtcc tcaatcgctg cttcttgtgc ccgctctttc cctgtccttt 120

gcgctgctct ttgtcacttc gtcggaagag ggagggcgag tgaagcccgg cggatgccct 180

tgggattggg ccggcaaaca actgtgcaaa acatcggcaa attgcaagtg caaggatggc 240

aaaagttggg ccaaatgtgt aaagtcggaa ggctacgcgg ccagcaattg ttgcgacaaa 300

aattacgtgt gggcatgttg cgggaagaag cccaaacatt gatgagagaa gagaacggga 360

gagaatgaga agcgagttcc cataattcat tctgttttgg atttttagtc tttttttctt 420

tttgggtttt ttttggtaaa tattttcttt agttattaaa aattgtttgg tctaattatg 480

ccattggaaa aaatgtattt gttttttaca aaaaaaaaaa aaaaaaaaaa 530

<210> 22
<211> 673
<212> DNA
<213> Heterodera glycines

<400> 22
ggctccaaaa aaaatccatt ccacattcca gcaaccaata ccattaattt cataaatccg 60

aagaaatccc tacaaaaatg tcttcttccc cgtccgtctc tgtgctcgcc atcgtcgcaa 120

ttgtctgttt gatgtgccaa tgttgctttt cggcaccgca tccgtgctgt cccggcagtc 180

aaaaagtggt ttcacttatg tccaattacg ttggcacttt tgccaattcc atttccaagt 240

catcgctttg ttcggatgcc caaaatgttg cggacgcgtt gaaaggccaa ctgatcggct 300

gctcgaatgg cggcgatcga actcttttgg ccgacatcga agcatctctt gccagtcatt 360

ctgctgatga gtgtgcccac agcctcggct tcgtccgtgc catgttcgcc attgccgcct 420

ctgcttcttc tcatgccagc aacaacagcg aatggcaggc attgagtaca cagtttgttc 480

agaaagtcac tgaaattgac tcgaaatgtg cagagtttgg cattagcatt ggaaaagtgc 540

ccatcgatgg ccccaaggga gaccactccc aacgaaatgt gcctagtacg gacagtgtga 600

tttccatgcc cggattgacc ggctcacaca aacattgaac tgaatgatga gtgacggaat 660

ggataaacta att 673

<210> 23
<211> 1614

<212>  DNA
<213>  Heterodera glycines


<220>
<221>  misc_feature
<222>  (1157)..(1157)
<223>  n is a, c, g, or t

<400>  23
atggccctct ctgcccttct gcttcttctc cccctgcttc tcaatgttca aaatatccca      60

gatgagtcag tccaatcgga tatgaaggct gtttattcgg ctatttcatc gccggaacaa     120

tggaagaact cacaaaattc attggcttca ctcgaatcac aactgacaga gccccaaaga     180

gcactggcaa aaatgcattg ggaattggag accatccaaa aggaaaagcc ggaggcaccg     240

ccacaattcg acttgggact tttctcggaa gctttggaag tgatggtcga aatggacgaa     300

gaagcgaaag aagtgaagct gaggaaggac aaactgatcc gaatgggcag gaggagagga     360

agcaaaaaaa aattgaagga aaagatgacg gaggaggaca cagtgccgga agtgagggtg     420

aacgagaatg gtaaggttga agtgaaggac ggcgccggag gggaaggacg gttggaagtc     480

agacgaacaa aggacgagaa aggcagagag caggtggtga tcacccttat gaagaatggc     540

ggaacggagg gaccagcgga aggaaccgcg gagaagccac aagagaaggc caagacggag     600

gaggaggtac agaaaaagaa tgatgacaaa agtacacgac aagaacagga ggaagcgaag     660

aagacggagc aggaaaacgc cggaggggtg ccaaaaactg actcggccaa cagccacatt     720

ccggtaatgc caatgcacac cattttgtca tccccgtctc caccggtgga ggagaagggc     780

aaagcgagtg cagaggaggc acaaaaaaag gaggaggaca aaaacacacg acgtggaggg     840

gaggaggcaa acaacacgga gcagaacaaa gtcggaggag tgccaaaaat gtcagttgac     900

agtcctaagt cggtcgtgcc aatgccaatg cacaccattt tgtcctcacc agccccaccg     960

gtcgaggagc aggacaaggc aagggatgcg ctcacagaag gagcaaatgg aaggaaaaag    1020

gcgcaaaaca acgaagaaat gttgctggtt gcgacggaga acaacggaag catgagaaat    1080

acgaataatg gaggaatatt tgattttgtc cgaaaattta tctccaatgt gtttggacgt    1140

aagaaaaggg acacggnaag cggtgcacag agaaaatatg acgggggaac gaaggaaaat    1200

tcgcagcatt caaaatttga ttatgagcac tgtagggaag tccgactcag agccgaagag    1260

cgaagacaaa cagcagcagg aagagaaaaa gaaggcggag atcaagcagt acctggagaa    1320

gggagtggtc aacacgggtg gaatcaaaaa ggccgagaaa ctggcatatg cttggtactc    1380

ggagcttctg tactggacaa ccaagtggat cgaggcatta gaaaatcggg tggcgggagt    1440

caaacccgaa ttggcacagc aattcttatt ctccaaaacg ggatcagctg cttaccagga    1500

gctgaaggaa gaggtggaca aatgcgaggc gaagttggcc aaactaaagg aatggatcgg    1560

cgactctttc aagtgaaaat ggaagaaacg gctcttagag gtgtattttt ctga        1614

<210>  24
<211>  909
<212>  DNA
<213>  Heterodera glycines

<220>
<221>  misc_feature
<222>  (613)..(613)
<223>  n is a, c, g, or t

<400>  24
ttcccttatg gccatggttt gcttggtgat ggctgtccta atggaaatgg caaattcaaa      60

agcagtcaaa aaagacaata aaaaaggagc agtggcggca tcaccagcaa aaggaaaagc     120

atcgccaaaa ggaggcaaaa gcccagcaaa aggaaaagcg gcaaaagtta gtaaaaaaga     180

taacaaattg aaagcaaaga aagaaaccaa aggcattaaa gttaaaaatg caaagccaac     240

aaaaaaaggt aaatcggcaa aaggcgcacc aaaaacagcc aagaaagtcc aagccgccaa     300

aaaagcacca ggaaaggaca aaaagtcgct agttaagcca atcgtcctta aagcaccggt     360

accaccccat aaaatgcacc cgatgattga atccgaagct gtgccaccgc ccgcccatgc     420

gcgttcgctt gcgaccgttc cttacagtac accgggggca gccgaccgta actcattgcc     480

atcgtacact tcggctggca ccaatttgga catggccgat gataatgatg attatcagaa     540

ttattactac ggaacggaag acggcagcag ggaatttgat gcatcggcgg aggaggagga     600

tgaactatat gangagggag gtcggggcaa tgagtgggca gacggacgac tacggtattg     660

gcagtggaca ggatgagtac ggtcatcaac cggtggacgt gccgccatag tcggtgatgc     720

tcgactgaag agcatcaatg aaatcaatca gagatcaccg gaaaatcatt ttttcgtaat     780

atagaccaaa atttatagtt gctaattttt gtatattttt atttgtaaaa atagtttttt     840

attaataaat ataactctta caattatctc atttataatt ttgctgaagt aaaacaacga     900

aaaaaaaaa                                                             909

<210>  25
<211>  1278
<212>  DNA
<213>  Heterodera glycines

<400>  25
attttatttt atctacaact caataaaaaa tgtccgcttg gatttgtcct gggtttttgc      60

tgttttttgt gctcattctg atcactgatc actgtccgat gacccaagct gtcactcaac     120

cgccctatgg tcaattggct gtcagtggaa aaaatctagt acaaaaaagt acgaaaaaag     180

ccgtggcatt gcacggcctg tcaatgtact ggagccaatg ggtgccgaag ttttgggtga 240

aacagaccgt caacaaatta aaatgttcat gcaatgccaa cgttgtacgc gcagcaatgg 300

catccagctt tggcggttac atttcaaacc caactgctga atataaaaaa atgactgcaa 360

tcattgatgg ggcaattgac caaggccttt acgtggttgt cgattggcac acgggcgacg 420

atttggccac caccgaaata aaatatgcga ctgaattctt tacaaaaatt gctaaagcct 480

ataataaata cccgcacatt ttgtacgaaa tttggaatga gcccaacaaa tttgtggcat 540

gggaagcggt ggtcaaacca tacgcaaaga cgatgattga cgtgattcgt aaatatgaca 600

aaaacaatgt gatcattgtt ggcacgccaa actgggacca agatgttgac attgttgcaa 660

aaagtccgtt gaaagacgca aatattattt acactatgca cttttacgcc ggccaacaca 720

aagatgacat ccgaaacaaa gtcaaaacgg cttataaatt gggccttcca atgtttgtga 780

ctgagtacgg ttgctactcg gctgacggca atgaaaaaac aaatttggaa gagttgaaaa 840

agtggatgga acttttggac ggctatggca tttcctacgc cgcgtggcac gtggcggaca 900

ttggcgaaca gtcgtccata ctgacaaaga cttccgaagt gaacaacatc tgtgacccgg 960

cccatttgac caactacgga aaagccatca ttaacaaatt gaaatcgcaa aacaatggtg 1020

tgaaatgcag tggttagcca accaatttca attaatcggc ggatgttatc gaaaatgtat 1080

tcaaaagtta aatattcaaa aaagtttact aacccaatta ttgttattaa tttcggtgtc 1140

aataattgta attgtaattt tgaaaactct taataattat gtactattat tttaattcaa 1200

ttcactagca aattttttaa atctaaaaac tttgaaatgt gaatttaaat catttaaaaa 1260

cgaaaaaaaa aaaaaaaa 1278

<210> 26
<211> 676
<212> DNA
<213> Heterodera glycines

<400> 26
atcctctaag aattcaaatc ctctcagaaa atggcttcat ctttctgctc ttcactcatt 60

tccatcgtcg caattgtctg tttgctgtgc aaatgctgct tttcagcacc ccatccatgc 120

tgtcctggca gccaacatgt tgtttcgatg atgaaagatc acaccggcac attctccgct 180

tcgatgccaa agtcttcgct ttgtctgagt gccgaaagag tcgccgctgc ggtggaaaac 240

caactgaaaa caatttggtg ccctggcaat ggtggccaaa cactcatcaa cgagatcaac 300

gcagctcaat catcatctga tgagtgtgct cgctctctcg gcttcgtccg tgccatgttc 360

gaaattgccg cttccgccgc ttcccatgtc ggtgccaacg ccgaattggc caatttggct 420

gtccagttcc gagaacaagt tggcacaatt gacaccaact gtgctgcgct gggcattcat 480

gttgggcaaa tcagcttggg cactcccaaa ggagaccatc cgcaagtgca tgactctgag 540

agtgtgctta gtaaccctgg caccagcggg tctcacaagc gcatttaagt gcattgcgac 600

gattccgatg atgtcatcat ttgttgattg atatgctata gaaattattt tattagataa 660

aaaatgaatc gttgct 676

<210> 27
<211> 978
<212> DNA
<213> Heterodera glycines

<400> 27
gattaaaaaa gaatttcttc aactaaaatc acaaacacat acaataagaa aatgattctt 60

ttcattctat tggtcataac ttttgtccaa attggtcaac tgagtgctgg catatgcaaa 120

tttccaaatc cgtcaaaaag tgttacggtc caatcgatga tgactgtttc atcgaatacc 180

gactataaga acactttgtt tgtcggcggt tccggcattt tgaacggcgc gtgtgatgtg 240

aacaatggca aactgaaata tttgatgaca ttgaagaatg gcgtgaccat caaaaatgcc 300

attctcgaca cgcccggttt gggcatttac tgcgagggca attgtgtgct ggaaaatatt 360

tactacaagc gattgtgtta ccatgctact gggttcgggt ataagagcac cagcacttca 420

tacacttacc aagtgattgg cggtgctggc caaggttcac cggacaaata tttcacccaa 480

tcgggcaaag gcacaaccat catcaagaat ttctgcgcag aaggcaaata cggcaaattg 540

tggtgttcat gcgggaattg tgctttccaa acggcgcgta ccgtacaaat ttcaaacacc 600

gtccttaagg ggcccggact ttcggttgtc tcgcttaatt ccaactacgg tgacaaaatg 660

tcactttcgg gactgacttt gcatggccaa aaatcatcga gcacaaaaac taattacatt 720

tgccaagagt acaaaggcct cactcacatg gccgccatga gtcctcaagc gaattatgag 780

ccaaccaaat cgggcagtgg cacatgcgcg tacagtgcct cggcagttaa aattgcgagc 840

taatttggag aaataaatga ataaataatg gagggttgag tgatgaaagt aaatttagaa 900

attaaatgat gttaatacaa cctgttaatt gtcggacgaa tataaataaa gattaaaaaa 960

aaaaaaaaaa aaaaaaaa 978

<210> 28
<211> 632
<212> DNA
<213> Heterodera glycines

<400> 28
aatggcattt ctcctgttgt cacttgcacc cctcttcgtt gcccttagcg ggattgatgc 60

aaataaagtg ccactgcaat tgtgctgtgc tggtggccaa agcttggctg gccttatcta 120

cgactattct gaccatttca gcgaagtttt gcaacagtcg ggaactgaga tgtgcactag 180

cctgaaaaag gacatgacca aattgacaac tgcaattgaa aaagctgaag gatgcaagtg 240

caatcttgtt gaaaaaattg agaaggaatt ggatgatgtt gaggatgatt gcgcctacaa 300

tttgtcactt cattcgcgca ctgttccaca ttgtggccat ggcctcggaa catttgggcg 360

aaagttggaa ctccgaagcg gccgagttca acgagcaaat tgaagccatt gatagcattg 420

gacatgatga acttaagaag cttgagtttg aagcggaatc ggaattgaaa acacatcctc 480

gtgttggccg aaaaagaatg aacagatgct aattgggctg ggcaaaattt gaagatgtca 540

atgtgtattt agacgaatta atttgtcgat ctttcattag tttaatgaca cattatggca 600

ataattaata gaagaataaa tatatttttt tg 632

<210> 29
<211> 679
<212> DNA
<213> Heterodera glycines

<400> 29
tattttatta aaaatgcatt taaaaatatt tttattaatt ttatttgtga caatttgctc 60

ggtttggtca ctttcaccca aaatccacca caactaccac caacagcagc aacagcacaa 120

cattaagaat aaattgccac caaaaaatat tgggacaaaa catttgaaaa cggaaattaa 180

tgggcacaac aaacacaagc acaacccggc gagcagacac gggcatgctt tgcttcaaaa 240

agtgaaagac caattgaata atgatggatg ggccagtgaa gatgcaaaca aaacatcggt 300

attgctctct tcactgttgg ccatcgaatg ccatatgaaa aaattggaca agaaaaggac 360

caacgactcg gccgagatca aacatttgac ggagcaaatc tgtgccgccg agaacaaaga 420

acaaattggc attcataaac tgataaatga agttgcgaaa acatttgaaa aagatggaaa 480

gaaaatggta gaacaaatga aaagggacga gcatacggta aaggtgcatc ttgacgggca 540

ctacggtacg ttggccgaga ttttgccaag cgccaaaaag cacgcgaagg caacaaggct 600

tcgaacgtat catgcagtgc tccatttgtt gcatttgatc actccgaaga tggaggaggg 660

accggccaaa gaagcgatg 679

<210> 30
<211> 848
<212> DNA
<213> Heterodera glycines

<400> 30
atggggtttt taaaaactat cgctttcctt tcgcttttgg cacttttttg cgcaacaatt 60

gctgaagaaa atggggcgac agaaggcaca gaagcggagg cgacgacagt gccaacactc 120

accgcagcgg cggagggagg caaagggggc gaagaggaag tgaccacggc gaagccgacg 180

gagacgagcg aaacggcacg ggaggagggg gaggagaagc acaaggcgaa cgaggggaaa 240

gcgactgagg gaagtggaac cagatcgttg tggcaaaaag tcacctgtat gttgttcggc 300

tttttcccat ttcgcgccgg ctgtccgggc aaaaacgata taaaagcggg gtcatcatca 360

catgtgagca gcacaattat gataattgag gaaaaagagg acagcgaatc ggacgaacaa 420

atggacggcg ccggagggga cgaggagaaa cggggaggag agaacggcag cgaagcgcag 480

ggcggcaaag gcgccgcaag ggacaacgaa atggaagacg aagaattgaa agtgaaagcc 540

gtggtggaag agacgacgac aaatgaacag ggcaaaactg agacaaacgt cgaacagagc 600

gaaaaggagg ggccgatgaa ctgaccgaaa gggaccgact gatcggcagg aacgtcatcc 660

ctgacgggga tttgtcacgg agaatagaaa caaaaaaagc cacttgcatt tgcgttgttt 720

tattttgttg ctacagtaat cacatagttc acctttgtag atgctttact gtactctttt 780

catttttctc tctttctgtc cctctctttc ttttctttcc gcttttggct tataaattcc 840

tttttctc 848

<210> 31
<211> 805
<212> DNA
<213> Heterodera glycines

<400> 31
tgatccgcta attattattt tgaattgaga tttattaaaa cctaattaat tttttttcta 60

ctcgaatttc ttttatggca tttgccattt caagtttaag cgctttaatt tcgctcgctt 120

tattggcagc accacaaaac tcgtaccaac agaaacctaa cgatgaaccg aaggtcttta 180

atgatatcca aaacgggtgt gatttgataa attgtaccga tggtcgaacc tgcggcattc 240

gagttgggct cgcaaaattc ggcaacagaa attttgagaa atttgctttt ccaaaatgta 300

taacaaatga aacggaacta aacatgaaca cggacaacga tggaaatgcg cacattgttc 360

ctaatggacc agggtgccaa atgatacaat gttctgttgg ctaccaatgt caagttcgaa 420

tttcaatttc caagcttggc aatttgccat atgctcagtc aattggaacc ttcccgcaat 480

gcgtggggcc taatgtaagc ttcaactcat caagttcaac tatcgagaat ggcccagggt 540

gtgaccaact tccgacgaaa tgtgacgaag gcacaaaatg cgtaactgcc gtcggaattg 600

cgaaatatgg caatttgcca tggtcgcaat tttctggccc cttttgtaca tgaaacgatg 660

aaaagcgctg caactgtttt atattcagag gagccaaaaa acgacggaaa atttattttt 720

acctaattta acaactcttg tgagtaaatt tgtgatttaa aatgtattgt gatttatgat 780

tttttggtaa actaaaattc atttc 805

<210> 32

<211>  868
<212>  DNA
<213>  Heterodera glycines

<400>  32
   aaaatgcgca ccattctctt atagccatgg tttgcttggt gatggctgtc ctaatggaaa      60
   tggcaaattc aaaggctgtc aaaaaataca acaaaaaagg agcagtggcg gcatcaccag     120
   caaaaggaaa agcatcgcca aaaggaggca aaagcccagc aaaaggaaaa gcggcaaaaa     180
   aattgaaacc taaaaaggat gctaaaggca ttaaagctaa aaaagcaaag ccagcaaaga     240
   aaagcaaagc ggcaaaagca gtaaagggag cgcctaagac agtcaaaaaa ctcgcatttg     300
   ccaaaacagc agcgcaagtg aaagaaataa agtcaccagc caagccaatg gttctaaaag     360
   cagtgccgcc ccaccaaatg catttaatga atgagaaagt tcaaactgct gtgtctccac     420
   ccgcccatgc tcgttcgctt gcgaccgttc cttacagtac accggtggca gccgaccgta     480
   actcactgcc atcgtacact tcggatggca ccaatttgga catggcccat gagaatgatg     540
   attatcagaa ttattactac ggatcgggag acagcagcaa agaatttgat gcatcggctg     600
   aagaggaaga tggactgtat gaggaggggg acggggcaat gaattgggca aacggacgac     660
   taccggattg ggcattgaac aggaatacgg gcatcaatcg gggggagtgg ccaccaaagt     720
   cggggatgct ccaactgaaa aacaccaaga attttggaaa aaacttattt tccaaaattt     780
   tgaaacattt ggattttaat attcccgtta aaaaactaaa ttttttttta cccagcctct     840
   aattttgtaa aaaaaaaaaa aaaaaaaa                                         868


<210>  33
<211>  455
<212>  DNA
<213>  Heterodera glycines

<400>  33
tcagtgccca acgcaacgca atggccactt acctattcct cgctgcagct gccttcggag      60

cgggaattta ctgttggaag acgtaccttg gcccgatgct tgaccaaccg aagagcgacg     120

acgaggagaa gcccaagccg gaaggaggcg aagaagcgac gaccgccggc gaggaggaga     180

agaaggctgg cagcaagggc gacaagcaca agaagaagga caagaagggc gaaggcgcag     240

cagcagcacc agcggcggac aaaaaggagg agaagcgcaa ggacaaacac aaatccaagg     300

agtcggggaa gagcgacgac agcaaagcgg gcaagaagga caagcacggg aagaaggaca     360

agaagggcaa gaagaaggac accgccgatg catccaccgt ggacgaagcg ctgggagaac     420

agagcacagc ggagacgagc caagcagagg aatag                                455


<210>  34
<211>  549

<212>  DNA
<213>  Heterodera glycines

<400>  34
atggcttcat ctttccgctc ctcaatcatt tccatcgtcg caattgtctg tttgctgtgc      60

aaatgctgct tttcggcacc ccatccatgc tgtcctggca gccaacatgt cgtttcgatg     120

atgaaagatc acaccggcac attctccgct tcgatgccaa agtcttcgct ttgtatgagt     180

gccgaaagag tcgccgctgc ggtggaaaac caactgaaaa caatttggtg ccctggcaat     240

ggtagtcaaa cactcatcaa cgagatcaac gcaactcaat catcatcaga tgagtgtgct     300

cgcagtctcg gcttcgtccg tgccatgttc gaaattgccg cttccgccgc ttcccatgcc     360

ggtgccaacg ccgatttggc tgtccagttc cgagaacaag ttggcacaat tgacagcaat     420

tgtgccgcgt tgggcattca tgttgggcaa atcagcttgg atgcccccaa gggagaccat     480

ccgcaagtgc atgactctga gagtgtgctt agtaaccctg gcaccagcgg gtctcacaag     540

cgcttttaa                                                             549

<210>  35
<211>  657
<212>  DNA
<213>  Heterodera glycines

<400>  35
tctctctact aactgttgca catcccaatt ttactttctt ccttagtaag tagggcttgg      60

tgcacaatgc gcacttttct gttcatagcc gtagtcgggt tgatgttggc cgtcatcttg     120

gagaatgtaa atgcgacagg gaaaaattcg ccgacaaagg aacttataca tgagcaaggt     180

aacctacaga accatgctac cggacaatcg ccgccagaca gtccaaacca taaaaatgac     240

catgaaaacg agattagtaa cctacagaac cgtgctaccg gacaatcgcc gccagacagt     300

acaaaacggg aaacagaaca taaaattgag aaaggaaaac cgtcgccaga cagtccaaag     360

tatgatgagc aagccttaaa atggtgaaga aactgtgtgg aacgatgatg ccaaccatga     420

acaaacccaa agctgttgta gtagatgtag aaaaaaactg tggtatcatc acgctattat     480

tgttgtgtaa aaaacgatcc gtttttacct cttttcggga aaaaaaactg gaaaaaattc     540

acaaaaaaaa aaatctcaca ttgtgcagat tttattgaaa ttagccaatt aaaaaaaaca     600

gtgacgaaaa aaaaaaaaaa aaaaaaaaaa aaaaaaaaaa aaaaaaaaaa aaaaaaa        657

<210>  36
<211>  441
<212>  DNA
<213>  Heterodera glycines

<400>  36
atgtcttctt ccccgtccgt ctctgtgctc gccgtcgtcg caattgtctg tttgatgtgc      60

caatgttgct tttcggcacc gcatccgtgc tgtcccggca gtcaaaaagt ggtttcactt 120

atgtccaatt acgttggcac ttttgccaat tccatttcca agtcatcgct ttgttcggat 180

gcccaaaatg ttgcggacgc gttgaaaggc caactgatcg gctgctccgc ttcttcccat 240

gccagcaaca acagcgaatg gcagacattg agtgggcagt ttggtcagaa agtcactgag 300

attgactcga aatgtgcaga gtttggcatt agcattggca aagtgcccat aaacggtccc 360

aagggtgtcc atgttcaaaa tgtgcccaac tcggaaagtg tgatttctat gcctggattg 420

gccggctcac acacccaatg a 441

<210> 37
<211> 774
<212> DNA
<213> Heterodera glycines

<400> 37
aattattttt tttaataata attttttaca aaatgagcaa ctttatattt gtcgcctttt 60

taactgcagc gttttttagc tcaagcctcg ctctaccggc tccttatgat gctgaatcgg 120

tggtatcttc tgaattaaat gttccactac tttcagctga cgcaaatgtt caagcagctg 180

aaaatgttga gatacaaatt ccatcaattc cagcaccaat tgaacaacaa actgctgctc 240

acattactca tccaactgaa actggcaatg agccttctat tgcatcatca tcatccgcgc 300

cgaaaagtga gcagacgcca aaaaaagtga tgaacatgaa aagcgcgatg gaaggcgcgg 360

cggccaacgt gtacgggggg cttccgttag accagcagcc caagacgatc gctgaagcgg 420

cagcaaaagc aaagcagacg cccgccaaac ttccggctga ctacgacgcg aaccgcgtgg 480

cggaacgcgc ggcggcccgt gtgtacgggt ggcttccgga agacaagcag cctaaggcga 540

tctatgacgc ggcggagaag gcaaagaaca cgcccaaacc gccgggcgac tacgacgtgg 600

agcgcgtggc gcaaaaggcg gcacggctcg tctacggtgt gctgcccatc ggcatgcagc 660

ccagcttcgc cggccctagc actgacaaga gcaatgtcga cgactcggag aaaccttctg 720

ctgctgcggc tggtgatgat gatgaagtcg aaaaaaaaaa aaaaaaaaaa aaaa 774

<210> 38
<211> 1451
<212> DNA
<213> Heterodera glycines

<400> 38
aattcgcttt catcgttcaa atgtgccgac tccaagcaac tcatctgctc gctcgactct 60

ttctgcttct tgcgctttgc actgctctcg ttagctctct cactgctgtt gccccgccat 120

tcggccaatt gtccgtttcc ggcaccaatt tggtcggcgc caacggacaa cccgtacagc 180

tgatcggcaa ctcactgttc tggcaccaat ggtacccgca gttttggaac gctgacacag 240

tgaaggcact caaatgcaat tggaatgcca atgtcatccg gggggccatg ggcgtggacg 300

agggcggcta tctgagtgac gcgaacacgg cttacaatct gatggtggca gtgatcgaag 360

cggccatttc caatggcatt tacgtgatcg tcgattggca tgcccacaat tcacatccgg 420

acgaagcggt caaattcttc acccgaattg ctcaagcgta cggctcctac cctcacattt 480

tgtacgagga tttcaacgag ccgctgagcg tttcgtggac cgatgtgctg gtgccatacc 540

acaaaaaggt cattgctgcc atccgagcta ttgacaagaa aaatgtgatc attctcggca 600

ctccaacatg gtcccaagac gtggatgtgg catcacagaa cccaatcaaa gactaccaaa 660

atctgatgta cactctccac ttttacgcgt ccagtcactt cacgaatgat cttggtgcca 720

agctcaaaac agccgtgaac aacggtttgc ctgtgttcgt cactgagtac ggcacatgcg 780

aagcgtcggg caacggcaac ttgaacactg actcgatgtc cagctggtgg actctgctgg 840

acagcttgaa gatttcatac gccaactggg caatctccga caaaagtgag gcctgctcag 900

cactgagccc cggtacaact gctgccaatg tcggtgtttc gtcccgttgg acatcctccg 960

gaaatatggt tgcttcgtac tacaagaaaa aatccaccgg cgtaagctgt agcggctcaa 1020

gttccggcag ctcttcgggc tcgagttctg gctcttccgg ttcaagttcc ggcagctctt 1080

ccggctcaag ttccggcagc tcttccggct caagttcggg ctcctctggt tcgagctcag 1140

ggtccagctc gggttcggga tccgccagca tctctgtagt cccatccaac acgtggaatg 1200

gcggtggtca ggtcaacttc gaaatcaaaa acatcgggtc cgtgccattg tgtggcgttg 1260

tgttcagcgt ctctcttccc tcagggacca cgcttggtgg atcgtggaac atggaatccg 1320

caggctccgg ccaatacagc ttgccaagtt gggtcagaat tgaggccgga aaatcgagca 1380

aagacgcggg gctgacattc aacggaaaag ataagccaac ggcgaaaatt gtgacgacga 1440

agaaatgtta g 1451

<210> 39
<211> 1149
<212> DNA
<213> Heterodera glycines

<400> 39
gaatttcatt atttcactga tttgcccatt acttttctta atgaactcct catttgtcat 60

ctgctgctgc ttccacaact ttgtgttgtt ccttcttttg agttgtgttg tgccacatgc 120

ttttgcttgt tgtcctcatg gaaatctcag agtgaaaggt acgcatttgg ccgatgaaaa 180

gggcgaaact gtgcagctgc gagggatgtc tttgtactgg agccaatggg agtacggctc 240

aaagttcttc aacgagaaga cggtcaactg cctgaagtgc agttggcatg ccgacattgt 300

ccgtgctccg ttagctgtgg accagggcgg ctatttgtca aatccagaac aggagtatgc 360

gaaggtgaag agcgtagtcc aagcagcaat cgacaaatgc atatatgtgc tgattgattg 420

gcattacacg agcagtgaga aatatacgga caaagcgaag gagttcttcg gcagaattag 480

cactctgtgc gctggcaaat gcaactgttt gtacgaaaca tggaacgaac ccatacaaaa 540

cgattggtcc tcgcagttga agccgtacca cgaggagctg gttaaagtga ttcggcagaa 600

tgacaagaac ggagtgataa tcgctggaac gccaaattat gaccaggatg tgaaggcggt 660

agtcaatgac ccaatcaaag agcacaatat aatgtacaca ttgcatttgt acgcggcatc 720

gcataagcaa gaactgcgca acacggcaca acaggcaatc ggcgcgggag tgccgttgtt 780

tgtcacggag tatggcacaa gttcgtacac aggcgatggc gggccagacc ttgtcgaaac 840

acagaattgg tacgactttt tcaacaaaaa ctccctctcg tacatcaact gggccatcga 900

cgacaagcag gaagcgtcgg tagcgctgca gaacacacag cccccagtcg ggccggccga 960

cgtgtgcagc accgatcggc tgaccaaatc gggcaaattt gtgcacgacc acctcatcgg 1020

tgtaaaccag aagccggatg gctgttaaac atcagaaaat ggcaaatgat cggaatcgcg 1080

cgctcttttt tttacttttc tgggtcattt catctttagc attaaaccta ataaaaaaaa 1140

aaaaaaaaa 1149

<210> 40
<211> 880
<212> DNA
<213> Heterodera glycines

<400> 40
cgcccattct cttatagcca tggtttgctt ggtgatggct gtcctaatgg aaatggcaaa 60

ttcaaaggct gtcaaaaaag acaacaaaaa aggagcagtg gcggcatcac cagcaaaagg 120

aaaatcatcg ccaaaaggag gcaaaagccc agcaaaagga aaagcggcaa aaaaattgaa 180

acctaaaaag gatgctaaag gcattaaagc taaaaaagca aagccagcaa agaaaagcaa 240

agcggcaaaa gcagtaaagg gagcgcctaa gacagtcaaa aaactcgcaa ttgccaaaac 300

agcagcgcaa gtgaaagaaa taaagtcacc agccaagcca atggttctaa aagcagtgcc 360

gccccaccaa atgcatttaa tgaatgagaa agttcaaact gctgtgtctc cacccgccca 420

tgctcgttca cttgcgaccg ttccttacag tacaccgatg gcagccgacc gtaactcact 480

gccatcgtac acttcggatg gcaccaattt ggacatggcc catgagaatg atgattatca 540

gaattattac tacggaacgg aagacagcag cagagaattt gatgcatcgg cggaggagga 600

ggatgaactg tatgaggagg gggacggggc aatgagttgg cagacggacg actacggtat 660

tggcattgga caggagtacg gtcatcaatc ggtggaagtg ccaccaaagt cggtgatgct 720

cgactgaaga acaccagcag gatttttgta aaaacatatt tctcagattt ttgaacattt 780

gcattttata ttcgcttaga agctaatttt ttttatctag ctctaattat gtaaaaatta 840

ataattcttc gaaaaaaaaa aaaaaaaaaa aaaaaaaaaa 880

<210> 41
<211> 1523
<212> DNA
<213> Heterodera glycines

<400> 41
catgaacatt ctgctgaagt ctgttctatt catctctctt ctgtcactgt ttggtgattg 60

catgaaaaac tcagatcaaa ttggagtcaa aagaaaatca gcaggaataa gaattgagga 120

accaaatgat gtaaaaggca aaggaaaagt cgtcgaaaca agcagaaaag gcaaagaaaa 180

agtcggcgaa aaaataagcg ttatgaacac agctgaacgc attgaaaaaa tggaccttgc 240

ccaagacaaa gctaaaatgg atatagacac caatggcaaa cccaaacaaa atgaggaaaa 300

tgctcctcca aaatatgttt tcatttggag cggtaacaat gagaagaaga accttgaggt 360

tgacctcaga gtgatccgtc actccaaaaa actcagcgaa ttattccatg ccaaaagtga 420

tcaatccgtc aatgcaaaac acggaaagca ggaagcatta ccggttccga aacaggaagc 480

tgaaaaaatg ctgaaggaag tggaagaatg gtacgcacat cacaaaaatg atgaacataa 540

aaaagctcca gtttttgtgt tcaagtgtaa agacaatgtc agtctggtga taaatgttca 600

tcttgatgca gtccggcttt ccaaggagtt gtcaaatatg cttgaagata ctgccgacga 660

aggatatccg aagccaatcc cgagtccagt tcagaatttc aaaagtgaca tcatgctaaa 720

actgattaaa ttgtgtgaag aatacaagta caaattcgat ggcctaaaga aaacaactga 780

tgtgccaact ggagaagtcg cggaggcatc ggcttcaaat gtccaagagg gtgacgttgg 840

ggaggcatcg gcttcaaagg tcaaggctgg aacaattgcg gaggaaatcc aaaaattcgt 900

tcctccgcca cacattatgc aatgggacgt gaaaacggac cttgagatgg ttcgggccgc 960

cgacttttat aacatcaatc gtttgatcaa cgatgattac attgacaatg tgtacgaaat 1020

cgtaaaagtc aaatggatca atggcaaaac gccgcaggaa attcgcaagg gattcggcgt 1080

cgaagagccg tacccgccgg gacatccgga atgggcacga gttgagaagg agaacgagtg 1140

ggaagaatcg gacgaggaac gtgaggcacg ccatgcaaag gaacgagagg aggaagagga 1200

gcgtgagaga aaggaagaac agaagcgtaa ggaagaggaa gcggaacgcc tccatcagga 1260

acaactgcag caacaacaga atcaggaaca gcaacctcag tagggacagc agcatggtga 1320

agaactggaa cacgatgaag ttatgcatga tgtggaggaa gagcaagatg atgaatggcg 1380

aggaagaaga tgatgagtga tgaggatgag gaggatgtaa aatgatgtgg tgattagtga 1440

ttttgatgaa ccgatgattc acttttcttg gatcctgttg cataaaactt gttgcaaaaa 1500

aaaaaaaaaa aaaaaaaaaa aaa 1523

<210> 42
<211> 711
<212> DNA
<213> Heterodera glycines

<400> 42
atgtcttctc tgctgctctc catcttccca attgtgtttt tggtctgttg caatgcaatg 60

ccaaatttcc cgtgctgccc gggcagtcag caagtggttg ctgtgatgtc catttacatt 120

gacactttct cttctgctgc tgacgagtct acagtatgct ttttcgctaa aagtactgtg 180

gatggaataa aaaatgaact gtcctctcgc ggcggatgcc aaaacggagg agaagcacaa 240

attgtggatg aaatcgatcg acagctgaag aatattgcaa aaatggagat caattatgag 300

gacgagtgcc cgtacaattt gggctttgcc cgtgccatgt tcgacttggc cgctgctgct 360

gctggccatg cgggcaacgg cacagaatgg caatacatga aagtacaatt tgagcaggaa 420

agccaagcaa tcaaagcaat tggacaagaa aagaactttg aagttacgga tgtgcatttt 480

ggagtcccaa gcaaaggggt ttctgcacat caaaatgtgc cgagtccgag ccatgtgatt 540

gccaatcctg gccaacacag ttcggttggg ccaaggaaag aaagaagaac cgttgtcatc 600

ggacttcgat ttttgaggac aaacaaatca ggaggaaata gaatagaaaa ccattttgtt 660

gacatgtcga acatttattt aaagtaataa atatttggta ataaggaaaa a 711

<210> 43
<211> 492
<212> DNA
<213> Heterodera glycines

<400> 43
atgaggaaac ttccattctt gctcttattt tctgcttgtt gctacttgca acggaaggaa 60

gtatatttcg ccgaaaaaac agaaagaaaa attcatcgag cagcgaacaa aaaacacaaa 120

gaagaaaccg agggtatggc cgaagtcgca ctttttcaaa tggcaatggc atgtatggcc 180

aatcgaatgg attttcaaat ggccgatttg gcggctccag tgtctattca aatagcggcc 240

ggtcgaatag ctttccaaat ggtggatttg gcggctccag tggcttttca tcgatgggac 300

ataactcggg cggacttcac ggctttgggt ccggcccacc ttctggaggc tacggatacg 360

gaagcggttt tggtggaaga aaatgaaaaa aagaccgaaa gtgatcggcg agagaatgat 420

ttgtgctgtt gccttttatg catgcaaaat ttaaatggaa aaacgcattt caccagcaga 480

acactttttt aa 492

<210>  44
<211>  669
<212>  DNA
<213>  Heterodera glycines

<400>  44
acaaccgctt cagtgcgcaa cgcaacgcaa tggccactta cctattcatc gccgctgctg      60

ccttcggagc aggaatttac tgttggaagg cgtacctcgg cccgatgctc gaccaaccga     120

agagcgacga cgaggagaag aaggctggca gcaagggcga caagcacaag aagaaggaca     180

agaagggcga aggcgcagca gcagcaccag cggcggacaa aaaggaggag aagcgcaagg     240

acaaacaccg atcgaaggag gcggggaaga gcgacgacag caaagcgggc aagaaggaca     300

agcacgggaa gaaggacaag aagggcaaga agaaggacac cgccgatgca tccaccgtgg     360

acgaagcgct gggagaacag agcacagcgg agacgagcca agcagaggaa tagccaggcc     420

ctgctgcgtg caaatgacag tgttcgacga cgacgtctcg cgacaccaac acacccgtcc     480

cttgctttcg accattccct tcccatccga ttgctgtgtt ttgtctgttc caatgcttcc     540

taaatgttcc ccttgtgaac agtgatcctt cctcatgtga cctgaatatg tatatattgt     600

atacccatta atatattgtg cttcgcttgt tcgatatttg tgaaatctca ttcatattca     660

tcgtcattg                                                             669

<210>  45
<211>  700
<212>  DNA
<213>  Heterodera glycines

<400>  45
atcctctaag aattcacatc ctctcagaaa atggcttcat ctttctgctc ctcaatcatt      60

tccatcgtcg caattgtctg tttgctgtgc aaatgctgct tttcggcacc ccatccatgc     120

tgtcctggca gccaacatgt tgtttcgatg atgaaagatc acaccggcac attctccgct     180

tcgatgccaa agtcttcgct ttgtctgagt gccgaaagag tcgccgctgc ggtggaaaac     240

caactgaaaa caatttggtg ccctggcaat ggtggtcaaa cactcatcaa cgagatcaac     300

gcagctcaat catcatctga tgagtgtgct cgctctctcg gcttcatccg tgccatgttc     360

gaaattgccg cttccgccgc ttcccatgcc ggtgccaacg ccgaattggc caatttggct     420

gtccagttcc gagaacaagt tggcacaatt gacaccaact gtgctgcgct gggcattcat     480

gttgggcaaa tcagcttggg cactcccaaa ggagaccatc cgcaagtgca tgactctgag     540

agtgtgctta gtaaccctgg caccagcggg tctcacaagc gcatttaagt gcattgcgac     600

gattccgatg atgtcatcat ttgttgattg atatgctata gaaattattt tattagataa     660

aaaatgaatc attacaaaaa aaaaaaaaaa aaaaaaaaaa                           700

<210>  46
<211>  900
<212>  DNA
<213>  Heterodera glycines


<220>
<221>  misc_feature
<222>  (433)..(433)
<223>  n is a, c, g, or t

<400>  46
taataaataa gtttttttgt tcaaaatgag caactttata tttgtttcct ttttaactgc      60

agcgtttttt agctcaggcc tcgctctacc ggctccttat gatgctgaat cggtggtatc     120

ttttgtttta aatgttccac tactttcagc tgacgcaaat gttgaagcat ctcctcccaa     180

tgaaagtgat gctgtttttg agatacaagc tccatcaatt ccggtaccaa ttgaacatca     240

aactgctgct gacattactc atccaactga aactggcaat gagtcttcta ttgcatcatc     300

atcatccacg ccgaaaagtg agcagacgcc aaaaaaagtg atgaacatga aaagcgcgct     360

ggaaggcgcg gcggccaacg tgtacggggg gcttccgtta gacaagcagc ccaagacgat     420

cgctgaagcg gcngcaaaag caaagcagac gcccgccaaa cttccggctg actacgacgt     480

gaaccgcgtg gcggaacgcg cagcagcccg tgtgtacggg tggcttccgg aagacaagca     540

gcctaaggcg atctatgacg cggcggagaa tgcaaagaac acgcccaaac cgccgggcga     600

ctacgacgtg gagcgcgtgg cgcaaaaggc ggcacggctc gtctacggtg tgctgcccat     660

cggcatgcag cccaacttcg ccggccctag cactgacaag agcaatgtcg acgactcgga     720

gaaaccttct gctgctgcgg ctggtgatga tgatgatgaa gtcgaaaaag agaagaagga     780

ataagcaaaa aacaatgtga atttattata ggaaaaaaag aaaaatggaa gcttagaaat     840

ttaatatttt catttttgaa catttataaa atttcaaaat taaaaaaaaa aaaaaaaaaa     900


<210>  47
<211>  730
<212>  DNA
<213>  Heterodera glycines

<400>  47
ttaaaatgcg cgccgttctc ttcctggcca tggtttgctt ggtgatggct gttattcttg      60

agacagccaa ctcaaaggca gtgaaaaaag acaataagaa aggagcaatt acaacgccag     120

caaaaggaaa agcggcgccg aaaggagcag cgaaaggagg aattaaaaaa gacgcaaaat     180

caaaggaaaa aggaaaaaag gacgccaaag gcaaaaagga caaaaaagct aaaccagcca     240

aagggaaagc atcgcctaaa aatgacaaaa aacccccagc tgccaaagca aatgacaaaa     300

agagcccaac caaaccaatg gcagcagtga aagccgtgcc aaaggcggcg gagaatgctc     360

agaagggacc gtcgagtgcg gaagtggtgg cggaggaaag caacttggac actgaggcgg 420

tcgacgactt gggggttgga cctgaggcgg tcgacgactt gggggcggaa gagtcggact 480

ttgaccaatt ggcagaggac gaactgctcg aggacgacgc catgggcggc gaggcggacg 540

aatgggaagg catgggggag gagacggaga tggacagcca aatggcactt tgatggccaa 600

ctaattggac aaattacaaa cggaacaaaa cgggacaaat gacacaaaat attatttatc 660

atttttggca tatcattgaa aataaataga atttattttg caaaaaaaaa aaaaaaaaaa 720

aaaaaaaaaa 730

<210> 48
<211> 270
<212> DNA
<213> Heterodera glycines

<400> 48
atgaatttga tttttgaaat attcgccttt ggaatggaca atttctcatc ggaaaaaaat 60

gtttctcttc ttcgtctttt cttattcttt ttctcgaccg tagtattatt tggaagcgca 120

caccctatgg taaacaaatt atgcgaagac ctagataaac ctgaaggttg gcaattattg 180

aaaaagttaa caatcgaaaa gtgccggatg agtataatgt ggaattcccc aaatggaaag 240

acagctacaa aagcattaaa aagtttataa 270

<210> 49
<211> 684
<212> DNA
<213> Heterodera glycines

<400> 49
atgcgcactt ttctgttcat agctgtagtc gggttggtgt tggctgtcat catggagagt 60

gtaaatgcga ctgggaataa ttcgccgaca aaggggcaaa ctccgcctgg cagtccgaaa 120

ggtgggcacg gcagcaacaa atcgccaacg acgccgcaca gtgccggttc gccacacggc 180

acgcagcatg gtaaccaaca gaaccatgct gccggacaaa cgccgcctgg cagtccgaaa 240

ggtgggcacg gcagcaacaa atcgccaacg acgccgcaca gtgccggttc gccacacggc 300

acgcagcatg gtaaccaaca gaaaaatgct accggacaaa cgccgcctgg cagtccgaaa 360

ggtgggcacg gcagcaacaa atcggcaacg acgccgcaca gtgccggttc gccacacggc 420

acgcagcatg gtaaccaaca gaaaaatgct accggacaaa cgccgcctgg cagtccgaaa 480

ggtgggcacg gcaacaacaa atcgccaacg acgccgcaca gtgccggttc gccacacggc 540

acgcagcatg gtaaccaaca gaaccatgct gccgtttcaa atgtggtgca gaacgttgac 600

aacaaacctc ggccatctgc tcctgcttca caccttgtgc caccaacacc agggaacagc 660

cctactgcgc atgacagtcg ctga                                          684

<210>  50
<211>  999
<212>  DNA
<213>  Heterodera glycines

<220>
<221>  misc_feature
<222>  (974)..(974)
<223>  n is a, c, g, or t

<400>  50
ctgcacattc cccaatttta tttcttcctt agtaagtagt attggtgcac aatgcgcact      60

tttctgttca tagccgtagt cgggttgatg ttggccgtca tcttggagaa tgtaaatgcg     120

actgggaaaa attcgccgac aaagggacaa tcgccgccag gcagtccaaa acatgaaaaa     180

gaccgtaaaa atgagcatgg taaccaacag aaccatgcta ccggaaaatc gccgccaggc     240

agtccgagag tacaatcgcc gccaggcagt ccgagaggaa aatcgccgcc aggcagtccg     300

agagtacaat cgccgccagg cagtccgaga ggacaatcgc cgccaggcag tccaaaacat     360

gaaaaagacc ataaaaatga gcatggtaac caacagaacc atgctaccgt tccaaatgtg     420

gtgcagaaag ttgaagtcca caaccaacct acgccatctg ctgctgcttc acaccctgtg     480

ccaccaacac cagggaacag ccctactgcg catgacagtc accacacaag cccaaaagtt     540

gcccatgttg ctgccgccgc cactttgggc catggacgcc aagaatgtgc caatgtgtcg     600

tgtggtgatg atgtaaccga ggaaatggac cgcgacgatg ataagacagt ggaacgcgcg     660

gtcggcgagt caccaatcgg cgtttcggaa tccagcgcca cggatgagga tgattctgct     720

gccgtttcgg cttacaacaa aaacaacagc tcggccgccg catcagcgtc agttgctgat     780

gactgagatg gggtgatggt tgaaggaact gcgtggaacg atgatgccaa ccatgaacaa     840

acacaaagct gttgtagtag atgtagaaaa aaactgtggt atcatcacgc tattattgtt     900

gtgtaaaaaa cgatccgttt ttacctcttt tcgggaaaaa aactggaaaa aattcacaaa     960

aaaaaaatct cacntaaaaa aaaaaaaaaa aaaaaaaaa                            999

<210>  51
<211>  954
<212>  DNA
<213>  Heterodera glycines

<400>  51
ggcaattaat tgtccaatta agcggatgaa ttcgtccgtc gtttcatttt cgctgttact      60

ttttgggtta ttcacaattc gcactgcaaa atcgcaatgc gagaaacatt gcactaaaag     120

caaaccgatg ggccaatgca atgaggcgga agaagtgatt ctgcgcaact ccgactgtgc     180

cttcatgaag aaaacggaaa cggcattcga atttgtggtc ggaatgaatg gccaaacgga 240

ggacaaaacg gcgcccgggg ccaatgccaa tggggcattt ttgtgctgta aggcaaccca 300

aggaaccgca acacttttca tcgtcggcgt ggccaacaaa cggctgatgt tggccaagga 360

cgtggtcaat tacaagtttc atcaaaacat ctcaatcgat gattttgaac gggaaaagca 420

agtgttggaa agtgtttcgg cgcagggaca aaaagccggc attggggaca attatggaga 480

aaaattcttc caggaccaaa tggacgccaa caaaatgatt cagaaaggct ctgtgaagct 540

gtggaccgcc aacaaatcgt tgcctcctca aaatgtgccg gatttacaga aagatacccg 600

gcccaaagtg acggcggcga cagaagaaat gattttggca ctgaaggtgt ttcaacagtt 660

tcgaaagaac aaaaattgtt gggcatctgt ggaaaaggaa ctcttaaagt cgcaaagctt 720

tttgagtttg gccgaaccca acggaaagga cgcaatgcga aaagcggcgg tgcgattgtg 780

cgcaaaaatg gaagcgaaaa ttgaggcaca aattgacgaa acggcaaaga aattgttggc 840

ctgaggagga cgaattgacg agcattttag gaaaaagatc ctttcgttcc gattttgatt 900

ctttgatcag tgaaagtgcg gtattgtcaa aaaaaaaaaa aaaaaaaaaa aaaa 954

<210> 52
<211> 612
<212> DNA
<213> Heterodera glycines

<400> 52
gaaggcgtgt ccaagttgat caacggtatt ccagtggctg agaccgtggt ttacccagcc 60

atcggcgaga ccaagactca tatcacggtg ttcaccgata ccacctgccc gtactgccac 120

aagctgcacg ccgaagtgcc tgcgctgaac aaaatgggga tcgaagtgcg ctacgtcgcg 180

ttcccgcgcc agggcctggg ctcgccgggt gacgaacagc tgcaagccgt atggtgctcg 240

gccgacaaga aagcggccat ggacaaaatg gtcgatggca aggaaatcaa atcggccaaa 300

tgcgccaacc cggtttccaa gcagttcgcc ctgggccagt cgattggtgt gaacggtaca 360

ccggccatcg ttttggctga tggccaggtc attccgggct accagcctgc gccacaagtt 420

gccaaactgg cactgggtgc gaagtaagca gtcatcgtcg agcctttgac gatcatgccc 480

cgtcgaacag tcgacggggc attaattgag agccgcagtt gtgcggctgt ttaccacggc 540

cgaccttgag tcggccgttt catggggagt tcttcagtga aaccggtcaa agtaggcatc 600

tgtgggttag gg 612

<210> 53
<211> 356
<212> DNA
<213> Heterodera glycines

<400>  53
agaagaattt ctcaaccatc ataggaccga atagatgaat agtgttttat cgatctccat      60

tttattcctg ctcagccagg ttctttccac tgtctcctct agcgatgtgt tggaatacac     120

ggatgctagc tttgactcgg gaatgcagca gcacgacatc gcattggcag aattctatgc     180

cccctggtgt ggacattgca aaaaactcgc tccggaatac gaaaaagcgg ccactaaact     240

gaagaacaac gacccaccaa ttccactcat caaagtcgat tgtactgcgg aaaaagagac     300

ttgcgataaa tttggagtta gcggttttcc aacattgaaa atcttcagga aggggc         356


<210>  54
<211>  261
<212>  DNA
<213>  Heterodera glycines

<400>  54
gcaataaaga gtaaattatg gataggagat tcactgtctt ccttgtgatc gcattggtta      60

cttctattta tgaagtgctt agtaatggaa atttgaacga tggcgacgac tcatttaaac     120

aattcgatga actcgaagaa aacccagcgc ataaatattc aaaagaggcc cagaaggggt     180

tcgaaatgga ggaggaggaa gtcacaatcc gagaaccttc ggggacgaag gaatcgttca     240

aattgcccat caatatgccg t                                               261


<210>  55
<211>  602
<212>  DNA
<213>  Heterodera glycines


<220>
<221>  misc_feature
<222>  (4)..(4)
<223>  n is a, c, g, or t

<220>
<221>  misc_feature
<222>  (11)..(11)
<223>  n is a, c, g, or t

<400>  55
cgtntcagtt ngagtttcat tgaaaactca aaaaatgaca acagtcaaat tgctatgcct      60

aattgcccta tttgcggtag tgcaacttcg ttttgcattt gctatgatga atcaaaacaa     120

ccaacaagtg agccaaaaca acagctctga cgaaggagaa gatgatgatg atgctggtaa     180

cggcgggcaa tatgacacgg ttgctccatt gcgtagcgac agcactagtt tcgttagcag     240

cggcacctta ccgccgcagg gcagcaccgg tttcagcggg gtcggcatga tgccgccgca     300

tgcccaaaac atgggagcaa tgggcatggg gatgggcaat accagtttcg gcaatttgca     360

acaacccact atgcaaatgc aatatcaaaa ccagcaaatg atgcaaaccc cgcaaatgat 420

gcaaaaccag caaatgccgt ttaaccagag tggcagtttc agtggggttg gcatgatgcc 480

tatgcagcac caaaacatgg gagcaatggg catgatgaat gttggcagta ccagttttgg 540

caatttgcaa caatccaata tgcaaatgca acatcaaaac cagcaaatga tgcaaaaccc 600

gc 602

<210> 56
<211> 320
<212> DNA
<213> Heterodera glycines

<400> 56
acaaaaatta attggccgtg acgaaaacaa tatcatcaac cacagaaaag tgaactgctc 60

cagcgctgca atgttgatgc tgttgatgac cattttcact gtgctgtcca atgtcaacga 120

ggtagcagcc gaggagcacg agctggtcag ccgcattaag cgcaacggct acggatacag 180

cagcggttat ggtggtggct gttcagcctg ccaaagcagc tgcaccacct gtggttacac 240

atcctaccat accgtgcctg cggctcccgc tcccccgcca ccaccgccgc ctccgcctcc 300

gcctgcgccc gtctacagct 320

<210> 57
<211> 643
<212> DNA
<213> Heterodera glycines

<220>
<221> misc_feature
<222> (477)..(477)
<223> n is a, c, g, or t

<400> 57
caaatttttg catataacca aaaaaaaatg catttccaaa ctgccttcct tctcctggtg 60

ccgttcattg ttgccaacat tgtgttggct gacatgccca aagatgatgc gccacgcccg 120

gcggtgcaaa tccgcacccg tcgttccgtg tatgtggcaa tgatggacgg catcggcgtc 180

accaaggagg ccatgagata tgcatgccgg ggatggcaaa cgagcttctg cacaaagttt 240

ggcactacgc tggccgttga gcgtacacga cgtgacacaa atgccgagat gatgggtacc 300

aagatgactg ccaatgccga gcgggaggca aagaaggaaa tgaatggcga gatgggtgcc 360

aagaaagaaa tgaatggcga gatgatgaac accaagaaag acacgaatgc cgaagtgatg 420

ggcaccaaga cgaatgctaa ccccgagcgg gacaccaaga tggacgccgt ggtgatnaac 480

accaagacga atgccaaccc cgagcgggac accaagacgg aagcgaatgc tgtcatggtg 540

aacaccaaga cggattctgc caaccccgag cgggacacca ataccgacgc cgtcatggtg 600

aacaccaaga caaatgttaa ccccgagcgg tacaccaaga ttg                    643

<210>  58
<211>  601
<212>  DNA
<213>  Heterodera glycines

<220>
<221>  misc_feature
<222>  (227)..(227)
<223>  n is a, c, g, or t

<220>
<221>  misc_feature
<222>  (458)..(458)
<223>  n is a, c, g, or t

<220>
<221>  misc_feature
<222>  (590)..(590)
<223>  n is a, c, g, or t

<220>
<221>  misc_feature
<222>  (599)..(599)
<223>  n is a, c, g, or t

<400>  58
aagaataaca tgaaaataat aactctaatt ccaatcttat tttcattaat aaattcggta     60

gttggacaaa caacatgcgc aacaccgaca acacccccag tttgttactg tacatgtgaa     120

tcaacacctg gagtgtcatc aacagcacca acaacgacag ttggaccaac aggaacaaca     180

acaactggca caccattaac aactggacag ggcacatctg gaccatntac accagcacca     240

acaggcactc ccggacaatc tacaacagca ccaacaggca cgcctggacc atcaacacca     300

gcaccaactg gcacgcctgg accatctaca ccagcaccaa caggcacacc cggaccatca     360

acaccaacac caacaggcac gcctggacca tctacaccag caccaacagg aacacccgga     420

ccatcaacac cagcaccaac aggcacgcct ggaccatnaa caccagcacc aacaggcacg     480

cctggaccat ctacaccagc accaacaggc acacccggac catcaacacc agcaccaaca     540

ggcacgcctg gaccatctac accagcacca acaggcacac ccggaccatn aacaccagna     600

c                                                                    601

<210>  59
<211>  584
<212>  DNA
<213>  Heterodera glycines

<220>

<221>  misc_feature
<222>  (145)..(145)
<223>  n is a, c, g, or t

<220>
<221>  misc_feature
<222>  (221)..(221)
<223>  n is a, c, g, or t

<220>
<221>  misc_feature
<222>  (487)..(487)
<223>  n is a, c, g, or t

<220>
<221>  misc_feature
<222>  (569)..(569)
<223>  n is a, c, g, or t

<400>  59
tgtcgacaga tttcaaactt gatacttgtt gttgttgaac atatttctat acctcacagt      60

tcgtccacat tttgactttc ggacaaaaac aaaagacaaa aaacggtggc aatgatctcc     120

aggagggcaa ttattatttg ggccnttgct ttgttggcat tagcagcgat ttcgcccaac     180

ttttccaccg ctgacaaagg agttgacgct gtcgatgcgg nggacgaaat cattgacgac     240

ccaaaagtgg aggtgccgaa gaacggcgtt ggcaaaggca ccgatgacca aacagtgcag     300

tgggaggagg aggccatcaa actggaggga ctatcagtgg ccgagttcaa gcagctcagg     360

gagagtgcgg agaagcatca attccaagcc gaggtcaacc ggatggtgaa gctgatcatc     420

aattcattgt accggaacaa ggagattttc ctccgtgagc tcatctccaa cgcgtcggat     480

gccctgnaca aaattcggct catttcgctg acaaattcga cagcacttgc ggccaccgaa     540

gaattgtcca tcaaaattaa ggccgacana gaaaatcaca tttt                      584


<210>  60
<211>  483
<212>  DNA
<213>  Heterodera glycines


<220>
<221>  misc_feature
<222>  (5)..(5)
<223>  n is a, c, g, or t

<400>  60
gtcgnaaatt gcaatgcctt catttaaatt tgtatgtttc caatttttgc taatttttgt      60

gctcacggaa ttggcacatt cagagaaagg gtgcccaaaa acagaagatt taattgaaat     120

gaccaaacat ttgctgacaa ctgggaagaa tgtaatttct ggtgaagcag cttcatcatc     180

caccgaagga aagtgcagtg atacggtaat ggaaagtgtt gtaacaaaag ttattgaatt     240

tgggtgttat gacgaaatgg aaaagaatga cggcgaaaga gcgaaaagca aaacgaaaaa 300

cgcatttatt gaaagcattg aaacacaacg aaaaggtttt tgccaacttt ggcaaaacgg 360

caaaagcatt ggggagaagg agcaaaaaga tggcattttg aaaatagtga aaggattggg 420

aggaattgag ctgaagaaaa ttgaacgaat tcttgaaaat gttcaaaaca ttggattatc 480

gcc 483

<210> 61
<211> 364
<212> DNA
<213> Heterodera glycines

<400> 61
aatattaaaa gatccgaaaa aatgccaaac attttcaaaa tccttctgat tgtgcttttg 60

gccgtcgtct cattccgtct ctcggcttct actggtgaca aaaaaactgc taatgatggg 120

agtggaaaca actcatcagc tgggattggt acgaagatca aaagaattgt caccgctgga 180

ctgctcttca cttccctggc gacgggtggg gcggaagtga ttgggcgaag caatgctcag 240

ggaggaaatg ccgccggatt ggtgccatcg catgtgacca atcgctcaat ggctccacca 300

cctcctcctg tgcaatttga aatgggggca aatcgattag aaaaaatgag ggcacaccta 360

cgcg 364

<210> 62
<211> 647
<212> DNA
<213> Heterodera glycines

<400> 62
agtcctaaag aaaaacgttc aaatgtttcg cttttcgctg cttctgctgc ttgtccctct 60

cgtccgctgt gtccccaacc caaagccttt gttcggaatt ggcatcggtc gcaagcaaag 120

cgcgggagcg gaggggaagc tgacctgcgc tggagagccg ttggcggatg tgaaggtgaa 180

actgtatgat gacgaccgcg gggtggacac ggatgatctg atgggcgaga cgagaacgga 240

ctcagaggga cgcttcagat tggaggggta cacgcacgaa atcaccacca tcgaccccaa 300

aattaacatt tatcacgact gcaacgacgg gctgaagcca tgccagcgca aaatctcaat 360

tatgatcccg gacaaataca tcgcctcggg cgaacatccc aatacgtatt acgacgcggg 420

cacagtcgaa ttggaggcca aattcagtgg cgagacaagg gactgtctgc actaattgca 480

ttggaactga tcatttggca ctttctgatc agttgggact gaccaattgg aactctctgc 540

actgatcact ttcccatttt tgtttctgtt ttgtcaccga ttttccccaa agtcttacac 600

cacccaatgt gaatttgcat aaatttgtcc gcaaaaaaaa aaaaaaa 647

<210>  63
<211>  857
<212>  DNA
<213>  Heterodera glycines

<400>  63
aacaaaacaa aacgaacatt tgccagcagt tggctaggac aacggcccaa agagaagtga      60

agtgaattgc ctctgctctg aacactgatg atgatggttc gttcttcctc ctcttccttt     120

tcccttccgt cgtacttcct ttctctcctc cccctccttc tcctcctttt ctcccaatgt     180

tcccaaattt cttttgcttg ccaatgccaa tgggccccgc caaaggacaa ttattgttcc     240

tccgattggg ttgcccacgt gcaggtgatc aaacgacagg acggcgtgcg aatgccggcg     300

gggatcaccg accgacagac ggacttaaac tcgcgacacg aagtcaaata tttaaggatg     360

tttaagatca gcaaacaaat gccggtcaat cagcagaacc aagtgattct ccctgtcaat     420

gtttatacgg ccaccgagga tgccgcctgt ggcattttgc tcgagtcggg acaccaatat     480

ttgttggccg gcgattacgt caacggcaca atgctcaccg gactgtgccg gcaaattctg     540

ctcgaagacc ttaaggagtc acgaaagcac gacattctcg agtggacaga agtgccgcaa     600

aagctgaaag aacagctgga aaagcaggaa tttgatcaga aatgcaactg aacagaatgg     660

aaaaagaaag agaggacaaa caattgcaaa tgaatgatca gaaaaatcaa acggacagag     720

aaatggccca atcgggcact ttttgctgaa cgaacatttc caataaacga cgaataaatt     780

tttaccgggt tagcccgtga aaaacgactt gcttgtaaag gttatttgct gcttatttaa     840

aaaaaaaaaa aaaaaaa                                                    857


<210>  64
<211>  24
<212>  DNA
<213>  Heterodera glycines

<400>  64
catttcctcc ctgagcattg ctta                                             24


<210>  65
<211>  27
<212>  DNA
<213>  Heterodera glycines

<400>  65
ttgtcaccgc tggactgctc ttcactt                                          27


<210>  66
<211>  17
<212>  DNA
<213>  Heterodera glycines

<400>  66

atgtcccttt tccgtcc                                                    17

<210>  67
<211>  20
<212>  DNA
<213>  Heterodera glycines

<400>  67
atgctccaaa acggccttac                                                 20

<210>  68
<211>  20
<212>  DNA
<213>  Heterodera glycines

<400>  68
atgctccaaa acggccttac                                                 20

<210>  69
<211>  21
<212>  DNA
<213>  Heterodera glycines

<400>  69
atgttcagct cttccaattt g                                               21

<210>  70
<211>  20
<212>  DNA
<213>  Heterodera glycines

<400>  70
caatgctcag ggaggaaatg                                                 20

<210>  71
<211>  20
<212>  DNA
<213>  Heterodera glycines

<400>  71
cactcggtga cagacgctta                                                 20

<210>  72
<211>  20
<212>  DNA
<213>  Heterodera glycines

<400>  72
aagcaggcgt atgagcagtt                                                 20

<210>  73
<211>  20
<212>  DNA
<213>  Heterodera glycines

<400>  73
gtcgtgtgcc aatacaatgc                                                    20


<210>  74
<211>  20
<212>  DNA
<213>  Heterodera glycines

<400>  74
cggctaatga aaagggaaaa                                                    20


<210>  75
<211>  20
<212>  DNA
<213>  Heterodera glycines

<400>  75
tggcatcatt ccactgactc                                                    20


<210>  76
<211>  20
<212>  DNA
<213>  Heterodera glycines

<400>  76
gtcctcaatc gctgcttctt                                                    20


<210>  77
<211>  20
<212>  DNA
<213>  Heterodera glycines

<400>  77
tcaatgtttg ggcttcttcc                                                    20


<210>  78
<211>  15
<212>  PRT
<213>  Heterodera glycines

<400>  78

Ser Thr Gly Asp Lys Lys Thr Ala Asn Asp Gly Ser Gly Asn Asn
1               5                   10                  15


<210>  79
<211>  16
<212>  PRT
<213>  Heterodera glycines

<400>  79

Pro Val Asn Glu Ser Lys Arg Leu Ser Pro Ser Gly Pro Asp Pro His
1               5                   10                  15

<210>  80
<211>  15
<212>  PRT
<213>  Heterodera glycines

<400>  80

Glu Glu Asp Ile Lys Ala Lys Thr Lys Arg Thr Leu Pro Lys Ser
1               5                   10                  15


<210>  81
<211>  15
<212>  PRT
<213>  Heterodera glycines

<400>  81

Lys Gly Leu Gly Thr Arg Asp Ser Asp Leu Ile Arg Leu Val Ile
1               5                   10                  15


<210>  82
<211>  15
<212>  PRT
<213>  Heterodera glycines

<400>  82

Ser Lys Pro Asn Pro Gly Gln Lys Pro Ser Gly Glu Arg Arg Lys
1               5                   10                  15


<210>  83
<211>  16
<212>  PRT
<213>  Heterodera glycines

<400>  83

Val Asn Arg Asn Gly Trp Glu Asn Thr Gly Thr Pro Thr Gly Gly Arg
1               5                   10                  15


<210>  84
<211>  26
<212>  DNA
<213>  artificial sequence

<220>
<223>  Synthetic primer

<400>  84
aaggatccat gcgcactttt ctgttc                                          26


<210>  85
<211>  27
<212>  DNA

<213>  artificial sequence

<220>
<223>  Synthetic primer

<400>  85
aagagctctc accattttaa ggcttgc                                          27


<210>  86
<211>  27
<212>  DNA
<213>  artificial sequence

<220>
<223>  Synthetic primer

<400>  86
aagaattcac agggaaaaat tcgtgac                                          27


<210>  87
<211>  28
<212>  DNA
<213>  artificial sequence

<220>
<223>  Synthetic primer

<400>  87
aaggatcctt caccatttta aggcttgc                                         28


<210>  88
<211>  23
<212>  DNA
<213>  artificial sequence

<220>
<223>  Synthetic primer

<400>  88
agttggacag aaggcatcag cac                                              23


<210>  89
<211>  23
<212>  DNA
<213>  artificial sequence

<220>
<223>  Synthetic primer

<400>  89
agaagcgttg cggacatatt tga                                              23


<210>  90
<211>  24
<212>  DNA
<213>  artificial sequence

<220>
<223>  Synthetic primer

<400>  90
gacgaagaag ataaaagttg agag                                              24


<210>  91
<211>  24
<212>  DNA
<213>  artificial sequence

<220>
<223>  Synthetic primer

<400>  91
ttgataaatt acaagcagat tgga                                              24


<210>  92
<211>  26
<212>  DNA
<213>  artificial sequence

<220>
<223>  Synthetic primer

<400>  92
tatctagagg catttgccat ttcaag                                            26


<210>  93
<211>  25
<212>  DNA
<213>  artificial sequence

<220>
<223>  Synthetic primer

<400>  93
taggatcctc atgtagaaaa gggcc                                             25


<210>  94
<211>  29
<212>  DNA
<213>  artificial sequence

<220>
<223>  Synthetic primer

<400>  94
tatctagagc gcacttttct gttcatagc                                         29


<210>  95
<211>  29
<212>  DNA
<213>  artificial sequence

<220>
<223>  Synthetic primer

<400>  95
ataagctttc accattttaa ggcttgctc                                         29


<210>  96
<211>  24
<212>  DNA
<213>  artificial sequence

<220>
<223>  Synthetic primer

<400>  96
ttgataaatt acaagcagat tgga                                              24


<210>  97
<211>  24
<212>  DNA
<213>  artificial sequence

<220>
<223>  Synthetic primer

<400>  97
gacgaagaag ataaaagttg agag                                              24


<210>  98
<211>  36
<212>  DNA
<213>  artificial sequence

<220>
<223>  Synthetic primer

<400>  98
atctcgagtt tggatgcatc gcttatcact gacctt                                 36


<210>  99
<211>  36
<212>  DNA
<213>  artificial sequence

<220>
<223>  Synthetic primer

<400>  99
atgaattcca ttgtttttaa accacccatc aacgca                                 36


<210>  100
<211>  36
<212>  DNA
<213>  artificial sequence

<220>

<223>  Synthetic primer

<400>  100
attctagatt tggatgcatc gcttatcact gacctt                                36


<210>  101
<211>  36
<212>  DNA
<213>  artificial sequence

<220>
<223>  Synthetic primer

<400>  101
ataagcttca ttgtttttaa accacccatc aacgca                                36


<210>  102
<211>  26
<212>  DNA
<213>  artificial sequence

<220>
<223>  Synthetic primer

<400>  102
atctcgagga aatgggaaga gaaaca                                           26


<210>  103
<211>  26
<212>  DNA
<213>  artificial sequence

<220>
<223>  Synthetic primer

<400>  103
atgaattcaa gataacccgg aaaagg                                           26


<210>  104
<211>  26
<212>  DNA
<213>  artificial sequence

<220>
<223>  Synthetic primer

<400>  104
attctagaga aatgggaaga gaaaca                                           26


<210>  105
<211>  26
<212>  DNA
<213>  artificial sequence

<220>
<223>  Synthetic primer

<400>  105
atatcgataa gataacccgg aaaagg                                          26


<210>  106
<211>  26
<212>  DNA
<213>  artificial sequence

<220>
<223>  Synthetic primer

<400>  106
atctcgagac gagttgagaa ggagaa                                          26


<210>  107
<211>  26
<212>  DNA
<213>  artificial sequence

<220>
<223>  Synthetic primer

<400>  107
atgaattcct cttcctcctc tcgttc                                          26


<210>  108
<211>  26
<212>  DNA
<213>  artificial sequence

<220>
<223>  Synthetic primer

<400>  108
attctagaac gagttgagaa ggagaa                                          26


<210>  109
<211>  26
<212>  DNA
<213>  artificial sequence

<220>
<223>  Synthetic primer

<400>  109
ataagcttct cttcctcctc tcgttc                                          26


<210>  110
<211>  26
<212>  DNA
<213>  artificial sequence

<220>
<223>  Synthetic primer

<400>  110
atctcgagaa ccatattccc caaatg                                          26


<210>  111
<211>  35
<212>  DNA
<213>  artificial sequence

<220>
<223>  Synthetic primer

<400>  111
atgaattcta tttggtttgg catttgattc ggctg                                35


<210>  112
<211>  26
<212>  DNA
<213>  artificial sequence

<220>
<223>  Synthetic primer

<400>  112
attctagaaa ccatattccc caaatg                                          26


<210>  113
<211>  35
<212>  DNA
<213>  artificial sequence

<220>
<223>  Synthetic primer

<400>  113
ataagcttta tttggtttgg catttgattc ggctg                                35


<210>  114
<211>  279
<212>  DNA
<213>  Heterodera schachtii

<400>  114
atgtcccttt tccgtcctca atcgctgctt cttctggccg ctctttgcct gtcctttgcg      60

ctgctttttg tcacttcgtc ggaagaggga gggcgagtga agcgcggcgg atggccttgg    120

gattgggccg gcaaacaact gtgcaaaaca tcggcaaatt gcaagtgcaa ggatggcaaa    180

aattgggcca aatgtgtaaa gtcggaaggc tacgcggcca gcaattgttg cgacaaaaat    240

tacgtgtggg catgttgcgg gaagaagccc aaacattaa                           279


<210>  115
<211>  420
<212>  DNA
<213>  Heterodera schachtii

<220>
<221>  misc_feature
<222>  (79)..(85)
<223>  n is a, c, g, or t

<400>  115
atgtcaaaca ttttcaaaat ccttctcatt gtgcttttgg ccgtcctctc attcagttct      60

tcggtttcca ctgatggcnn nnnnnctgct aatgatggca gtggaagcaa ctcatcagct     120

gggattggta cgaagatcaa acgaattgtc accgctgggc tgctcttcac ttccttggcg     180

acgggtgggg cggaagtgat tgggcgaagc aatgctcagg gaggaaatgc cgcgggactg     240

gtgccatcgc atgtgaccaa tcgctcaatg gctccaccac ctcctcctgt gcaatttgaa     300

atgggggcaa atcgattaga aaaaatgagg gcacacctac gcgaacttgc tgagaaaatg     360

ccgccggtca atgaatcgaa gcgactggca ccgagtggac ccgacccacg tcatcattag     420


<210>  116
<211>  1023
<212>  DNA
<213>  Heterodera schachtii

<400>  116
atgctccaaa acggccttac cattctgctt ctgatcagcg ttgtgatcgg ccattccttg      60

gccaaccttg gcccaaccat caaacataat cctcaattta aagccgtaca aactgcgcat     120

catttgcatg atgccattgc gaaaaagcac gaggccgaag ttacgcagat catttgctcc     180

attagcaacg aacaacgaca agcattggca tcggagttca aaaaacaatt cggcactgat     240

ctgattgcca tgctgaaaaa ggagttcaaa agcgactttg aagaactgat catttctttg     300

atgcaaacgc ccgccgttta cgatgccaac caaatgcgtg ccgcattgtc cggctccaac     360

gagacggtgc taatcgaaat tttggcgacg cgcacaaacc gacaaataac ggcgctgaag     420

caggcgtatg agcagttgga cagaaggcat cagcacaatc agctggagga ggacatcaaa     480

gcgaagacga agggcgcctt tcaaaatctg ttggtgtctt tgctcagctg ctctcgcgaa     540

gaaagtgcgc ccgcaagcat tgttttggca caccacgagg ccatgaaact gttcagagag     600

ggcgagggcc gaagaggcgt taacgccgtg gtgttcaacc aggtgttggc cactcgcagc     660

ttcgcccagc ttcgagaaac tttcgagttt taccgacaag ccgcgcacca cgagattgag     720

aagggcattg agcaagaatt cagcggtcac aacgaagcgg gtttcttggc actaatcaaa     780

tatgtccgca acgcttctgt gttttttgcg gatttgttgt tcaattcgat gaaagggctc     840

ggcacacgcg actcggattt gattcgtctg gtcatttctc ggtctgaggt tgacctggct     900

gacatcaaac acgcttttca cacgttgcac aagaagagcc tggaggaggc gatcaaaggg     960

gacaccagcg gagcttaccg agacgcactt ttggcactgg tcaagggcaa cacggagcag    1020

tga                                                                   1023

<210>  117
<211>  411
<212>  DNA
<213>  Heterodera schachtii

<400>  117
atgttcagct cttccaattt gtctgctctc tttttggcct cctccgtttt tgccgtgttt      60

ataattggca ttaaaatgga cggaccgacg gaggcaaaag gcgccgcccc tccaaacgcc     120

gcggggccaa tgggactttt gcttttattg aatggcaaac aatcggcggc caatgaaaag     180

ggaaaagcgc cctctggcga aagtaagcca aatccggggc agaagccgaa cggagaacgg     240

caaaagaggg acgttttggg gcacgccggc ggatacgtcg gaggatggga ccatcccatt     300

gactcgacag ttgattgggc aaagagtcag tggaatgatg ccaattggct cgccgatgtt     360

gtcaacagaa acggatggga aaacaccggc gctccaaccg gcggacgatg a              411